(19) Europäisches Patentamt
European Patent Office
Office européen des brevets

(11) EP 4 464 709 A1

(12) **EUROPEAN PATENT APPLICATION**
published in accordance with Art. 153(4) EPC

(43) Date of publication:
**20.11.2024 Bulletin 2024/47**

(21) Application number: **23740080.9**

(22) Date of filing: **13.01.2023**

(51) International Patent Classification (IPC):
***C07D 513/04*** *(2006.01)* ***C07D 417/12*** *(2006.01)*
***C07D 417/14*** *(2006.01)* ***A61K 31/428*** *(2006.01)*
***A61K 31/429*** *(2006.01)* ***A61P 35/00*** *(2006.01)*

(52) Cooperative Patent Classification (CPC):
**A61K 31/428; A61K 31/429; A61P 35/00; C07D 417/12; C07D 417/14; C07D 513/04**

(86) International application number:
**PCT/CN2023/072041**

(87) International publication number:
**WO 2023/134739 (20.07.2023 Gazette 2023/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.01.2022 CN 202210038783**
**30.09.2022 CN 202211207114**

(71) Applicant: **Nanjing Zaiming Pharmaceutical Co., Ltd.**
**Nanjing, Jiangsu 210032 (CN)**

(72) Inventors:
- **LI, Zhen**
  **Shanghai 201318 (CN)**
- **TANG, Feng**
  **Shanghai 201318 (CN)**
- **LIU, Le**
  **Nanjing, Jiangsu 210042 (CN)**
- **LIU, Lu**
  **Shanghai 201318 (CN)**
- **JIANG, Lei**
  **Shanghai 201318 (CN)**
- **ZHOU, Feng**
  **Shanghai 201318 (CN)**
- **TANG, Renhong**
  **Shanghai 201318 (CN)**
- **REN, Jinsheng**
  **Nanjing, Jiangsu 210042 (CN)**

(74) Representative: **Mewburn Ellis LLP**
**Aurora Building**
**Counterslip**
**Bristol BS1 6BX (GB)**

(54) **SIX-MEMBERED CYCLOTHIAZOLE COMPOUND AND USE THEREOF**

(57) Provided in the present disclosure are a six-membered cyclothiazole compound as represented by formula (I) or a pharmaceutically acceptable salt thereof, a pharmaceutical composition containing same, and the use thereof in the prevention or treatment of diseases mediated by DNA polymerase θ.

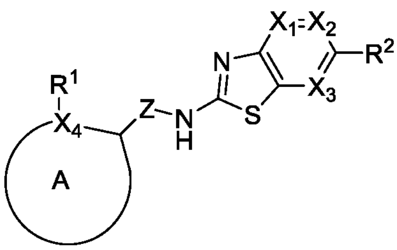

(I)

EP 4 464 709 A1

## Description

### Cross Reference to Related Application

The present application claims the benefit and the right of priority for Chinese invention patent application No. 202210038783.7 and No. 202211207114.4 submitted to the China National Intellectual Property Administration on January 13, 2022 and on September 30, 2022, respectively, which are hereby incorporated into the present application by reference in their entireties.

### Technical Field

The present application relates to a six-membered cyclothiazole compound or a pharmaceutically acceptable salt thereof, a pharmaceutical composition containing same, and the use thereof as a DNA polymerase θ inhibitor in the prevention or treatment of related diseases.

### Background

DNA double-strand break repair is critical for the maintenance of genome stability and cell survival. There are three main repair pathways for DNA double-strand breaks: homologous recombination (HR), non-homologous end joining (NHEJ), and alternative non-homologous end joining (alt-MEJ). Microhomology-mediated end joining (MMEJ) is the most common non-homologous end joining and alternative non-homologous end joining. Homologous recombination is a high-fidelity, error-free repair mechanism that maintains genome stability and avoids cancer induction. Non-homologous end joining and microhomology-mediated end joining are error-prone repair pathways that lead to mutations at repair sites.

Unlike normal cells, the survival of tumour cells often depends on the misregulation of DNA double-strand break repair. At the same time, the abnormal DNA double-strand break repair may make tumour cells more sensitive to specific types of DNA damage. Thus, defects in DNA double-strand break repair can be exploited to develop targeted tumour therapies. Tumour cells with impaired homologous recombination or non-homologous end joining repair will be more dependent on microhomology-mediated end joining repair. Multiple lines of evidence from genetics, cell biology, and biochemistry point to DNA polymerase θ (POLQ or POLθ) as a key protein in the process of microhomology-mediated end joining repair (Kent et al. Nature Structural & Molecular Biology (2015), 22(3), 230-237, Mateos-Gomez et al. Nature (2015), 518(7538), 254-257).

POLQ is a multifunctional enzyme consisting of an N-terminal helicase domain (SF2 HEL308-type) and a C-terminal low-fidelity DNA polymerase domain (A-type) (Wood & Doublie DNA Repair (2016), 44, 22-32). The helicase domain mediates the removal of RPA proteins from single-stranded DNA and promotes annealing, and the polymerase domain can extend single-stranded DNA ends and fill gaps, and the two domains work together to function in the process of microhomology-mediated end joining repair.

Studies have shown that POLQ is essential for homologous recombination-deficient cells (e.g., synthetic lethality associated with FA/BRCA deficiency), and that POLQ protein levels are upregulated in homologous recombination-deficient tumour cells (Ceccaldi et al. Nature (2015), 518(7538), 258-262). Results from *in vivo* studies also have shown that POLQ is overexpressed in a series of ovarian, uterine and breast cancers with deficient homologous recombination and poor prognosis (Higgins et al. Oncotarget (2010), 1, 175-184, Lemee et al. PNAS (2010), 107(30), 13390-13395, Ceccaldi et al. (2015), supra). More importantly, the expression of POLQ is suppressed in normal tissues compared with tumour tissues (Kawamura et al. International Journal of Cancer (2004), 109(1), 9-16).

In summary, POLQ is essential for homologous recombination-deficient cells, and there is still an unmet market demand for the treatment of homologous recombination-deficient tumours. Inhibiting the function of POLQ can inhibit the microhomology-mediated end joining repair in cells, and the development of inhibitors of POLQ function can provide a new strategy for the targeted therapy of homologous recombination-deficient tumours.

### Summary of the Invention

In an aspect, the present application relates to a compound represented by formula (I) or a pharmaceutically acceptable salt thereof,

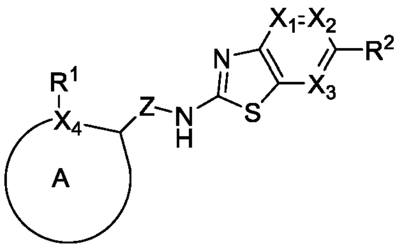

(I)

wherein,

$X_1$, $X_2$ and $X_3$ are independently selected from CH or N;
$X_4$ is selected from C or N;
Z is selected from C(=O) or $CH_2$;
ring A is selected from 5- to 10-membered heteroaryl, $C_6$-$C_{14}$ aryl or 4- to 12-membered heterocyclyl, wherein the 5- to 10-membered heteroaryl, $C_6$-$C_{14}$ aryl or 4- to 12-membered heterocyclyl is optionally substituted with $R^{1a}$;
each $R^{1a}$ is independently selected from halogen, hydroxyl, -NRR', cyano, carboxyl, =O, -C(=O)NRR', $C_1$-$C_{10}$ alkyl, $C_1$-$C_{10}$ alkoxy, $C_1$-$C_{10}$ alkoxyacyl, $C_3$-$C_{10}$ cycloalkyl, $C_2$-$C_{10}$ alkynyl, $C_2$-$C_{10}$ alkenyl, 4- to 12-membered heterocyclyl or 5- to 10-membered heteroaryl, wherein the $C_1$-$C_{10}$ alkyl, $C_1$-$C_{10}$ alkoxy, $C_1$-$C_{10}$ alkoxyacyl, $C_3$-$C_{10}$ cycloalkyl, $C_2$-$C_{10}$ alkynyl, $C_2$-$C_{10}$ alkenyl, 4- to 12-membered heterocyclyl or 5- to 10-membered heteroaryl is optionally substituted with $R^{1b}$;
$R^1$ is selected from $C_6$-$C_{14}$ aryl, 5- to 10-membered heteroaryl, 3- to 18-membered heterocyclyl or $C_4$-$C_{10}$ cycloalkenyl, wherein the $C_6$-$C_{14}$ aryl, 5- to 10-membered heteroaryl, 3- to 18-membered heterocyclyl or $C_4$-$C_{10}$ cycloalkenyl is optionally substituted with $R^{2a}$;
each $R^{2a}$ is independently selected from halogen, cyano, =O, hydroxyl, -NRR', -C(=O)NRR', $C_1$-$C_{10}$ alkyl, $C_1$-$C_{10}$ alkoxy, $C_3$-$C_{10}$ cycloalkyloxy, $C_1$-$C_{10}$ alkylacyl, $C_1$-$C_{10}$ alkylsulfonyl, $C_2$-$C_{10}$ alkynyl, $C_2$-$C_{10}$ alkenyl, $C_3$-$C_{10}$ cycloalkyl, 4- to 12-membered heterocyclyl, 4- to 8-membered heterocyclylalkyl or 4- to 8-membered heterocyclyloxy, wherein the $C_1$-$C_{10}$ alkyl, $C_1$-$C_{10}$ alkoxy, $C_3$-$C_{10}$ cycloalkyloxy, $C_1$-$C_{10}$ alkylacyl, $C_1$-$C_{10}$ alkylsulfonyl, $C_2$-$C_{10}$ alkynyl, $C_2$-$C_{10}$ alkenyl, $C_3$-$C_{10}$ cycloalkyl, 4- to 12-membered heterocyclyl, 4- to 8-membered heterocyclylalkyl or 4- to 8-membered heterocyclyloxy is optionally substituted with $R^{2b}$;
$R^2$ is selected from $C_6$-$C_{14}$ aryl, 5- to 10-membered heteroaryl, $C_3$-$C_{10}$ cycloalkyl or 4- to 12-membered heterocyclyl, wherein the $C_6$-$C_{14}$ aryl, 5- to 10-membered heteroaryl, $C_3$-$C_{10}$ cycloalkyl or 4- to 12-membered heterocyclyl is optionally substituted with $R^{3a}$;
each $R^{3a}$ is independently selected from halogen, -NRR', hydroxyl, cyano, =O, $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5-to 10-membered heteroaryl or 4- to 8-membered heterocyclyl, wherein the $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5- to 10-membered heteroaryl or 4- to 8-membered heterocyclyl is optionally substituted with $R^{3b}$;
R and R' are independently selected from hydrogen, $C_3$-$C_{10}$ cycloalkyl, $C_1$-$C_{10}$ alkylcarbonyl, 4- to 8-membered heterocyclyl or $C_1$-$C_{10}$ alkyl, wherein the $C_3$-$C_{10}$ cycloalkyl, $C_1$-$C_{10}$ alkylcarbonyl, 4- to 8-membered heterocyclyl or $C_1$-$C_{10}$ alkyl is optionally substituted with $R^{4b}$;
$R^{1b}$, $R^{2b}$, $R^{3b}$, and $R^{4b}$ are each independently selected from deuterium, halogen, carboxyl, hydroxyl, =O, cyano, -C(=O)NRR', sulfonyl, -S(=O)$_2$NRR', -NRR', $C_1$-$C_{10}$ alkyl or $C_1$-$C_{10}$ alkoxy.

In another aspect, the present application provides a pharmaceutical composition, comprising the compound represented by formula (I) or the pharmaceutically acceptable salt thereof according to the present application and a pharmaceutically acceptable excipient.

In still another aspect, the present application provides a method for preventing or treating diseases mediated by DNA polymerase θ in a mammal, comprising administering a therapeutically effective amount of a compound represented by formula (I) or a pharmaceutically acceptable salt thereof, or a pharmaceutical composition comprising same to the mammal, preferably a human in need of the treatment.

In yet another aspect, the present application provides the use of a compound represented by formula (I) or a pharmaceutically acceptable salt thereof, or a pharmaceutical composition comprising same in the preparation of a medicament for preventing or treating diseases mediated by DNA polymerase θ.

In still another aspect, the present application provides the use of a compound represented by formula (I) or a pharmaceutically acceptable salt thereof, or a pharmaceutical composition comprising same in the prevention or

treatment of diseases mediated by DNA polymerase θ.

In yet another aspect, the present application provides a compound represented by formula (I) or a pharmaceutically acceptable salt thereof, or a pharmaceutical composition comprising same, for use in preventing or treating diseases mediated by DNA polymerase θ.

In a further aspect, the present application provides the use of a compound represented by formula (I) or a pharmaceutically acceptable salt thereof, or a pharmaceutical composition comprising same in the preparation of a medicament for preventing or treating cancer.

## Detailed Description

The present application relates to a compound represented by formula (I) or a pharmaceutically acceptable salt thereof,

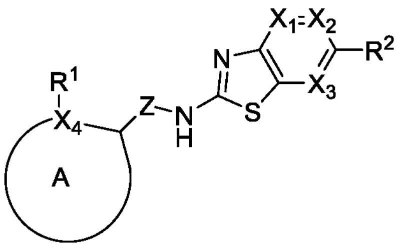

(I)

wherein,

- $X_1$, $X_2$ and $X_3$ are independently selected from CH or N;
- $X_4$ is selected from C or N;
- Z is selected from C(=O) or $CH_2$;
- ring A is selected from 5- to 10-membered heteroaryl, $C_6$-$C_{14}$ aryl or 4- to 12-membered heterocyclyl, wherein the 5- to 10-membered heteroaryl, $C_6$-$C_{14}$ aryl or 4- to 12-membered heterocyclyl is optionally substituted with $R^{1a}$;
- each $R^{1a}$ is independently selected from halogen, hydroxyl, -NRR', cyano, carboxyl, =O, -C(=O)NRR', $C_1$-$C_{10}$ alkyl, $C_1$-$C_{10}$ alkoxy, $C_1$-$C_{10}$ alkoxyacyl, $C_3$-$C_{10}$ cycloalkyl, $C_2$-$C_{10}$ alkynyl, $C_2$-$C_{10}$ alkenyl, 4- to 12-membered heterocyclyl or 5- to 10-membered heteroaryl, wherein the $C_1$-$C_{10}$ alkyl, $C_1$-$C_{10}$ alkoxy, $C_1$-$C_{10}$ alkoxyacyl, $C_3$-$C_{10}$ cycloalkyl, $C_2$-$C_{10}$ alkynyl, $C_2$-$C_{10}$ alkenyl, 4- to 12-membered heterocyclyl or 5- to 10-membered heteroaryl is optionally substituted with $R^{1b}$;
- $R^1$ is selected from $C_6$-$C_{14}$ aryl, 5- to 10-membered heteroaryl, 3- to 18-membered heterocyclyl or $C_4$-$C_{10}$ cycloalkenyl, wherein the $C_6$-$C_{14}$ aryl, 5- to 10-membered heteroaryl, 3- to 18-membered heterocyclyl or $C_4$-$C_{10}$ cycloalkenyl is optionally substituted with $R^{2a}$;
- each $R^{2a}$ is independently selected from halogen, cyano, =O, hydroxyl, -NRR', -C(=O)NRR', $C_1$-$C_{10}$ alkyl, $C_1$-$C_{10}$ alkoxy, $C_3$-$C_{10}$ cycloalkyloxy, $C_1$-$C_{10}$ alkylacyl, $C_1$-$C_{10}$ alkylsulfonyl, $C_2$-$C_{10}$ alkynyl, $C_2$-$C_{10}$ alkenyl, $C_3$-$C_{10}$ cycloalkyl, 4- to 12-membered heterocyclyl, 4- to 8-membered heterocyclylalkyl or 4- to 8-membered heterocyclyloxy, wherein the $C_1$-$C_{10}$ alkyl, $C_1$-$C_{10}$ alkoxy, $C_3$-$C_{10}$ cycloalkyloxy, $C_1$-$C_{10}$ alkylacyl, $C_1$-$C_{10}$ alkylsulfonyl, $C_2$-$C_{10}$ alkynyl, $C_2$-$C_{10}$ alkenyl, $C_3$-$C_{10}$ cycloalkyl, 4- to 12-membered heterocyclyl, 4- to 8-membered heterocyclylalkyl or 4- to 8-membered heterocyclyloxy is optionally substituted with $R^{2b}$;
- $R^2$ is selected from $C_6$-$C_{14}$ aryl, 5- to 10-membered heteroaryl, $C_3$-$C_{10}$ cycloalkyl or 4- to 12-membered heterocyclyl, wherein the $C_6$-$C_{14}$ aryl, 5- to 10-membered heteroaryl, $C_3$-$C_{10}$ cycloalkyl or 4- to 12-membered heterocyclyl is optionally substituted with $R^{3a}$;
- each $R^{3a}$ is independently selected from halogen, -NRR', hydroxyl, cyano, =O, $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5-to 10-membered heteroaryl or 4- to 8-membered heterocyclyl, wherein the $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5- to 10-membered heteroaryl or 4- to 8-membered heterocyclyl is optionally substituted with $R^{3b}$.
- R and R' are independently selected from hydrogen, $C_3$-$C_{10}$ cycloalkyl, $C_1$-$C_{10}$ alkylcarbonyl, 4- to 8-membered heterocyclyl or $C_1$-$C_{10}$ alkyl, wherein the $C_3$-$C_{10}$ cycloalkyl, $C_1$-$C_{10}$ alkylcarbonyl, 4- to 8-membered heterocyclyl or $C_1$-$C_{10}$ alkyl is optionally substituted with $R^{4b}$;
- $R^{1b}$, $R^{2b}$, $R^{3b}$, and $R^{4b}$ are each independently selected from deuterium, halogen, carboxyl, hydroxyl, =O, cyano, -C(=O)NRR', sulfonyl, -S(=O)$_2$NRR', -NRR', $C_1$-$C_{10}$ alkyl or $C_1$-$C_{10}$ alkoxy.

In some embodiments, at least one of $X_1$, $X_2$ and $X_3$ is N.
In some embodiments, $X_1$ and $X_2$ are independently selected from CH or N, and $X_3$ is N.
In some embodiments, $X_1$ and $X_2$ are independently CH, and $X_3$ is N.
In some embodiments, $X_1$ and $X_3$ are independently N, and $X_2$ is CH.
In some embodiments, $X_1$ is CH, and $X_2$ and $X_3$ are independently N.
In some embodiments, $X_1$ is N, and $X_2$ and $X_3$ are independently CH.
In some embodiments, $X_4$ is C.
In some embodiments, Z is C(=O).
In some embodiments, ring A is selected from 5- to 10-membered heteroaryl, $C_6$-$C_{10}$ aryl or 4- to 10-membered heterocyclyl, wherein the 5- to 10-membered heteroaryl, $C_6$-$C_{10}$ aryl or 4- to 10-membered heterocyclyl is optionally substituted with $R^{1a}$.
In some embodiments, ring A is selected from 5- to 10-membered heteroaryl, $C_6$-$C_{10}$ aryl or 6- to 10-membered heterocyclyl, wherein the 5- to 10-membered heteroaryl, $C_6$-$C_{10}$ aryl or 6- to 10-membered heterocyclyl is optionally substituted with $R^{1a}$. In some embodiments, ring A is selected from 5- to 9-membered heteroaryl, phenyl or 9-membered heterocyclyl, wherein the 5- to 9-membered heteroaryl, phenyl or 5- to 9-membered heterocyclyl is optionally substituted with $R^{1a}$.
In some embodiments, ring A is selected from 5- to 9-membered heteroaryl having 1, 2 or 3 nitrogen atoms, phenyl or 9-membered heterocyclyl, wherein the 5-to 9-membered heteroaryl, phenyl or 9-membered heterocyclyl is optionally substituted with $R^{1a}$.
In some embodiments, ring A is selected from imidazolyl, 6- to 9-membered heteroaryl, phenyl or 9-membered heterocyclyl, wherein the imidazolyl, 6- to 9-membered heteroaryl, phenyl or 9-membered heterocyclyl is optionally substituted with $R^{1a}$.
In some embodiments, ring A is selected from 6-membered heteroaryl, wherein the 6-membered heteroaryl is optionally substituted with $R^{1a}$.
In some embodiments, ring A is selected from pyridyl, imidazolyl, imidazo[1,2-a]pyridyl, pyrimidyl, [1,2,4]triazolo[1,5-a]pyridyl, phenyl or benzo[d][1,3]dioxinyl, wherein the pyridyl, imidazolyl, imidazo[1,2-a]pyridyl, pyrimidyl, [1,2,4]triazolo[1,5-a]pyridyl, phenyl or benzo[d][1,3]dioxinyl is optionally substituted with $R^{1a}$.
In some embodiments, ring A is selected from pyridyl, imidazolyl, imidazo[1,2-a]pyridyl, pyrimidyl, [1,2,4]triazolo[1,5-a]pyridyl, phenyl or

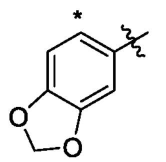

,

wherein the pyridyl, imidazolyl, imidazo[1,2-a]pyridyl, pyrimidyl, [1,2,4]triazolo[1,5-a]pyridyl, phenyl or

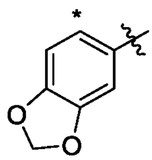

is optionally substituted with $R^{1a}$.
In some embodiments, ring A is selected from pyridyl, wherein the pyridyl is optionally substituted with $R^{1a}$.
In some embodiments, ring A is selected from

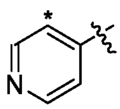 or 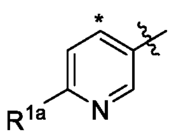

,

wherein * indicates linking to $R^1$.
In some embodiments, $R^{1a}$ is selected from halogen, cyano or $C_1$-$C_6$ alkyl, wherein the $C_1$-$C_6$ alkyl is optionally substituted with $R^{1b}$.
In some embodiments, $R^{1a}$ is selected from halogen, cyano or $C_1$-$C_3$ alkyl, wherein the $C_1$-$C_3$ alkyl is optionally

substituted with halogen.

In some embodiments, $R^{1a}$ is selected from $C_1$-$C_6$ alkyl, wherein the $C_1$-$C_6$ alkyl is optionally substituted with $R^{1b}$.

In some embodiments, $R^{1a}$ is selected from $C_1$-$C_3$ alkyl.

In some embodiments, $R^{1b}$ is selected from halogen.

In some embodiments, $R^{1b}$ is selected from fluorine.

In some embodiments, $R^{1a}$ is selected from fluorine, chlorine, cyano, $CF_3$ or methyl.

In some embodiments, $R^{1a}$ is selected from methyl or cyano.

In some embodiments, $R^{1a}$ is selected from methyl.

In some embodiments, ring A is selected from

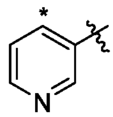 , 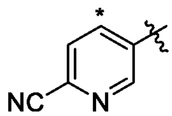 ,

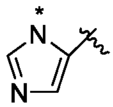 , 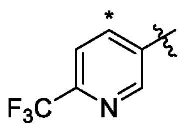 , 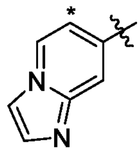 , 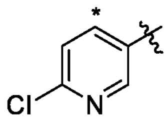 , 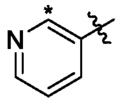 , 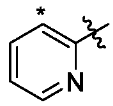 , 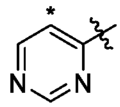 ,

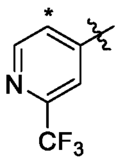 , 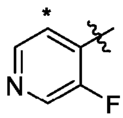 , 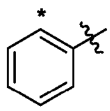 , 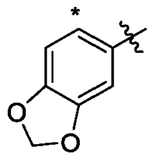 , 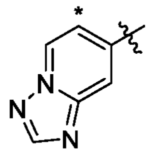 , 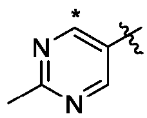 , 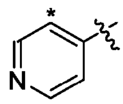 ,

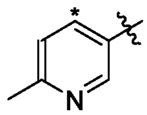 or 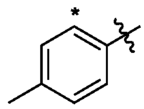 ,

wherein * indicates linking to $R^1$.

In some embodiments, ring A is selected from

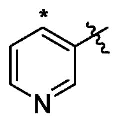 , 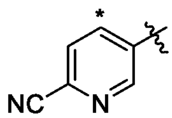 ,

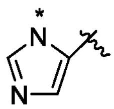 , 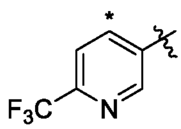 , 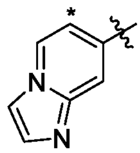 , 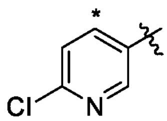 , 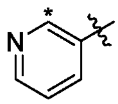 , 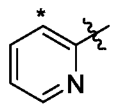 , 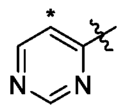 ,

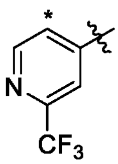 , 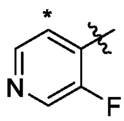 , 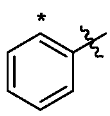 , 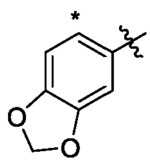 , 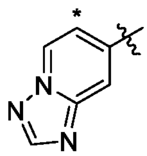 , 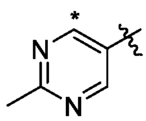 , 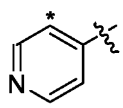 ,

or

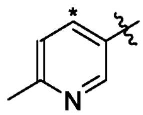,

wherein * indicates linking to $R^1$.

In some embodiments, ring A is selected from

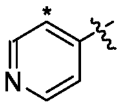 or 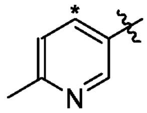,

wherein * indicates linking to $R^1$.

In some embodiments, $R^1$ is selected from $C_6$-$C_{10}$ aryl, 5- to 10-membered heteroaryl, 4- to 10-membered heterocyclyl or $C_5$-$C_7$ cycloalkenyl, wherein the $C_6$-$C_{10}$ aryl, 5- to 10-membered heteroaryl, 4- to 10-membered heterocyclyl or $C_5$-$C_7$ cycloalkenyl is optionally substituted with $R^{2a}$.

In some embodiments, $R^1$ is selected from $C_6$-$C_{10}$ aryl, 5- to 10-membered heteroaryl or 4- to 10-membered heterocyclyl, wherein the $C_6$-$C_{10}$ aryl, 5- to 10-membered heteroaryl or 4- to 10-membered heterocyclyl is optionally substituted with $R^{2a}$.

In some embodiments, $R^1$ is selected from phenyl, 6-membered heteroaryl or 6-membered heterocyclyl, wherein the phenyl, 6-membered heteroaryl or 6-membered heterocyclyl is optionally substituted with $R^{2a}$.

In some embodiments, $R^1$ is selected from phenyl, pyridyl or morpholinyl, wherein the phenyl, pyridyl or morpholinyl is optionally substituted with $R^{2a}$.

In some embodiments, $R^1$ is selected from phenyl or pyridyl, wherein the phenyl or pyridyl is optionally substituted with $R^{2a}$.

In some embodiments, $R^1$ is selected from phenyl, wherein the phenyl is optionally substituted with $R^{2a}$.

In some embodiments, $R^1$ is selected from

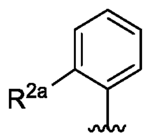 or 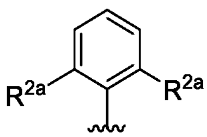

In some embodiments, $R^{2a}$ is selected from halogen, cyano, $C_1$-$C_6$ alkyl, $C_2$-$C_6$ alkynyl, $C_3$-$C_6$ cycloalkyl, $C_1$-$C_6$ alkoxy or $C_3$-$C_6$ cycloalkyloxy, wherein the $C_1$-$C_6$ alkyl, $C_2$-$C_6$ alkynyl, $C_3$-$C_6$ cycloalkyl, $C_1$-$C_6$ alkoxy or $C_3$-$C_6$ cycloalkyloxy is optionally substituted with $R^{2b}$.

In some embodiments, $R^{2a}$ is selected from halogen, cyano, $C_1$-$C_3$ alkyl, $C_2$-$C_4$ alkynyl, $C_3$-$C_4$ cycloalkyl, $C_1$-$C_3$ alkoxy or $C_3$-$C_4$ cycloalkyloxy, wherein the $C_1$-$C_3$ alkyl, $C_2$-$C_4$ alkynyl, $C_3$-$C_4$ cycloalkyl, $C_1$-$C_3$ alkoxy or $C_3$-$C_4$ cycloalkyloxy is optionally substituted with $R^{2b}$.

In some embodiments, $R^{2a}$ is selected from halogen, cyano, $C_1$-$C_3$ alkyl, $C_2$-$C_4$ alkynyl, $C_3$-$C_4$ cycloalkyl, $C_1$-$C_3$ alkoxy or $C_3$-$C_4$ cycloalkyloxy, wherein the $C_1$-$C_3$ alkyl or $C_1$-$C_3$ alkoxy is optionally substituted with $R^{2b}$.

In some embodiments, $R^{2a}$ is selected from halogen, cyano, $C_1$-$C_6$ alkyl, $C_2$-$C_6$ alkynyl, $C_3$-$C_6$ cycloalkyl or $C_1$-$C_6$ alkoxy, wherein the $C_1$-$C_6$ alkyl, $C_2$-$C_6$ alkynyl, $C_3$-$C_6$ cycloalkyl or $C_1$-$C_6$ alkoxy is optionally substituted with $R^{2b}$.

In some embodiments, $R^{2a}$ is selected from halogen, cyano, $C_1$-$C_3$ alkyl, $C_2$-$C_4$ alkynyl, $C_3$-$C_4$ cycloalkyl or $C_1$-$C_3$ alkoxy, wherein the $C_1$-$C_3$ alkyl, $C_2$-$C_4$ alkynyl, $C_3$-$C_4$ cycloalkyl or $C_1$-$C_3$ alkoxy is optionally substituted with $R^{2b}$.

In some embodiments, $R^{2a}$ is selected from halogen, cyano, $C_1$-$C_3$ alkyl, $C_2$-$C_4$ alkynyl, $C_3$-$C_4$ cycloalkyl or $C_1$-$C_3$ alkoxy, wherein the $C_1$-$C_3$ alkyl or $C_1$-$C_3$ alkoxy is optionally substituted with $R^{2b}$.

In some embodiments, $R^{2a}$ is selected from halogen or $C_1$-$C_6$ alkoxy, wherein the $C_1$-$C_6$ alkoxy is optionally substituted with $R^{2b}$.

In some embodiments, $R^{2a}$ is selected from fluorine, chlorine, bromine, iodine or $C_1$-$C_3$ alkoxy.

In some embodiments, $R^{2b}$ is selected from halogen or deuterium.

In some embodiments, $R^{2b}$ is selected from fluorine or deuterium.

In some embodiments, $R^{2b}$ is selected from halogen.

In some embodiments, $R^{2b}$ is selected from fluorine.

In some embodiments, $R^{2a}$ is selected from fluorine, chlorine, cyano, $OCHF_2$, methyl, ethynyl, cyclopropyl, methoxy, $CF_3$, $OCF_3$, $OCD_3$ or

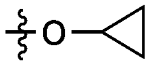

In some embodiments, $R^{2a}$ is selected from fluorine, chlorine, cyano, $OCHF_2$, methyl, ethynyl, cyclopropyl or methoxy.

In some embodiments, $R^{2a}$ is selected from fluorine or methoxy.

In some embodiments, $R^1$ is selected from

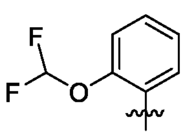, 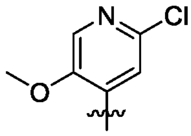,

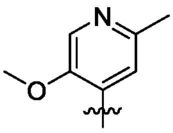, 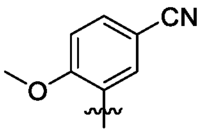, 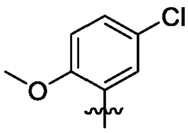, 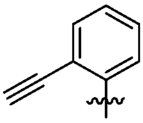, 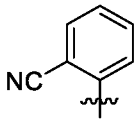, 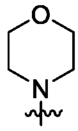,

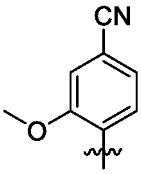, 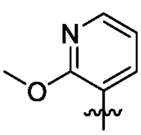, 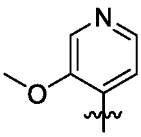, 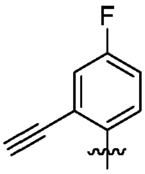, 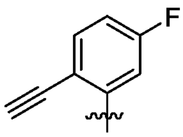, 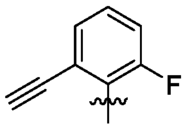,

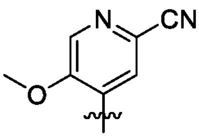, 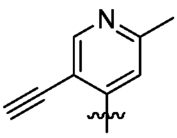, 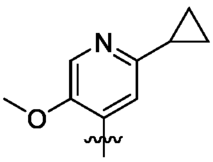, 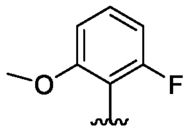, 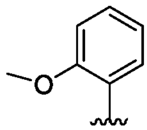,

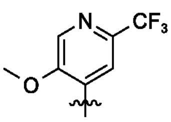, 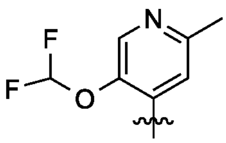, 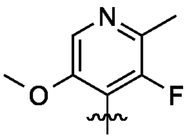, 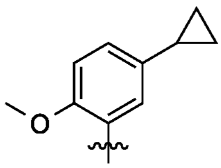,

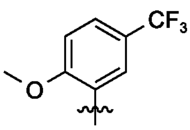, 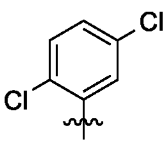, 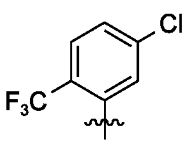, 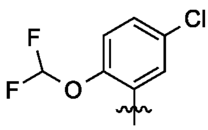, 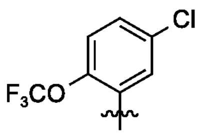,

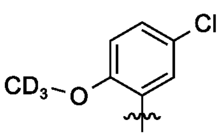 or 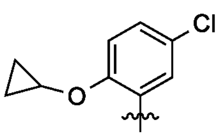

In some embodiments, $R^1$ is selected from

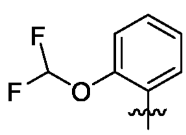, 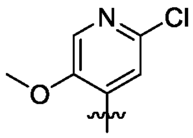,

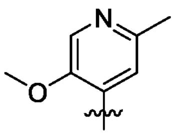, 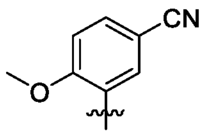, 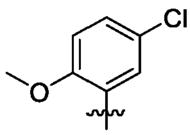, 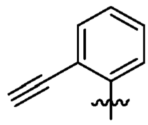, 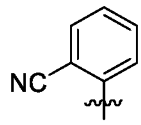, 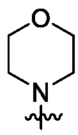,

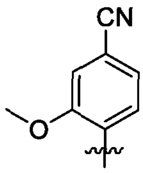, 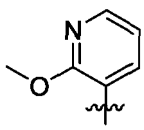, 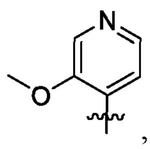, 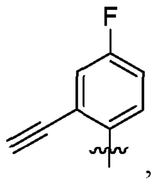, 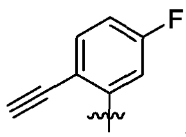, 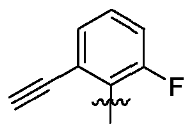,

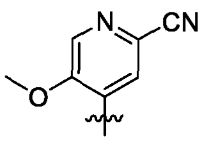, 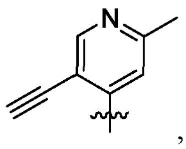, 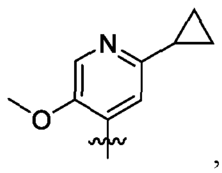, 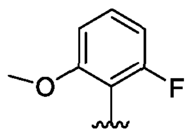 or 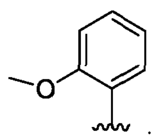.

In some embodiments, $R^1$ is selected from

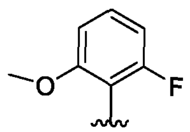 or 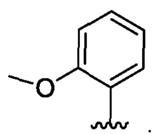.

In some embodiments, $R^2$ is selected from $C_6$-$C_{10}$ aryl, 5- to 10-membered heteroaryl, $C_3$-$C_6$ cycloalkyl or 4- to 10-membered heterocyclyl, wherein the $C_6$-$C_{10}$ aryl, 5- to 10-membered heteroaryl, $C_3$-$C_6$ cycloalkyl or 4- to 10-membered heterocyclyl is optionally substituted with $R^{3a}$.

In some embodiments, $R^2$ is selected from $C_6$-$C_{10}$ aryl, 5- to 10-membered heteroaryl or 4- to 10-membered heterocyclyl, wherein the $C_6$-$C_{10}$ aryl, 5- to 10-membered heteroaryl or 4- to 10-membered heterocyclyl is optionally substituted with $R^{3a}$.

In some embodiments, $R^2$ is selected from phenyl, 5- to 6-membered heteroaryl or 5- to 6-membered heterocyclyl, wherein the phenyl, 5- to 6-membered heteroaryl or 5- to 6-membered heterocyclyl is optionally substituted with $R^{3a}$.

In some embodiments, $R^2$ is selected from phenyl, 6-membered heteroaryl or 5- to 6-membered heterocyclyl, wherein the phenyl, 6-membered heteroaryl or 5- to 6-membered heterocyclyl is optionally substituted with $R^{3a}$.

In some embodiments, $R^2$ is selected from phenyl, pyridyl, tetrahydrofuryl or

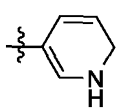,

wherein the phenyl, pyridyl, tetrahydrofuryl or

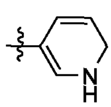

is optionally substituted with $R^{3a}$. In some embodiments, $R^2$ is selected from phenyl, pyridyl or tetrahydrofuryl, wherein the

phenyl, pyridyl or tetrahydrofuryl is optionally substituted with $R^{3a}$.

In some embodiments, $R^2$ is selected from phenyl or pyridyl, wherein the phenyl or pyridyl is optionally substituted with $R^{3a}$.

In some embodiments, $R^2$ is selected from phenyl, wherein the phenyl is optionally substituted with $R^{3a}$.

In some embodiments, $R^2$ is selected from

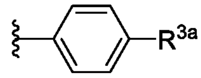 or 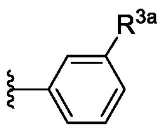

In some embodiments, each $R^{3a}$ is independently selected from halogen, -NRR', hydroxyl, cyano, $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5- to 10-membered heteroaryl or 4- to 8-membered heterocyclyl, wherein the $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5- to 10-membered heteroaryl or 4- to 8-membered heterocyclyl is optionally substituted with $R^{3b}$.

In some embodiments, each $R^{3a}$ is independently selected from halogen, cyano, =O, $C_1$-$C_6$ alkyl, $C_1$-$C_6$ alkoxy or $C_3$-$C_6$ cycloalkyl, wherein the $C_1$-$C_6$ alkyl, $C_1$-$C_6$ alkoxy or $C_3$-$C_6$ cycloalkyl is optionally substituted with $R^{3b}$.

In some embodiments, each $R^{3a}$ is independently selected from halogen, cyano, =O, $C_1$-$C_6$ alkyl, $C_1$-$C_6$ alkoxy or $C_3$-$C_6$ cycloalkyl, wherein the $C_1$-$C_6$ alkyl or $C_1$-$C_6$ alkoxy is optionally substituted with $R^{3b}$.

In some embodiments, each $R^{3a}$ is independently selected from halogen, cyano, $C_1$-$C_6$ alkyl or $C_1$-$C_6$ alkoxy, wherein the $C_1$-$C_6$ alkyl or $C_1$-$C_6$ alkoxy is optionally substituted with $R^{3b}$.

In some embodiments, each $R^{3a}$ is independently selected from fluorine, chlorine, bromine, iodine, cyano, $C_1$-$C_3$ alkyl or $C_1$-$C_3$ alkoxy.

In some embodiments, $R^{3b}$ is selected from halogen, cyano or hydroxyl.

In some embodiments, $R^{3b}$ is selected from fluorine, cyano or hydroxyl.

In some embodiments, $R^{3b}$ is selected from halogen or cyano.

In some embodiments, $R^{3b}$ is selected from fluorine or cyano.

In some embodiments, each $R^{3a}$ is independently selected from fluorine, chlorine, cyano, =O, methyl, cyclopropyl, $OCHF_2$, $CF_3$, methoxy,

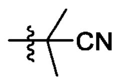,

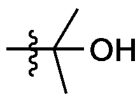,

$OCF_3$ or tert-butyl.

In some embodiments, each $R^{3a}$ is independently selected from cyano, cyclopropyl, $OCHF_2$, $OCF_3$ or $CF_3$.

In some embodiments, each $R^{3a}$ is independently selected from fluorine, chlorine, cyano, =O, methyl, cyclopropyl, $OCHF_2$, $CF_3$, methoxy or

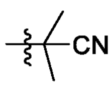

In some embodiments, each $R^{3a}$ is independently selected from chlorine, cyano, methyl or methoxy.

In some embodiments, $R^2$ is selected from

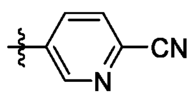, 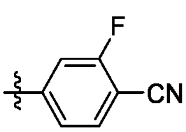,

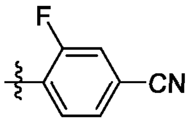, 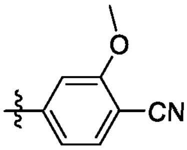, 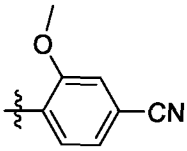, 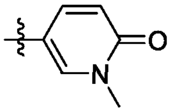, 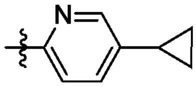,

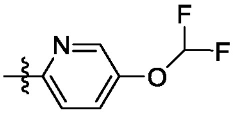, 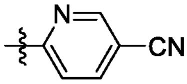, 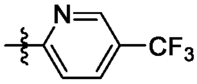, 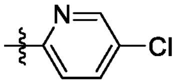,

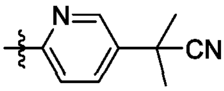, 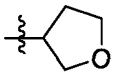, 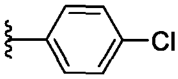, 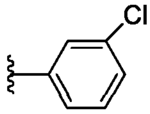, 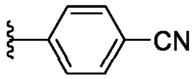,

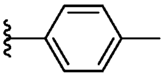, 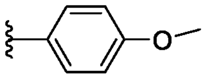, 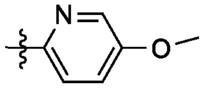, 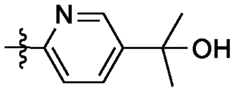,

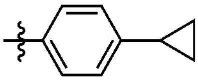, 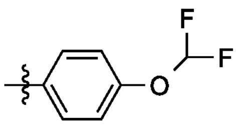 or 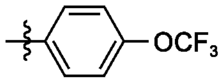

In some embodiments, $R^2$ is selected from

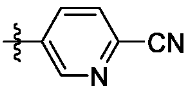, 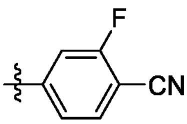,

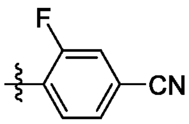, 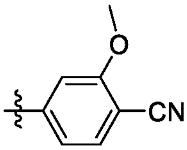, 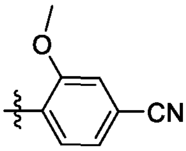, 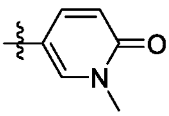, 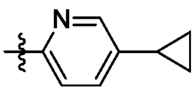,

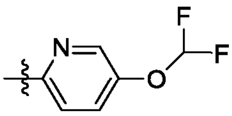, 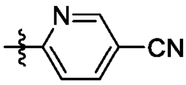, 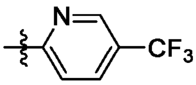, 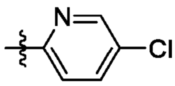,

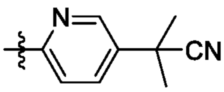, 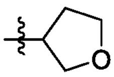, 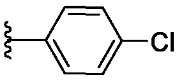, 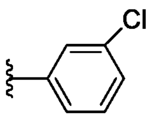, 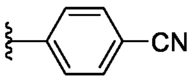,

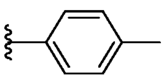 or 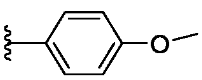

In some embodiments, $R^2$ is selected from

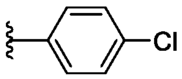 , 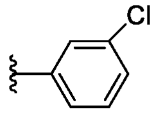 ,

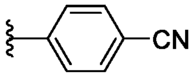 , 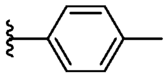 or 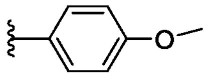

In some embodiments, $R^{3a}$ is selected from chlorine or cyano.

In some embodiments, $R^{3a}$ is cyano.

In some embodiments, $R^2$ is selected from

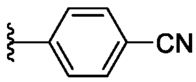

In some embodiments, R and R' are independently selected from hydrogen.

In some embodiments, at least one of $X_1$, $X_2$ and $X_3$ is N; $X_4$ is selected from C or N; Z is C(=O); ring A is selected from 5- to 10-membered heteroaryl, $C_6$-$C_{10}$ aryl or 6- to 10-membered heterocyclyl, wherein the 5- to 10-membered heteroaryl, $C_6$-$C_{10}$ aryl or 6- to 10-membered heterocyclyl is optionally substituted with $R^{1a}$; each $R^{1a}$ is independently selected from halogen, cyano or $C_1$-$C_3$ alkyl, wherein the $C_1$-$C_3$ alkyl is optionally substituted with halogen; $R^1$ is selected from phenyl, 6-membered heteroaryl or 6-membered heterocyclyl, wherein the phenyl, 6-membered heteroaryl or 6-membered heterocyclyl is optionally substituted with $R^{2a}$; each $R^{2a}$ is independently selected from halogen, cyano, $C_1$-$C_6$ alkyl, $C_2$-$C_6$ alkynyl, $C_3$-$C_6$ cycloalkyl, $C_1$-$C_6$ alkoxy or $C_3$-$C_6$ cycloalkyloxy, wherein the $C_1$-$C_6$ alkyl, $C_2$-$C_6$ alkynyl, $C_3$-$C_6$ cycloalkyl, $C_1$-$C_6$ alkoxy or $C_3$-$C_6$ cycloalkyloxy is optionally substituted with $R^{2b}$; each $R^{2b}$ is selected from halogen or deuterium; $R^2$ is selected from phenyl, 5- to 6-membered heteroaryl or 5- to 6-membered heterocyclyl, wherein the phenyl, 5- to 6-membered heteroaryl or 5- to 6-membered heterocyclyl is optionally substituted with $R^{3a}$; each $R^{3a}$ is independently selected from halogen, cyano, =O, $C_1$-$C_6$ alkyl, $C_1$-$C_6$ alkoxy or $C_3$-$C_6$ cycloalkyl, wherein the $C_1$-$C_6$ alkyl, $C_1$-$C_6$ alkoxy or $C_3$-$C_6$ cycloalkyl is optionally substituted with $R^{3b}$; and $R^{3b}$ is selected from halogen, cyano or hydroxyl.

In some embodiments, the compound represented by formula (I) or the pharmaceutically acceptable salt thereof according to the present application is selected from the following compound or a pharmaceutically acceptable salt thereof:

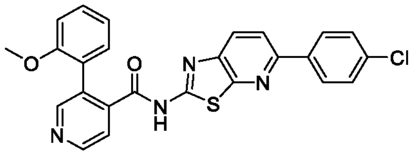 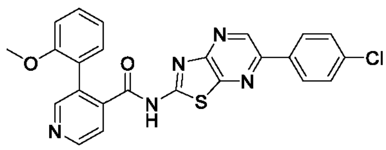

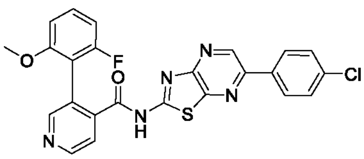 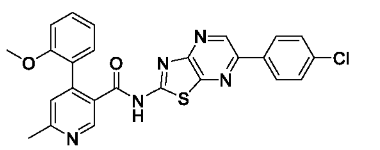

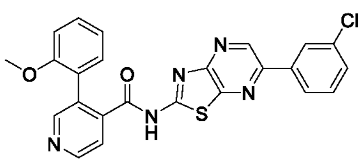 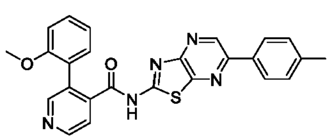

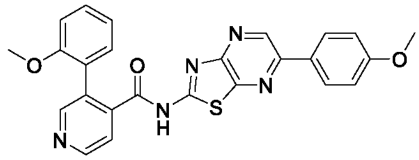
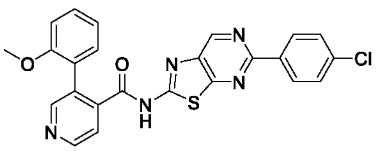

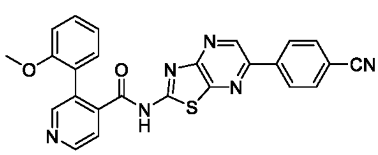
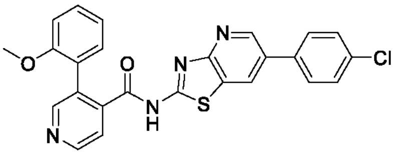

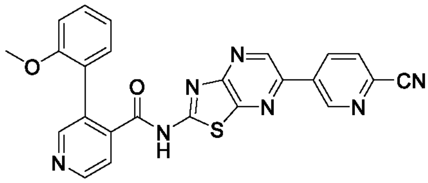
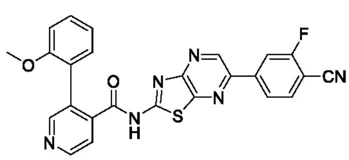

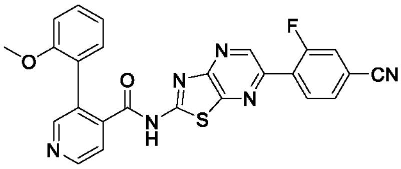
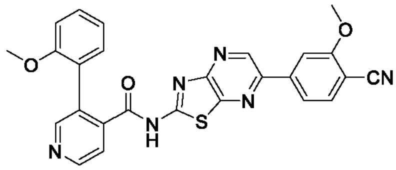

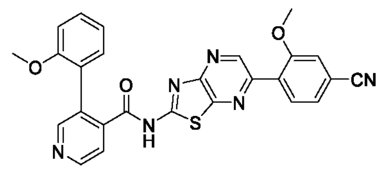
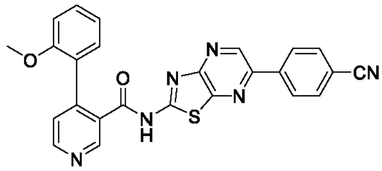

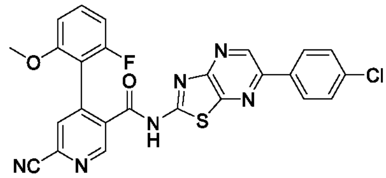
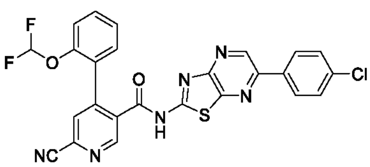

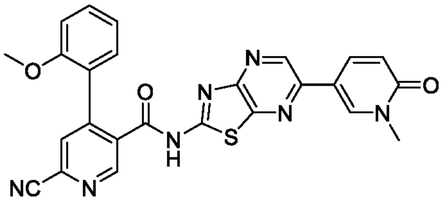
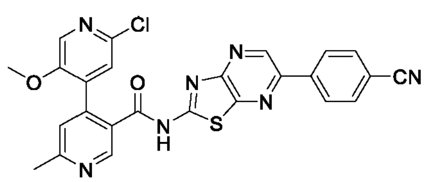

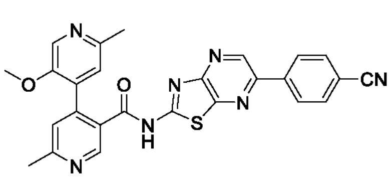
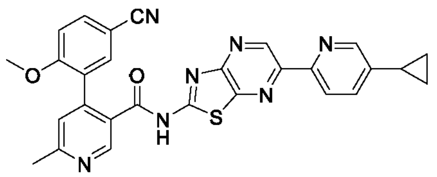

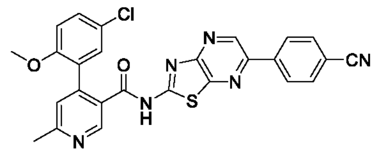
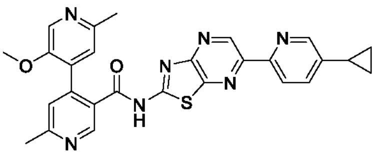

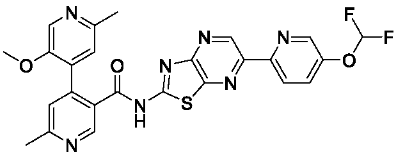
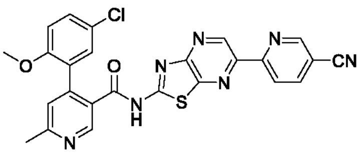

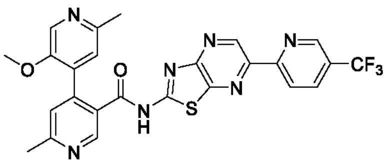
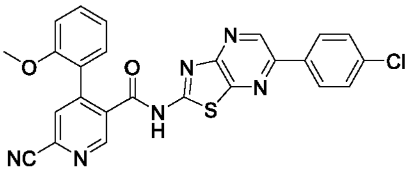

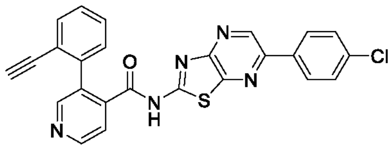
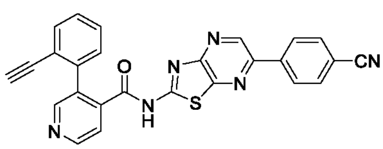

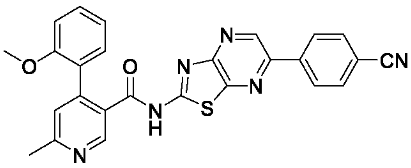
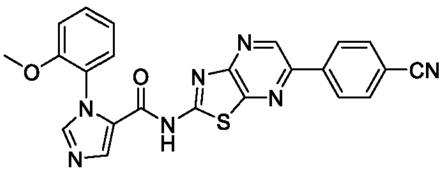

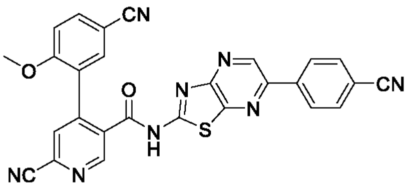
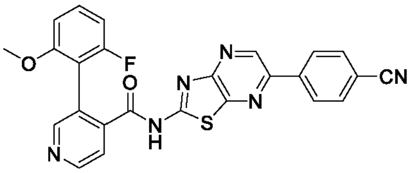

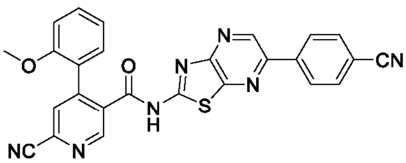
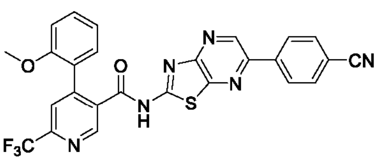

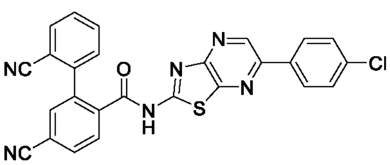
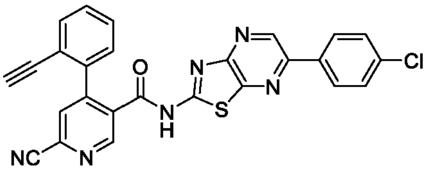

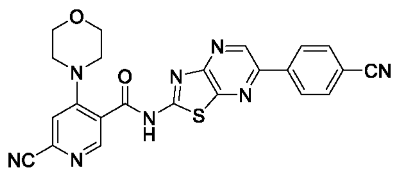 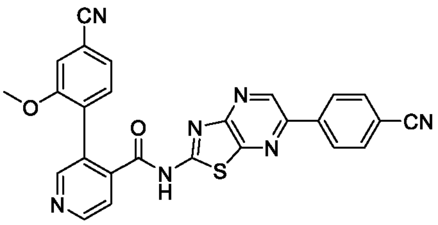

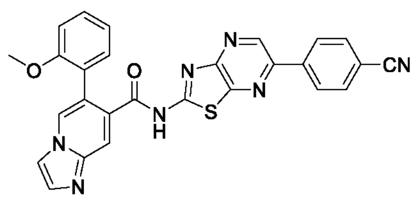 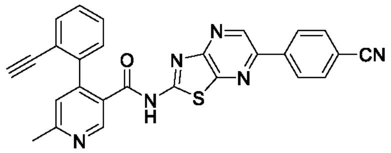

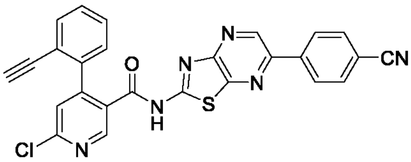 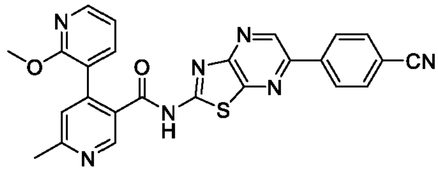

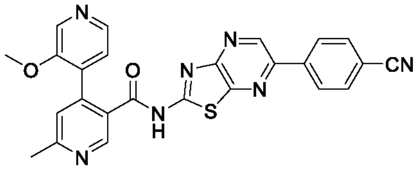 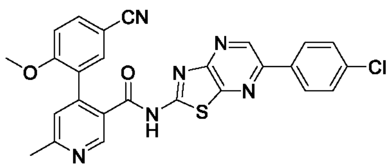

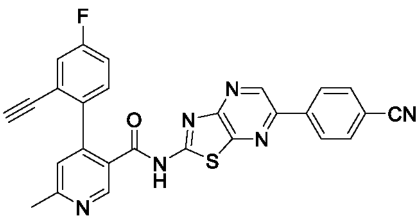 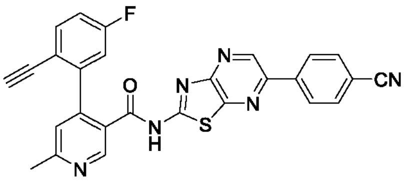

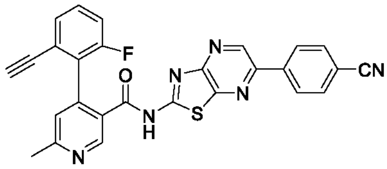 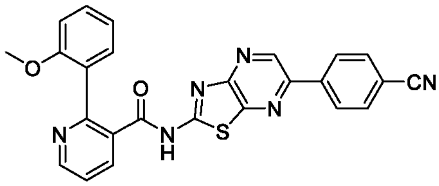

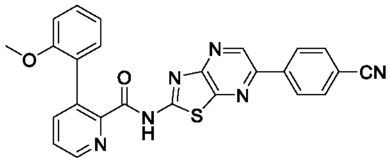 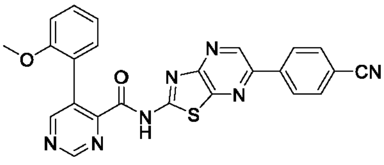

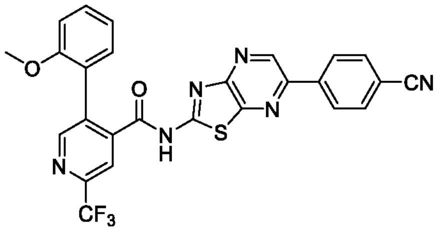
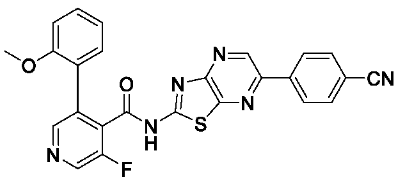

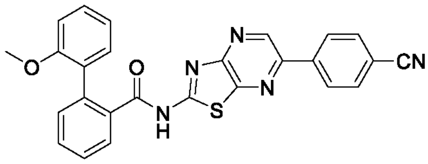
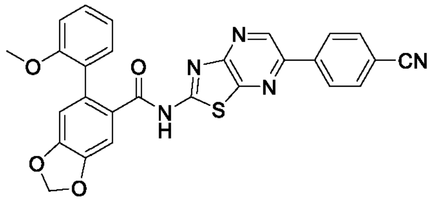

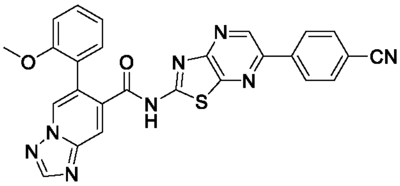
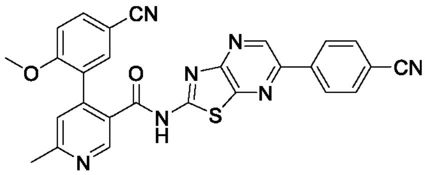

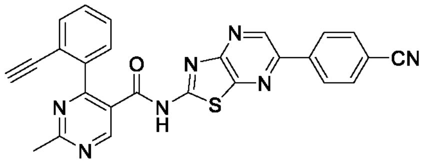
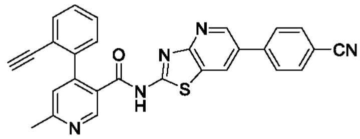

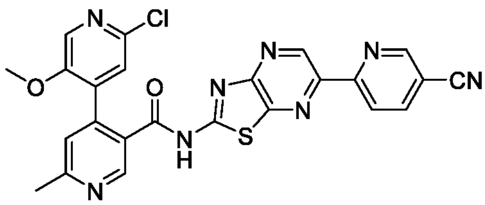
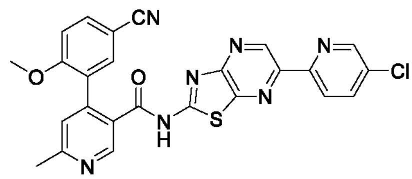

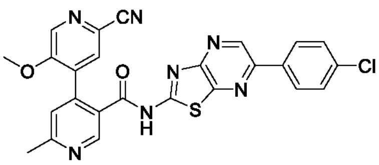
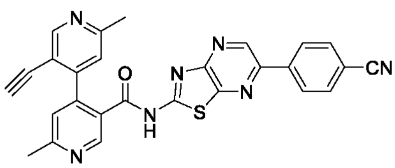

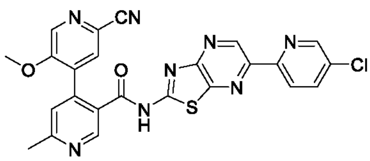
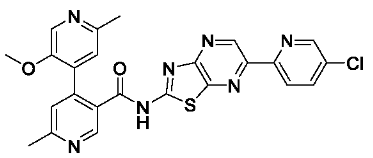

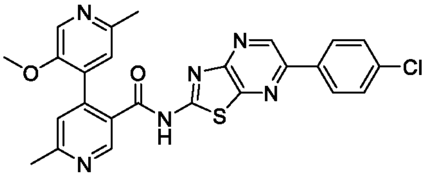
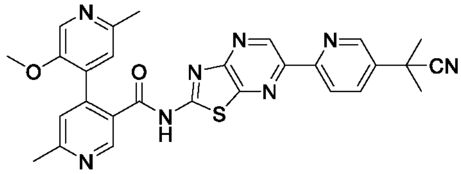

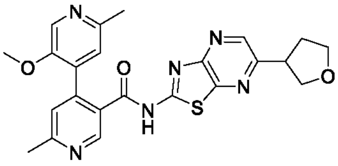
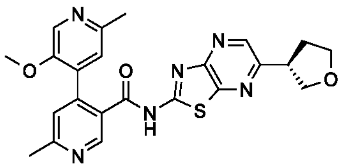

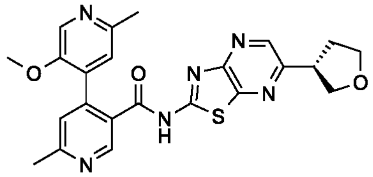
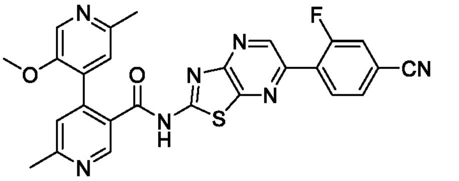

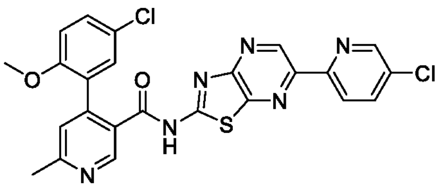
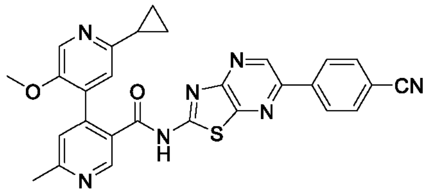

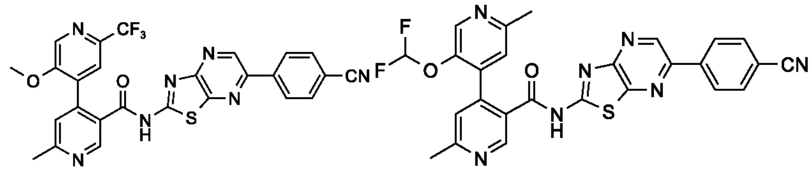

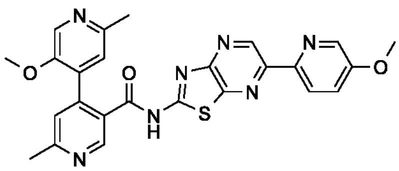
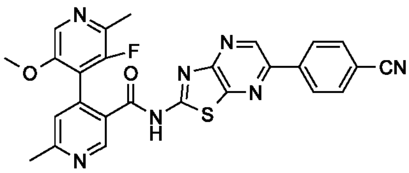

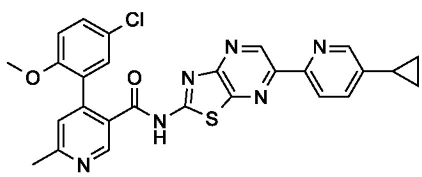
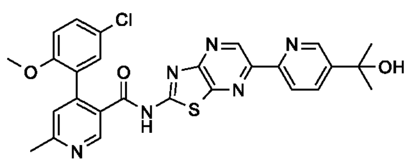

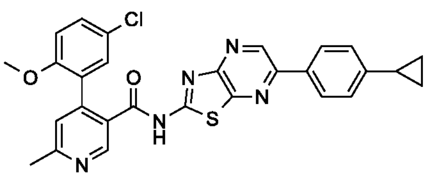
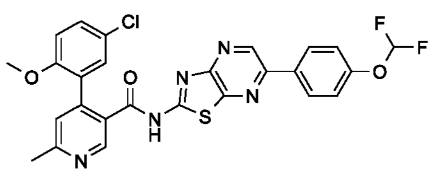

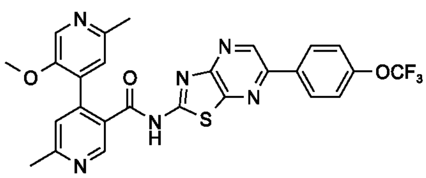
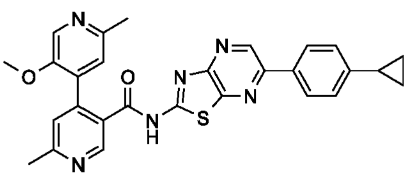

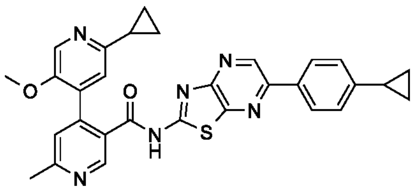
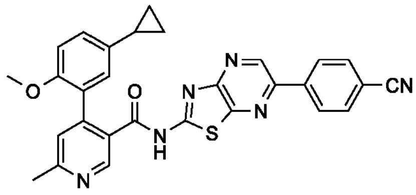
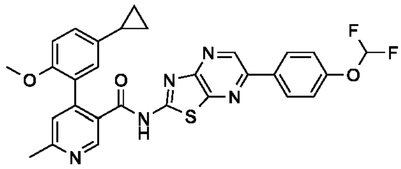
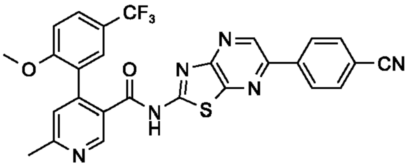
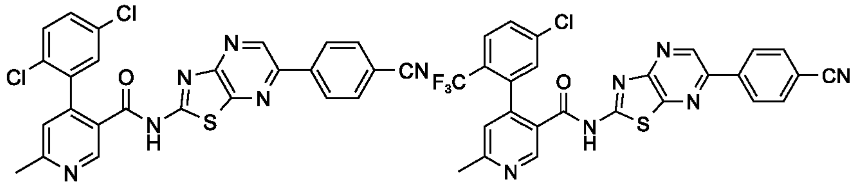
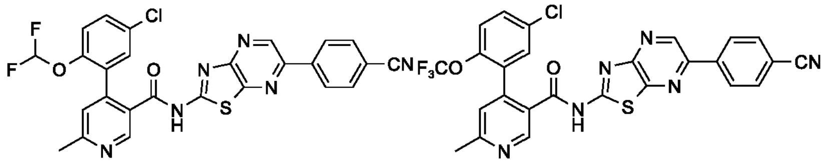
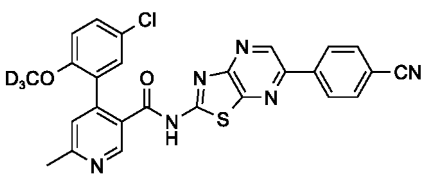
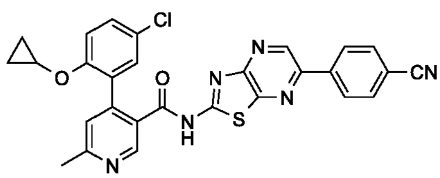
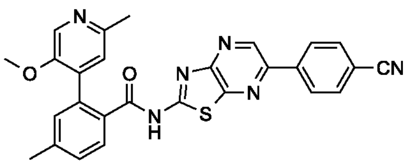

or

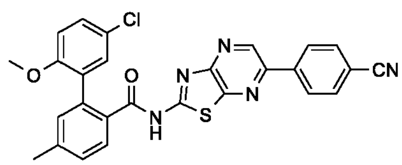

In another aspect, the present application provides a pharmaceutical composition, comprising the compound represented by formula (I) or the pharmaceutically acceptable salt thereof according to the present application and a pharmaceutically acceptable excipient.

In another aspect, the present application provides a method for preventing or treating diseases mediated by DNA polymerase θ in a mammal, comprising administering a therapeutically effective amount of a compound represented by formula (I) or a pharmaceutically acceptable salt thereof, or a pharmaceutical composition comprising same to the mammal, preferably a human in need of the treatment.

In another aspect, the present application provides the use of a compound represented by formula (I) or a pharmaceutically acceptable salt thereof, or a pharmaceutical composition comprising same in the preparation of a medicament for preventing or treating diseases mediated by DNA polymerase θ.

In another aspect, the present application provides the use of a compound represented by formula (I) or a pharmaceutically acceptable salt thereof, or a pharmaceutical composition comprising same in the prevention or treatment of diseases mediated by DNA polymerase θ.

In another aspect, the present application provides a compound represented by formula (I) or a pharmaceutically acceptable salt thereof, or a pharmaceutical composition comprising same, for use in preventing or treating diseases mediated by DNA polymerase θ.

In some embodiments, the diseases mediated by DNA polymerase θ are diseases in which DNA polymerase θ is overexpressed.

In some embodiments, the diseases mediated by DNA polymerase θ are cancer.

In some embodiments, the cancer is with a reduction or absence of BRCA gene expression, the absence of the BRCA gene, or reduced function of BRCA protein.

In some embodiments, the cancer is colorectal adenocarcinoma.

In another aspect, the present application provides the use of a compound represented by formula (I) or a pharmaceutically acceptable salt thereof, or a pharmaceutical composition comprising same in the preparation of a medicament for preventing or treating cancer.

Definitions and Description of Terms

Unless otherwise stated, the terms used in the present application have the following meanings; the definitions of groups and terms described in the present application, including their definitions as examples, exemplary definitions, preferred definitions, definitions listed in tables, definitions of specific compounds in the examples, etc., may be arbitrarily combined or incorporated with one another. A specific term should not be considered uncertain or unclear unless specifically defined, but should be understood in its ordinary meaning in the art. When a trade name appears herein, it is intended to refer to the corresponding commodity or an active ingredient thereof.

$X_4$ is located at an ortho position to the ring atom in ring A to which Z is linked.

"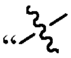"

herein indicates a linking site.

In the present application, "*" indicates that the atom identified thereby is a linking site, for example, linking group

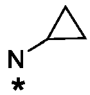

indicates that the N atom in the linking group is the linking site.

The diagrammatic presentation of the racemate or enantiomerically pure compound herein is from Maehr, J.Chem.Ed. 1985, 62:114-120. Unless otherwise stated, the wedged solid bond and wedged dashed bond

(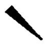 and 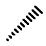)

are used to represent the absolute configuration of a stereocentre, and the straight solid bond and straight dashed bond

(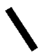 and 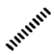)

are used to represent the relative configuration of a stereocentre (such as cis or trans configuration of alicyclic compounds).

The term "tautomer" refers to a functional group isomer resulting from the rapid movement of an atom in two positions in a molecule. The compounds of the present application may exhibit tautomerism. Tautomeric compounds can exist as two or more interconvertible forms. Tautomers generally exist in equilibrium form, so that the attempts to separate a single tautomer usually result in the formation of a mixture, whose chemical and physical properties are consistent with a mixture of the compounds. The position of equilibrium depends on the chemical properties within the molecule. For example, in many aliphatic aldehydes and ketones such as acetaldehyde, the ketone form is dominant; and in phenols, the enol form is dominant. The present application encompasses all tautomeric forms of the compounds.

The term "stereoisomer" refers to an isomer created as a result of different spatial arrangement of atoms in molecules, including cis and trans isomers, enantiomers and diastereomers.

The compounds of the present application may have asymmetric atoms, such as carbon atoms, sulphur atoms, nitrogen atoms, phosphorus atoms or asymmetric double bonds, and therefore the compounds of the present application may exist in specific geometric or stereoisomeric forms. Specific geometric or stereoisomeric forms may be cis and trans isomers, E- and Z-geometric isomers, (-)- and (+)-enantiomers, (R)- and (S)-enantiomers, diastereomers, (D)-isomers, (L)-isomers, and racemic mixtures or other mixtures thereof, such as enantiomerically or diastereomerically enriched

mixtures, and all of the above isomers and mixtures thereof fall within the scope of the definition of the compounds of the present application. Additional asymmetric carbon atoms, asymmetric sulphur atoms, asymmetric nitrogen atoms, or asymmetric phosphorus atoms may be present in substituents such as an alkyl group, and these isomers and mixtures thereof involved in all substituents are also included in the scope of the definition of the compounds of the present application. The compounds containing asymmetric atoms of the present application can be separated in optically active-pure or racemic forms, and the optically active-pure forms can be resolved from the racemic mixture or synthesized by utilizing chiral raw materials or chiral reagents.

The term "substituted" means that any one or more hydrogen atoms on the designated atom are substituted with a substituent, provided that the valence state of the designated atom is normal, and the substituted compound is stable. When the substituent is oxo (i.e., =O), it means that two hydrogen atoms are substituted.

The term "optional" or "optionally" means that the subsequently described event or circumstance may or may not occur, and the description includes the occurrence and the non-occurrence of the event or circumstance. For example, the expression "ethyl is "optionally" substituted with halogen" means that ethyl may be unsubstituted ($CH_2CH_3$), mono-substituted ($CH_2CH_2F$, $CH_2CH_2Cl$, etc.), polysubstituted ($CHFCH_2F$, $CH_2CHF_2$, $CHFCH_2Cl$, $CH_2CHCl_2$, etc.), or completely substituted ($CF_2CF_3$, $CF_2CCl_3$, $CCl_2CCl_3$, etc.). With respect to any group containing one or more substituents, it will be understood by those skilled in the art that any substitution or substitution patterns that are sterically impractical and/or synthetically non-feasible are not intended to be introduced into such group.

Where any variable (such as $R^a$ and $R^b$) appears more than once in the constitution or structure of a compound, its definition in each case is independent. For example, if a group is substituted with two $R^b$, each $R^b$ has an independent option.

$C_m$-$C_n$ herein means that it has an integer number of carbon atoms, wherein the integer number is within the range of m-n. For example, "$C_1$-$C_{10}$" means that the group may have 1, 2, 3, 4, 5, 6, 7, 8, 9 or 10 carbon atoms.

The term "alkyl" refers to a hydrocarbon group of general formula $C_nH_{2n+1}$, which may be linear or branched. The term "$C_1$-$C_{10}$ alkyl" is to be understood as denoting a linear or branched, saturated hydrocarbon group having 1, 2, 3, 4, 5, 6, 7, 8, 9 or 10 carbon atoms. Specific examples of the alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc. The term "$C_1$-$C_6$ alkyl" is to be understood as denoting an alkyl group having 1, 2, 3, 4, 5 or 6 carbon atoms. Specific examples include, but are not limited to, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc. The term "$C_1$-$C_3$ alkyl" is to be understood as denoting a linear or branched, saturated alkyl group having 1, 2 or 3 carbon atoms. The "$C_1$-$C_{10}$ alkyl" may include "$C_1$-$C_6$ alkyl" or "$C_1$-$C_3$ alkyl" and other ranges, and the "$C_1$-$C_6$ alkyl" may further include "$C_1$-$C_3$ alkyl".

The term "alkoxy" refers to a group produced by the loss of a hydrogen atom on a hydroxyl group of a linear or branched alcohol, and is to be understood as "alkyloxy" or "alkyl-O-". The term "$C_1$-$C_{10}$ alkoxy" is to be understood as "$C_1$-$C_{10}$ alkyloxy" or "$C_1$-$C_{10}$ alkyl-O-". The term "$C_1$-$C_6$ alkoxy" is to be understood as "$C_1$-$C_6$ alkyloxy" or "$C_1$-$C_6$ alkyl-O-". The "$C_1$-$C_{10}$ alkoxy" may include "$C_1$-$C_6$ alkoxy" and "$C_1$-$C_3$ alkoxy" and other ranges, and the "$C_1$-$C_6$ alkoxy" may further include "$C_1$-$C_3$ alkoxy".

The term "alkenyl" refers to a linear or branched, unsaturated aliphatic hydrocarbon group consisting of carbon atoms and hydrogen atoms and having at least one double bond. The term "$C_2$-$C_{10}$ alkenyl" is to be understood as denoting a linear or branched, unsaturated hydrocarbon group, comprising one or more double bonds and having 2, 3, 4, 5, 6, 7, 8, 9 or 10 carbon atoms. The "$C_2$-$C_{10}$ alkenyl" is preferably "$C_2$-$C_6$ alkenyl", further preferably "$C_2$-$C_4$ alkenyl", and more further preferably $C_2$ or $C_3$ alkenyl. It is to be understood that where the alkenyl group contains more than one double bond, the double bonds may be isolated from, or conjugated with, each other. Specific examples of the alkenyl include, but are not limited to, vinyl, allyl, (E)-2-methylvinyl, (Z)-2-methylvinyl, (E)-but-2-enyl, (Z)-but-2-enyl, (E)-but-1-enyl, (Z)-but-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, etc.

The term "alkynyl" refers to a linear or branched, unsaturated aliphatic hydrocarbon group consisting of carbon atoms and hydrogen atoms and having at least one triple bond. The term "$C_2$-$C_{10}$ alkynyl" is to be understood as denoting a linear or branched, unsaturated hydrocarbon group, comprising one or more triple bonds and having 2, 3, 4, 5, 6, 7, 8, 9 or 10 carbon atoms. Examples of the "$C_2$-$C_{10}$ alkynyl" include, but are not limited to, ethynyl (-C=CH), prop-1-ynyl (-C≡$CCH_3$), prop-2-ynyl (-$CH_2$C≡CH), but-1-ynyl, but-2-ynyl or but-3-ynyl. The "$C_2$-$C_{10}$ alkynyl" is preferably "$C_2$-$C_6$ alkynyl", further preferably "$C_2$-$C_4$ alkynyl", and more further preferably $C_2$ or $C_3$ alkynyl.

The term "cycloalkyl" refers to a carbocyclic group that is fully saturated and exists as a monocyclic ring, fused ring, bridged ring or spirocyclic ring and other forms. Unless otherwise indicated, the carbocyclic group is typically a 3- to 10-membered ring. The term "$C_3$-$C_{10}$ cycloalkyl" is to be understood as denoting a saturated monocyclic ring, fused ring, spirocyclic ring or bridged ring, having 3, 4, 5, 6, 7, 8, 9 or 10 carbon atoms. Specific examples of the cycloalkyl include, but are not limited to, cyclopropyl, cyclobutyl, cyclopentyl, cyclohexyl, cycloheptyl, cyclooctyl, cyclononyl, cyclodecyl,

norbornyl (bicyclo[2.2.1]heptyl), bicyclo[2.2.2]octyl, adamantyl, spiro[4.5]decanyl, etc. The term "$C_3$-$C_{10}$ cycloalkyl" may include "$C_3$-$C_6$ cycloalkyl". The term "$C_3$-$C_6$ cycloalkyl" is to be understood as denoting a saturated monocyclic or bicyclic hydrocarbon ring, having 3, 4, 5 or 6 carbon atoms. Specific examples include, but are not limited to, cyclopropyl, cyclobutyl, cyclopentyl, cyclohexyl, etc.

The term "cycloalkyloxy" is to be understood as "cycloalkyl-O-".

The term "cycloalkenyl" refers to a non-aromatic carbocyclic group that is not fully saturated and exists as a monocyclic ring, fused ring, bridged ring or spirocyclic ring and other forms. Unless otherwise indicated, the carbocyclic group is typically a 4- to 10-membered ring, having 4, 5, 6, 7, 8, 9 or 10 carbon atoms. Specific examples of the cycloalkenyl include, but are not limited to, cyclopentenyl, cyclopentadienyl, cyclohexenyl, cyclohexadienyl, cycloheptenyl, cycloheptadienyl, etc.

The term "heterocyclyl" refers to a fully saturated or partially saturated monocyclic ring, fused ring, spirocyclic ring or bridged ring group, the ring atoms therein containing 1, 2, 3, 4 or 5 heteroatoms or heteroatomic groups (namely, atomic groups containing heteroatoms). The "heteroatoms or heteroatomic groups" include, but are not limited to, nitrogen atoms (N), oxygen atoms (O), sulphur atoms (S), phosphorus atoms (P), boron atoms (B), $-S(=O)_2-$, -S(=O)-, $-P(=O)_2-$, -P(=O)-, -NH-, -S(=O)(=NH)-, -C(=O)NH-, -NHC(=O)NH-, etc. The term "3- to 18-membered heterocyclyl" refers to a heterocyclyl group having 3, 4, 5, 6, 7, 8, 9, 10, 11, 12, 13, 14, 15, 16, 17 or 18 ring atoms, the ring atoms therein containing 1, 2, 3, 4 or 5 heteroatoms or heteroatomic groups independently selected from the above-mentioned heteroatoms or heteroatomic groups. The term "4- to 12-membered heterocyclyl" refers to a heterocyclyl group having 4, 5, 6, 7, 8, 9, 10, 11 or 12 ring atoms, the ring atoms therein containing 1, 2, 3, 4 or 5 heteroatoms or heteroatomic groups independently selected from the above-mentioned heteroatoms or heteroatomic groups. The term "4- to 10-membered heterocyclyl" refers to a heterocyclyl group having 4, 5, 6, 7, 8, 9 or 10 ring atoms, the ring atoms therein containing 1, 2, 3, 4 or 5 heteroatoms or heteroatomic groups independently selected from the above-mentioned heteroatoms or heteroatomic groups. Specific examples of 4-membered heterocyclyl include, but are not limited to, azetidinyl, thietanyl or oxetanyl. Specific examples of 5-membered heterocyclyl include, but are not limited to, tetrahydrofuryl, dioxolyl, pyrrolidyl, imidazolidinyl, pyrazolidinyl, pyrrolinyl, 4,5-dihydrooxazolyl or 2,5-dihydro-1H-pyrrolyl. Specific examples of 6-membered heterocyclyl include, but are not limited to, tetrahydropyranyl, piperidyl, morpholinyl, dithianyl, thiomorpholinyl, piperazinyl, trithianyl, tetrahydropyridyl or 4H-[1,3,4]thiadiazinyl. Specific examples of 7-membered heterocyclyl include, but are not limited to, diazepanyl. Specific examples of 8-membered heterocyclyl include, but are not limited to, hexahydrocyclopenta[c]pyrrol-2(1H)-yl. Specific examples of 9-membered heterocyclyl include, but are not limited to, benzo[d][1,3]dioxolyl. Specific examples of 10-membered heterocyclyl include, but are not limited to, dihydroisoquinolinyl. The heterocyclyl may also be a bicyclic group. Specific examples of 5,5-membered bicyclic group include, but are not limited to, hexahydrocyclopenta[c]pyrrol-2(1H)-yl. Specific examples of 5,6-membered bicyclic group include, but are not limited to, hexahydropyrrolo[1,2-*a*]pyrazin-2(1H)-yl, 5,6,7,8-tetrahydro-[1,2,4]triazolo[4,3-*a*]pyrazinyl or 5,6,7,8-tetrahydroimidazo[1,5-*a*]pyrazinyl. Optionally, the heterocyclyl may be a benzo-fused ring group of the above-mentioned 4- to 7-membered heterocyclyl. Specific examples include, but are not limited to, dihydroisoquinolinyl, benzo[d][1,3]dioxolyl, etc. "4- to 10-membered heterocyclyl" may include "5- to 10-membered heterocyclyl", "4- to 7-membered heterocyclyl", "5- to 6-membered heterocyclyl", "6- to 8-membered heterocyclyl", "4- to 10-membered heterocycloalkyl", "5- to 10-membered heterocycloalkyl", "4- to 7-membered heterocycloalkyl", "5- to 6-membered heterocycloalkyl", "6- to 8-membered heterocycloalkyl" and other ranges, and "4- to 7-membered heterocyclyl" may further include "4-6-membered heterocyclyl", "5- to 6-membered heterocyclyl", "4-to 7-membered heterocycloalkyl", "4- to 6-membered heterocycloalkyl", "5- to 6-membered heterocycloalkyl" and other ranges.

The term "heterocyclyloxy" is to be understood as "heterocyclyl-O-".

The term "heterocycloalkyl" refers to a cyclic group that is fully saturated and exists as a monocyclic ring, fused ring, bridged ring or spirocyclic ring and other forms, the ring atoms therein containing 1, 2, 3, 4 or 5 heteroatoms or heteroatomic groups (namely, atomic groups containing heteroatoms). The "heteroatoms or heteroatomic groups" include, but are not limited to, nitrogen atoms (N), oxygen atoms (O), sulphur atoms (S), phosphorus atoms (P), boron atoms (B), $-S(=O)_2-$, - S(=O)-, -NH-, -S(=O)(=NH)-, -C(=O)NH-, -NHC(=O)NH-, etc.

The term "aryl" refers to an all-carbon monocyclic or fused polycyclic aromatic ring group having a conjugated $\pi$-electron system. The aryl can have 6-20 carbon atoms, 6-14 carbon atoms or 6-10 carbon atoms. The term "$C_6$-$C_{14}$ aryl" is to be understood as an aryl group having 6 to 14 carbon atoms, especially a ring having 6 carbon atoms ("$C_6$ aryl"), such as phenyl; or a ring having 9 carbon atoms ("$C_9$ aryl"), such as indanyl or indenyl; or a ring having 10 carbon atoms ("$C_{10}$ aryl"), such as tetrahydronaphthyl, dihydronaphthyl or naphthyl; or a ring having 13 carbon atoms ("$C_{13}$ aryl"), such as fluorenyl; or a ring having 14 carbon atoms ("$C_{14}$ aryl"), such as anthracenyl. The term "$C_6$-$C_{10}$ aryl" is to be understood as an aryl group having 6 to 10 carbon atoms, especially a ring having 6 carbon atoms ("$C_6$ aryl"), such as phenyl; or a ring having 9 carbon atoms ("$C_9$ aryl"), such as indanyl or indenyl; or a ring having 10 carbon atoms ("$C_{10}$ aryl"), such as tetrahydronaphthyl, dihydronaphthyl or naphthyl.

The term "heteroaryl" refers to an aromatic monocyclic or fused polycyclic ring system, containing at least one ring atom selected from N, O, and S, the remaining ring atoms being aromatic ring groups of C. The term "5- to 10-membered heteroaryl" is to be understood as including a monocyclic or bicyclic aromatic ring system having 5, 6, 7, 8, 9 or 10 ring atoms, especially 5 or 6 or 9 or 10 ring atoms, and containing 1, 2, 3, 4 or 5 heteroatoms, preferably 1, 2 or 3 heteroatoms independently selected from N, O and S. Especially, the heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, etc., and the benzo derivatives thereof, such as benzofuryl, benzothienyl, benzothiazolyl, benzoxazolyl, benzisoxazolyl, benzoimidazolyl, benzotriazolyl, indazolyl, indolyl or isoindolyl; or pyridyl, pyridazinyl, pyrimidyl, pyrazinyl, triazinyl, etc., and the benzo derivatives thereof, such as quinolinyl, quinazolinyl or isoquinolinyl; or azocinyl, indolizinyl, purinyl, etc., and the benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, etc. The term "5- to 6-membered heteroaryl" refers to an aromatic ring system having 5 or 6 ring atoms, and containing 1, 2 or 3 heteroatoms, preferably 1 to 2 heteroatoms independently selected from N, O and S. The term "9- to 10-membered heteroaryl" refers to an aromatic ring system having 9 or 10 ring atoms, and containing 1, 2 or 3 heteroatoms, preferably 1 to 2 heteroatoms independently selected from N, O and S. "6-membered heteroaryl" refers to an aromatic ring system having 6 ring atoms, and containing 1, 2 or 3 heteroatoms, preferably 1 to 2 heteroatoms independently selected from N, O and S.

The term "alkoxyacyl" refers to -COO-alkyl. The term "$C_1$-$C_{10}$ alkoxyacyl" refers to -COO-$C_1$-$C_{10}$ alkyl, such as methoxyacyl, ethoxyacyl, propoxyacyl, isopropoxyacyl, n-butoxyacyl, isobutoxyacyl or tert-butoxyacyl. The term "$C_1$-$C_6$ alkoxyacyl" refers to -COO-$C_1$-$C_6$ alkyl. The term "$C_1$-$C_3$ alkoxyacyl" refers to - COO-$C_1$-$C_3$ alkyl.

The term "alkylsulfonyl" refers to -S(=O)$_2$-alkyl, such as methylsulfonyl, ethylsulfonyl, or 2-propylsulfonyl. The term "$C_1$-$C_{10}$ alkylsulfonyl" refers to - S(=O)$_2$-$C_1$-$C_{10}$ alkyl. The term "$C_1$-$C_6$ alkylsulfonyl" refers to -S(=O)$_2$-$C_1$-$C_6$ alkyl. The term "$C_1$-$C_3$ alkylsulfonyl" refers to -S(=O)$_2$-$C_1$-$C_3$ alkyl.

The term "alkylacyl" refers to -C(=O)-alkyl, such as methylacyl or ethylacyl. The term "$C_1$-$C_{10}$ alkylacyl" refers to -C(=O)-$C_1$-$C_{10}$ alkyl.

The term "heterocyclylalkyl" refers to -(alkylene)-heterocyclyl.

The term "halo" or "halogen" refers to fluorine, chlorine, bromine or iodine.

The term "hydroxyl" refers to -OH group.

The term "cyano" refers to -CN group.

The term "amino" refers to -$NH_2$ group.

The term "therapeutically effective amount" means an amount of the compound of the present application that (i) treats a particular disease, condition or disorder, (ii) attenuates, ameliorates or eliminates one or more symptoms of a particular disease, condition or disorder, or (iii) delays the onset of one or more symptoms of a particular disease, condition or disorder described herein. The amount of the compound of the present application which constitutes a "therapeutically effective amount" will vary depending on the compound, the disease states and the severity thereof, the manner of administration, and the age of the mammal to be treated, but can be determined routinely by those skilled in the art according to their own knowledge and the present disclosure.

The term "pharmaceutically acceptable" refers to compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The term "pharmaceutically acceptable salt" refers to salts of pharmaceutically acceptable acids or bases, including salts formed between compounds and inorganic or organic acids, and salts formed between compounds and inorganic or organic bases.

The term "pharmaceutical composition" refers to a mixture of one or more of the compounds or the salts thereof according to the present application and a pharmaceutically acceptable excipient. The pharmaceutical composition is intended to facilitate administering the compound of the present application to an organism.

The term "pharmaceutically acceptable excipient" refers to excipients which have no significant irritating effect on the organism and do not impair the bioactivity and properties of the active compound. Suitable excipients are well known to those skilled in the art, such as a carbohydrate, a wax, a water-soluble and/or water-swellable polymer, a hydrophilic or hydrophobic material, gelatine, an oil, a solvent, and water.

The wording "comprise" and its variants such as "comprises" or "comprising" are to be understood as an open, non-exclusive meaning, i.e., "including, but not limited to".

The present application also includes isotopically-labelled compounds of the present application which are identical to those recited herein, but have one or more atoms replaced by an atom having an atomic mass or mass number different from the atomic mass or mass number usually found in nature. Examples of isotopes that can be incorporated into the compounds of the present application include isotopes of hydrogen, carbon, nitrogen, oxygen, phosphorus, sulphur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, respectively.

Certain isotopically-labelled compounds of the present application (e.g., those labelled with $^{3}H$ and $^{14}C$) are useful in tissue distribution assays of compounds and/or substrates. Tritiated (i.e., $^{3}H$) and carbon-14 (i.e., $^{14}C$) isotopes

are particularly preferred for their ease of preparation and detectability. Positron emitting isotopes such as $^{15}O$, $^{13}N$, $^{11}C$, and $^{18}F$ are useful for positron emission tomography (PET) studies to examine substrate occupancy. The isotopically-labelled compounds of the present application can generally be prepared according to following procedures analogous to those disclosed in the Schemes and/or in the Examples herein below by substituting a non-isotopically-labelled reagent with an isotopically-labelled reagent.

The pharmaceutical composition of the present application may be prepared by combining the compound of the present application with an appropriate pharmaceutically acceptable excipient. For example, the pharmaceutical composition of the present application may be formulated into solid, semi-solid, liquid or gaseous preparations, such as tablets, pills, capsules, powders, granules, ointments, emulsions, suspensions, suppositories, injections, inhalants, gels, microspheres and aerosols.

Typical administration routes of the compound or the pharmaceutically acceptable salt thereof, or the pharmaceutical composition comprising same according to the present application include, but are not limited to, oral administration, rectal administration, topical administration, administration by inhalation, parenteral administration, sublingual administration, intravaginal administration, intranasal administration, intraocular administration, intraperitoneal administration, intramuscular administration, subcutaneous administration, and intravenous administration.

The pharmaceutical composition of the present application can be manufactured by using well-known methods in the art, such as conventional mixing method, dissolution method, granulation method, emulsification method, and freezedrying method.

In some embodiments, the pharmaceutical composition is in oral form. For oral administration, the pharmaceutical composition may be formulated by mixing the active compound with a pharmaceutically acceptable excipient well-known in the art. Such excipients enable the compounds of the present application to be formulated into tablets, pills, lozenges, dragees, capsules, liquids, gels, syrups, suspensions, etc., for oral administration to patients.

A solid oral composition can be prepared by a conventional mixing, filling or tableting method. For example, it can be obtained by mixing the active compound with a solid excipient, optionally grinding the resulting mixture, adding other suitable excipients, if necessary, and then processing the mixture into granules to obtain cores of tablets or dragees. Suitable excipients include, but are not limited to, binders, diluents, disintegrants, lubricants, glidants, flavouring agents, etc.

The pharmaceutical composition can also be suitable for parenteral administration, such as sterile solutions, suspensions or lyophilized products in a suitable unit dosage form.

The daily administration dose of the compound of general formula (I) in all the administration manners described herein is from 0.1 mg/kg body weight to 500 mg/kg body weight, preferably from 0.5 mg/kg body weight to 400 mg/kg body weight, and more preferably from 1 mg/kg body weight to 200 mg/kg body weight, in the form of a single dose or divided doses.

The compounds of the present application can be prepared by various synthetic methods well known to those skilled in the art, including the specific examples listed below, the embodiments formed by the combination with other chemical synthesis methods, and equivalent alternative embodiments well known to those skilled in the art, wherein the preferred embodiments include, but are not limited to, the examples of the present application.

The chemical reactions described in the specific examples of the present application are completed in a suitable solvent, wherein the solvent must be suitable for the chemical changes of the present application and the reagents and materials required thereby. In order to obtain the compounds of the present application, sometimes those skilled in the art need to modify or select synthesis steps or reaction schemes based on the existing examples.

The present application uses the following abbreviations:

| Abbreviation | Chemical name | Abbreviation | Chemical name |
|---|---|---|---|
| HATU | O-(7-azabenzotriazol-1-yl)-*N,N,N',N'*-tetramethyluronium hexafluorophosphate | DIEA or DIPEA | N,N-diisopropylethylamine |
| $Pd(dppf)Cl_2$ or $PdCl_2$ (dppf) | 1,1'-bis(diphenylphosphino)ferrocene palladium dichloride (II) or 1,1-bis(diphenylphosphine)ferrocene palladium chloride | TFA | Trifluoroacetic acid |
| $Pd(dtbpf)Cl_2$ or $PdCl_2$ (dtbpt) | 1,1'-bis(di-tert-butylphosphino)ferrocene palladium dichloride (II) or 1,1-bis(tert-butylphosphine)ferrocene palladium chloride | DMF | *N,N*-dimethylformamide |
| $PIn_2B_2$ | Bis(pinacolato)diboron | DMAP | 4-dimethylaminopyridine |
| DME | 1,2-dimethoxy ethane | $NiCl_2$ glyme | Nickel (II) chloride ethylene glycol dimethyl ether complex |

(continued)

| Abbreviation | Chemical name | Abbreviation | Chemical name |
|---|---|---|---|
| Bipyridine | 2,2'-bipyridine | $Pd(dba)_2$ | Bis(dibenzylideneacetone)pallad ium |
| Xantphos | 4,5-bis(diphenylphosphine)-9,9-dimethyl-xanthene | $Pd(PPh_3)_4$ | Tetrakistriphenylphosphine palla-dium |
| cataCXium A-Pd-G2 | Chloro[(n-butylbis(1-adamantyl)phosphine)-2-(2-aminobiphenyl)]palladium (II) | $PdCl_2(PPh_3)_2$ | Dichlorobis(triphenylphosphine) palladium |
| LDA | Lithium diisopropylamide | Xphos-Pd-$G_2$ | Chloro(2-dicyclohexylphosphi-no-2,4,6-triisopropyl-1,1-biphenyl) [2-(2-amino-1,1-biphenyl)]palla-dium (II) |
| $Pd_2(dba)_3$ | Tris(dibenzylideneacetone)dipalladium | EDCI | 1-(3-dimethylaminopropyl)-3-ethylcarbodiimide hydrochloride |
| HOBt | 1-hydroxybenzotriazole | TosMIC | 1-(isocyanomethylsulfonyl)-4-methylbenzene |
| DMF-DMA | N,N-dimethylformamide dimethyl acetal | TFAA | Trifluoroacetic anhydride |
| DCM | Dichloromethane | dioxane | Dioxane |
| TEA | Triethylamine | TBAI | Tetrabutylammonium iodide |
| DMA | *N,N*-dimethylacetamide | | |

**Specific Examples**

The present invention will be described in detail with the following examples, but not imply any adverse limitation to the present application. The present application has been described in detail herein, and the specific examples thereof are also disclosed. Various changes and improvements to the specific examples of the present application would be obvious to those skilled in the art without departing from the spirit and scope of the present application. All reagents used in the present application are commercially available and can be used without further purification.

Unless otherwise specified, the ratios indicated for mixed solvents are volume mixing ratios. For example, "petroleum ether : ethyl acetate = 1 : 2" means that the volume ratio of petroleum ether to ethyl acetate is 1 : 2.

Compounds are named by hand or ChemDraw® software, and commercially available compounds are named by the supplier catalogue names.

The structures of the compounds are determined by nuclear magnetic resonance (NMR) and/or mass spectrometry (MS). The NMR shifts are calculated in $10^{-6}$ (ppm). The solvents for NMR assay are deuterated dimethyl sulfoxide, deuterated chloroform, deuterated methanol, etc., and the internal standard is tetramethylsilane (TMS). "$IC_{50}$" refers to the half inhibitory concentration, the concentration at which half of the maximal inhibitory effect is achieved.

In the following purification using high performance liquid chromatography, unless otherwise specified, the amount "%" of acid or base in mobile phase A refers to the volume fraction, for example, "water (0.05% formic acid)" means that the volume of formic acid is 0.05% of the total volume of formic acid and water. B% indicates the proportion of the volume of mobile phase B in the total volume of mobile phase A and mobile phase B during gradient elution, and "B%: 50%-70%" indicates that the proportion of the volume of mobile phase B in the total volume of mobile phase A and mobile phase B varies from 50% to 70% during gradient elution.

**Example 1, *N*-(5-(4-chlorophenyl)thiazolo[5,4-b]pyridin-2-yl)-3-(2-methoxyphenyl)pyridine-4-carboxamide (compound 1)**

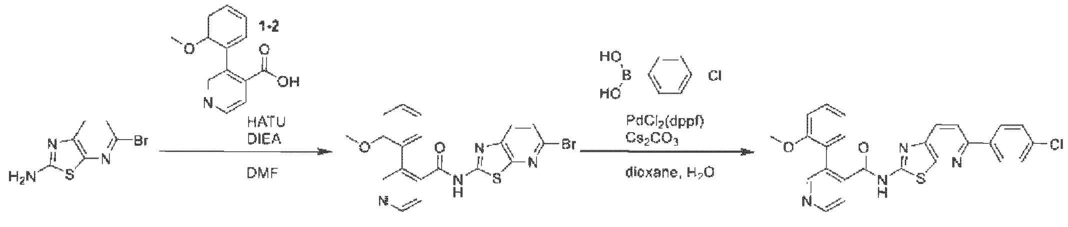


1-1

1-3

Compound 1

**Step 1: Synthesis of *N*-(5-bromothiazolo[5,4-b]pyridin-2-yl)-3-(2-methoxyphenyl)pyridine-4-carboxamide (intermediate 1-3)**

At 20°C, **intermediate 1-1** (100 mg) was dissolved in DMF (2 mL), and to the reaction solution were added **intermediate 1-2** (99.63 mg, which could be synthesized according to the method reported in International Publication No. WO 2020243459), HATU (247.88 mg) and DIEA (112.34 mg). The reaction solution was stirred at 20°C for 1 hour. After the reaction was completed, to the reaction solution were added ethyl acetate (10 mL) and water (20 mL), and the organic phase was washed with water (10 mL*2), then separated, and dried over anhydrous sodium sulphate. After filtration, the organic phase was concentrated to dryness under reduced pressure. The residue was purified by thin-layer chromatography (petroleum ether : ethyl acetate = 1 : 2) to obtain the title compound (22 mg).
MS m/z (ESI): = 441.3 $[M+H]^+$.

**Step 2: Synthesis of *N*-(5-(4-chlorophenyl)thiazolo[5,4-b]pyridin-2-yl)-3-(2-methoxyphenyl)pyridine-4-carboxamide (compound 1)**

At 20°C, **intermediate 1-3** (20 mg) was dissolved in a solution of dioxane (1 mL)/water (0.2 mL), and to the reaction solution were added p-chlorophenylboronic acid (9.21 mg), caesium carbonate (29.53 mg) and 1,1'-bis(diphenylphosphino)ferrocene palladium dichloride (II) ($PdCl_2$(dppf)) (1.64 mg). Under nitrogen protection, the reaction solution was stirred at 80°C for 15 minutes. After the reaction was completed, the reaction solution was concentrated to dryness under reduced pressure. The residue was purified by high performance liquid chromatography (chromatographic column: C18-2 (100*30 mm*5 um); mobile phase: A: water (0.05% ammonia water), B: acetonitrile; gradient elution, B%: 19%-59%, 10 minutes) to obtain the title compound (1.09 mg).
**$^1$H NMR (400 MHz, Methanol-$d_4$)** $\delta$ = 8.75-8.72 (m, 1H), 8.66 (s, 1H), 8.16-8.08 (m, 3H), 7.99 (d, *J*= 8.6 Hz, 1H), 7.76-7.73 (m, 1H), 7.54-7.50 (m, 2H), 7.48-7.37 (m, 2H), 7.14 (t, *J* = 7.4 Hz, 1H), 7.00 (d, *J* = 8.0 Hz, 1H), 3.64 (s, 3H)
MS m/z (ESI): = 473.1 $[M+H]^+$.

**Example 2, *N*-(6-(4-chlorophenyl)thiazolo[4,5-b]pyrazin-2-yl)-3-(2-methoxyphenyl)pyridine-4-carboxamide (compound 2)**

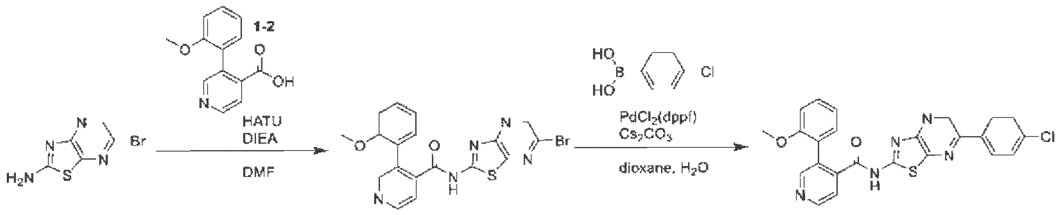


2-1

2-2

Compound 2

**Step 1: Synthesis of *N*-(6-bromothiazolo[4,5-b]pyrazin-2-yl)-3-(2-methoxyphenyl)pyridine-4-carboxamide (intermediate 2-2)**

At 20°C, **intermediate 2-1** (100 mg) was dissolved in DMF (2 mL), and to the reaction solution were added **intermediate 1-2** (66.14 mg), HATU (164.55 mg) and DIEA (74.58 mg). The reaction solution was stirred at 20°C for 1 hour. After the reaction was completed, to the reaction solution were added ethyl acetate (10 mL) and water (20 mL), and the organic phase was washed with water (10 mL*2), then separated, and dried over anhydrous sodium sulphate. After filtration, the organic phase was concentrated to dryness under reduced pressure. The residue was purified by thin-layer

chromatography (petroleum ether : ethyl acetate = 1 : 5) to obtain the title compound (25 mg).

MS m/z (ESI): = 442.0 [M+H]+.

**Step 2: Synthesis of *N*-(6-(4-chlorophenyl)thiazolo[4,5-b]pyrazin-2-yl)-3-(2-methoxyphenyl)pyridine-4-carboxamide (compound 2)**

At 20°C, **intermediate 2-2** (25 mg) was dissolved in a solution of dioxane (1 mL)/water (0.2 mL), and to the reaction solution were added p-chlorophenylboronic acid (11.49 mg), caesium carbonate (36.83 mg) and 1,1'-bis(diphenylphosphino)ferrocene palladium dichloride (II) (2.07 mg). Under nitrogen protection, the reaction solution was stirred at 80°C for 15 minutes. After the reaction was completed, the reaction solution was concentrated to dryness under reduced pressure. The residue was purified by high performance liquid chromatography (chromatographic column: Boston Prime C18 (150*30 mm*5 um); mobile phase: A: water (0.05% formic acid), B: acetonitrile; gradient elution, B%: 50%-70%, 11 minutes) to obtain the title compound (2.03 mg).

**$^1$H NMR (400 MHz, Methanol-$d_4$)** δ = 9.12 (s, 1H), 8.75 (d, *J*= 5.0 Hz, 1H), 8.67 (s, 1H), 8.17 (d, *J*= 8.6 Hz, 2H), 7.76 (d, *J*= 5.3 Hz, 1H), 7.56 (d, *J*= 8.6 Hz, 2H), 7.47-7.41 (m, 2H), 7.17-7.11 (m, 1H), 7.02-6.98 (m, 1H), 3.65 (s, 3H)

MS m/z (ESI): = 474.1 [M+H]+.

**Example 3, *N*-(6-(4-chlorophenyl)thiazolo[4,5-b]pyrazin-2-yl)-3-(2-fluoro-6-methoxyphenyl)pyridine-4-carboxamide (compound 3)**

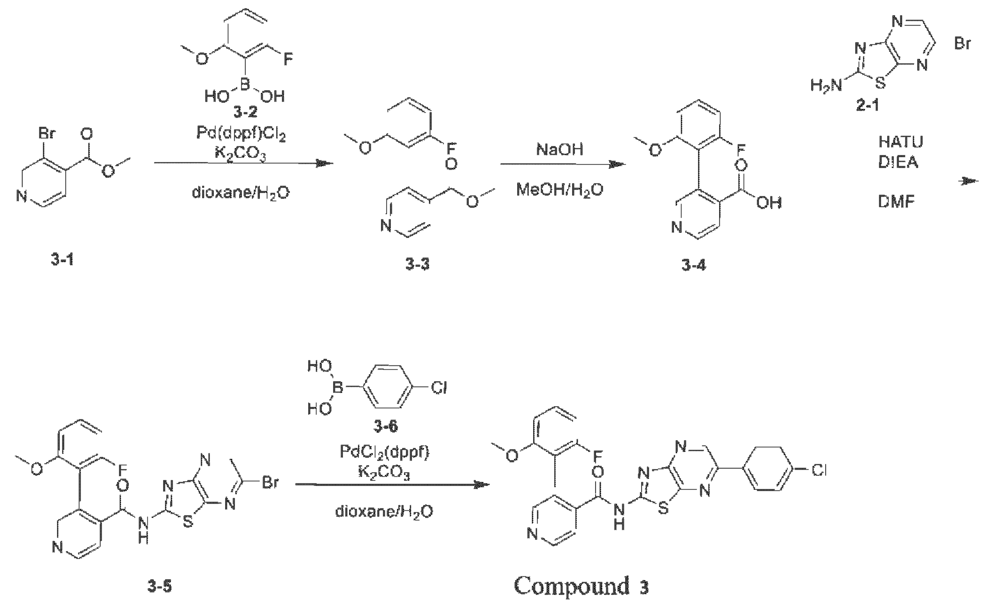


3-2
3-1
3-3
3-4
2-1
3-6
3-5
Compound 3

**Step 1: Synthesis of methyl 3-(2-fluoro-6-methoxyphenyl)pyridine-4-carboxylate (intermediate 3-3)**

At 20°C, **intermediate 3-1** (500 mg) was dissolved in a solution of dioxane (15 mL)/water (3 mL), and to the reaction solution were added **intermediate 3-2** (786.67 mg), potassium carbonate (639.75 mg) and 1,1'-bis(diphenylphosphino)ferrocene palladium dichloride (II) (169.35 mg). Under nitrogen protection, the reaction solution was stirred at 80°C for 15 minutes. After the reaction was completed, the reaction solution was filtered, and the filtrate was concentrated to dryness under reduced pressure. The residue was purified by column chromatography (petroleum ether : ethyl acetate = 1 : 1) to obtain the title compound (600 mg).

MS m/z (ESI): = 262.1 [M+H]+.

**Step 2: Synthesis of 3-(2-fluoro-6-methoxyphenyl)pyridine-4-carboxylic acid (intermediate 3-4)**

At 20°C, **intermediate 3-3** (300 mg) was dissolved in a solution of methanol (5 mL)/water (5 mL), and to the reaction solution was added sodium hydroxide (137.79 mg). The reaction solution was stirred at 50°C for 2 hours. After the reaction was completed, the reaction solution was concentrated to dryness under reduced pressure. The residue was

dissolved in a small amount of methanol. After filtration, the filtrate was concentrated to dryness under reduced pressure to obtain the title compound (220 mg).

MS m/z (ESI): = 248.1 [M+H]+.

**Step 3: Synthesis of *N*-(6-bromothiazolo[4,5-b]pyrazin-2-yl)-3-(2-fluoro-6-methoxyphenyl)pyridine-4-carboxamide (intermediate 3-5)**

At 20°C, **intermediate 3-4** (80 mg) was dissolved in DMF (3 mL), and to the reaction solution were added **intermediate 2-1** (74.77 mg), HATU (135.35 mg) and DIEA (83.65 mg). The reaction solution was stirred at 20°C for 1 hour. After the reaction was completed, to the reaction solution were added ethyl acetate (5 mL) and water (10 mL), and the mixture was extracted with ethyl acetate (5 mL*2). The organic phases were combined, and dried over anhydrous sodium sulphate. After filtration, the organic phase was concentrated to dryness under reduced pressure. The residue was purified by thin-layer chromatography (petroleum ether : ethyl acetate = 1 : 2) to obtain the title compound (50 mg).

MS m/z (ESI): = 460.0 [M+H]+.

**Step 4: Synthesis of *N*-(6-(4-chlorophenyl)thiazolo[4,5-b]pyrazin-2-yl)-3-(2-fluoro-6-methoxyphenyl)pyridine-4-carboxamide (compound 3)**

At 20°C, **intermediate 3-5** (50 mg) was dissolved in a solution of dioxane (1 mL)/water (0.2 mL), and to the reaction solution were added intermediate **3-6** p-chlorophenylboronic acid (33.97 mg), potassium carbonate (30.03 mg) and 1,1'-bis(diphenylphosphino)ferrocene palladium dichloride (II) (7.95 mg). Under nitrogen protection, the reaction solution was stirred at 80°C for 15 minutes. After the reaction was completed, the reaction solution was filtered, and the filtrate was concentrated to dryness under reduced pressure. The residue was purified by high performance liquid chromatography (chromatographic column: Waters Xbridge BEH C18 (100*30 mm*10 um); mobile phase: A: water (0.05% formic acid), B: acetonitrile; gradient elution, B%: 40%-80%, 10 minutes) to obtain the title compound (16.4 mg).

**$^1$H NMR (400 MHz, Methanol-*$d_4$*)** δ = 13.62 (s, 1H), 9.29 (s, 1H), 8.82 (d, *J* = 5.0 Hz, 1H), 8.70 (s, 1H), 8.22 (d, *J*= 8.5 Hz, 2H), 7.85 (d, *J*= 5.3 Hz, 1H), 7.62 (d, *J*= 8.5 Hz, 2H), 7.46-7.40 (m, 1H), 6.98 (t, *J*= 9.0 Hz, 1H), 6.91 (d, *J*= 8.5 Hz, 1H), 3.60 (s, 3H)

MS m/z (ESI): = 492.1 [M+H]+.

**Example 4, *N*-(6-(4-chlorophenyl)thiazolo[4,5-b]pyrazin-2-yl)-4-(2-methoxyphenyl)-6-methylpyridine-3-carboxamide (compound 4)**

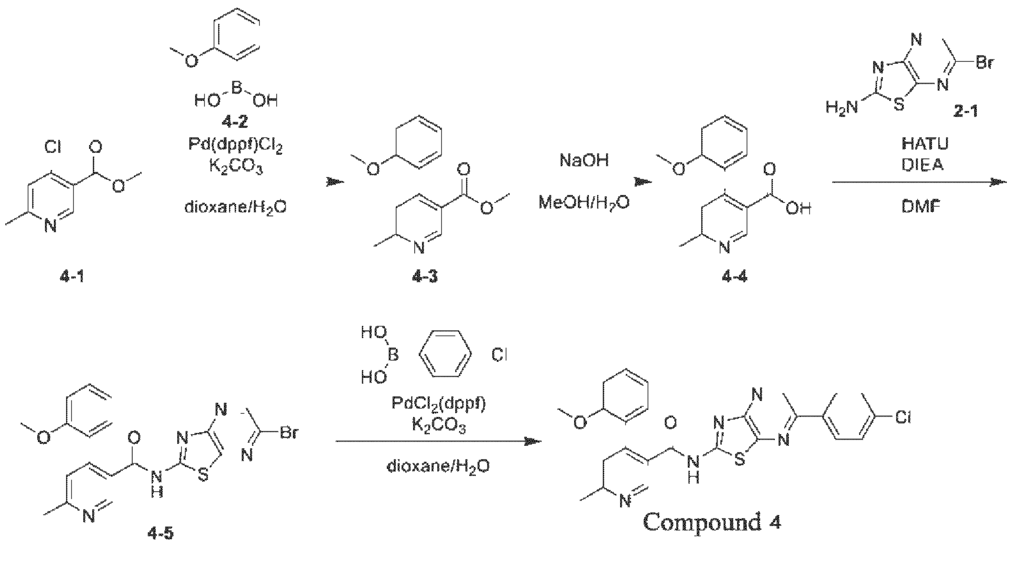


**Step 1: Synthesis of methyl 4-(2-methoxyphenyl)-6-methylpyridine-3-carboxylate (intermediate 4-3)**

At 20°C, **intermediate 4-1** (500 mg) was dissolved in a solution of dioxane (15 mL)/water (3 mL), and to the reaction solution were added intermediate 4-2 (818.69 mg), potassium carbonate (744.62 mg) and 1,1'-bis(diphenylphosphino)ferrocene palladium dichloride (II) (197.11 mg). Under nitrogen protection, the reaction solution was stirred at 80°C for 15 minutes. After the reaction was completed, the reaction solution was filtered, and the filtrate was concentrated to dryness under reduced pressure. The residue was purified by column chromatography (petroleum ether : ethyl acetate = 1 : 1) to obtain the title compound (490 mg).

MS m/z (ESI): = 258.1 $[M+H]^+$.

**Step 2: Synthesis of 4-(2-methoxyphenyl)-6-methylpyridine-3-carboxylic acid (intermediate 4-4)**

At 20°C, **intermediate 4-3** (300 mg) was dissolved in a solution of methanol (5 mL)/water (5 mL), and to the reaction solution was added sodium hydroxide (139.91 mg). The reaction solution was stirred at 50°C for 2 hours. After the reaction was completed, the reaction solution was concentrated to dryness under reduced pressure. The residue was dissolved in a small amount of methanol. After filtration, the filtrate was concentrated to dryness under reduced pressure to obtain the title compound (280 mg).

MS m/z (ESI): = 244.1 $[M+H]^+$.

**Step 3: Synthesis of *N*-(6-bromothiazolo[4,5-b]pyrazin-2-yl)-4-(2-methoxyphenyl)-6-methylpyridine-3-carboxamide (intermediate 4-5)**

At 20°C, **intermediate 4-4** (100 mg) was dissolved in DMF (3 mL), and to the reaction solution were added **intermediate 2-1** (94.99 mg), HATU (187.57 mg) and DIEA (106.26 mg). The reaction solution was stirred at 20°C for 1 hour. After the reaction was completed, to the reaction solution were added ethyl acetate (5 mL) and water (10 mL), and the mixture was extracted with ethyl acetate (5 mL*2). The organic phases were combined, and dried over anhydrous sodium sulphate. After filtration, the organic phase was concentrated to dryness under reduced pressure. The residue was purified by thin-layer chromatography (petroleum ether : ethyl acetate = 1 : 1) to obtain the title compound (130 mg).

MS m/z (ESI): = 456.0 $[M+H]^+$.

**Step 4: Synthesis of *N*-(6-(4-chlorophenyl)thiazolo[4,5-b]pyrazin-2-yl)-4-(2-methoxyphenyl)-6-methylpyridine-3-carboxamide (compound 4)**

At 20°C, **intermediate 4-5** (50 mg) was dissolved in a solution of dioxane (1 mL)/water (0.2 mL), and to the reaction solution were added p-chlorophenylboronic acid (34.27 mg), potassium carbonate (30.29 mg) and 1,1'-bis(diphenylphosphino)ferrocene palladium dichloride (II) (8.02 mg). Under nitrogen protection, the reaction solution was stirred at 80°C for 15 minutes. After the reaction was completed, the reaction solution was filtered, and the filtrate was concentrated to dryness under reduced pressure. The residue was purified by high performance liquid chromatography (chromatographic column: Waters Xbridge BEH C18 (100*30 mm*10 um); mobile phase: A: water (0.05% formic acid), B: acetonitrile; gradient elution, B%: 34%-74%, 10 minutes) to obtain the title compound (15 mg).

**$^1$H NMR (400 MHz, DMSO-*d*$_6$)** $\delta$ = 13.35 (s, 1H), 9.27 (s, 1H), 8.77 (s, 1H), 8.22 (d, *J* = 8.5 Hz, 2H), 7.62 (d, *J* = 8.8 Hz, 2H), 7.44-7.40 (m, 2H), 7.36 (s, 1H), 7.12 (t, *J*= 7.5 Hz, 1H), 6.99 (d, *J*= 7.8 Hz, 1H), 3.52 (s, 3H), 2.60 (s, 3H)

MS m/z (ESI): = 488.1 $[M+H]^+$.

**Example 5, *N*-(6-(3-chlorophenyl)thiazolo[4,5-b]pyrazin-2-yl)-3-(2-methoxyphenyl)pyridine-4-carboxamide (compound 5)**

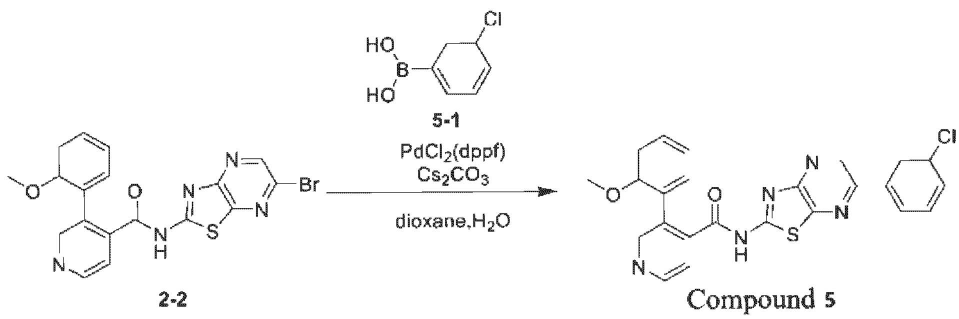


At 20°C, **intermediate 2-2** (30 mg) and **intermediate 5-1** (31.82 mg) were dissolved in dioxane (1.5 mL) and water (0.3 mL), and then 1,1'-bis(diphenylphosphino)ferrocene palladium dichloride (II) (2.48 mg) and caesium carbonate

(44.2 mg) were added to the mixture. Under nitrogen protection, the resulting mixture was stirred at 100°C for 3 hours. After the reaction was completed, the organic phase was concentrated to dryness under reduced pressure. Ethyl acetate (10 mL) and water (10 mL) were added, and the mixture was washed with water (10 mL*2). The organic phase was separated, and dried over anhydrous sodium sulphate. After filtration, the organic phase was concentrated to dryness under reduced pressure. The residue was purified by high performance liquid chromatography (chromatographic column: Boston Green ODS (150*30 mm*5 um); mobile phase: A: water (0.05% formic acid), B: acetonitrile, gradient elution, B%: 55%-85%, 12 minutes) to obtain the title compound (5.4 mg).

**$^1$H NMR (400MHz, DMSO-$d_6$)** δ = 13.49 (s, 1H), 9.29 (s, 1H), 8.77 (d, *J* = 4.8 Hz, 1H), 8.67 (s, 1H), 8.23 (s, 1H), 8.16 (d, *J* = 6.9 Hz, 1H), 7.74 (d, *J* = 4.9 Hz, 1H), 7.66-7.51 (m, 2H), 7.48-7.34 (m, 2H), 7.17-7.09 (m, 1H), 7.00 (d, *J*= 8.3 Hz, 1H), 3.53 (s, 3H)

MS m/z (ESI): = 474.1 [M+H]+.

**Example 6, 3-(2-methoxyphenyl)-*N*-(6-(4-methylphenyl)thiazolo[4,5-b]pyrazin-2-yl)pyridine-4-carboxamide (compound 6)**

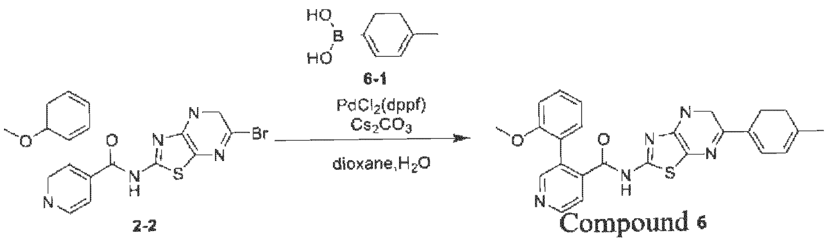


2-2 Compound 6

At 25°C, **intermediate 6-1** (27.67 mg) and **intermediate 2-2** (30 mg) were dissolved in a solution of dioxane (1.5 mL) and water (0.3 mL), and then caesium carbonate (44.20 mg) and 1,1'-bis(diphenylphosphino)ferrocene palladium dichloride (II) (2.48 mg) were added. Under nitrogen protection, the reaction solution was reacted at 100°C for 3 hours. After the reaction was completed, to the reaction solution were added ethyl acetate (20 mL) and water (60 mL), and the mixture was washed with water (20 mL*3). The organic phase was dried over anhydrous sodium sulphate. After filtration, the organic phase was concentrated to dryness under reduced pressure, and the residue was purified by high performance liquid chromatography (chromatographic column: Boston Green ODS (150*30 mm*5 um); mobile phase: A: water (0.05% formic acid), B: acetonitrile; gradient elution, B%: 52%-82%, 12 minutes) to obtain the title compound (4.3 mg).

**$^1$H NMR (400 MHz,DMSO-$d_6$):** δ = 9.16 (s, 1H), 8.75 (d, *J* = 4.8 Hz, 1H), 8.64 (s, 1H), 8.07 (d, *J* = 8.0 Hz, 2H), 7.74 (d, *J* = 4.9 Hz, 1H), 7.44-7.34 (m, 4H), 7.09 (t, *J*= 7.3 Hz, 1H), 7.00 (d, *J*= 8.0 Hz, 1H), 3.53 (s, 3H), 2.39 (s, 3H)

MS m/z (ESI): = 454.2 [M+H]+.

**Example 7, 3-(2-methoxyphenyl)-*N*-(6-(4-methoxyphenyl)thiazolo[4,5-b]pyrazin-2-yl)pyridine-4-carboxamide (compound 7)**

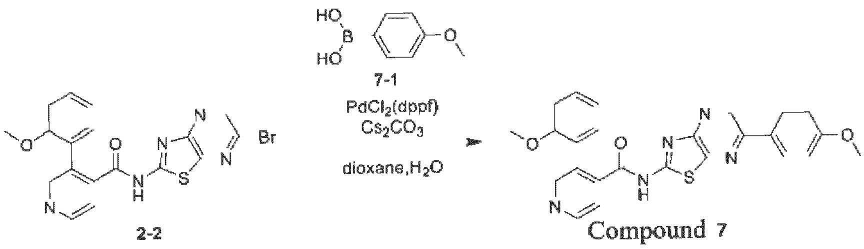


2-2 Compound 7

At 25°C, **intermediate 7-1** (30.92 mg) and **intermediate 2-2** (30 mg) were dissolved in a solution of dioxane (1.5 mL) and water (0.3 mL), and then caesium carbonate (44.20 mg) and 1,1'-bis(diphenylphosphino)ferrocene palladium dichloride (II) (2.48 mg) were added. Under nitrogen protection, the reaction solution was reacted at 100°C for 3 hours. After the reaction was completed, to the reaction solution were added ethyl acetate (20 mL) and water (60 mL), and the mixture was washed with water (20 mL*3). The organic phase was dried over anhydrous sodium sulphate. After filtration, the organic phase was concentrated to dryness under reduced pressure, and the residue was purified by high performance

liquid chromatography (chromatographic column: Boston Green ODS (150*30 mm*5 um); mobile phase: A: water (0.05% formic acid); B: acetonitrile; gradient elution, B%: 50%-80%, 12 minutes) to obtain the title compound (4.8 mg).

**$^1$H NMR (400 MHz, DMSO-$d_6$)** δ = 13.38 (s, 1H), 9.19 (s, 1H), 8.84-8.74 (m, 1H), 8.68 (s, 1H), 8.14 (d, *J* = 8.9 Hz, 2H), 7.73 (d, *J* = 4.8 Hz, 1H), 7.53-7.33 (m, 2H), 7.15-7.08 (m, 3H), 7.03-6.95 (m, 1H), 3.85 (s, 3H), 3.53 (s, 3H).

MS m/z (ESI): = 470.2 [M+H]$^+$.

**Example 8: *N*-(5-(4-chlorophenyl)thiazolo[5,4-*d*]pyrimidin-2-yl)-3-(2-methoxyphenyl)pyridine-4-carboxamide (compound 8)**

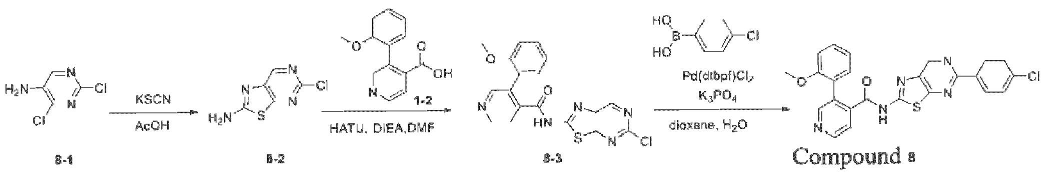


8-1 8-2 8-3 Compound 8

**Step 1: Synthesis of 5-chlorothiazolo[5,4-*d*]pyrimidin-2-amine (intermediate 8-2)**

**Intermediate 8-1** (5.0 g) was added to anhydrous acetic acid (40 mL). At 0°C, to the reaction solution was added potassium thiocyanate (6 g). The reaction solution was stirred at 80°C for 12 hours. After the reaction was completed, the reaction was cooled to room temperature, and the reaction solution was poured into water. The mixture was filtered, and the filter cake was dried to obtain the title compound (5 g).

MS m/z (ESI): 187.1 [M+H]$^+$.

**Step 2: Synthesis of *N*-(5-chlorothiazolo[5,4-*d*]pyrimidin-2-yl)-3-(2-methoxyphenyl)pyridine-4-carboxamide (intermediate 8-3)**

**Intermediate 8-2** (170 mg), **intermediate 1-2** (208 mg), HATU (345.8 mg) and DIEA (236 mg) were added to DMF (2 mL), and the reaction solution was stirred at 25°C under nitrogen protection for 12 hours. After the reaction was completed, the reaction was cooled to room temperature, and water (50 mL) and ethyl acetate (100 mL) were successively added. The organic phase was washed with water (50 mL *2), and dried over anhydrous sodium sulphate. After filtration, the filtrate was concentrated under reduced pressure, and the residue was purified by preparative high performance liquid chromatography (chromatographic column: Gemini NX C18 (3.5 μm*4.6*100 mm); mobile phase: A: water (0.05% TFA), B: acetonitrile; gradient elution, B%: 10%-90%, 10 min) to obtain the title compound (210 mg).

MS m/z (ESI): 398.2 [M+H]$^+$.

**Step 3: Synthesis of *N*-(5-(4-chlorophenyl)thiazolo[5,4-*d*]pyrimidin-2-yl)-3-(2-methoxyphenyl)pyridine-4-carboxamide (compound 8)**

**Intermediate 8-3** (100 mg), p-chlorophenylboronic acid (78 mg), Pd(dtbpf)$Cl_2$ (16.35 mg) and potassium phosphate (105 mg) were added to dioxane (1 mL) and water (0.2 mL), and the reaction solution was stirred at 100°C under nitrogen protection for 12 hours. After the reaction was completed, the reaction was cooled to room temperature, and water (50 mL) and ethyl acetate (100 mL) were successively added. The organic phase was washed with water (50 mL *2), and dried over anhydrous sodium sulphate. After filtration, the filtrate was concentrated under reduced pressure, and the residue was purified by preparative high performance liquid chromatography (chromatographic column: Gemini NX C18 (3.5 μm*4.6*100 mm); mobile phase: A: water (0.05% TFA), B: acetonitrile; gradient elution, B%: 10%-90%, 10 min) to obtain the title compound (11 mg).

MS m/z (ESI): 474.1 [M+H]$^+$.

**$^1$H NMR (400 MHz, DMSO-$d_6$)** δ 13.33 (s, 1H), 9.17 (s, 1H), 8.73 (d, *J* = 5.0 Hz, 1H), 8.61 (s, 1H), 8.47-8.40 (m, 2H), 7.72 (d, *J* = 5.0 Hz, 1H), 7.63-7.56 (m, 2H), 7.42-7.33 (m, 2H), 7.12-7.03 (m, 1H), 6.99 (d, *J*= 8.1 Hz, 1H), 3.52 (s, 3H).

**Example 9, *N*-(6-(4-cyanophenyl)thiazolo[4,5-b]pyrazin-2-yl)-3-(2-methoxyphenyl)pyridine-4-carboxamide (compound 9)**

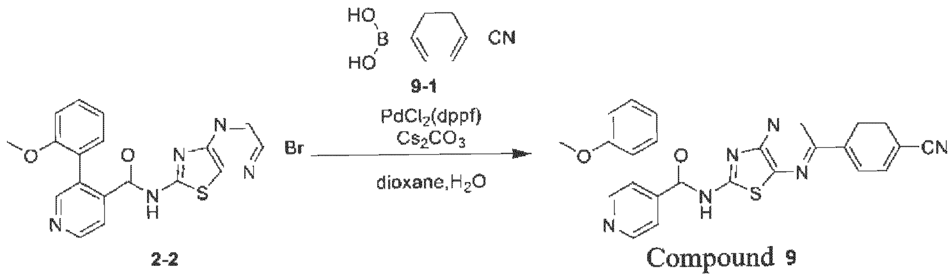


At 100°C, **intermediate 2-2** (50 mg) was dissolved in a mixed solution of dioxane and water (dioxane : water = 10 : 1, 0.55 ml), and then to the reaction solution were slowly added **intermediate 9-1** (21.59 mg), caesium carbonate (73.67 mg) and 1,1'-bis(diphenylphosphino)ferrocene palladium dichloride (4.14 mg). Under nitrogen protection, the reaction solution was stirred at 100°C for 15 minutes. After the reaction was completed, to the reaction solution were added ethyl acetate (9 mL) and water (18 mL), and the mixture was extracted with ethyl acetate (9 mL*3). The organic phases were combined, and dried over anhydrous sodium sulphate. After filtration, the organic phase was concentrated to dryness under reduced pressure. The residue was purified by high performance liquid chromatography (chromatographic column: Boston Green ODS (150*30 mm*5 um); mobile phase: A: water (0.05% ammonia water), B: acetonitrile; gradient elution, B%: 20%-40%, 11 minutes) to obtain the title compound (13 mg).

**$^1$H NMR (400MHz, Methanol-*d*$_4$)** $\delta$ = 9.00-8.97 (m, 1H), 8.62 (d, *J*= 5.0 Hz, 1H), 8.55 (s, 1H), 8.34-8.29 (m, 2H), 7.88 (d, *J*= 8.5 Hz, 2H), 7.81 (d, *J* = 5.0 Hz, 1H), 7.41-7.35 (m, 2H), 7.10-7.04 (m, 1H), 6.98 (d, *J*= 8.3 Hz, 1H), 3.64 (s, 3H)

MS m/z (ESI): = 465.1 [M+H]$^+$.

**Example 10, *N*-(6-(4-chlorophenyl)thiazolo[4,5-*b*]pyridin-2-yl)-3-(2-methoxyphenyl)isonicotinamide (compound 10)**

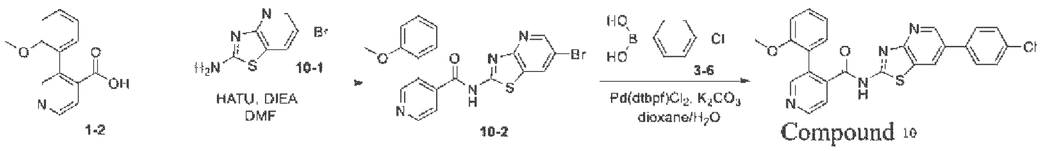


**Step 1: Synthesis of *N*-(6-bromothiazolo[4,5-*b*]pyridin-2-yl)-3-(2-methoxyphenyl)isonicotinamide (intermediate 10-2)**

**Intermediate 1-2** (200 mg), **intermediate 10-1** (200 mg), HATU (328 mg) and DIEA (225 mg) were dissolved in DMF (4 mL), and the reaction solution was stirred and reacted at 25°C for 12 h. After the reaction was completed, the reaction solution was poured into water (20 mL), and the mixture was extracted with ethyl acetate (15 mL*3). The organic layers were combined, washed with water (15 mL * 2), and dried over anhydrous sodium sulphate. After filtration, the filtrate was concentrated under reduced pressure to remove the solvent, and the residue was purified by preparative thin-layer chromatography (dichloromethane : methanol = 15 : 1) to obtain the title compound (160 mg).

MS m/z (ESI): 441.0/443.0 [M+H]$^+$.

**Step 2: Synthesis of *N*-(6-(4-chlorophenyl)thiazolo[4,5-*b*]pyridin-2-yl)-3-(2-methoxyphenyl)isonicotinamide (compound 10)**

**Intermediate 10-2** (80 mg) and **intermediate 3-6** (42 mg) were dissolved in dioxane (1.2 mL) and water (0.3 mL), and to the mixture were added potassium carbonate (50 mg) and Pd(dtbpf)$Cl_2$ (17 mg). The reaction solution was reacted at 80°C under nitrogen protection for 4 h. After the reaction was completed, the reaction was cooled to room temperature. The reaction solution was poured into water (15 mL), and the mixture was extracted with ethyl acetate (10 mL * 3). The organic layers were combined, washed with water (10 mL*2), and dried over anhydrous sodium sulphate. After filtration, the filtrate was concentrated under reduced pressure to remove the solvent, and the residue was purified by preparative high performance liquid chromatography (column: Gemini NX C18 3.5 $\mu$m*4.6*100 mm; mobile phase: A: **water** (0.05% TFA), B: acetonitrile; B%: 10%-90%, 10 min) to obtain the title compound (28 mg).

MS m/z (ESI): 473.0 [M+H]$^+$.

**$^1$H NMR (400 MHz, DMSO-*d*$_6$)** $\delta$ 13.24 (br, s, 1H), 8.88 (d, *J*= 2.3 Hz, 1H), 8.80-8.73 (m, 2H), 8.65 (s, 1H), 7.81 (d,

*J* = 8.6 Hz, 2H), 7.72 (d, *J* = 4.9 Hz, 1H), 7.58 (d, *J*= 8.6 Hz, 2H), 7.47-7.33 (m, 2H), 7.12-7.08 (m, 1H), 6.99 (d, *J*= 8.1 Hz, 1H), 3.50 (s, 3H).

**Example 11, *N*-(6-(6-cyanopyridin-3-yl)thiazolo[4,5-b]pyrazin-2-yl)-3-(2-methoxyphenyl)isonicotinamide (compound 11)**

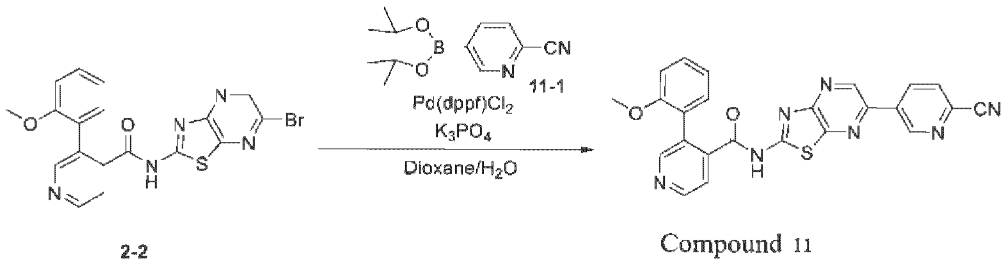


2-2

Compound 11

Under nitrogen atmosphere, **intermediate 2-2** (50 mg), **intermediate 11-1** (52.02 mg), Pd(dppf)$Cl_2$ (8.27 mg), and potassium phosphate (47.98 mg) were dissolved in dioxane (1 mL) and water (0.2 mL), and the mixture was stirred and reacted at 100°C for 3 h. The reaction solution was diluted by adding water (5 mL), and extracted with ethyl acetate (5 mL*2). The organic phase was concentrated to dryness under reduced pressure, and the residue was purified by preparative high performance liquid chromatography (column: Gemini NX C18 5 μm*10*150 mm; mobile phase: A: water (0.225% formic acid), B: acetonitrile; B%: 30%-50%, 11 min) to obtain the title compound (5.5 mg).
MS m/z (ESI): 466.1 $[M+H]^+$.
**$^1$H NMR (400 MHz,** DMSO-*d*$_6$) δ 13.59 (s, 1H), 9.61-9.49 (m, 1H), 9.43 (s, 1H), 8.84-8.74 (m, 2H), 8.67 (s, 1H), 8.23 (d, *J* = 8.2 Hz, 1H), 7.75 (d, *J*= 5.0 Hz, 1H), 7.48-7.35 (m, 2H), 7.11 (t, *J*= 7.5 Hz, 1H), 7.00 (d, *J*= 8.4 Hz, 1H), 3.52 (s, 3H).

**Example 12: *N*-(6-(4-cyano-3-fluorophenyl)thiazolo[4,5-b]pyrazin-2-yl)-3-(2-methoxyphenyl)isonicotinamide (compound 12)**

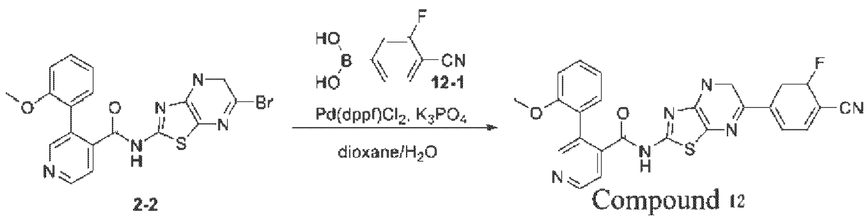


2-2

Compound 12

**Intermediate 2-2** (30 mg), **intermediate 12-1** (22.37 mg), Pd(dppf)$Cl_2$ (4.96 mg), and potassium phosphate (28.80 mg) were added to dioxane (1 mL) and water (0.2 mL), and the reaction solution was stirred at 80°C for 3 h. The reaction solution was poured into water (5 mL), and the mixture was extracted with ethyl acetate (5 mL*3). The organic layers were combined, washed with saturated sodium chloride aqueous solution (10 mL*2), dried over anhydrous sodium sulphate, and filtered. The filtrate was concentrated to dryness under reduced pressure. The residue was purified by preparative high performance liquid chromatography (column: Gemini NX C18 3.5 μm*4.6*100 mm; mobile phase: A: **water** (0.05% TFA), B: acetonitrile; B%: 10%-90%, 8 min) to obtain the title compound (13 mg).
MS m/z (ESI): 483.0 $[M+H]^+$.
**$^1$H NMR (400 MHz, DMSO-*d*$_6$)** δ 13.59 (br, s, 1H), 9.36 (s, 1H), 8.86-8.68 (m, 1H), 8.64 (s, 1H), 8.36-8.28 (m, 1H), 8.28-8.21 (m, 1H), 8.15-8.06 (m, 1H), 7.78-7.72 (m, 1H), 7.45-7.35 (m, 2H), 7.14-7.06 (m, 1H), 7.03-6.96 (m, 1H), 3.53 (s, 3H).

**Example 13, *N*-(6-(4-cyano-2-fluorophenyl)thiazolo[4,5-b]pyrazin-2-yl)-3-(2-methoxyphenyl)isonicotinamide (compound 13)**

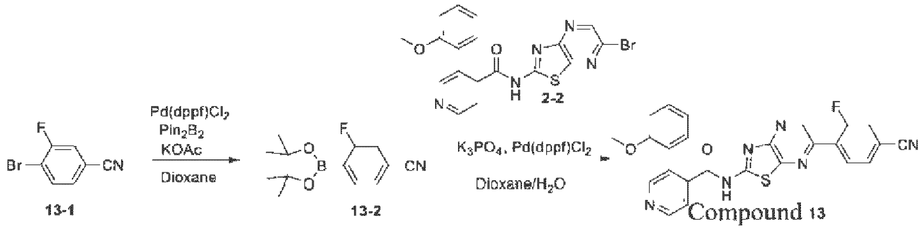


**Step 1: Synthesis of 3-fluoro-4-(4,4,5,5-tetramethyl-1,3,2-dioxaborolan-2-yl)benzonitrile (intermediate 13-2)**

Under nitrogen atmosphere, **starting material 13-1** (1.0 g) was dissolved in dioxane (10 mL), and potassium acetate (981 mg), bis(pinacolato)diboron (1.52 g) and [1,1'-bis(diphenylphosphine)ferrocene]palladium dichloride (362 mg) were added. After the addition, the mixture was warmed to 100°C and stirred for 2 hours. The reaction solution was filtered, and the filtrate was concentrated to dryness under reduced pressure. The residue was purified by column chromatography (PE/EA=10/1) to obtain the title compound (1.0 g).

MS m/z (ESI): 248.1 $[M+H]^+$.

**Step 2: Synthesis of *N*-(6-(4-cyano-2-fluorophenyl)thiazolo[4,5-b]pyrazin-2-yl)-3-(2-methoxyphenyl)isonicotinamide (compound 13)**

Under nitrogen atmosphere, **intermediate 13-2** (80 mg) was dissolved in dioxane (1 mL), and **intermediate 2-2** (54 mg), potassium phosphate (77 mg), water (0.2 mL) and [1,1'-bis(diphenylphosphine)ferrocene]palladium dichloride (13 mg) were added. After the addition, the mixture was warmed to 100°C and stirred for 2 h. The reaction solution was concentrated to dryness under reduced pressure, and the residue was purified by preparative high-pressure liquid chromatography [YMC-Actus Triart C18 column, 5 um silica, 30 mm in diameter, 150 mm in length; using a decreasingly polar mixture of water (containing 0.05% $NH_4HCO_3$) and acetonitrile as the eluent; acetonitrile gradient proportion: 55%-80%, elution time: 13 minutes] to obtain the title compound (7.71 mg).

MS m/z (ESI): 483.1 $[M+H]^+$.

**$^1$H NMR (400 MHz, DMSO-*d*$_6$)** δ 13.60 (s, 1H), 9.09 (s, 1H), 8.78 (d, *J* = 5.0 Hz, 1H), 8.67 (s, 1H), 8.22-8.18 (m, 1H), 8.12 (d, *J*= 11.0 Hz, 1H), 7.89 (d, *J* = 8.6 Hz, 1H), 7.75 (d, *J*= 5.0 Hz, 1H), 7.61-7.35 (m, 2H), 7.13-7.09 (m, 1H), 7.00 (d, *J* = 8.2 Hz, 1H), 3.52 (s, 3H).

**Example 14: *N*-(6-(4-cyano-3-methoxyphenyl)thiazolo[4,5-b]pyrazin-2-yl)-3-(2-methoxyphenyl)isonicotinamide (compound 14)**

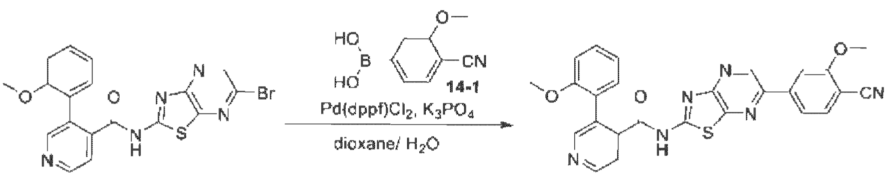


2-2

Compound 14

**Intermediate 2-2** (40 mg), **intermediate 14-1** (32 mg), Pd(dppf)$Cl_2$ (6.60 mg), and potassium phosphate (38.40 mg) were added to dioxane (1 mL) and water (0.2 mL), and the mixture was stirred and reacted at 80°C for 3 h. The reaction solution was poured into water (10 mL), and the mixture was extracted with ethyl acetate (8 mL*3). The organic layers were combined, washed with saturated sodium chloride aqueous solution (10 mL*2), dried over anhydrous sodium sulphate, and filtered to remove the desiccant. The filtrate was concentrated to dryness under reduced pressure. The residue was purified by preparative high performance liquid chromatography (column: Gemini NX C18 3.5 μm*4.6*100 mm; mobile phase: A: water (0.05% TFA), B: acetonitrile; B%: 20%-90%, 10 min) to obtain the title compound (8 mg).

MS m/z (ESI): 495.1 $[M+H]^+$.

**$^1$H NMR (400 MHz, DMSO-*d*$_6$)** δ 13.54 (br, s, 1H), 9.32 (s, 1H), 8.73 (d, *J*= 4.9 Hz, 1H), 8.62 (s, 1H), 7.94 (s, 1H), 7.89 (s, 2H), 7.78-7.71 (m, 1H), 7.46-7.33 (m, 2H), 7.13-7.04 (m, 1H), 7.03-6.96 (m, 1H), 4.06 (s, 3H), 3.53 (s, 3H).

**Example 15: *N*-(6-(4-cyano-2-methoxyphenyl)thiazolo[4,5-b]pyrazin-2-yl)-3-(2-methoxyphenyl)isonicotinamide (compound 15)**

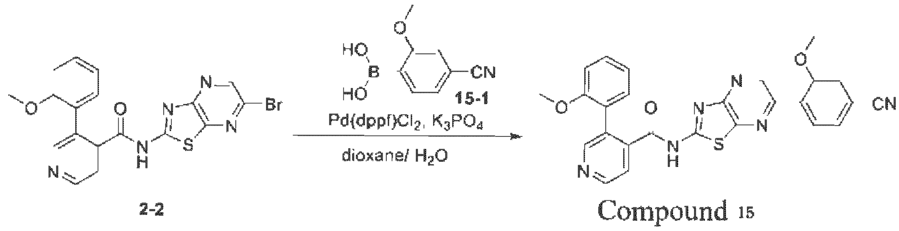


2-2

Compound 15

**Intermediate 2-2** (30 mg), **intermediate 15-1** (24 mg), Pd(dppf)$Cl_2$ (4.96 mg), and potassium phosphate (28.80 mg) were added to dioxane (1 mL) and water (0.2 mL), and the mixture was stirred and reacted at 80°C for 3 h. The reaction solution was poured into water (10 mL), and the mixture was extracted with ethyl acetate (5 mL*3). The organic layers were combined, washed with saturated sodium chloride aqueous solution (10 mL * 2), dried over anhydrous sodium sulphate, and filtered. The filtrate was concentrated to dryness under reduced pressure. The residue was purified by preparative high performance liquid chromatography (column: Gemini NX C18 3.5 μm*4.6*100 mm; mobile phase: A: 0.05% TFA v/v, B: acetonitrile; B%: 10%-90%, 8 min) to obtain the title compound (8 mg).

MS m/z (ESI): 495.1 $[M+H]^+$.

**$^1$H NMR (400 MHz, DMSO-*d*$_6$)** δ 13.51 (br, s, 1H), 9.08 (s, 1H), 8.77-8.71 (m, 1H), 8.63 (s, 1H), 8.02-7.95 (m, 1H), 7.77-7.69 (m, 2H), 7.62-7.54 (m, 1H), 7.44-7.34 (m, 2H), 7.13-7.04 (m, 1H), 7.03-6.96 (m, 1H), 3.96 (s, 3H), 3.52 (s, 3H).

**Example 16: *N*-(6-(4-cyanophenyl)thiazolo[4,5-b]pyrazin-2-yl)-4-(2-methoxyphenyl)nicotinamide (compound 16)**

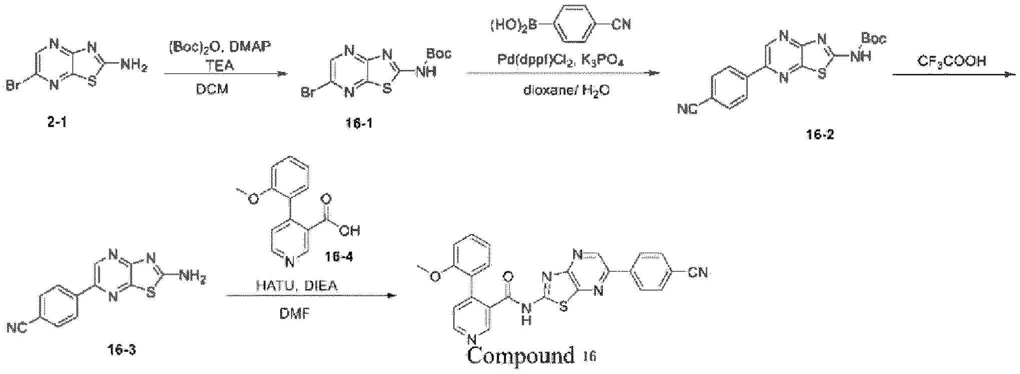


2-1 16-1 16-2 16-3 Compound 16

**Step 1: Synthesis of tert-butyl (6-bromothiazolo[4,5-b]pyrazin-2-yl)carbamate (intermediate 16-1)**

**Starting material 2-1** (790 mg), triethylamine (691.90 mg), and DMAP (41.77 mg) were dissolved in dichloromethane (10 mL), and to the reaction solution was dropwise added $(Boc)_2O$ (820.78 mg). The mixture was stirred at room temperature for 16 h. Subsequently, the reaction solution was washed with saturated sodium chloride aqueous solution, and the organic phase was dried over anhydrous sodium sulphate, and filtered. The filtrate was concentrated under reduced pressure to obtain the title compound (1 g).

MS m/z (ESI): 331.2/333.2 $[M+H]^+$.

**Step 2: Synthesis of tert-butyl (6-(4-cyanophenyl)thiazolo[4,5-b]pyrazin-2-yl)carbamate (intermediate 16-2)**

**Intermediate 16-1** (1 g), 4-cyanophenylboronic acid (887.34 mg), Pd(dppf)$Cl_2$ (220.93 mg), and potassium phosphate (1.28 g) were added to dioxane (10 mL) and water (2 mL), and the mixture was stirred at 80°C for 4 h. The reaction solution was poured into water, and the mixture was extracted with ethyl acetate (10 mL*3). The organic phase was dried over anhydrous sodium sulphate, and filtered. The filtrate was concentrated to dryness under reduced pressure,

and the residue was slurried with ethyl acetate, and filtered. The filter cake was dried to obtain the title compound (0.5 g).

MS m/z (ESI): 354.0 $[M+H]^+$.

**Step 3: Synthesis of 4-(2-aminothiazolo[4,5-*b*]pyrazin-6-yl)benzonitrile (intermediate 16-3)**

**Intermediate 16-2** (0.48 g) was dissolved in trifluoroacetic acid (2 mL), and the mixture was stirred at room temperature for 1 h. To the reaction solution was added ethyl acetate, and the mixture was filtered. The filtrate was concentrated to dryness under reduced pressure to obtain the title compound (230 mg).

MS m/z (ESI): 254.0 $[M+H]^+$.

**Step 4: Synthesis of *N*-(6-(4-cyanophenyl)thiazolo[4,5-b]pyrazin-2-yl)-4-(2-methoxyphenyl)nicotinamide (compound 16)**

**Intermediate 16-4** (30 mg), HATU (52 mg), and DIEA (36 mg) were added to DMF (1 mL), and the mixture was stirred at room temperature for 1 h. **Intermediate 16-3** (70.00 mg) was then added, and the resulting mixture was stirred at room temperature for 16 h. The reaction solution was poured into water (10 mL), and the mixture was extracted with ethyl acetate (5 mL * 3). The organic layers were combined, washed with saturated sodium chloride aqueous solution (10 mL * 2), dried over anhydrous sodium sulphate, and filtered. The filtrate was concentrated to dryness under reduced pressure. The residue was purified by preparative high performance liquid chromatography (column: Gemini NX C18 3.5 μm*4.6*100 mm; mobile phase: A: **water** (0.05% TFA), B: acetonitrile; B%: 10%-90%, 8 min) to obtain the title compound (5 mg).

MS m/z (ESI): 465.0 $[M+H]^+$.

**$^1$H NMR (400 MHz, DMSO-*d*$_6$)** δ 13.49 (brs, 1H), 9.32 (s, 1H), 8.92 (s, 1H), 8.78 (s, 1H), 8.41-8.34 (m, 1H), 8.04-7.97 (m, 1H), 7.49-7.37 (m, 3H), 7.21 (s, 1H), 7.16-7.06 (m, 1H), 7.04-6.97 (m, 1H), 6.67 (s, 1H), 3.53 (s, 3H).

**Example 17: *N*-(6-(4-chlorophenyl)thiazolo[4,5-b]pyrazin-2-yl)-6-cyano-4-(2-fluoro-6-methoxyphenyl)nicotinamide (compound 17)**

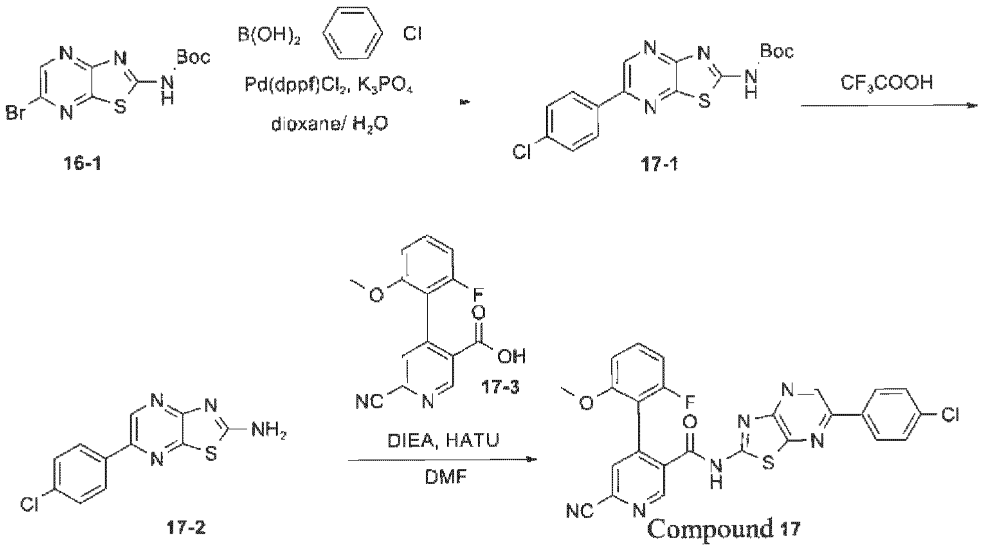


**Step 1: Synthesis of tert-butyl (6-(4-chlorophenyl)thiazolo[4,5-b]pyrazin-2-yl)carbamate (intermediate 17-1)**

**Intermediate 16-1** (1 g), 4-chlorophenylboronic acid (887.34 mg), Pd(dppf)$Cl_2$ (220.93 mg), and potassium phosphate (1.28 g) were added to dioxane (10 mL) and water (2 mL), and the mixture was stirred at 80°C for 4 h. The reaction solution was poured into water, and the mixture was extracted with ethyl acetate (10 mL*3), and washed with saturated sodium chloride aqueous solution (10 mL*3). The organic phases were combined, dried over anhydrous sodium sulphate, and filtered. The filtrate was concentrated to dryness under reduced pressure, and the residue was slurried with ethyl acetate, and filtered. The filter cake was dried to obtain the title compound (0.5 g).

MS m/z (ESI): 363.0 $[M+H]^+$.

**Step 2: Synthesis of 6-(4-chlorophenyl)thiazolo[4,5-*b*]pyrazin-2-amine (intermediate 17-2)**

**Intermediate 17-1** (0.48 g) was dissolved in trifluoroacetic acid (2 mL), and the mixture was stirred at room temperature for 1 h. To the reaction solution was added ethyl acetate, and the mixture was filtered. The filtrate was concentrated to dryness under reduced pressure to obtain the title compound (230 mg).
MS m/z (ESI): 263.0 $[M+H]^+$.

**Step 3: Synthesis of *N*-(6-(4-chlorophenyl)thiazolo[4,5-b]pyrazin-2-yl)-6-cyano-4-(2-fluoro-6-methoxyphenyl) nicotinamide (compound 17)**

**Intermediate 17-3** (30 mg), HATU (62.21 mg), and DIEA (28.35 mg) were added to DMF (1 mL), and the mixture was reacted at room temperature for 1 h. **Intermediate 17-2** (28.95 mg) was then added, and the resulting mixture was reacted for additional 2 h. The reaction solution was poured into water (10 mL), and the mixture was extracted with ethyl acetate (5 mL * 3). The organic layers were combined, washed with saturated sodium chloride aqueous solution (10 mL* 2), dried over anhydrous sodium sulphate, and filtered. The filtrate was concentrated to dryness under reduced pressure. The residue was purified by preparative high performance liquid chromatography (column: Gemini NX C18 3.5 μm*4.6*100 mm; mobile phase: A: **water** (0.05% TFA), B: acetonitrile; B%: 10%-90%, 8 min) to obtain the title compound (15 mg).
MS m/z (ESI): 514.90$[M-H]^-$.
**$^1$H NMR (400 MHz, DMSO-*d*$_6$)** $^1$H NMR (400 MHz, DMSO-*d*$_6$) δ 13.78 (brs, 1H), 9.27 (s, 1H), 9.20 (s, 1H), 8.29 (s, 1H), 8.21 (d, *J* = 8.6 Hz, 2H), 7.62 (d, *J* = 8.5 Hz, 2H), 7.50 (q, *J* = 8.5 Hz, 1H), 7.01 (t, *J* = 9.1 Hz, 1H), 6.95 (d, *J* = 8.5 Hz, 1H), 3.62 (s, 3H).

**Example 18: N-(6-(4-chlorophenyl)thiazolo[4,5-b]pyrazin-2-yl)-6-cyano-4-(2-(difluoromethoxy)phenyl)nicotinamide (compound 18)**

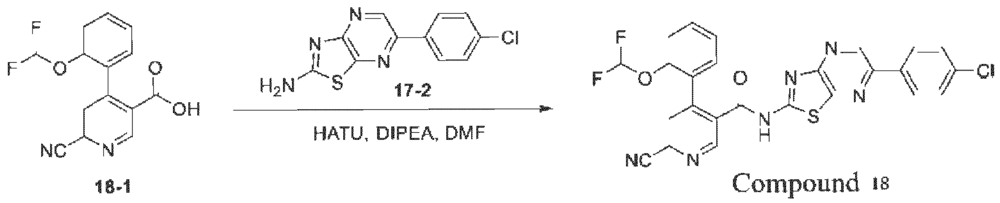

18-1
Compound 18

**Intermediate 18-1** (30 mg, which was synthesized according to the method reported in International Publication No. WO 2020243459, using the raw material 2-(2-(difluoromethoxy)phenyl)-4,4,5,5-tetramethyl-1,3,2-dioxaborane instead of 2-(2-(difluoromethoxy)-5-fluorophenyl)-4,4,5,5-tetramethyl-1,3,2-dioxaboran) was dissolved in *N*,*N*-dimethylformamide (3 mL), and **intermediate 17-2** (27 mg), *N,N*-diisopropylethylamine (26 mg) and HATU (39.28 mg) were successively added. The reaction solution was stirred and reacted at 30°C for 2 hours. The reaction solution was concentrated to dryness under reduced pressure, and the residue was purified by column chromatography (dichloromethane : methanol =20 : 1) to obtain the title compound (3 mg).
MS m/z (ESI): 535.0 $[M+H]^+$;
**$^1$H NMR (400 MHz, DMSO-*d*$_6$)** δ 13.76 (brs, 1H), 9.22 (s, 1H), 9.11 (s, 1H), 8.20 (s, 1H), 8.18-8.08 (m, 2H), 7.58-7.47 (m, 4H), 7.37-7.30 (m, 1H), 7.19-7.14 (m, 1H), 7.25-6.86 (m, 1H).

**Example 19, 6-cyano-4-(2-methoxyphenyl)-N-(6-(1-methyl-6-oxo-1,6-dihydropyridin-3-yl)thiazolo[4,5-b]pyrazin-2-yl)nicotinamide (compound 19)**

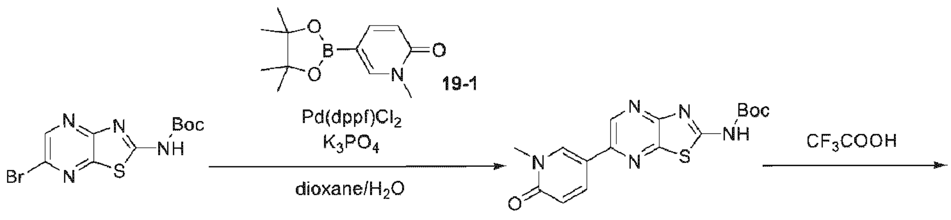


16-1 19-2

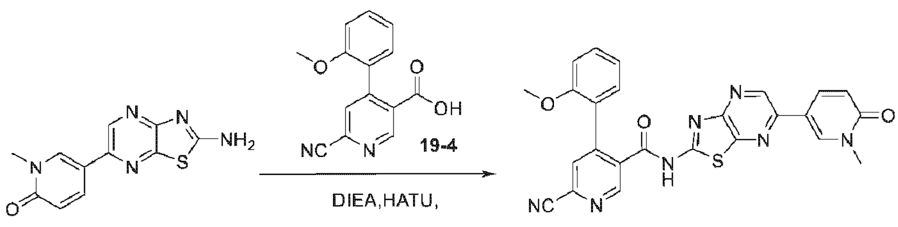


19-3 Compound 19

**Step 1: Synthesis of tert-butyl (6-(1-methyl-6-oxo-1,6-dihydropyridin-3-yl)thiazolo[4,5-b]pyrazin-2-yl)carbamate (intermediate 19-2)**

Under nitrogen atmosphere, **intermediate 16-1** (150 mg), Pd(dppf)$Cl_2$ (33.11 mg), potassium phosphate (192.04 mg), and **intermediate 19-1** (159.71 mg) were dissolved in dioxane (5 mL) and water (1 mL), and the mixture was stirred at 100°C for 3 h. The reaction solution was diluted by adding water (5 mL), and extracted with ethyl acetate (10 mL*3). The organic phases were combined, dried over anhydrous sodium sulphate, and filtered. The filtrate was concentrated to dryness under reduced pressure. The residue was purified by preparative thin-layer chromatography (DCM : $CH_3OH$=10 : 1) to obtain the title compound (140 mg).
MS m/z (ESI): 360.1 $[M+H]^+$.

**Step 2: Synthesis of 5-(2-aminothiazolo[4,5-*b*]pyrazin-6-yl)-1-methylpyridine-2(1*H*)-one (intermediate 19-3)**

**Intermediate 19-2** (140 mg) was dissolved in trifluoroacetic acid (2 mL), and the mixture was stirred at room temperature for 2 h. The reaction solution was concentrated to dryness to obtain the title compound (120.0 mg).
MS m/z (ESI): 260.1 $[M+H]^+$.

**Step 3: Synthesis of 6-cyano-4-(2-methoxyphenyl)-*N*-(6-(1-methyl-6-oxo-1,6-dihydropyridin-3-yl)thiazolo[4,5-b]pyrazin-2-yl)nicotinamide (compound 19)**

To a solution of **intermediate 19-4** (10 mg) in DMF (1 mL) were added DIEA (10.17 mg) and HATU (17.81 mg), and the mixture was stirred at room temperature for 30 min. **Intermediate 19-3** (12.24 mg) was then added, and the resulting mixture was stirred at room temperature for 8 h. The reaction solution was purified by preparative high performance liquid chromatography (column: Gemini NX C18 5 μm*10*150 mm; mobile phase: A: water (0.225% formic acid v/v), B: acetonitrile; B%: 30%-50%, 11 min) to obtain the title compound (2.0 mg).
MS m/z (ESI): 496.1 $[M+H]^+$.
**$^1$H NMR (400 MHz, Methanol-*$d_4$*)** δ 8.88 (d, *J* = 3.0 Hz, 2H), 8.49 (d, *J* = 2.6 Hz, 1H), 8.24-8.21 (m, 1H), 7.92 (s, 1H), 7.37 (t, *J* = 7.8 Hz, 2H), 7.09-7.02 (m, 1H), 6.90 (d, *J*= 8.1 Hz, 1H), 6.59 (d, *J*= 9.5 Hz, 1H), 3.60 (s, 3H), 3.53 (s, 3H).

**Example 20, 2'-chloro-*N*-(6-(4-cyanophenyl)thiazolo[4,5-b]pyrazin-2-yl)-5'-methoxy-6-methyl-[4,4'-bipyridine]-3-carboxamide (compound 20)**

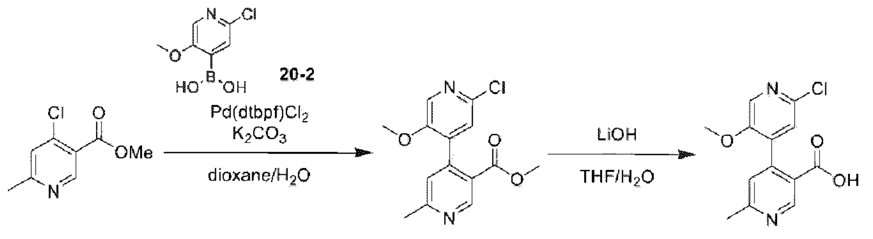


20-1 20-3 20-4

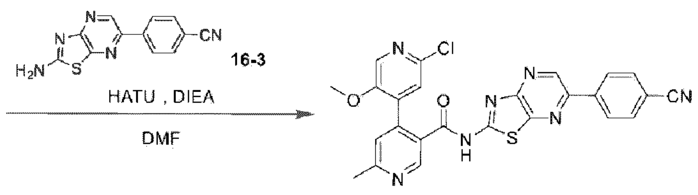


Compound 20

**Step 1: Synthesis of methyl 2'-chloro-5'-methoxy-6-methyl-[4,4'-bipyridine]-3-carboxylate (intermediate 20-3)**

At 20°C, **intermediate 20-1** (3.96 g) was dissolved in a solution of dioxane (40 mL)/water (8 mL), and to the mixture were added **intermediate 20-2** (4 g), potassium carbonate (5.90 g) and 1,1-bis(tert-butylphosphine)ferrocene palladium chloride (Pd(dtbpf)$Cl_2$) (1.39 g). Under nitrogen protection, the reaction solution was stirred and reacted at 80°C for 1 hour. After the reaction was completed, to the reaction solution was added water (100 mL), and the mixture was extracted with ethyl acetate (50 mL*3). The organic phases were combined, and dried over anhydrous sodium sulphate. After filtration, the organic phase was concentrated under reduced pressure to remove the solvent. The residue was purified by silica gel column chromatography (petroleum ether/ethyl acetate =1/1) to obtain the title compound (3.2 g).

MS m/z (ESI): = 292.9 $[M+H]^+$.

**Step 2: Synthesis of 2'-chloro-5'-methoxy-6-methyl-[4,4'-bipyridine]-3-carboxylic acid (intermediate 20-4)**

At 20°C, **intermediate 20-3** (190 mg) was dissolved in a solution of tetrahydrofuran (2 mL)/water (2 mL), and to the reaction solution was added lithium hydroxide (31.09 mg). The reaction solution was stirred and reacted at 20°C for 2 hours. After the reaction was completed, the reaction solution was adjusted to pH 4 with dilute hydrochloric acid, and water (5 mL) was added. The mixture was extracted with ethyl acetate (5 mL*3). The organic phases were combined, and concentrated under reduced pressure to remove the solvent, to obtain the title compound (180 mg).

MS m/z (ESI): = 278.9 $[M+H]^+$.

**Step 3: Synthesis of 2'-chloro-*N*-(6-(4-cyanophenyl)thiazolo[4,5-b]pyrazin-2-yl)-5'-methoxy-6-methyl-[4,4'-bipyridine]-3-carboxamide (compound 20)**

At 20°C, **intermediate 20-4** (180 mg) was dissolved in *N,N*-dimethylformamide (3 mL), and to the reaction solution were added **intermediate 16-3** (163.59 mg), HATU (248.58 mg) and *N,N*-diisopropylethylamine (166.95 mg). The reaction solution was stirred and reacted at 20°C for 1 hour. After the reaction was completed, the reaction solution was purified by high performance liquid chromatography (chromatographic column: Boston Prime C18 150*30 mm*5 um; mobile phase: A: water (0.05% ammonia water), B: acetonitrile; B%: 23%-43%, 11 minutes) to obtain the title compound (90 mg).

**$^1$H NMR (400 MHz, DMSO-$d_6$)** $\delta$ = 13.58 (s, 1H), 9.35 (s, 1H), 8.91 (s, 1H), 8.38 (d, *J* = 8.5 Hz, 2H), 8.19 (s, 1H), 8.02 (d, *J* = 8.5 Hz, 2H), 7.62 (s, 1H), 7.49 (s, 1H), 3.64 (s, 3H), 2.62 (s, 3H)

MS m/z (ESI): = 514.0 $[M+H]^+$.

**Example 21, *N*-(6-(4-cyanophenyl)thiazolo[4,5-b]pyrazin-2-yl)-5'-methoxy-2',6-dimethyl-[4,4'-bipyridine]-3-carboxamide (compound 21)**

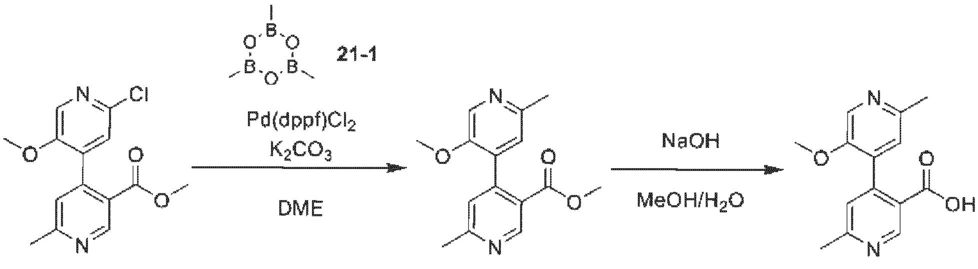


20-3 21-2 21-3

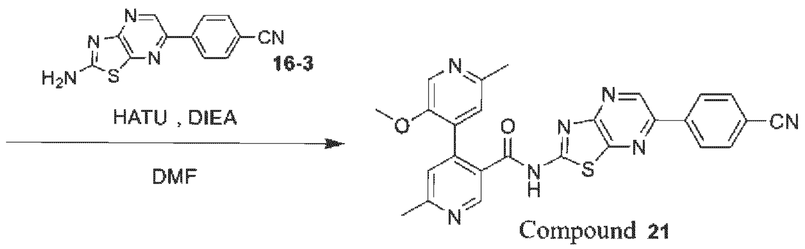


Compound 21

**Step** 1: **Synthesis of methyl 5'-methoxy-2',6-dimethyl-[4,4'-bipyridine]-3-carboxylate (intermediate 21-2)**

**Intermediate 20-3** (250 mg) and **intermediate 21-1** (321.64 mg) were dissolved in 1,2-dimethoxyethane (2 mL), and to the mixture was added potassium carbonate (295.10 mg). To the reaction solution was then added Pd(dppf)$Cl_2$ (62.49 mg). The reaction solution was stirred at 115°C under nitrogen atmosphere for 2 h. After the reaction was completed, the reaction solution was filtered, and the filtrate was concentrated under reduced pressure to remove the solvent. The residue was purified by flash silica gel column chromatography (ISCO®; 12 g SepaFlash® flash silica gel column, gradient: 0-40% petroleum ether/ethyl acetate, flow rate: 50 mL/min) to obtain the title compound (200.0 mg).

MS m/z (ESI): 273.0 $[M+H]^+$.

**Step 2: Synthesis of 5'-methoxy-2',6-dimethyl-[4,4'-bipyridine]-3-carboxylic acid (intermediate 21-3)**

**Intermediate 21-2** (200 mg) was dissolved in anhydrous methanol (4 mL), and to the mixture were added sodium hydroxide (88.13 mg) and water (1 mL). The reaction solution was stirred at 25°C for 2 h. After the reaction was completed, the reaction solution was concentrated under reduced pressure, and the residue was adjusted to pH 3 with an appropriate amount of hydrochloric acid. The reaction solution was concentrated to dryness under reduced pressure, and the residue was stirred for 10 min in the presence of dichloromethane : methanol (10: 1, 10 mL), and filtered. The filtrate was concentrated under reduced pressure to remove the solvent, to obtain the title compound (120.0 mg).

MS m/z (ESI): 259.0 $[M+H]^+$.

**Step 3: Synthesis of N-(6-(4-cyanophenyl)thiazolo[4,5-b]pyrazin-2-yl)-5'-methoxy-2',6-dimethyl-[4,4'-bipyridine]-3-carboxamide (compound 21)**

**Intermediate 21-3** (50.00 mg) was dissolved in anhydrous *N,N*-dimethylformamide (1 mL), and to the mixture were added HATU (77.29 mg) and *N,N*-diisopropylethylamine (75.06 mg). The reaction solution was stirred at 25°C under nitrogen atmosphere for 30 min. **Intermediate 16-3** (53.94 mg) was then added, and the reaction solution was stirred at 25°C under nitrogen atmosphere for 2 h. After the reaction was completed, the reaction solution was filtered, and the filtrate was purified by preparative high performance liquid chromatography (Boston Green ODS C18 column, 5 um silica, 30 mm in diameter, 150 mm in length; using a decreasingly polar mixture of water (containing 0.225% formic acid) and acetonitrile as the eluent; acetonitrile gradient proportion: 15%-45%, elution time: 12 minutes) to obtain the title compound (12 mg).

MS m/z (ESI): 494.0 $[M+H]^+$.

**$^1$H NMR (400MHz, DMSO-$d_6$)** $\delta$ = 13.56 (brs, 1H), 9.37 (s, 1H), 8.88 (s, 1H), 8.39 (d, *J*= 8.4 Hz, 2H), 8.24 (s, 1H), 8.02 (d, *J*= 8.4 Hz, 2H), 7.45 (s, 1H), 7.43 (s, 1H), 3.62 (s, 3H), 2.63 (s, 3H), 2.54 (s, 3H)

**Example 22, 4-(5-cyano-2-methoxyphenyl)-*N*-(6-(5-cyclopropylpyridin-2-yl)thiazolo[4,5-b]pyrazin-2-yl)-6-methylnicotinamide (compound 22)**

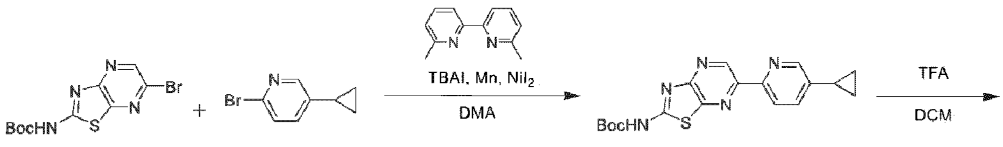


16-1 22-1

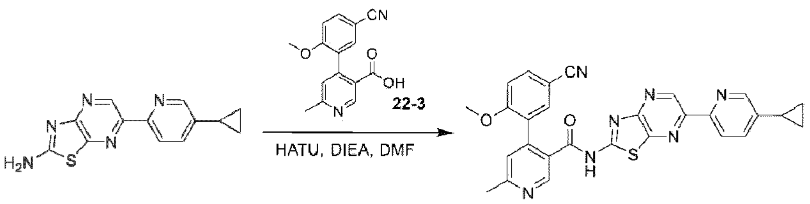


22-2 Compound 22

**Step 1: Synthesis of tert-butyl (6-(5-cyclopropylpyridin-2-yl)thiazolo[4,5-b]pyrazin-2-yl)carbamate (intermediate 22-1)**

**Intermediate 16-1** (500 mg), 2-bromo-5-cyclopropylpyridine (299.01 mg), 6,6'-dimethyl-2,2'-bipyridine (27.81 mg), tetrabutylammonium iodide (836.46 mg), manganese powder (331.76 mg), and nickel iodide (47.18 mg) were dissolved in N,N-dimethylacetamide (20 mL). Under nitrogen atmosphere, the reaction solution was stirred and reacted at 90°C for 16 h. After the reaction was completed, the reaction solution was filtered, and to the filtrate was added water (60 mL). The mixture was extracted with ethyl acetate (30 mL*2), and the organic phases were combined, and dried over anhydrous sodium sulphate. After filtration, the organic phase was concentrated under reduced pressure to remove the solvent. The residue was purified by flash silica gel column chromatography (ISCO®; 8 g SepaFlash® flash silica gel column, gradient: 0-34% ethyl acetate/petroleum ether, flow rate: 20 mL/min) to obtain the title compound (140 mg).
MS m/z (ESI): = 370.2 $[M+H]^+$.

**Step 2: Synthesis of 6-(5-cyclopropylpyridin-2-yl)thiazolo[4,5-*b*]pyrazin-2-amine (intermediate 22-2)**

**Intermediate 22-1** (180 mg) was dissolved in dichloromethane (2 mL) and trifluoroacetic acid (2 mL), and the reaction solution was stirred and reacted at 20°C for 4 hours. After the reaction was completed, the reaction solution was concentrated to dryness under reduced pressure to obtain the title compound (80 mg).
MS m/z (ESI): = 270.0 $[M+H]^+$.

**Step 3: Synthesis of 4-(5-cyano-2-methoxyphenyl)-*N*-(6-(5-cyclopropylpyridin-2-yl)thiazolo[4,5-b]pyrazin-2-yl)-6-methylnicotinamide (compound 22)**

**Intermediate 22-3** (48.99 mg, which was synthesized according to the method reported in International Publication No. WO 2020243459) and HATU (69.43 mg) were dissolved in *N,N*-dimethylformamide (2 mL). Under nitrogen atmosphere, the reaction solution was stirred and reacted at 40°C for 30 minutes, and then **intermediate 22-2** (70 mg) and *N,N*-diisopropylethylamine (47.20 mg) were added. The reaction solution was stirred and reacted at 40°C for 16 hours. After the reaction was completed, the reaction solution was purified by high performance liquid chromatography (chromatographic column: Boston Green ODS 150*30 mm*5 um; mobile phase: A: water (0.225% formic acid), B: acetonitrile; B%: 40%-70%, 14 minutes) to obtain the title compound (5 mg).
**$^1$H NMR (400 MHz, DMSO-$d_6$)** $\delta$ = 13.44 (brs, 1H), 9.51 (s, 1H), 8.83 (s, 1H), 8.56 (s, 1H), 8.25 (d, *J* = 8.0 Hz, 1H), 7.96-7.88 (m, 2H), 7.62 (d, J=8.4 Hz, 1H), 7.46 (s, 1H), 7.19 (d, *J*= 9.4 Hz, 1H), 3.61 (s, 3H), 2.61 (s, 3H), 2.11-2.02 (m, 1H), 1.14-1.05 (m, 2H), 0.90-0.80 (m, 2H)
MS m/z (ESI): = 520.2 $[M+H]^+$

**Example 23, 4-(5-chloro-2-methoxyphenyl)-N-[6-(4-cyanophenyl)thiazolo[4,5-b]pyrazin-2-yl]-6-methylnicotinamide (compound 23)**

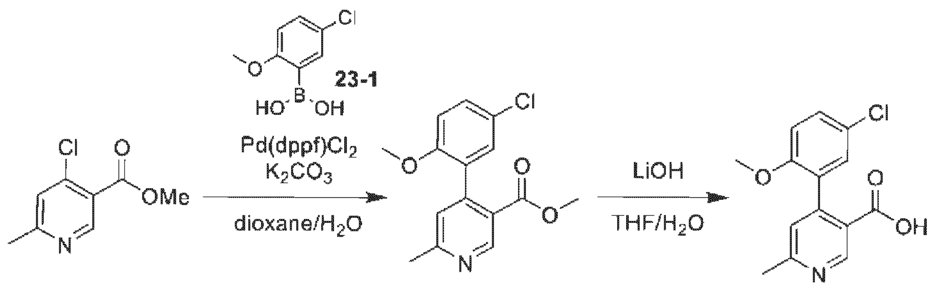


20-1 23-2 23-3

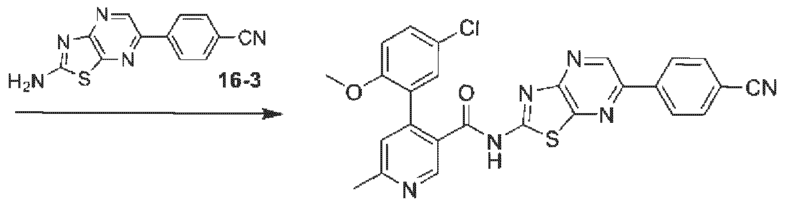


Compound 23

**Step 1: Synthesis of methyl 4-(5-chloro-2-methoxyphenyl)-6-methylpyridine-3-carboxylate (intermediate 23-2)**

Under nitrogen atmosphere, **intermediate 23-1** (1 g) was dissolved in dioxane (20 mL) and water (5 mL), and to the reaction solution were added **intermediate 20-1** (995.75 mg), Pd(dppf)$Cl_2$ (349.65 mg) and potassium carbonate (1.48 g). Subsequently, the reaction solution was stirred at 90°C under nitrogen atmosphere for 2 hours. After the reaction was completed, to the reaction solution was added water (50 mL), and the mixture was extracted with ethyl acetate (50 mL*3). The organic phases were combined, and dried over anhydrous sodium sulphate. After filtration, the organic phase was concentrated under reduced pressure to remove the solvent, and the residue was purified by silica gel column chromatography (ISCO®; 20 g SepaFlash® Silica Flash chromatographic column, gradient: 0-50% ethyl acetate/petroleum ether, flow rate: 20 mL/minute) to obtain the title compound (870 mg).

MS m/z (ESI): 292.1 $[M+H]^+$.

**Step 2: Synthesis of 4-(5-chloro-2-methoxyphenyl)-6-methylpyridine-3-carboxylic acid (intermediate 23-3)**

**Intermediate 23-2** (870 mg) was added to tetrahydrofuran (8 mL) and water (4 mL), and to the reaction solution was added lithium hydroxide (157.13 mg). Subsequently, the reaction solution was stirred at 25°C for 16 hours. After the reaction was completed, the reaction solution was adjusted to pH 3, and concentrated under reduced pressure to remove the solvent. The residue was washed with a mixed solvent of dichloromethane/methanol (10/1, 20 mL), and filtered. The filtrate was then concentrated to dryness under reduced pressure to obtain the title compound (1.2 g).

MS m/z (ESI): 277.9 $[M+H]^+$.

**Step 3: Synthesis of 4-(5-chloro-2-methoxyphenyl)-*N*-[6-(4-cyanophenyl)thiazolo[4,5-b]pyrazin-2-yl]-6-methylnicotinamide (compound 23)**

Under nitrogen atmosphere, **intermediate 23-3** (300 mg) was added to *N,N*-dimethylformamide (5 mL), and to the reaction solution were added **intermediate 16-3** (273.62 mg), HATU (410.76 mg) and *N,N*-diisopropylethylamine (279.24 mg). Subsequently, the reaction solution was stirred at 25°C under nitrogen atmosphere for 2 hours. After the reaction was completed, the reaction solution was purified by preparative high performance liquid chromatography

(chromatographic column: Boston Prime C18 150*30 mm*5 um; mobile phase: [A: water (0.225% formic acid), B: acetonitrile]; B%: 48%-68%, 11 minutes) to obtain the title compound (51.96 mg).

MS m/z (ESI): 513.1 $[M+H]^+$.

**$^1$H NMR (400MHz, DMSO-*d*$_6$)** δ 13.40 (brs, 1H), 9.27 (s, 1H), 8.84 (s, 1H), 8.36 (d, *J* = 8.4 Hz, 2H), 8.00 (d, *J* = 8.3 Hz, 2H), 7.50-7.42 (m, 2H), 7.36 (s, 1H), 7.03-6.99 (m, 1H), 3.52 (s, 3H), 2.59 (s, 3H)

**Example 24, *N*-(6-(5-cyclopropylpyridin-2-yl)thiazolo [4,5-b] pyrazin-2-yl)-5'-methoxy-2',6-dimethyl-[4,4'-bipyridine]-3-carboxamide (compound 24)**

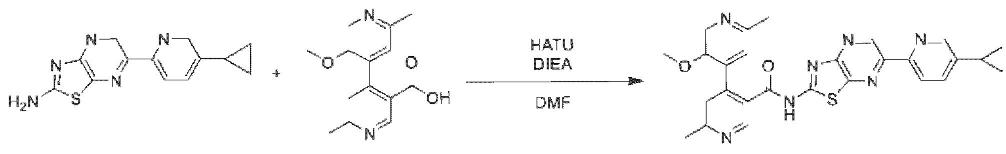


22-2 21-3 Compound 24

At 20°C, **intermediate 22-2** (200 mg) was dissolved in *N,N*-dimethylformamide (3 mL), and to the mixture were added **intermediate 21-3** (230.15 mg), HATU (338.83 mg) and *N,N*-diisopropylethylamine (191.95 mg). The reaction solution was stirred and reacted at 40°C for 6 hours. After the reaction was completed, the reaction solution was purified by high performance liquid chromatography (chromatographic column: Boston Prime C18 150*30 mm*5 um; mobile phase: A: water (0.05% ammonia water), B: acetonitrile; B%: 20%-40%, 11 minutes) to obtain the title compound (39.9 mg).

**$^1$H NMR (400 MHz, DMSO-*d*$_6$)** δ = 13.54 (brs, 1H), 9.48 (s, 1H), 8.85 (s, 1H), 8.55 (s, 1H), 8.29-8.11 (m, 2H), 7.69-7.55 (m, 1H), 7.49-7.23 (m, 2H), 3.59 (s, 3H), 2.61 (s, 3H), 2.16-1.99 (m, 1H), 1.09-0.99 (m, 2H), 0.89-0.79 (m, 2H)

MS m/z (ESI): = 510.2 $[M+H]^+$.

**Example 25, *N*-(6-(5-(difluoromethoxy)pyridin-2-yl)thiazolo[4,5-b]pyrazin-2-yl)-5'-methoxy-2',6-dimethyl-[4,4'-bipyridine]-3-carboxamide (compound 25)**

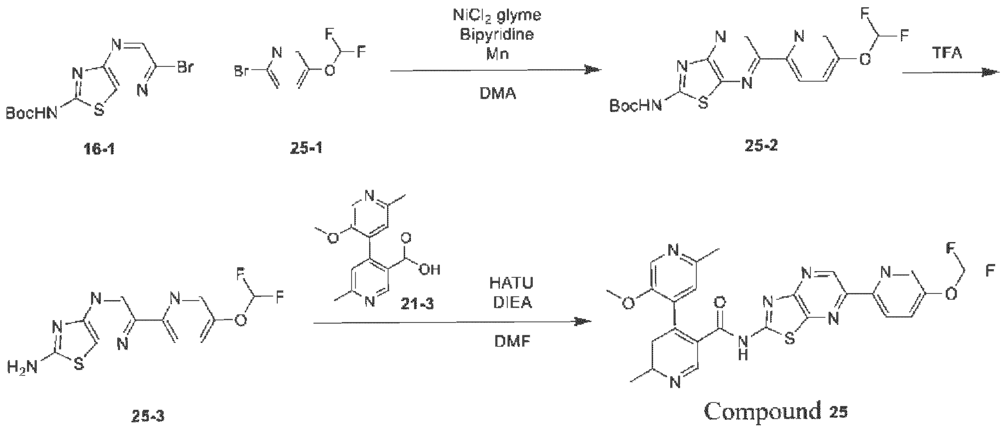


16-1 25-1 25-2

25-3 Compound 25

**Step 1: Synthesis of tert-butyl (6-(5-(difluoromethoxy)pyridin-2-yl)thiazolo[4,5-b]pyrazin-2-yl)carbamate (intermediate 25-2)**

At 20°C, **intermediate 16-1** (1.33 g) was dissolved in *N,N*-dimethylacetamide (10 mL), and to the reaction solution were added **intermediate 25-1** (900 mg), nickel (II) chloride ethylene glycol dimethyl ether complex ($NiCl_2$ glyme) (176.56 mg), 2,2'-bipyridine (62.75 mg) and manganese powder (220.73 mg). Under nitrogen atmosphere, the reaction solution was stirred and reacted at 90°C for 4 hours. After the reaction was completed, to the reaction solution were added dichloromethane (100 mL) and methanol (10 mL). After filtration, the filtrate was concentrated under reduced pressure to remove the solvent. To the residue was added water (100 mL), and the mixture was filtered. The filter cake was collected, dried, and then purified by silica gel column chromatography (dichloromethane/methanol = 15/1) to obtain the title compound (140 mg).

MS m/z (ESI): = 396.3 $[M+H]^+$.

**Step 2: Synthesis of 6-(5-(difluoromethoxy)pyridin-2-yl)thiazolo[4,5-b]pyrazin-2-amine (intermediate 25-3)**

At 20°C, **intermediate 25-2** (140 mg) was dissolved in dichloromethane (3 mL), and to the reaction solution was added trifluoroacetic acid (262.17 μL). The reaction solution was stirred and reacted at 20°C for 2 hours. After the reaction was completed, the reaction solution was concentrated under reduced pressure to remove the solvent. Subsequently, to the residue was added a saturated sodium bicarbonate solution, and the mixture was adjusted to pH 7, and filtered to obtain the title compound (60 mg).

MS m/z (ESI): = 296.1 $[M+H]^+$.

**Step 3: Synthesis of *N*-(6-(5-(difluoromethoxy)pyridin-2-yl)thiazolo[4,5-b]pyrazin-2-yl)-5'-methoxy-2',6-dimethyl-[4,4'-bipyridine]-3-carboxamide (compound 25)**

At 20°C, **intermediate 25-3** (100 mg) was dissolved in *N,N*-dimethylformamide (1 mL), and to the mixture were added **intermediate 21-3** (122.46 mg), HATU (180.28 mg) and *N,N*-diisopropylethylamine (61.28 mg). The reaction solution was stirred and reacted at 45°C for 6 hours. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 23%-43%, 11 minutes) to obtain the title compound (55 mg).

**$^1$H NMR (400 MHz, DMSO-*$d_6$*)** δ = 13.56 (brs, 1H), 9.50 (s, 1H), 8.85 (s, 1H), 8.65 (d, *J*= 2.8 Hz, 1H), 8.44 (d, *J*= 8.9 Hz, 1H), 8.21 (s, 1H), 7.86 (dd, *J*= 2.8, 8.7 Hz, 1H), 7.62-7.24 (m, 3H), 3.60 (s, 3H), 2.62 (s, 3H)

MS m/z (ESI): = 536.1 $[M+H]^+$.

**Example 26, 4-(5-chloro-2-methoxyphenyl)-N-(6-(5-cyanopyridin-2-yl)thiazolo[4,5-b]pyrazin-2-yl)-6-methylnicotinamide (compound 26)**

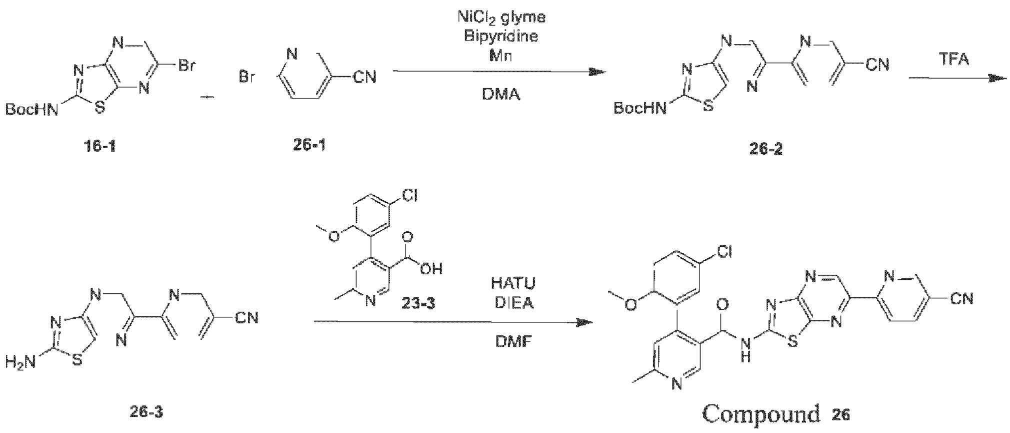


**Step 1: Synthesis of tert-butyl (6-(5-cyanopyridin-2-yl)thiazolo[4,5-b]pyrazin-2-yl)carbamate (intermediate 26-2)**

At 20°C, **intermediate 16-1** (3 g) was dissolved in *N,N*-dimethylacetamide (25 mL), and to the mixture were added **intermediate 26-1** (1.66 g), nickel (II) chloride ethylene glycol dimethyl ether complex (398.06 mg), 2,2'-bipyridine (141.48 mg) and manganese powder (497.64 mg). Under nitrogen atmosphere, the reaction solution was stirred and reacted at 90°C for 4 hours. After the reaction was completed, to the reaction solution were added dichloromethane (250 mL) and methanol (25 mL). After filtration, the organic phase was concentrated under reduced pressure to remove the solvent. To the residue was added water (250 mL), and the mixture was filtered. The residue was purified by silica gel column chromatography (dichloromethane/methanol = 15/1) to obtain the title compound (320 mg).

MS m/z (ESI): = 355.1 $[M+H]^+$.

**Step 2: Synthesis of 6-(2-aminothiazolo[4,5-b]pyrazin-6-yl)-3-cyanopyridine (intermediate 26-3)**

At 20°C, **intermediate 26-2** (320 mg) was dissolved in dichloromethane (5 mL), and to the reaction solution was

added trifluoroacetic acid (668.57 μL). The reaction solution was stirred and reacted at 20°C for 2 hours. After the reaction was completed, the reaction solution was concentrated under reduced pressure to remove the solvent. Subsequently, to the residue was added a saturated sodium bicarbonate solution, and the mixture was adjusted to pH 7, and filtered to obtain the title compound (150 mg).

MS m/z (ESI): = 255.1 $[M+H]^+$.

**Step 3: Synthesis of 4-(5-chloro-2-methoxyphenyl)-*N*-(6-(5-cyanopyridin-2-yl)thiazolo[4,5-b]pyrazin-2-yl)-6-methylnicotinamide (compound 26)**

At 20°C, **intermediate 26-3** (150 mg) was dissolved in *N,N*-dimethylformamide (2.5 mL), and to the reaction solution were added **intermediate 23-3** (327.65 mg), HATU (448.61 mg) and *N,N*-diisopropylethylamine (152.48 mg). The reaction solution was stirred and reacted at 20°C for 6 hours. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 45%-65%, 11 minutes) to obtain the title compound (53.3 mg).

**$^1$H NMR (400 MHz, DMSO-$d_6$)** δ = 13.56 (brs, 1H), 9.58 (s, 1H), 9.19 (s, 1H), 8.81 (s, 1H), 8.60-8.44 (m, 2H), 7.54-7.42 (m, 3H), 7.08-6.98 (m, 1H), 3.52 (s, 3H), 2.61 (s, 3H)

MS m/z (ESI): = 514.1 $[M+H]^+$.

**Example 27, 5'-methoxy-2',6-dimethyl-*N*-(6-(5-(trifluoromethyl)pyridin-2-yl)thiazolo[4,5-b]pyrazin-2-yl)-[4,4'-bipyridine]-3-carboxamide (compound 27)**

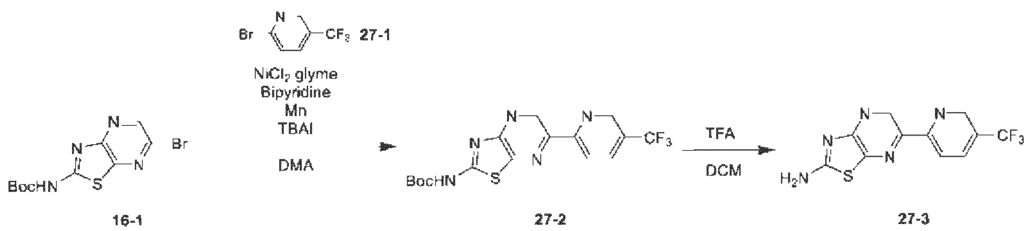


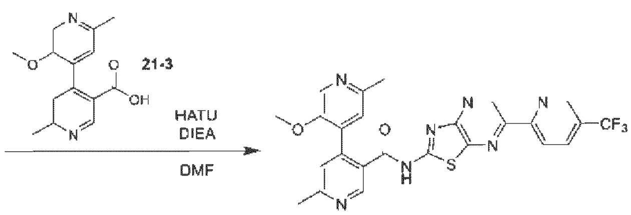


Compound 27

**Step 1: Synthesis of tert-butyl (6-(5-(trifluoromethyl)pyridin-2-yl)thiazolo[4,5-b]pyrazin-2-yl)carbamate (intermediate 27-2)**

At 25°C, **intermediate 16-1** (3 g) was dissolved in *N,N*-dimethylacetamide (40 mL), and to the mixture were added nickel (II) chloride ethylene glycol dimethyl ether complex (199.03 mg), manganese powder (1.99 g), 2,2'-bipyridine (141.48 mg), tetrabutylammonium iodide (5.02 g) and **intermediate 27-1** (2.05 g). The reaction solution was stirred and reacted at 90°C under nitrogen atmosphere for 6 hours. After the reaction was completed, the reaction solution was cooled to room temperature, and filtered. Subsequently, to the reaction solution were added ethyl acetate (50 mL) and water (100 mL), and the mixture was extracted twice with a saturated sodium chloride aqueous solution solution (100 mL). The organic phases were combined, and dried over anhydrous sodium sulphate. After filtration, the organic phase was concentrated under reduced pressure to remove the solvent. The residue was purified by silica gel column chromatography (mobile phase: 0-5% tetrahydrofuran/dichloromethane, flow rate: 60 mL/minute) to obtain the title compound (400 mg).

MS m/z (ESI): = 398.0 $[M+H]^+$.

**Step 2: Synthesis of 6-(5-(trifluoromethyl)pyridin-2-yl)thiazolo[4,5-*b*]pyrazin-2-amine (intermediate 27-3).**

At 20°C, **intermediate 27-2** (100 mg) was dissolved in dichloromethane (4 mL), and to the reaction solution was added trifluoroacetic acid (860.82 mg). Under nitrogen atmosphere, the reaction solution was stirred and reacted at 20°C for 0.5 hours. After the reaction was completed, the organic phase was concentrated under reduced pressure to remove the solvent. Subsequently, to the residue was added a saturated sodium bicarbonate solution, and the mixture was adjusted to pH 7, and filtered to obtain the title compound (100 mg).
MS m/z (ESI): = 298.0 $[M+H]^+$.

**Step 3: Synthesis of 5'-methoxy-2',6-dimethyl-*N*-(6-(5-(trifluoromethyl)pyridin-2-yl)thiazolo[4,5-b]pyrazin-2-yl)-[4,4'-bipyridine]-3-carboxamide (compound 27).**

At 20°C, **intermediate 21-3** (100 mg) was dissolved in *N,N*-dimethylformamide (1 mL), and to the reaction solution were added HATU (127.91 mg) and DIEA (86.96 mg). The mixture was stirred for 1 hour, and then **intermediate 27-3** (100 mg) was added. Under nitrogen atmosphere, the reaction solution was stirred and reacted at 20°C for 16 hours. After the reaction was completed, to the reaction solution were added ethyl acetate (5 mL) and water (10 mL), and the mixture was extracted twice with a saturated sodium chloride solution (10 mL). The organic phases were combined, and dried over anhydrous sodium sulphate. After filtration, the organic phase was concentrated under reduced pressure to remove the solvent. The residue was purified by high performance liquid chromatography (chromatographic column: Boston Prime C18 150*30 mm*5 um; mobile phase: A: water (0.05% ammonia water), B: acetonitrile; B%: 23%-43%, 11 minutes) to obtain the title compound (21.7 mg).
**$^1$H NMR (400MHz, DMSO-*d*$_6$)** $\delta$ = 9.56 (s, 1H), 9.12 (s, 1H), 8.87 (s, 1H), 8.56 (d, *J* = 8.6 Hz, 1H), 8.39 (d, *J* = 8.4 Hz, 1H), 8.19 (s, 1H), 7.41 (s, 1H), 7.32 (s, 1H), 3.59 (s, 3H), 2.61 (s, 3H).
MS m/z (ESI): = 538.1 $[M+H]^+$.

**Example 28, *N*-(6-(4-chlorophenyl)thiazolo[4,5-*b*]pyrazin-2-yl)-6-cyano-4-(2-methoxyphenyl)pyridine-3-carboxamide (compound 28)**

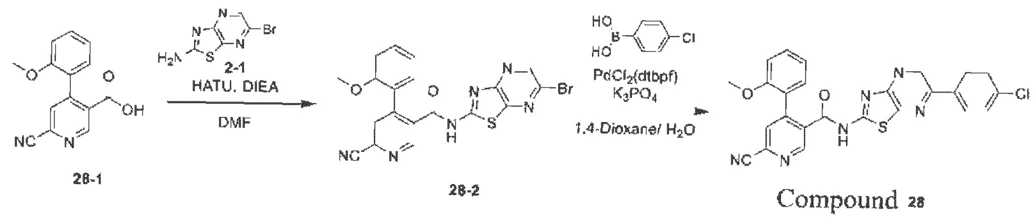

28-1
28-2
Compound 28

**Step** 1: **Synthesis of *N*-(6-bromothiazolo[4,5-*b*]pyrazin-2-yl)-6-cyano-4-(2-methoxyphenyl)pyridine-3-carboxamide (intermediate 28-2)**

**Intermediate 28-1** (150 mg, which could be synthesized according to the method reported in International Publication No. WO 2020243459), **intermediate 2-1** (119.97 mg), HATU (148.06 mg), and DIEA (67.10 mg) were dissolved in *N,N*-dimethylformamide (2 mL), and the reaction solution was stirred at 25°C under nitrogen atmosphere for 1 h. After the reaction was completed, the reaction solution was diluted by adding water (50 mL), and extracted with ethyl acetate (30 mL*2). The organic phases were combined, and dried over anhydrous sodium sulphate. After filtration, the organic phase was concentrated to dryness under reduced pressure, and the residue was purified by silica gel column chromatography (ISCO®; 20 g SepaFlash® flash silica gel column, gradient: 0-35% ethyl acetate/petroleum ether, flow rate: 30 mL/min) to obtain the title compound (75 mg).
MS m/z(ESI): = 467.1 $[M+H]^+$.

**Step 2: Synthesis of *N*-(6-(4-chlorophenyl)thiazolo[4,5-*b*]pyrazin-2-yl)-6-cyano-4-(2-methoxyphenyl)pyridine-3-carboxamide (compound 28)**

**Intermediate 28-2** (40 mg), (4-chlorophenyl)boronic acid (26.77 mg), 1,1'-bis(di-tert-butylphosphino)ferrocene palladium dichloride (5.58 mg), and potassium phosphate (36.34 mg) were dissolved in a mixed solvent of dioxane (0.5 mL) and water (0.1 mL), and the reaction solution was stirred at 60°C under nitrogen atmosphere for 30 min. After the reaction was completed, the reaction solution was cooled to room temperature, and slowly poured into water (20 ml). The

mixture was extracted with ethyl acetate (15 mL*2), and the organic phases were combined, and dried over anhydrous sodium sulphate. After filtration, the organic phase was concentrated to dryness under reduced pressure, and the residue was purified by preparative high performance liquid chromatography (chromatographic column: Boston Prime C18 150*30 mm*5 um; mobile phase: [A: water (0.225% formic acid), B: acetonitrile]; B%: 60%-80%, 11 min) to obtain the title compound (11 mg).

**$^1$H NMR (400 MHz, DMSO-*d*$_6$)** $\delta$ = 13.71-13.59(m, 1H), 9.28(s, 1H), 9.07(s, 1H), 8.24-8.19(m, 3H), 7.62(d, *J* = 8.7 Hz, 2H), 7.55-7.50(m, 1H), 7.50-7.45(m, 1H), 7.15(t, *J* = 7.5 Hz, 1H), 7.04(d, *J* = 8.3 Hz, 1H), 3.55(s, 3H)

MS m/z(ESI): = 499.0 [M+H]$^+$.

**Example 29, *N*-(6-(4-chlorophenyl)thiazolo[4,5-*b*]pyrazin-2-yl)-3-(2-ethynylphenyl)pyridine-4-carboxamide (compound 29)**

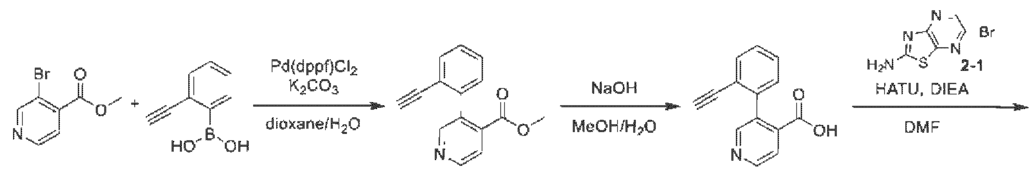


29-1 29-2 29-3 29-4

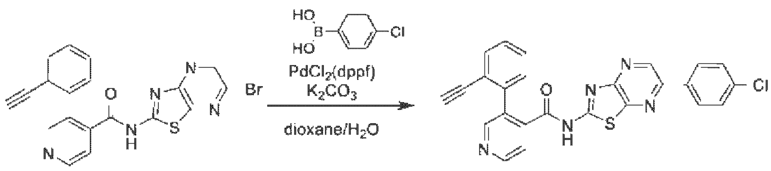


29-5 Compound 29

**Step 1: Synthesis of methyl 3-(2-ethynylphenyl)pyridine-4-carboxylate (intermediate 29-3)**

**Intermediate 29-1** (1.18 g), **intermediate 29-2** (800 mg), potassium carbonate (1.52 g) and 1,1'-bis(diphenylphosphino)ferrocene palladium dichloride (II) (401.07 mg) were dissolved in a solution of dioxane (20 mL)/water (4 mL). Under nitrogen atmosphere, the reaction solution was stirred and reacted at 70°C for 2 h. After the reaction was completed, the reaction solution was cooled to room temperature, diluted with water (20 mL), and extracted with ethyl acetate (15 ml*3). The organic phases were combined, and dried over anhydrous sodium sulphate. After filtration, the organic phase was concentrated to dryness under reduced pressure. The residue was purified by silica gel column chromatography (ISCO®; 40 g SepaFlash® flash silica gel column, gradient: 0-25% ethyl acetate/petroleum ether, flow rate: 50 mL/min) to obtain the title compound (536 mg).

MS m/z(ESI): = 238.1 [M+H]$^+$.

**Step 2: Synthesis of 3-(2-ethynylphenyl)pyridine-4-carboxylic acid (intermediate 29-4)**

**Intermediate 29-3** (350 mg) was dissolved in a solution of methanol (3 mL)/water (3 mL), and to the mixture was added sodium hydroxide (177.01 mg). The reaction solution was stirred and reacted at 50°C for 15 min. After the reaction was completed, the reaction solution was adjusted to pH 7 with dilute hydrochloric acid, and then concentrated to dryness under reduced pressure. The residue was dissolved in methanol (1 mL). After filtration, the filtrate was concentrated to dryness under reduced pressure to obtain the title compound (300 mg).

MS m/z(ESI): = 224.1 [M+H]$^+$.

**Step 3: Synthesis of *N*-(6-bromothiazolo[4,5-*b*]pyrazin-2-yl)-3-(2-ethynylphenyl)pyridine-4-carboxamide (intermediate 29-5)**

**Intermediate 29-4** (300 mg), **intermediate 2-1** (310.55 mg), HATU (562.10 mg) and DIEA (347.39 mg) were dissolved in *N,N*-dimethylformamide (8 mL). Under nitrogen atmosphere, the reaction solution was stirred and reacted at 25°C for 2 hours. After the reaction was completed, to the reaction solution was added water (100 mL), and the mixture was

extracted with ethyl acetate (45 mL*3). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: 0-50% ethyl acetate/petroleum ether, flow rate: 50 mL/min) to obtain the title compound (220 mg).

MS m/z(ESI): = 436.0 $[M+H]^+$.

**Step 4: Synthesis of N-(6-(4-chlorophenyl)thiazolo[4,5-b]pyrazin-2-yl)-3-(2-ethynylphenyl)pyridine-4-carboxamide (compound 29)**

**Intermediate 29-5** (100 mg), (4-chlorophenyl)boronic acid (71.68 mg), potassium carbonate (63.36 mg) and 1,1'-bis(diphenylphosphino)ferrocene palladium dichloride (II) (14.94 mg) were dissolved in a solution of dioxane (1 mL)/water (0.2 mL). Under nitrogen atmosphere, the reaction solution was stirred and reacted at 90°C for 1 h. After the reaction was completed, to the reaction solution was added water (50 mL), and the mixture was extracted with ethyl acetate (15 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 53%-73%, 11 minutes) to obtain the title compound (7.5 mg).

**$^1$H NMR(400 MHz, DMSO-*d*$_6$)** δ = 13.85-13.46(m, 1H), 9.27(s, 1H), 8.85(d, *J* = 5.0 Hz, 1H), 8.72(s, 1H), 8.21(d, *J* = 8.7 Hz, 2H), 7.87(d, *J* = 5.0 Hz, 1H), 7.64-7.57(m, 3H), 7.53-7.47(m, 1H), 7.46-7.40(m, 2H), 4.05(s, 1H)

MS m/z(ESI): = 468.1 $[M+H]^+$.

**Example 30, N-(6-(4-cyanophenyl)thiazolo[4,5-*b*]pyrazin-2-yl)-3-(2-ethynylphenyl)pyridine-4-carboxamide (compound 30)**

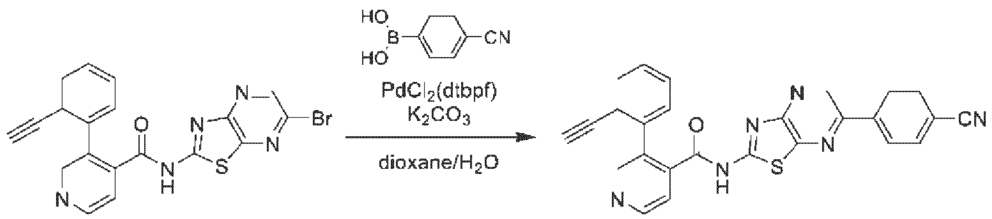


29-5

Compound 30

**Intermediate 29-5** (100 mg), (4-cyanophenyl)boronic acid (67.36 mg), 1,1'-bis(di-tert-butylphosphino)ferrocene palladium dichloride (14.94 mg), and potassium carbonate (63.36 mg) were dissolved in a mixed solvent of dioxane (5 mL) and water (1 mL), and the reaction solution was stirred and reacted at 90°C under nitrogen atmosphere for 1 h. After the reaction was completed, the reaction solution was cooled to room temperature, diluted with water (50 mL), and extracted with ethyl acetate (15 ml*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 25%-45%, 11 minutes) to obtain the title compound (8.5 mg).

**$^1$H NMR(400 MHz, DMSO-*d*$_6$)** δ = 13.84-13.60(m, 1H), 9.39(s, 1H), 8.86(d, *J* = 5.0 Hz, 1H), 8.72(s, 1H), 8.39(d, *J* = 8.5 Hz, 2H), 8.03(d, *J* = 8.5 Hz, 2H), 7.88(d, *J* = 5.1 Hz, 1H), 7.61-7.57(m, 1H), 7.53-7.49(m, 1H), 7.46-7.41(m, 2H), 4.05(s, 1H)

MS m/z(ESI): = 459.0 $[M+H]^+$.

**Example 31, *N*-(6-(4-cyanophenyl)thiazolo[4,5-*b*]pyrazin-2-yl)-4-(2-methoxyphenyl)-6-methylpyridine-3-carboxamide (compound 31)**

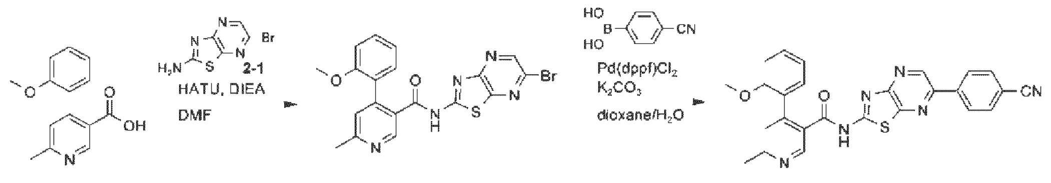


4-4 31-1 Compound 31

**Step 1: Synthesis of *N*-(6-bromothiazolo[4,5-*b*]pyrazin-2-yl)-4-(2-methoxyphenyl)-6-methylpyridine-3-carboxamide (intermediate 31-1)**

**Intermediate 4-4** (110 mg), HATU (189.13 mg), **intermediate 2-1** (104.49 mg) and DIEA (116.89 mg) were dissolved in *N,N*-dimethylformamide (2 mL), and the reaction solution was stirred and reacted at 25°C for 2 hours. After the reaction was completed, to the reaction solution was added water (50 mL), and the mixture was extracted with ethyl acetate (15 mL*3). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: 0-50% ethyl acetate/petroleum ether, flow rate: 30 mL/min) to obtain the title compound (100 mg).
MS m/z(ESI): = 456.3 $[M+H]^+$.

**Step 2: Synthesis of *N*-(6-(4-cyanophenyl)thiazolo[4,5-*b*]pyrazin-2-yl)-4-(2-methoxyphenyl)-6-methylpyridine-3-carboxamide (compound 31)**

**Intermediate 31-1** (90 mg), (4-cyanophenyl)boronic acid (69.55 mg), (diphenylphosphino)ferrocene palladium dichloride (II) (14.43 mg), and potassium carbonate (54.52 mg) were dissolved in a mixed solvent of dioxane (1 mL) and water (0.2 mL). Under nitrogen atmosphere, the reaction solution was stirred and reacted at 80°C for 15 minutes. After the reaction was completed, to the reaction solution was added water (50 mL), and the mixture was extracted with ethyl acetate (15 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 40%-60%, 11 minutes) to obtain the title compound (9.9 mg).
**$^1$H NMR(400 MHz, DMSO-*d*$_6$)** δ = 13.61-13.22(m, 1H), 9.42-9.22(m, 1H), 8.79(s, 1H), 8.38(d, *J* = 8.4 Hz, 2H), 8.01(d, *J* = 8.3 Hz, 2H), 7.46-7.37(m, 2H), 7.34(s, 1H), 7.11(t, *J* = 7.3 Hz, 1H), 7.03-6.94(m, 1H), 3.54-3.52(m, 3H), 2.60(s, 3H).
MS m/z(ESI): = 479.1 $[M+H]^+$

**Example 32, *N*-(6-(4-cyanophenyl)thiazolo[4,5-*b*]pyrazin-2-yl)-1-(2-methoxyphenyl)-1*H*-imidazole-5-carboxamide (compound 32)**

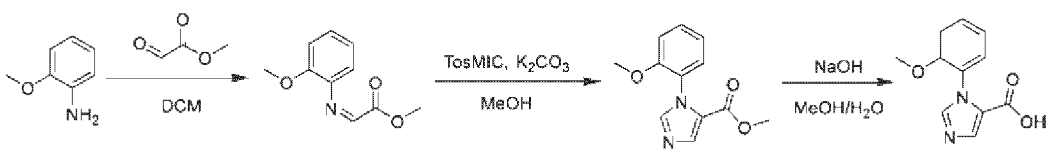


32-1 32-2 32-3 32-4

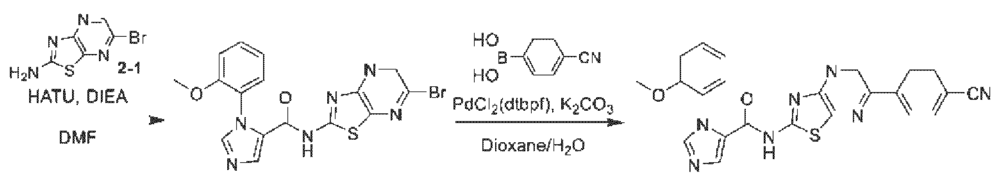


32-5 Compound 32

**Step 1: Synthesis of methyl (Z)-2-(2-methoxyphenyl)imino)acetate (intermediate 32-2)**

**Intermediate 32-1** (1 g) and methyl 2-oxoacetate (715.07 mg) were dissolved in dichloromethane (20 mL), and 4A molecular sieve (200 mg) was added. The reaction solution was stirred at 20°C for 16 h. After the reaction was completed, the reaction solution was filtered, and concentrated to dryness under reduced pressure to obtain the title compound (1.57 g).
MS m/z(ESI): = 194.1 $[M+H]^+$.

**Step 2: Synthesis of methyl 1-(2-methoxyphenyl)-1*H*-imidazole-5-carboxylate (intermediate 32-3)**

**Intermediate 32-2** (1.57 g) was dissolved in methanol (40 mL), and potassium carbonate (2.25 g) and 1-(isocyanomethylsulfonyl)-4-methylbenzene (TosMIC) (3.17 g) were added. The reaction solution was stirred at 20°C for 16 h. After the reaction was completed, the reaction solution was filtered, and concentrated to dryness under reduced pressure. The residue was purified by silica gel column chromatography (ISCO®; 20 g SepaFlash® flash silica gel column, gradient: 40%-50% ethyl acetate/petroleum ether, flow rate: 40 mL/min) to obtain the title compound (220 mg).
MS m/z(ESI): = 233.2 $[M+H]^+$.

**Step 3: Synthesis of 1-(2-methoxyphenyl)-1*H*-imidazole-5-carboxylic acid (intermediate 32-4)**

**Intermediate 32-3** (220 mg) was dissolved in a solution of methanol (2 mL)/water (2 mL), and to the mixture was added sodium hydroxide (113.67 mg). The reaction solution was stirred and reacted at 50°C for 2 hours. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (220 mg).
MS m/z(ESI): = 219.0 $[M+H]^+$.

**Step 4: Synthesis of *N*-(6-bromothiazolo[4,5-*b*]pyrazin-2-yl)-1-(2-methoxyphenyl)-1*H*-imidazole-5-carboxamide (intermediate 32-5)**

**Intermediate 32-4** (220 mg) and **intermediate 2-1** (232.97 mg) were dissolved in *N,N*-dimethylformamide (5 mL), and HATU (421.69 mg) and DIEA (260.61 mg) were added. The reaction solution was stirred and reacted at 20°C for 1 hour. After the reaction was completed, to the reaction solution were added ethyl acetate (30 mL) and water (20 mL), and the mixture was extracted with ethyl acetate (15 mL*3). The organic phases were combined, and dried over anhydrous sodium sulphate. After filtration, the organic phase was concentrated to dryness under reduced pressure. The residue was purified by silica gel column chromatography (ISCO®; 12 g SepaFlash® flash silica gel column, gradient: 40%-60% ethyl acetate/petroleum ether, flow rate: 30 mL/min) to obtain the title compound (320 mg).
MS m/z(ESI): = 431.0 $[M+H]^+$.

**Step 5: Synthesis of *N*-(6-(4-cyanophenyl)thiazolo[4,5-*b*]pyrazin-2-yl)-1-(2-methoxyphenyl)-1*H*-imidazole-5-carboxamide (compound 32)**

**Intermediate 32-5** (60 mg), (4-cyanophenyl)boronic acid (40.89 mg), 1,1'-bis(di-tert-butylphosphino)ferrocene palladium dichloride (9.07 mg), and potassium carbonate (38.46 mg) were dissolved in a solution of dioxane (1 mL)/water (0.2 mL). Under nitrogen atmosphere, the reaction solution was stirred and reacted at 90°C for 15 minutes. After the reaction was completed, to the reaction solution were added ethyl acetate (50 mL) and water (50 mL), and the mixture was extracted with ethyl acetate (15 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 40%-60%, 11 minutes) to obtain the title compound (3 mg).
**$^1$H NMR(400 MHz, DMSO-$d_6$)** $\delta$ = 13.43(s, 1H), 9.30(s, 1H), 8.38-8.34(m, 2H), 8.27-8.20(m, 1H), 8.09-8.04(m, 1H), 8.03-7.98(m, 2H), 7.53-7.48(m, 1H), 7.45-7.41(m, 1H), 7.26-7.21(m, 1H), 7.14-7.07(m, 1H), 3.71(s, 3H)
MS m/z(ESI): = 454.1 $[M+H]^+$

**Example 33, 6-cyano-4-(5-cyano-2-methoxyphenyl)-*N*-(6-(4-cyanophenyl)thiazolo[4,5-6]pyrazin-2-yl)pyridine-3-carboxamide (compound 33)**

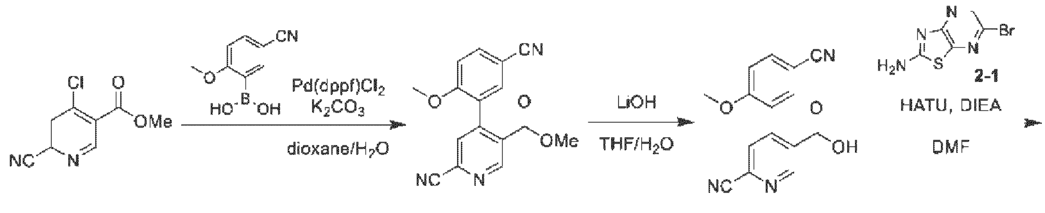


33-1 33-2 33-3

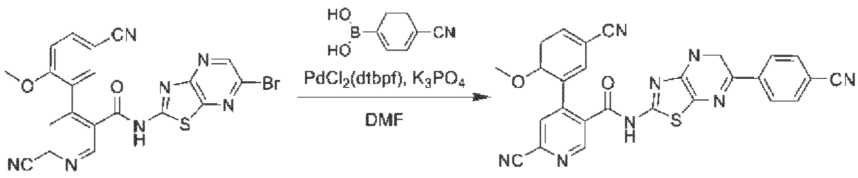


33-4 Compound 33

**Step 1: Synthesis of methyl 6-cyano-4-(5-cyano-2-methoxyphenyl)pyridine-3-carboxylate (intermediate 33-2)**

**Intermediate 33-1** (1 g), (5-cyano-2-methoxyphenyl)boronic acid (1.2 g), 1,1'-bis(diphenylphosphino)ferrocene palladium dichloride (II) (372.20 mg), and potassium carbonate (1.41 g, 10.17 mmol) were dissolved in a solution of dioxane (20 mL)/water (4 mL). Under nitrogen atmosphere, the reaction solution was stirred and reacted at 80°C for 15 minutes. After the reaction was completed, to the reaction solution was added water (500 mL), and the mixture was extracted with ethyl acetate (300 mL*2). The organic phases were combined, and dried over anhydrous sodium sulphate. After filtration, the organic phase was concentrated to dryness under reduced pressure. The residue was purified by silica gel column chromatography (ISCO®; 40 g SepaFlash® flash silica gel column, gradient: 60%-100% ethyl acetate/petroleum ether, flow rate: 40 mL/min) to obtain the title compound (1.1 g).

MS m/z(ESI): = 294.2 $[M+H]^+$

**Step 2: Synthesis of 6-cyano-4-(5-cyano-2-methoxyphenyl)pyridine-3-carboxylic acid (intermediate 33-3)**

**Intermediate 33-2** (1 g) was dissolved in tetrahydrofuran (8 mL), and a solution of lithium hydroxide (244.97 mg) in water (8 mL) was added. The reaction solution was stirred and reacted at 20°C for 1 hour. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (1.17 g).

MS m/z(ESI): = 280.2 $[M+H]^+$.

**Step 3: Synthesis of *N*-(6-bromothiazolo[4,5-*b*]pyrazin-2-yl)-6-cyano-4-(5-cyano-2-methoxyphenyl)pyridine-3-carboxamide (intermediate 33-4)**

**Intermediate 33-3** (150 mg), HATU (204.24 mg), and DIEA (138.85 mg) were dissolved in *N,N*-dimethylformamide (1 mL), and then **intermediate 2-1** (124.12 mg) was added. The reaction solution was stirred and reacted at 20°C for 1 hour. After the reaction was completed, to the reaction solution was added water (100 mL), and the mixture was extracted with ethyl acetate (60 mL*2). The organic phases were combined, and dried over anhydrous sodium sulphate. After filtration, the organic phase was concentrated to dryness under reduced pressure to obtain the title compound (750 mg).

MS m/z(ESI): = 492.1 $[M+H]^+$

**Step 4: Synthesis of 6-cyano-4-(5-cyano-2-methoxyphenyl)-*N*-(6-(4-cyanophenyl)thiazolo[4,5-*b*]pyrazin-2-yl) pyridine-3-carboxamide (compound 33)**

**Intermediate 33-4** (600 mg), (4-cyanophenyl)boronic acid (179.08 mg), 1,1'-bis(di-tert-butylphosphino)ferrocene palladium dichloride (79.43 mg), and potassium phosphate (517.40 mg) were dissolved in *N,N*-dimethylformamide (6 mL). Under nitrogen atmosphere, the reaction solution was stirred and reacted at 100°C for 1 h. After the reaction was completed, to the reaction solution was added water (30 mL), and the mixture was extracted with ethyl acetate (30 mL*2). The organic phases were combined, and dried over anhydrous sodium sulphate. After filtration, the organic phase was

concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 45%-65%, 11 minutes) to obtain the title compound (50 mg).

**$^1$H NMR(400 MHz, DMSO-$d_6$)**$\delta$ = 13.79(s, 1H), 9.38(s, 1H), 9.14(s, 1H), 8.42-8.36(m, 2H), 8.34-8.31(m, 1H), 8.08-7.96(m, 4H), 7.26-7.20(m, 1H), 3.63(s, 3H)

MS m/z(ESI): = 515.1 $[M+H]^+$.

**Example** 34, **N-(6-(4-cyanophenyl)thiazolo[4,5-*b*]pyrazin-2-yl)-3-(2-fluoro-6-methoxyphenyl)pyridine-4-carboxamide (compound 34)**

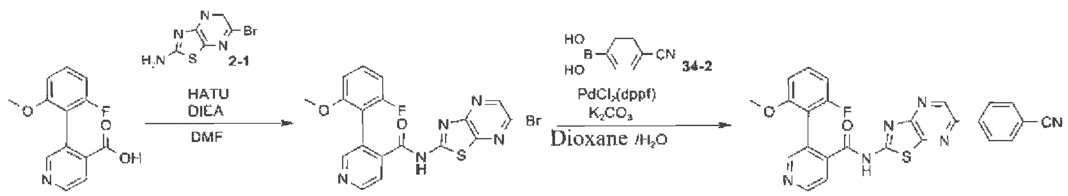


3-3

34-1

Compound 34

**Step 1: Synthesis of N-(6-bromothiazolo[4,5-b]pyrazin-2-yl)-3-(2-fluoro-6-methoxyphenyl)pyridine-4-carboxamide (intermediate 34-1)**

At 20°C, **intermediate 3-3** (120 mg) was dissolved in *N,N*-dimethylformamide (3 mL), and to the reaction solution were added **intermediate 2-1** (112.16 mg), HATU (203.02 mg) and N,N-diisopropylethylamine (125.46 mg). The reaction solution was stirred and reacted at 20°C for 1 hour. After the reaction was completed, to the reaction solution were added ethyl acetate (5 mL) and water (20 mL), and the mixture was extracted with ethyl acetate (5 mL*2). The organic phases were combined, and dried over anhydrous sodium sulphate. After filtration, the organic phase was concentrated to dryness under reduced pressure. The residue was purified by silica gel chromatography (petroleum ether/ethyl acetate =1/2) to obtain the title compound (160 mg).

MS m/z (ESI): = 460.0 $[M+H]^+$.

**Step 2: *N*-(**6-(**4-cyanophenyl)thiazolo[4,5-*b*]pyrazin-2-yl)-3-(2-fluoro-6-methoxyphenyl)pyridine-4-carboxamide (compound 34)**

At 20°C, **intermediate 34-1** (100 mg) was dissolved in a solution of dioxane (1 mL)/water (0.2 mL), and to the reaction solution were added **intermediate 34-2** (38.31 mg), potassium carbonate (36.03 mg) and 1,1'-bis(diphenylphosphino)ferrocene palladium dichloride (II) (9.54 mg). Under nitrogen atmosphere, the reaction solution was stirred and reacted at 80°C for 15 minutes. After the reaction was completed, the reaction solution was filtered, and concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 25%-45%, 11 minutes) to obtain the title compound (21 mg).

**$^1$H NMR (400 MHz, DMSO-$d_6$)** $\delta$ = 8.99 (s, 1H), 8.67-8.61 (m, 1H), 8.44-8.39 (m, 1H), 8.31-8.25 (m, 2H), 7.99-7.95 (m, 1H), 7.94-7.90 (m, 2H), 7.39-7.31 (m, 1H), 6.91-6.83 (m, 2H), 3.59 (s, 3H)

MS m/z (ESI): = 483.2 $[M+H]^+$.

**Example 35, 6-cyano-*N*-(6-(4-cyanophenyl)thiazolo[4,5-b]pyrazin-2-yl)-4-(2-methoxyphenyl)pyridine-3-carboxamide (compound 35)**

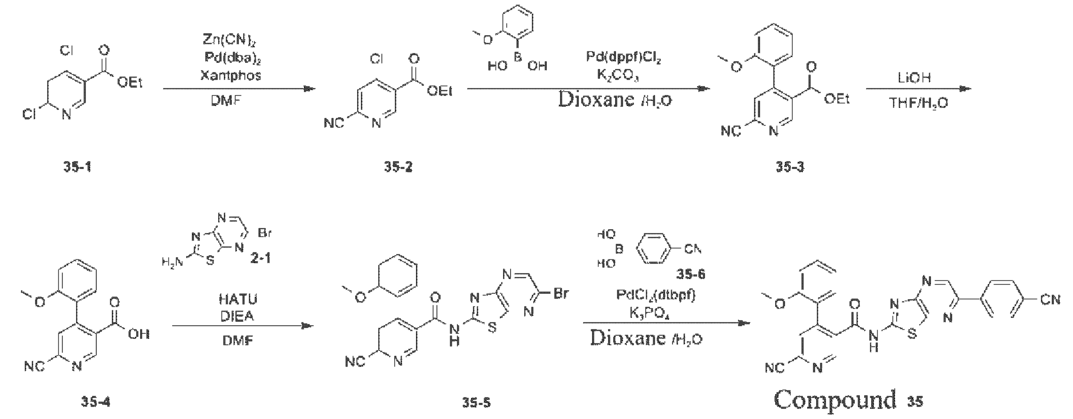


35-1 35-2 35-3 35-4 35-5 Compound 35

**Step 1: Synthesis of ethyl 4-chloro-6-cyanopyridine-3-carboxylate (intermediate 35-2)**

At 20°C, **intermediate 35-1** (5 g) was dissolved in *N,N*-dimethylformamide (50 mL), and to the reaction solution were added zinc cyanide (1.6 g), bis(dibenzylideneacetone)palladium (130.65 mg) and 4,5-bis(diphenylphosphine)-9,9-dimethylxanthene (262.95 mg). Under nitrogen atmosphere, the reaction solution was stirred and reacted at 130°C for 2 hours. After the reaction was completed, to the reaction solution was added water (200 mL), and the mixture was extracted with ethyl acetate (100 mL*3). The organic phases were combined, and dried over anhydrous sodium sulphate. After filtration, the organic phase was concentrated to dryness under reduced pressure. The residue was purified by column chromatography (petroleum ether/ethyl acetate = 2/1) to obtain the title compound (3.28 g).

MS m/z (ESI): = 211.1 $[M+H]^+$.

**Step 2: Synthesis of ethyl 6-cyano-4-(2-methoxyphenyl)pyridine-3-carboxylate (intermediate 35-3)**

At 20°C, **intermediate 35-2** (3.28 g) was dissolved in a solution of dioxane (40 mL)/water (8 mL), and to the reaction solution were added 2-methoxyphenylboronic acid (2.84 g), potassium carbonate (2.58 g) and 1,1'-bis(diphenylphosphino)ferrocene palladium dichloride (II) (683.7 mg). Under nitrogen atmosphere, the reaction solution was stirred and reacted at 80°C for 15 minutes. After the reaction was completed, the reaction solution was filtered, and concentrated to dryness under reduced pressure. The residue was purified by column chromatography (petroleum ether/ethyl acetate = 3/1) to obtain the title compound (4.4 g).

MS m/z (ESI): = 283.2 $[M+H]^+$

**Step 3: Synthesis of 6-cyano-4-(2-methoxyphenyl)pyridine-3-carboxylic acid (intermediate 35-4)**

At 20°C, **intermediate 35-3** (1 g) was dissolved in a solution of tetrahydrofuran (10 mL)/water (10 mL), and to the reaction solution was added lithium hydroxide (254.5 mg). The reaction solution was stirred and reacted at 0°C for 8 hours. After the reaction was completed, the reaction solution was concentrated to dryness under reduced pressure. The residue was dissolved in methanol (1 mL). After filtration, the filtrate was concentrated to dryness under reduced pressure to obtain the title compound (1 g).

MS m/z (ESI): = 255.2 $[M+H]^+$

**Step 4: Synthesis of *N*-(6-bromothiazolo[4,5-b]pyrazin-2-yl)-6-cyano-4-(2-methoxyphenyl)pyridine-3-carboxamide (intermediate 35-5)**

At 20°C, **intermediate 35-4** (100 mg) was dissolved in *N,N*-dimethylformamide (1.5 mL), and to the reaction solution were added **intermediate 2-1** (90.89 mg), HATU (164.51 mg) and *N,N*-diisopropylethylamine (101.67 mg). The reaction solution was stirred and reacted at 20°C for 1 hour. After the reaction was completed, to the reaction solution were added ethyl acetate (5 mL) and water (10 mL), and the mixture was extracted with ethyl acetate (5 mL*2). The organic phases were combined, and dried over anhydrous sodium sulphate. After filtration, the organic phase was concentrated to dryness under reduced pressure. The residue was purified by silica gel chromatography (petroleum ether/ethyl acetate = 2/1) to obtain the title compound (100 mg).

MS m/z (ESI): = 467.1 $[M+H]^+$.

**Step 5: Synthesis of 6-cyano-*N*-(6-(4-cyanophenyl)thiazolo[4,5-b]pyrazin-2-yl)-4-(2-methoxyphenyl)pyridine-3-carboxamide (compound 35)**

At 20°C, **intermediate 35-5** (90 mg) was dissolved in a solution of dioxane (0.5 mL)/water (0.1 mL), and to the reaction solution were added **intermediate 35-6** (56.60 mg), potassium phosphate (81.76 mg) and 1,1-bis(tert-butylphosphine)ferrocene palladium chloride (12.55 mg). Under nitrogen atmosphere, the reaction solution was stirred and reacted at 80°C for 15 minutes. After the reaction was completed, the reaction solution was filtered, and concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 48%-68%, 11 minutes) to obtain the title compound (12.3 mg).

**$^1$H NMR (400 MHz, DMSO-*$d_6$*)** δ = 13.72 (s, 1H), 9.38 (s, 1H), 9.07 (s, 1H), 8.39 (d, *J* = 8.6 Hz, 2H), 8.22 (s, 1H), 8.03 (d, *J* = 8.4 Hz, 2H), 7.53 (dd, *J* = 1.3, 7.6 Hz, 1H), 7.50-7.46 (m, 1H), 7.19-7.13 (m, 1H), 7.04 (d, *J* = 8.2 Hz, 1H), 3.55 (s, 3H)

MS m/z (ESI): = 490.1 $[M+H]^+$.

**Example 36, *N*-(6-(4-cyanophenyl)thiazolo[4,5-*b*]pyrazin-2-yl)-4-(2-methoxyphenyl)-6-(trifluoromethyl)pyridine-3-carboxamide (compound 36)**

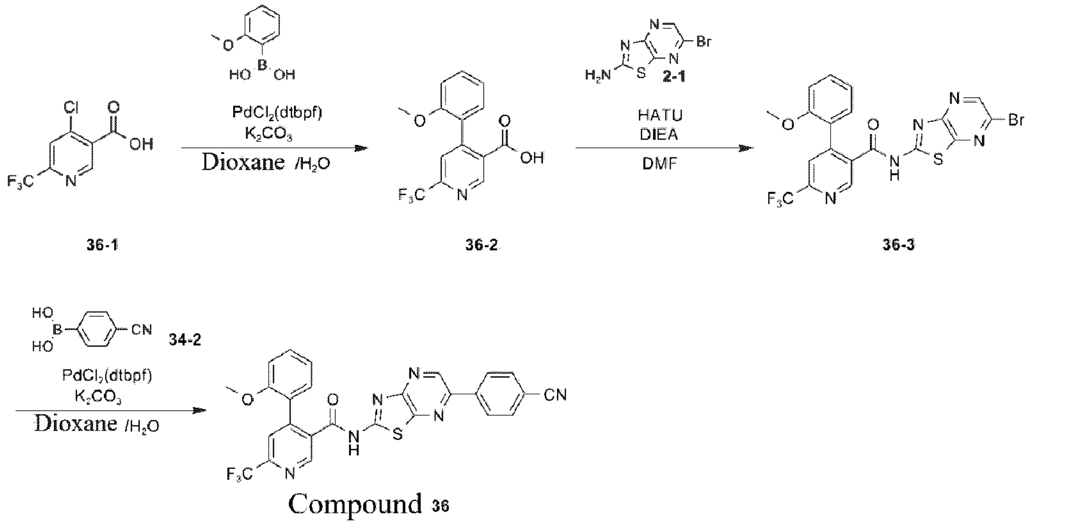


36-1 36-2 36-3

Compound 36

**Step 1: Synthesis of 4-(2-methoxyphenyl)-6-(trifluoromethyl)pyridine-3-carboxylic acid (intermediate 36-2)**

At 20°C, **intermediate 36-1** (250 mg) was dissolved in a solution of dioxane (5 mL)/water (1 mL), and to the reaction solution were added 2-methoxyphenylboronic acid (336.85 mg), potassium carbonate (306.37 mg) and 1,1-bis(tert-butylphosphine)ferrocene palladium chloride (72.24 mg). Under nitrogen atmosphere, the reaction solution was stirred and reacted at 80°C for 15 minutes. After the reaction was completed, the reaction solution was filtered, and concentrated to dryness under reduced pressure to obtain the title compound (700 mg).

MS m/z (ESI): = 297.9 $[M+H]^+$.

**Step 2: Synthesis of *N*-(6-bromothiazolo[4,5-b]pyrazin-2-yl)-4-(2-methoxyphenyl)-6-(trifluoromethyl)pyridine-3-carboxamide (intermediate 36-3)**

At 20°C, **intermediate 36-2** (600 mg) was dissolved in *N,N*-dimethylformamide (6 mL), and to the reaction solution were added **intermediate 2-1** (233.23 mg), HATU (422.15 mg) and *N,N*-diisopropylethylamine (260.90 mg). The reaction solution was stirred and reacted at 20°C for 1 hour. After the reaction was completed, to the reaction solution was added water (100 mL), and the mixture was extracted with ethyl acetate (100 mL*2). The organic phases were combined, and dried over anhydrous sodium sulphate. After filtration, the organic phase was concentrated to dryness under reduced pressure. The residue was purified by column chromatography (petroleum ether/ethyl acetate =3/1) to obtain the title compound (180 mg).

MS m/z (ESI): = 510.1 $[M+H]^+$

**Step 3: Synthesis of *N*-(6-(4-cyanophenyl)thiazolo[4,5-b]pyrazin-2-yl)-4-(2-methoxyphenyl)-6-(trifluoromethyl) pyridine-3-carboxamide (compound 36)**

At 20°C, **intermediate 36-3** (170 mg) was dissolved in a solution of dioxane (5 mL)/water (1 mL), and to the reaction solution were added **intermediate 34-2** (97.90 mg), potassium carbonate (92.09 mg) and 1,1-bis(tert-butylphosphine)ferrocene palladium chloride (21.71 mg). Under nitrogen atmosphere, the reaction solution was stirred and reacted at 80°C for 15 minutes. After the reaction was completed, the reaction solution was filtered, and concentrated to dryness under reduced pressure. The residue was purified by column chromatography (petroleum ether/ethyl acetate =1/1) to obtain the title compound (30 mg).

**$^1$H NMR (400 MHz, DMSO-*d*$_6$)** $\delta$ = 13.68 (s, 1H), 9.37 (s, 1H), 9.11 (s, 1H), 8.39 (d, *J* = 8.3 Hz, 2H), 8.07-7.95 (m, 3H), 7.55 (d, *J* = 7.5 Hz, 1H), 7.51-7.45 (m, 1H), 7.15 (s, 1H), 7.05 (d, *J* = 8.3 Hz, 1H), 3.56 (s, 3H)

MS m/z (ESI): = 533.1 $[M+H]^+$

**Example 37, *N*-(6-(4-chlorophenyl)thiazolo[4,5-b]pyrazin-2-yl)-2',5-dicyano-[1,1'-biphenyl]-2-carboxamide (compound 37)**

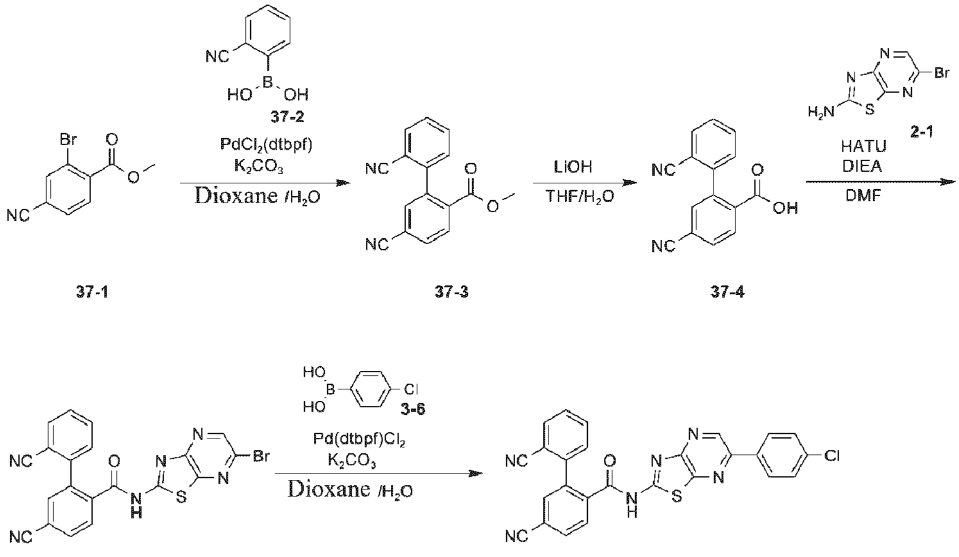


37-1 37-3 37-4

37-5 Compound 37

**Step 1: Synthesis of methyl 2',5-dicyano-[1,1' biphenyl]-2-carboxylate (intermediate 37-3)**

At 25°C, **intermediate 37-1** (500 mg), **intermediate 37-2** (1.22 g), 1,1-bis(tert-butylphosphine)ferrocene palladium chloride (135.75 mg), and potassium carbonate (575.73 mg) were dissolved in dioxane (5 mL) and water (1 mL), and the mixture was stirred at 90°C under nitrogen atmosphere for 2 hours. After the reaction was completed, the organic phase was concentrated to dryness under reduced pressure, and ethyl acetate (200 mL) and water (100 mL) were added. The mixture was washed with water (45 mL*3), and the organic phase was dried over anhydrous sodium sulphate. After filtration, the organic phase was concentrated to dryness under reduced pressure. The residue was purified by silica gel chromatography (petroleum ether/ethyl acetate = 1 : 1) to obtain the title compound (540 mg).

MS m/z (ESI): = 263.1 $[M+H]^+$.

**Step 2: Synthesis of 2',5-dicyano-[1,1'-biphenyl]-2-carboxylic acid (intermediate 37-4)**

At 20°C, **intermediate 37-3** (500 mg) was dissolved in a solution of tetrahydrofuran (3 mL) and water (3 mL), and a solution of lithium hydroxide (136.97 mg) in water (3 mL) was dropwise added. The reaction solution was stirred and reacted at 50°C for 2 hours. After the reaction was completed, the reaction solution was adjusted to pH 7 with dilute hydrochloric acid, and then concentrated to dryness under reduced pressure. The residue was dissolved in methanol (1 mL). After filtration, the filtrate was concentrated to dryness under reduced pressure to obtain the title compound (540 mg).

MS m/z (ESI): = 249.1 $[M+H]^+$.

**Step 3: Synthesis of *N*-(6-bromothiazolo[4,5-b]pyrazin-2-yl)-2',5-dicyano-[1,1'-biphenyl]-2-carboxamide (intermediate 37-5)**

At 20°C, **intermediate 37-4** (100 mg) and **intermediate 2-1** (93.09 mg) were dissolved in *N,N*-dimethylformamide (1 mL), and to the reaction solution were added HATU (168.49 mg) and *N,N*-diisopropylethylamine (104.13 mg). The reaction solution was stirred and reacted at 25°C for 2 hours. After the reaction was completed, to the reaction solution were added ethyl acetate (50 mL) and water (100 mL), and the mixture was washed with water (25 mL*3). The organic phase was dried over anhydrous sodium sulphate. After filtration, the organic phase was concentrated to dryness under reduced pressure. The residue was purified by silica gel chromatography (petroleum ether/ethyl acetate =1/1) to obtain the title compound (120 mg).
MS m/z (ESI): = 463.2 $[M+H]^+$

**Step 4: Synthesis of *N*-(6-(4-chlorophenyl)thiazolo[4,5-b]pyrazin-2-yl)-2',5-dicyano-[1,1'-biphenyl]-2-carboxamide (compound 37)**

At 20°C, **intermediate 37-5** (100 mg) and **intermediate 3-6** (50.85 mg) were dissolved in a solution of dioxane (1 mL) and water (0.2 mL), and to the reaction solution were added potassium carbonate (59.92 mg), and 1,1-bis(tert-butylphosphine)ferrocene palladium chloride. Under nitrogen atmosphere, the reaction solution was stirred and reacted at 90°C for 16 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 60%-80%, 11 minutes) to obtain the title compound (25.2 mg).
**$^1$H NMR (400MHz, DMSO-*d*$_6$)** $\delta$ = 13.79 (d, *J* = 1.0 Hz, 1H), 9.28 (s, 1H), 8.23-8.18 (m, 3H), 8.18-8.14 (m, 2H), 8.00-7.95 (m, 1H), 7.76 (dt, *J* = 1.3, 7.7 Hz, 1H), 7.62 (d, *J* = 8.6 Hz, 3H), 7.53 (d, *J* = 7.6 Hz, 1H)
MS m/z (ESI): = 493.0 $[M+H]^+$

**Example 38, *N*-(6-(4-chlorophenyl)thiazolo [4,5-*b*]pyrazin-2-yl)-6-cyano-4-(2-ethynylphenyl)pyridine-3-carboxamide (compound 38)**

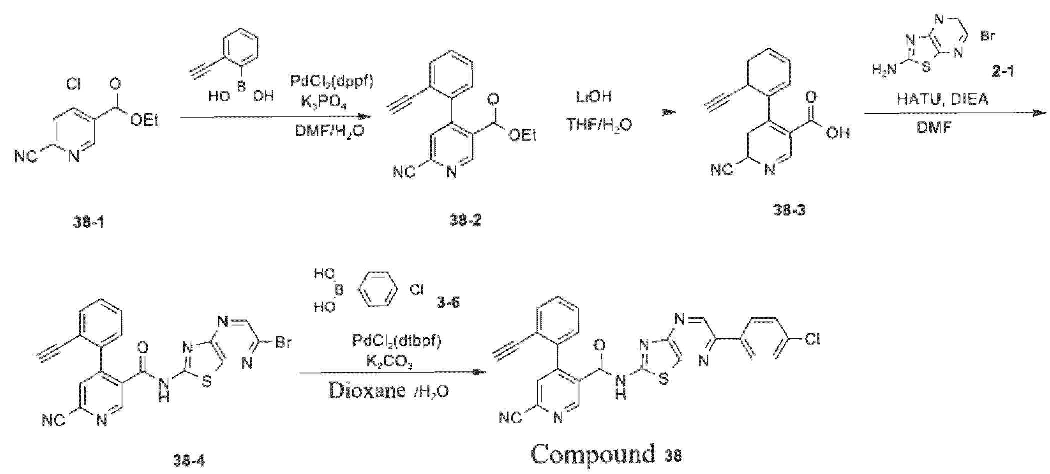


**Step 1: Synthesis of ethyl 6-cyano-4-(2-ethynylphenyl)-pyridine-3-carboxylate (intermediate 38-2)**

**Intermediate 38-1** (90 mg), (2-ethynylphenyl)boronic acid (124.73 mg), potassium phosphate (181.41 mg), and 1,1'-bis(diphenylphosphino)ferrocene palladium dichloride (II) (27.85 mg) were dissolved in a mixed solvent of *N,N*-dimethylformamide (3 mL) and water (0.2 ml). Under nitrogen atmosphere, the reaction solution was stirred and reacted at 60°C for 15 minutes. After the reaction was completed, the reaction solution was cooled to room temperature, and slowly poured into water (50 mL). The mixture was extracted with ethyl acetate (20 mL * 3), and the organic phases were combined, and dried over anhydrous sodium sulphate. After filtration, the organic layer was concentrated to dryness under reduced pressure, and the residue was purified by silica gel column chromatography (ISCO®; 20 g SepaFlash® flash silica

gel column, gradient: 0-30% ethyl acetate/petroleum ether, flow rate: 30 mL/min) to obtain the title compound (85 mg).
MS m/z (ESI): = 277.2 [M+H]$^+$.

**Step 2: Synthesis of 6-cyano-4-(2-ethynylphenyl)-pyridine-3-carboxylic acid (intermediate 38-3)**

**Intermediate 38-2** (85 mg) was dissolved in tetrahydrofuran (1 mL), and a solution of lithium hydroxide (22.10 mg) in water (1 mL) was added. The reaction solution was stirred at 0°C for 16 h. After the reaction was completed, the reaction solution was concentrated to dryness under reduced pressure, and the residue was dissolved in dichloromethane (3 mL), and filtered. The filtrate was concentrated to dryness under reduced pressure to obtain the title compound (50 mg).
MS m/z (ESI): = 249.1 [M+H]$^+$.

**Step 3: Synthesis of *N*-(6-bromothiazolo[4,5-b]pyrazin-2-yl)-6-cyano-4-(2-ethynylphenyl)pyridine-3-carboxamide (intermediate 38-4)**

At 20°C, **intermediate 38-3** (250 mg) was dissolved in *N,N*-dimethylformamide (4 mL), and to the reaction solution were added **intermediate 2-1** (232.72 mg), HATU (382.93 mg) and *N,N*-diisopropylethylamine (260.32 mg). The reaction solution was stirred and reacted at 20°C for 1 hour. After the reaction was completed, to the reaction solution was added water (30 mL), and the mixture was extracted with ethyl acetate (20 mL*2). The organic phases were combined, and dried over anhydrous sodium sulphate. After filtration, the organic phase was concentrated to dryness under reduced pressure. The residue was purified by silica gel column chromatography (petroleum ether/ethyl acetate =2/1) to obtain the title compound (110 mg).
MS m/z (ESI): = 461.1 [M+H]$^+$

**Step 4: Synthesis of *N*-(6-(4-chlorophenyl)thiazolo[4,5-b]pyrazin-2-yl)-6-cyano-4-(2-ethynylphenyl)pyridine-3-carboxamide (compound 38)**

At 20°C, **intermediate 38-4** (110 mg) was dissolved in a solution of dioxane (1 mL)/water (0.2 mL), and to the reaction solution were added **intermediate 3-6** (74.58 mg), potassium carbonate (65.91 mg) and 1,1-bis(tert-butylphosphine)ferrocene palladium chloride (15.54 mg). Under nitrogen atmosphere, the reaction solution was stirred and reacted at 80°C for 15 minutes. After the reaction was completed, the reaction solution was filtered, and the filtrate was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 54%-74%, 13 minutes) to obtain the title compound (13.2 mg).
**$^1$H NMR (400 MHz, DMSO-*d*$_6$)** δ = 13.85 (s, 1H), 9.29-9.26 (m, 1H), 9.25-9.22 (m, 1H), 8.30-8.26 (m, 1H), 8.24-8.19 (m, 2H), 7.64-7.59 (m, 3H), 7.58-7.44 (m, 3H), 4.17-4.15 (m, 1H)
MS m/z (ESI): = 493.2 [M+H]$^+$.

**Example 39, 6-cyano-*N*-(6-(4-cyanophenyl)thiazolo[4,5-b]pyrazin-2-yl)-4-morpholinopyridine-3-carboxamide (compound 39)**

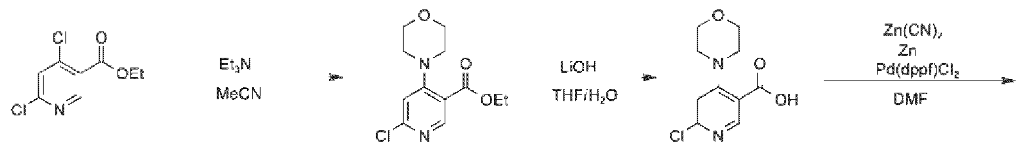


39-1 39-2 39-3

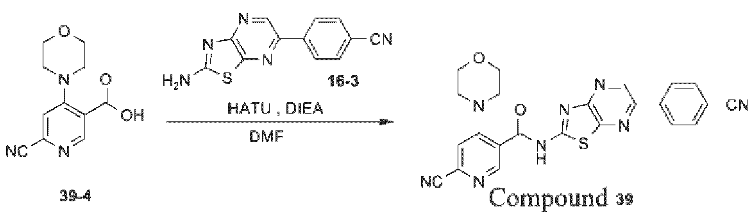


16-3

39-4 Compound 39

**Step 1: Synthesis of ethyl 6-chloro-4-morpholinopyridine-3-carboxylate (intermediate 39-2)**

**Intermediate 39-1** (2 g), morpholine (870.99 mg), and triethylamine (1.84 g) were dissolved in acetonitrile (30 mL). Under nitrogen atmosphere, the reaction solution was stirred and reacted at 25°C for 16 hours. After the reaction was completed, the reaction solution was concentrated to dryness under reduced pressure. The residue was purified by silica gel column chromatography (ISCO®; 20 g SepaFlash® flash silica gel column, gradient: 0-15% ethyl acetate/petroleum ether, flow rate: 80 mL/min) to obtain the title compound (1.0 g).

MS m/z (ESI): = 271.2 $[M+H]^+$

**Step 2: Synthesis of 6-chloro-4-morpholinopyridine-3-carboxylic acid (intermediate 39-3)**

**Intermediate 39-2** (1 g) was dissolved in tetrahydrofuran (12 mL), and a solution of lithium hydroxide (465.04 mg) in water (4 mL) was added. The reaction solution was stirred and reacted at 25°C for 1 hour. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (800 mg).

MS m/z (ESI): = 243.1 $[M+H]^+$.

**Step 3: Synthesis of 6-cyano-4-morpholinopyridine-3-carboxylic acid (intermediate 39-4)**

**Intermediate 39-3** (700 mg), zinc cyanide (0.77 mg), zinc powder (138.85 mg), and 1,1-bis(diphenylphosphine) ferrocene palladium chloride (316.61 mg) were dissolved in *N,N*-dimethylformamide (10 mL), and the reaction solution was stirred and reacted at 100°C for 2 hours. After the reaction was completed, to the reaction solution was added water (100 mL), and the mixture was extracted with ethyl acetate (60 mL*3). The organic phases were combined, and dried over anhydrous sodium sulphate. After filtration, the organic phase was concentrated under reduced pressure, and the residue was purified by silica gel column chromatography (ISCO®; 20 g SepaFlash® flash silica gel column, gradient: 0-20% acetonitrile/water, flow rate: 60 mL/min) to obtain the title compound (220 mg).

MS m/z (ESI): = 234.1 $[M+H]^+$

**Step 4: Synthesis of 6-cyano-*N*-(6-(4-cyanophenyl)thiazolo[4,5-b]pyrazin-2-yl)-4-morpholinopyridine-3-carboxamide (compound 39)**

**Intermediate 39-4** (50 mg), **intermediate 16-3** (54.30 mg), HATU (122.27 mg), and *N,N*-diisopropylethylamine (27.71 mg) were dissolved in *N,N*-dimethylformamide (5 mL). Under nitrogen atmosphere, the reaction solution was stirred and reacted at 25°C for 1 h. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 35%-55%, 12 minutes) to obtain the title compound (30 mg).

**$^1$H NMR (400 MHz, DMSO-$d_6$)** δ = 8.98 (s, 1H), 8.47 (s, 1H), 8.29-8.26 (m, 2H), 7.93-7.90 (m, 2H), 7.47 (s, 1H), 3.70-3.61 (m, 4H), 3.28-3.21 (m, 4H)

MS m/z (ESI): = 469.0 $[M+H]^+$.

**Example 40, 3-(4-cyano-2-methoxyphenyl)-*N*-(6-(4-cyanophenyl)thiazolo [4,5-b]pyrazin-2-yl)pyridine-4-carboxamide (compound 40)**

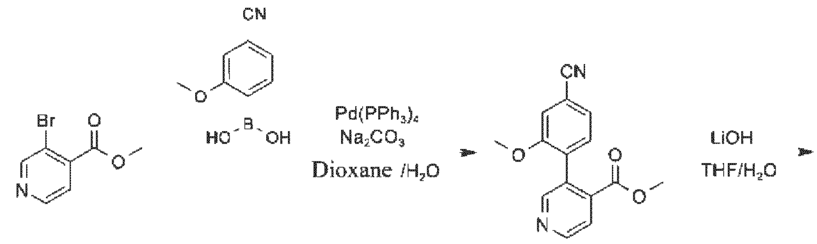


40-1 40-2

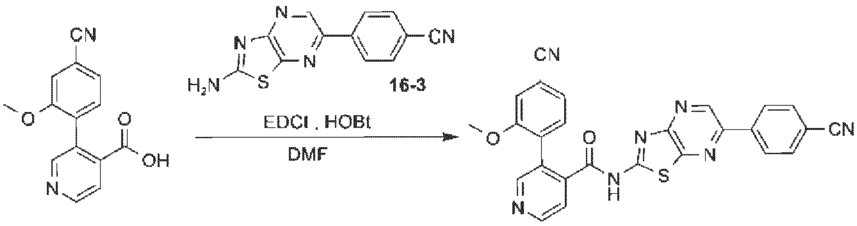


40-3

Compound 40

**Step 1: Synthesis of methyl 3-(4-cyano-2-methoxyphenyl)pyridine-4-carboxylate (intermediate 40-2)**

**Intermediate 40-1** (500 mg), (4-cyano-2-methoxyphenyl)boronic acid (614.37 mg), tetrakis(triphenylphosphine)palladium (267.45 mg), and sodium carbonate (490.62 mg) were dissolved in a solution of dioxane (20 mL)/water (4 mL). Under nitrogen atmosphere, the reaction solution was stirred and reacted at 80°C for 2 hours. After the reaction was completed, to the reaction solution was added water (50 mL), and the mixture was extracted with ethyl acetate (30 mL*2). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: -30% ethyl acetate/petroleum ether, flow rate: 40 mL/min) to obtain the title compound (500 mg).

MS m/z (ESI): = 269.4 $[M+H]^+$

**Step 2: Synthesis of 3-(4-cyano-2-methoxyphenyl)pyridine-4-carboxylic acid (intermediate 40-3)**

**Intermediate 40-2** (500 mg) was dissolved in tetrahydrofuran (8 mL), and a solution of lithium hydroxide (244.97 mg) in water (8 mL) was added. The reaction solution was stirred and reacted at 20°C for 1 hour. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (400 mg).

MS m/z (ESI): = 255.2 $[M+H]^+$.

**Step 3: Synthesis of 3-(4-cyano-2-methoxyphenyl)-A-(6-(4-cyanophenyl)thiazolo[4,5-b]pyrazin-2-yl)pyridine-4-carboxamide (compound 40)**

**Intermediate 40-3** (50 mg), **intermediate 16-3** (49.81 mg), 1-(3-dimethylaminopropyl)-3-ethylcarbodiimide hydrochloride (75.40 mg), pyridine (46.67 mg) and 1-hydroxybenzotriazole (53.15 mg) were dissolved in *N,N*-dimethylformamide (5 mL). Under nitrogen atmosphere, the reaction solution was stirred and reacted at 25°C for 1 h. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 43%-63%, 2 minutes) to obtain the title compound (5 mg).

**$^1$H NMR (400 MHz, DMSO-d6)** δ = 9.28 (s, 1H), 8.81 (s, 1H), 8.64 (s, 1H), 8.37 (d, *J* = 8.5 Hz, 2H), 8.00 (d, *J* = 8.3 Hz, 2H), 7.83 (d, *J* = 5.0 Hz, 1H), 7.59 (s, 2H), 7.49 (s, 1H), 3.59 (s, 3H)

MS m/z (ESI): = 490.1 $[M+H]^+$.

**Example 41, N (6-(4-cyanophenyl)thiazolo[4,5-b]pyrazin-2-yl)-6-(2-methoxyphenyl)imidazo[1,2-*a*]pyridine-7-carboxamide (compound 41)**

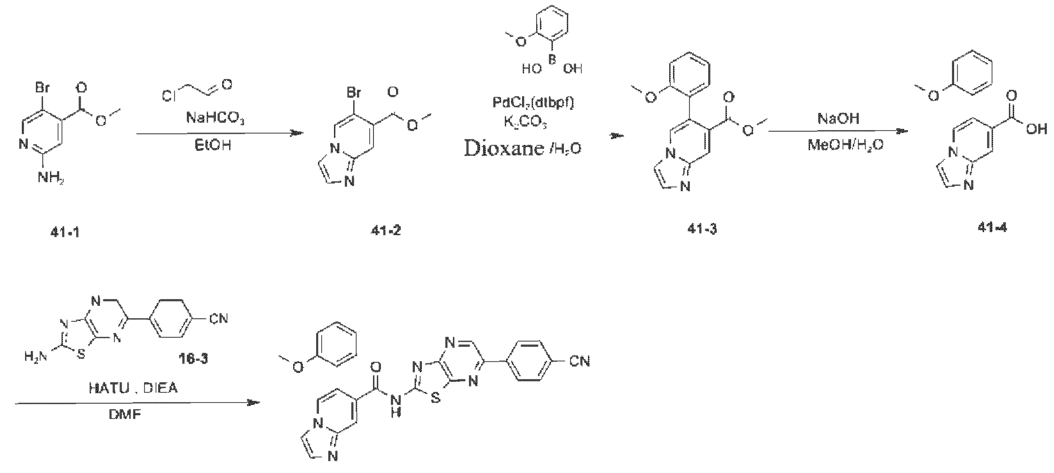


Compound 41

**Step 1: Synthesis of methyl 6-bromoimidazo[1,2-*a*]pyridine-7-carboxylate (intermediate 41-2)**

At 25°C, **intermediate 41-1** (300 mg) and 2-chloroacetaldehyde (509.62 mg) were dissolved in ethanol (5 mL), and then sodium bicarbonate (218.16 mg) was added. The reaction solution was stirred and reacted at 80°C for 16 hours. After the reaction was completed, the organic phase was concentrated to dryness under reduced pressure, and ethyl acetate (200 mL) and water (100 mL) were added. The mixture was washed with water (45 mL*3), and the organic phase was dried over anhydrous sodium sulphate. After filtration, the filtrate was concentrated to dryness under reduced pressure to obtain the title compound (200 mg).
MS m/z (ESI): = 255.2 $[M+H]^+$.

**Step 2: Synthesis of methyl 6-(2-methoxyphenyl)imidazo[1,2-*a*]pyridine-7-carboxylate (intermediate 41-3)**

At 20°C, **intermediate 41-2** (100 mg), 2-methoxyphenylboronic acid (89.36 mg), 1,1-bis(tert-butylphosphine) ferrocene palladium chloride (25.55 mg) and potassium carbonate (108.37 mg) were dissolved in a solution of dioxane (1 mL) and water (0.2 mL), and the reaction solution was stirred and reacted at 90°C for 2 hours. After the reaction was completed, the organic phase was concentrated to dryness under reduced pressure, and ethyl acetate (200 mL) and water (100 mL) were added. The mixture was washed with water (45 mL*3), and the organic phase was concentrated to dryness under reduced pressure. The residue was purified by silica gel chromatography (petroleum ether/ethyl acetate =1/3) to obtain the title compound (100 mg).
MS m/z (ESI): = 282.9 $[M+H]^+$.

Step **3:** Synthesis **of 6-(2-methoxyphenyl)imidazo[1,2-*a*]pyridine-7-carboxylic acid (intermediate 41-4)**

At 20°C, **intermediate 41-3** (100 mg) and sodium hydroxide (141.70 mg) were dissolved in methanol (1 mL) and water (1 mL). The reaction solution was stirred and reacted at 60°C for 2 hours. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (100 mg).
MS m/z (ESI): = 269.1 $[M+H]^+$.

**Step 4: Synthesis of *N*-(6-(4-cyanophenyl)thiazolo[4,5-*b*]pyrazin-2-yl)-6-(2-methoxyphenyl)imidazo[1,2-*a*]pyridine-7-carboxamide (compound 41)**

At 20°C, **intermediate 41-4** (80 mg) and **intermediate 16-3** (90.64 mg) were dissolved in *N,N*-dimethylformamide (1 mL), and to the reaction solution were added HATU (124.73 mg) and diisopropylethylamine (222.60 mg). The

reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 25%-55%, 10 minutes) to obtain the title compound (1 mg).

**$^1$H NMR (400MHz, DMSO-$d_6$)** $\delta$ = 13.52-13.41 (m, 1H), 8.63 (s, 1H), 8.41-8.35 (m, 2H), 8.15-8.05 (m, 3H), 8.04-7.99 (m, 2H), 7.85-7.73 (m, 1H), 7.49-7.35 (m, 2H), 7.14-7.06 (m, 1H), 6.97 (d, *J* = 8.3 Hz, 1H), 3.53-3.52 (m, 3H).

MS m/z (ESI): = 504.1 [M+H]$^+$.

**Example 42, *N*-(6-(4-cyanophenyl)thiazolo[4,5-b]pyrazin-2-yl)-4-(2-ethynylphenyl)-6-methylpyridine-3-carboxamide (compound 42)**

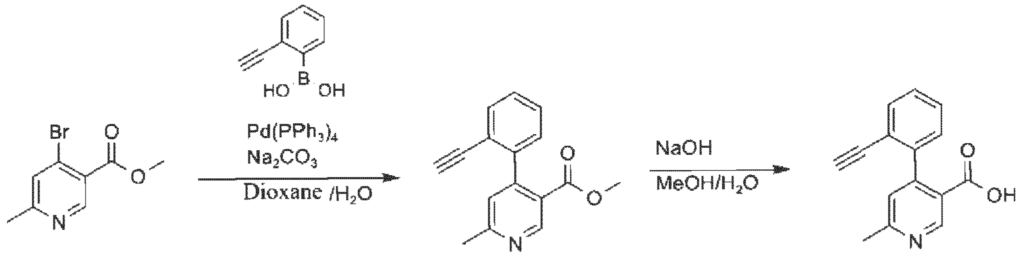


42-1 42-2 42-3

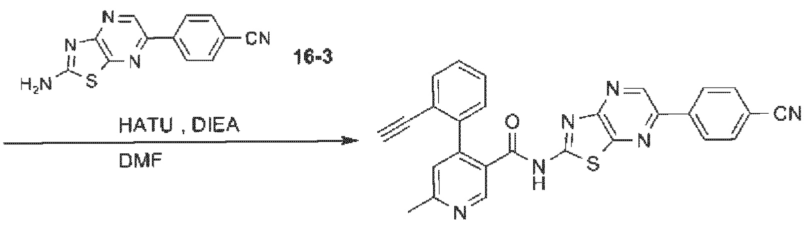


Compound 42

**Step 1: Synthesis of methyl 4-(2-ethynylphenyl)-6-methylpyridine-3-carboxylate (intermediate 42-2)**

**Intermediate 42-1** (4.50 g) and (2-ethynylphenyl)boronic acid (3.00 g) were dissolved in dioxane (45 mL) and water (9 mL), and to the mixture was added sodium carbonate (4.15 g). The reaction solution was subjected to nitrogen replacement three times. To the reaction solution was then added tetrakistriphenylphosphine palladium (2.26 g). The reaction solution was stirred at 80°C under nitrogen for 2 h. After the reaction was completed, the reaction was cooled to room temperature, and the reaction solution was filtered. The filtrate was poured into water, and ethyl acetate (30 mL) was added. The aqueous phase was washed with ethyl acetate (50 mL*2), and after washing, the organic phase was dried over anhydrous sodium sulphate, filtered, and concentrated to dryness under reduced pressure. The residue was purified by flash silica gel column chromatography (ISCO®; 25 g SepaFlash® flash silica gel column, gradient: 0-30% petroleum ether/ethyl acetate, flow rate: 60 mL/min) to obtain the title compound (2.8 g).

MS m/z (ESI): 252.0 [M+H]$^+$.

**Step 2: Synthesis of 4-(2-ethynylphenyl)-6-methylpyridine-3-carboxylic acid (intermediate 42-3)**

**Intermediate 42-2** (2.60 g) was dissolved in anhydrous methanol (26 mL), and to the mixture were added sodium hydroxide (1.24 g) and water (6 mL). The reaction solution was stirred at 30°C for 4 h. After the reaction was completed, the reaction solution was concentrated under reduced pressure to remove methanol. The remaining aqueous phase was adjusted to pH 3 with an appropriate amount of hydrochloric acid, with a large amount of solid precipitated. After filtration, the filter cake was dried to obtain the title compound (2.0 g).

MS m/z (ESI): 238.0 [M+H]+.

**Step 3: Synthesis of *N*-(6-(4-cyanophenyl)thiazolo[4,5-b]pyrazin-2-yl)-4-(2-ethynylphenyl)-6-methylpyridine-3-carboxamide (compound 42)**

**Intermediate 42-3** (30.00 mg) was dissolved in anhydrous *N' N*-dimethylformamide (0.5 mL), and to the mixture were added HATU (68.68 mg) and *N,N*-diisopropylethylamine (46.69 mg). The reaction solution was stirred at 0°C under nitrogen atmosphere for 30 min. **Intermediate 16-3** (30.50 mg) was then added, and the reaction solution was stirred at 25°C under nitrogen atmosphere for 2 h. After the reaction was completed, the reaction solution was filtered, and purified by preparative high performance liquid chromatography (Boston Prime C18 column, 5 um silica, 30 mm in diameter, 150 mm in length; using a decreasingly polar reaction solution of water (containing 0.225% formic acid) and acetonitrile as the eluent; acetonitrile gradient proportion: 39%-79%, elution time: 9 minutes) to obtain the title compound (16.0 mg).
MS m/z (ESI): 473.1 [M+H]+.
**$^1$H NMR (400MHz, DMSO-d6)** δ = 13.58 (s, 1H), 9.34 (s, 1H), 8.95 (s, 1H), 8.37 (d, *J* = 8.3 Hz, 2H), 8.01 (d, *J* = 8.3 Hz, 2H), 7.58-7.49 (m, 2H), 7.48-7.36 (m, 3H), 4.02 (s, 1H), 2.62 (s, 3H)

**Example** 43, **6-chloro-*N*-(6-(4-cyanophenyl)thiazolo[4,5-b]pyrazin-2-yl)-4-(2-ethynylphenyl)pyridine-3-carboxamide (compound 43)**

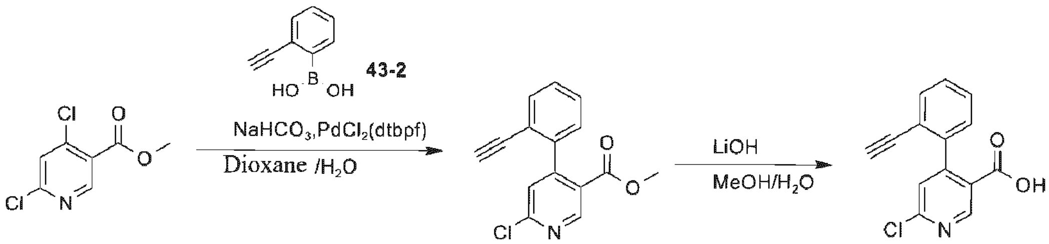


43-1 43-3 43-4

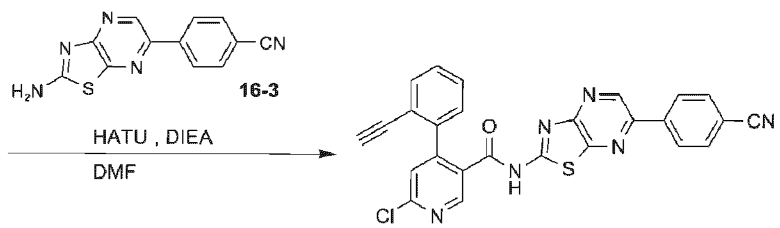


Compound 43

**Step 1: Synthesis of methyl 6-chloro-4-(2-ethynylphenyl)pyridine-3-carboxylate (intermediate 43-3)**

At 25°C, **intermediate 43-1** (200 mg) and **intermediate 43-2** (212.52 mg) were dissolved in dioxane (5 mL) and water (1 mL), and then sodium bicarbonate (268.33 mg) and 1,1-bis(tert-butylphosphine)ferrocene palladium chloride (63.27 mg) were added. The reaction solution was stirred and reacted at 90°C for 2 hours. After the reaction was completed, the organic phase was concentrated to dryness under reduced pressure, and ethyl acetate (200 mL) and water (100 mL) were added. The mixture was washed with water (45 mL*3), and the organic phase was dried over anhydrous sodium sulphate. After filtration, the filtrate was concentrated to dryness under reduced pressure, and the residue was purified by silica gel chromatography (petroleum ether/ethyl acetate =3/1) to obtain the title compound (50 mg).
MS m/z (ESI): = 272.3 [M+H]+.

**Step 2: Synthesis of 6-chloro-4-(2-ethynylphenyl)pyridine-3-carboxylic acid (intermediate 43-4)**

At 20°C, **intermediate 43-3** (25 mg) was dissolved in a solution of methanol (1 mL) and water (1 mL), and then to the reaction solution was added lithium hydroxide (19.31 mg). The reaction solution was stirred and reacted at 25°C for 2

hours. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (50 mg).

MS m/z (ESI): = 257.9 [M+H]$^+$.

**Step 3: Synthesis of 6-chloro-*N*-(6-(4-cyanophenyl)thiazolo[4,5-b]pyrazin-2-yl)-4-(2-ethynylphenyl)pyridine-3-carboxamide (compound 43)**

At 20°C, **intermediate 43-4** (30 mg) and **intermediate 16-3** (29.49 mg) were dissolved in *N,N*-dimethylformamide (1 mL), and to the reaction solution were added HATU (48.70 mg) and diisopropylethylamine (222.60 mg). The reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 41%-71%, 9 minutes) to obtain the title compound (1.5 mg).

**$^1$H NMR (400MHz, DMSO-*d*$_6$)** $\delta$ = 13.79-13.64 (m, 1H), 9.30 (s, 1H), 8.97 (s, 1H), 8.37 (d, *J* = 8.3 Hz, 2H), 8.00 (d, *J* = 8.4 Hz, 2H), 7.65 (s, 1H), 7.61-7.56 (m, 1H), 7.54-7.45 (m, 2H), 7.45-7.40 (m, 1H), 4.20-4.02 (m, 1H).

MS m/z (ESI): = 493.0 [M+H]$^+$.

**Example 44, *N*-(6-(4-cyanophenyl)thiazolo[4,5-b]pyrazin-2-yl)-2-methoxy-6'-methyl-[3,4'-bipyridine]-3'-carboxamide (compound 44)**

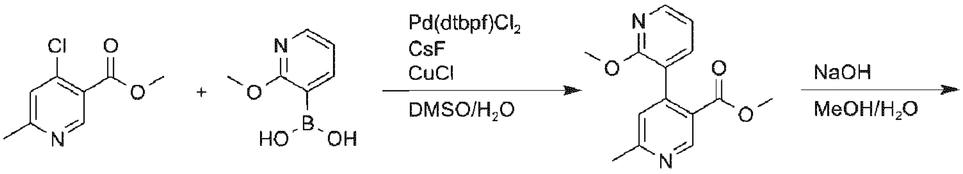


20-1 44-1 44-2

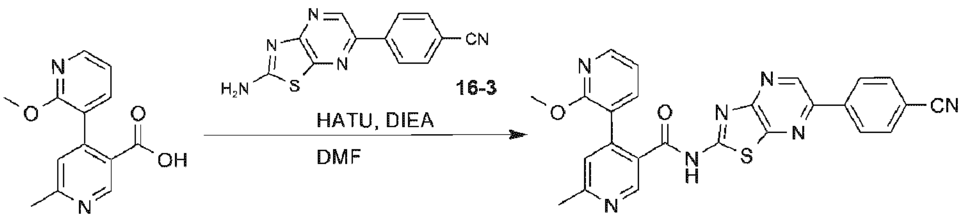


44-3

Compound 44

**Step 1: Synthesis of methyl 2-methoxy-6'-methyl-[3,4'-bipyridine]-3'-carboxylate (intermediate 44-2)**

**Intermediate 20-1** (1.83 g) and **intermediate 44-1** (1.00 g) were dissolved in DMSO (10 mL) and water (1 mL), and to the mixture were added cuprous chloride (1.29 g) and caesium fluoride (1.99 g). To the reaction solution was then added 1,1-bis(tert-butylphosphine)ferrocene palladium chloride (426.14 mg). The reaction solution was stirred at 90°C under nitrogen atmosphere for 16 h. After the reaction was completed, the reaction was cooled to room temperature, and the reaction solution was filtered. The filtrate was poured into water, and ethyl acetate (30 mL) was added. The aqueous phase was washed with ethyl acetate (20 mL *2). After washing, the organic phase was dried over anhydrous sodium sulphate, and concentrated to dryness under reduced pressure. The residue was purified by flash silica gel column chromatography (ISCO®; 12 g SepaFlash® flash silica gel column, gradient: 0-70% petroleum ether/ethyl acetate, flow rate: 60 mL/min) to obtain the title compound (300.0 mg).

MS m/z (ESI): 259.4 [M+H]$^+$

**Step 2: Synthesis of 2-methoxy-6'-methyl-[3,4'-bipyridine]-3'-carboxylic acid (intermediate 44-3)**

**Intermediate 44-2** (300 mg) was dissolved in anhydrous methanol (2.5 mL), and to the mixture were added sodium hydroxide (139.38 mg) and water (0.5 mL). The reaction solution was stirred at 30°C for 3 h. After the reaction was completed, the reaction solution was concentrated under reduced pressure to remove methanol. The remaining aqueous phase was adjusted to pH 3 with an appropriate amount of hydrochloric acid. The reaction solution was concentrated under reduced pressure, and to the residue was added dichloromethane : methanol (10 : 1, 10 mL). The mixture was stirred for 10 min, and filtered. The filtrate was concentrated to dryness under reduced pressure to obtain the title compound (250.0 mg).

MS m/z (ESI): 245.2 $[M+H]^+$.

**Step 3: Synthesis of *N*-(6-(4-cyanophenyl)thiazolo[4,5-b]pyrazin-2-yl)-2-methoxy-6'-methyl-[3,4'-bipyridine]-3'-carboxamide (compound 44)**

**Intermediate 44-3** (50.00 mg) was dissolved in anhydrous *N'N*-dimethylformamide (1 mL), and to the mixture were added HATU (81.73 mg) and diisopropylethylamine (79.37 mg). The reaction solution was stirred at 25°C under nitrogen atmosphere for 30 min. **Intermediate 16-3** (51.85 mg) was then added, and the reaction solution was stirred at 25°C under nitrogen atmosphere for 3 h. After the reaction was completed, the reaction solution was filtered, and purified by preparative high performance liquid chromatography (Boston Green ODS C18 column, 5 um silica, 30 mm in diameter, 150 mm in length; using a decreasingly polar reaction solution of water (containing 0.225% formic acid) and acetonitrile as the eluent; acetonitrile gradient proportion: 35%-65%, elution time: 14 minutes) to obtain the title compound (20 mg).

MS m/z (ESI): 480.0 $[M+H]^+$.

**$^1$H NMR (400MHz, DMSO-$d_6$)** $\delta$ = 13.52 (s, 1H), 9.37 (s, 1H), 8.84 (s, 1H), 8.39 (d, *J* = 8.4 Hz, 2H), 8.28-8.20 (m, 1H), 8.03 (d, *J* = 8.5 Hz, 2H), 7.89 (d, *J* = 5.6 Hz, 1H), 7.45 (s, 1H), 7.19 (dd, *J* = 5.1, 7.3 Hz, 1H), 3.62 (s, 3H), 2.62 (s, 3H)

**Example 45, *N*-(6-(4-cyanophenyl)thiazolo[4,5-b]pyrazin-2-yl)-3'-methoxy-6-methyl-[4,4'-bipyridine]-3-carboxamide (compound 45)**

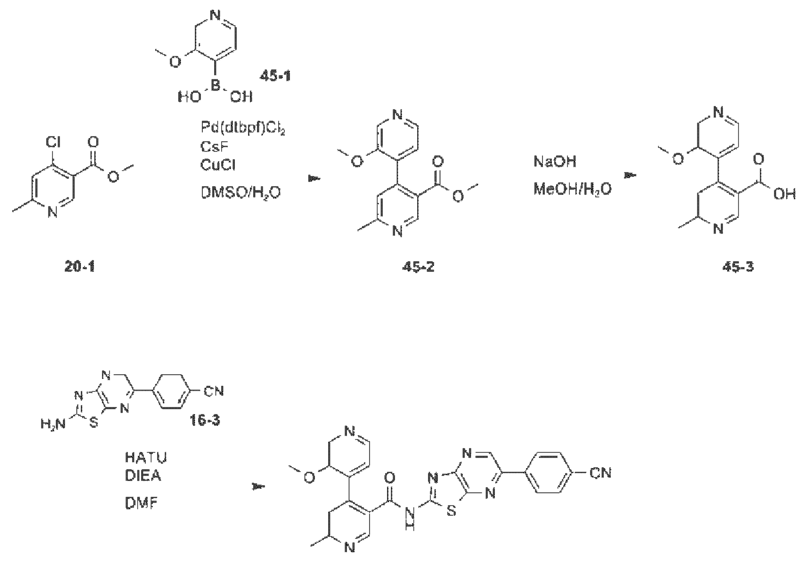


45-1

20-1

45-2

45-3

16-3

Compound 45

**Step 1: Synthesis of methyl 3'-methoxy-6-methyl-[4,4'-bipyridine]-3-carboxylate (intermediate 45-2)**

At 25°C, **intermediate 20-1** (500 mg), **intermediate 45-1** (618.01 mg), 1,1-bis(tert-butylphosphine)ferrocene palladium chloride (175.57 mg), cuprous chloride (33.38 mg) and caesium fluoride (818.41 mg) were dissolved in a solution of dimethyl sulfoxide (5 mL) and water (0.2 mL). The reaction solution was stirred and reacted at 90°C for 2 hours. After the reaction was completed, the organic phase was concentrated to dryness under reduced pressure, and ethyl acetate (200 mL) and water (100 mL) were added. The mixture was washed with water (45 mL*3), and after liquid separation was performed, the organic phase was concentrated to dryness under reduced pressure. The residue was

purified by silica gel chromatography (petroleum ether/ethyl acetate =1/1) to obtain the title compound (80 mg).

MS m/z (ESI): = 259.0 [M+H]+.

**Step 2: Synthesis of 3'-methoxy-6-methyl-[4,4'-bipyridine]-3-carboxylic acid (intermediate 45-3)**

At 25°C, **intermediate 45-2** (80 mg) and sodium hydroxide (37.17 mg) were dissolved in a solution of methanol (0.8 mL) and water (0.2 mL), and the reaction solution was stirred and reacted at 25°C for 2 hours. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (70 mg).

MS m/z (ESI): = 245.2 [M+H]+

**Step 3: Synthesis of *N*-(6-(4-cyanophenyl)thiazolo[4,5-b]pyrazin-2-yl)-3'-methoxy-6-methyl-[4,4'-bipyridine]-3-carboxamide (compound 45)**

At 20°C, **intermediate 45-3** (50 mg) and **intermediate 16-3** (77.77 mg) were dissolved in *N,N* dimethylformamide (1 mL), and to the reaction solution were added HATU (93.41 mg) and diisopropylethylamine (52.92 mg). The reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 18%-38%, 11 minutes) to obtain the title compound (5.5 mg).

**$^1$H NMR (400MHz, DMSO-*d*$_6$)** δ = 13.67-13.48 (m, 1H), 9.36 (s, 1H), 8.87 (s, 1H), 8.42-8.34 (m, 4H), 8.02 (d, *J* = 8.6 Hz, 2H), 7.49-7.40 (m, 2H), 3.65 (s, 3H), 2.63 (s, 3H).

MS m/z (ESI): = 480.0 [M+H]+.

**Example 46, *N*-(6-(4-chlorophenyl)thiazolo [4,5-b] pyrazin-2-yl)-4-(5-cyano-2-methoxyphenyl)-6-methylpyridine-3-carboxamide (compound 46)**

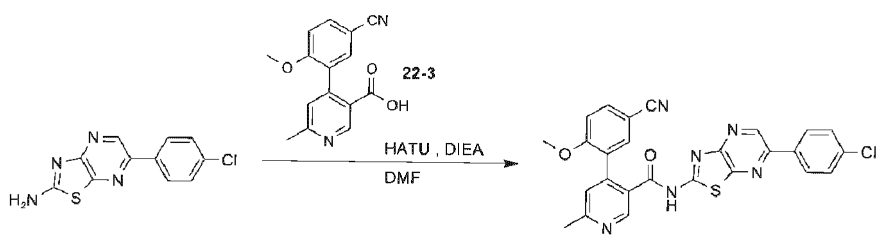


17-2

Compound 46

**Intermediate 22-3** (71.21 mg) and HATU (100.93 mg) were dissolved in *N,N*-dimethylformamide (2 mL). Under nitrogen atmosphere, the reaction solution was stirred and reacted at 40°C for 30 minutes, and then **intermediate 17-2** (110 mg) and *N,N*-diisopropylethylamine (68.61 mg) were added. The reaction solution was stirred and reacted at 40°C for 16 hours. After the reaction was completed, the reaction solution was purified by high performance liquid chromatography (chromatographic column: Boston Green ODS 150*30 mm*5 um; mobile phase: A: water (0.225% formic acid), B: acetonitrile; B%: 52%-82%, 12 minutes) to obtain the title compound (22 mg).

**$^1$H NMR (400 MHz, DMSO-*d*$_6$)** δ = 13.46-13.33 (m, 1H), 9.31-9.25 (m, 1H), 8.89-8.79 (m, 1H), 8.27-8.16 (m, 2H), 7.98-7.88 (m, 2H), 7.66-7.59 (m, 2H), 7.52-7.42 (m, 1H), 7.25-7.15 (m, 1H), 3.61 (s, 3H), 2.64-2.59 (m, 3H)

MS m/z (ESI): = 513.1 [M+H]+.

**Example 47, *N*-(6-(4-cyanophenyl)thiazolo[4,5-b]pyrazin-2-yl)-4-(2-ethynyl-4-fluorophenyl)-6-methylpyridine-3-carboxamide (compound 47)**

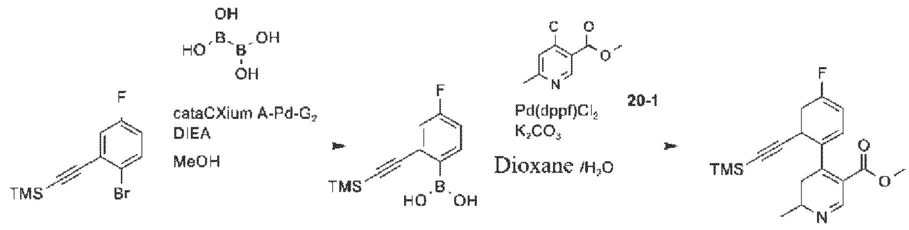


47-1 47-2 47-3

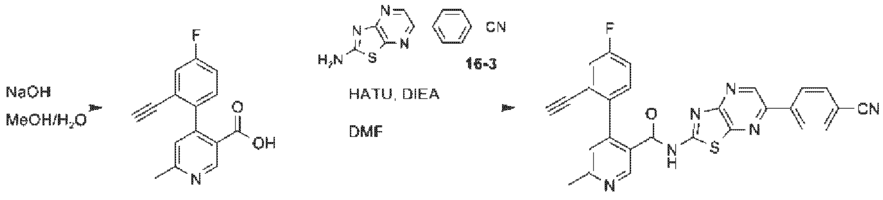


47-4 Compound 47

**Step 1: Synthesis of (4-fluoro-2-((trimethylsilyl)ethynyl)phenyl)boronic acid (intermediate 47-2)**

**Intermediate 47-1** (2 g) and tetrahydroxydiboron (1.98 g) were dissolved in anhydrous methanol (20 mL), and to the mixture was added *N,N*-diisopropylethylamine (4.77 g). The reaction solution was subjected to nitrogen replacement three times. To the reaction solution was then added chloro[(n-butylbis(1-adamantyl)phosphine)-2-(2-aminobiphenyl)] palladium (II) (493.09 mg). The reaction solution was stirred at 25°C under nitrogen atmosphere for 1 h. After the reaction was completed, the reaction solution was filtered, and the filtrate was concentrated to dryness under reduced pressure. The residue was purified by silica gel column chromatography (ISCO®; 12 g SepaFlash® flash silica gel column, gradient: 0-30% petroleum ether/ethyl acetate, flow rate: 50 mL/min) to obtain the title compound (500 mg).
MS m/z (ESI): 237.0 $[M+H]^+$.

**Step 2: Synthesis of methyl 4-(4-fluoro-2-((trimethylsilyl)ethynyl)phenyl)-6-methylpyridine-3-carboxylate (intermediate 47-3)**

**Intermediate 47-2** (500 mg) and **intermediate 20-1** (589.55 mg) were dissolved in dioxane (5 mL) and water (1 mL). Under nitrogen atmosphere, to the reaction solution were added (1,1'-bis(diphenylphosphino)ferrocene)palladium dichloride (154.94 mg) and potassium carbonate (585.32 mg), and the reaction solution was stirred at 90°C under nitrogen atmosphere for 2 h. After the reaction was completed, the reaction solution was cooled to room temperature, and filtered. The filtrate was poured into water, and ethyl acetate (30 mL) was added. The aqueous phase was extracted with ethyl acetate (30 mL *2), and the organic phase was dried over anhydrous sodium sulphate, and filtered. The filtrate was concentrated to dryness under reduced pressure. The residue was purified by silica gel column chromatography (ISCO®; 12 g SepaFlash® flash silica gel column, gradient: 0-30% ethyl acetate/petroleum ether, flow rate: 60 mL/min) to obtain the title compound (500 mg).
MS m/z (ESI): 342.2 $[M+H]^+$.

**Step 3: Synthesis of 4-(2-ethynyl-4-fluorophenyl)-6-methylpyridine-3-carboxylic acid (intermediate 47-4)**

**Intermediate 47-3** (500 mg) was dissolved in anhydrous methanol (5 mL), and to the mixture were added sodium hydroxide (175.20 mg, 4.38 mmol) and water (1 mL). The reaction solution was stirred at 25°C for 2 h. After the reaction was completed, the reaction solution was concentrated to dryness under reduced pressure, and to the residue was added 10 mL of dichloromethane : methanol (10 : 1). The mixture was stirred for 10 min, and filtered. The filtrate was concentrated to dryness under reduced pressure to obtain the title compound (250.0 mg).
MS m/z (ESI): 256.0 $[M+H]^+$.

**Step 4: Synthesis of *N*-(6-(4-cyanophenyl)thiazolo[4,5-b]pyrazin-2-yl)-4-(2-ethynyl-4-fluorophenyl)-6-methylpyridine-3-carboxamide (compound 47)**

**Intermediate 47-4** (50.00 mg) was dissolved in anhydrous *N'N*-dimethylformamide (1 mL), and to the mixture were added HATU (75.23 mg) and N,N-diisopropylethylamine (75.95 mg). The reaction solution was stirred under nitrogen atmosphere for 30 min. **Intermediate 16-3** (74.42 mg) was then added, and the reaction solution was stirred at 25°C under

nitrogen atmosphere for 3 h. After the reaction was completed, the reaction solution was filtered, and purified by preparative high performance liquid chromatography (Boston Green ODS C18 column, 5 um silica, 30 mm in diameter, 150 mm in length; using a decreasingly polar reaction solution of water (containing 0.225% formic acid) and acetonitrile as the eluent; acetonitrile gradient proportion: 46%-76%, elution time: 12 minutes) to obtain the title compound (10 mg).

MS m/z (ESI): 491.0 $[M+H]^+$.

**$^1$H** NMR **(400MHz, DMSO-*d*$_6$)** δ = 13.59 (s, 1H), 9.36 (s, 1H), 8.97 (s, 1H), 8.37 (d, *J* = 8.4 Hz, 2H), 8.01 (d, *J* = 8.5 Hz, 2H), 7.49-7.43 (m, 2H), 7.42-7.37 (m, 2H), 4.16 (s, 1H), 2.62 (s, 3H)

**Example 48, *N*-(6-(4-cyanophenyl)thiazolo[4,5-*b*]pyrazin-2-yl)-4-(2-ethynyl-5-fluorophenyl)-6-methylpyridine-3-carboxamide (compound 48)**

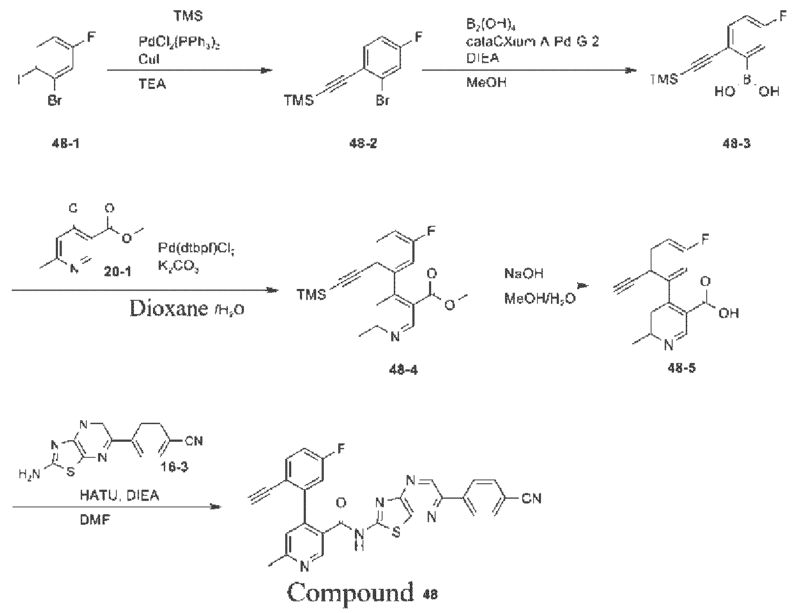


48-1 48-2 48-3 48-4 48-5 Compound 48

**Step 1: Synthesis of ((2-bromo-4-fluorophenyl)ethynyl)trimethylsilane (intermediate 48-2)**

At 25°C, **intermediate 48-1** (10 g), trimethylsilylacetylene (3.26 g), cuprous iodide (253.18 mg) and dichlorobis(triphenylphosphine)palladium (466.54 mg) were dissolved in triethylamine (90 mL), and the reaction solution was stirred and reacted at 40°C for 4 hours. After the reaction was completed, the organic phase was concentrated to dryness under reduced pressure, and ethyl acetate (200 mL) and water (100 mL) were added. The mixture was washed with water (45 mL*3), and the organic phase was dried over anhydrous sodium sulphate, and filtered. The filtrate was concentrated to dryness under reduced pressure, and the residue was purified by silica gel column chromatography (ISCO®; 120 g SepaFlash® flash silica gel column, gradient: 100% petroleum ether, flow rate: 50 mL/min) to obtain the title compound (6.89 g).

**$^1$H NMR (400MHz, DMSO-*d*$_6$)** δ = 7.48-7.41 (m, 2H), 7.40-7.33 (m, 1H), 0.28-0.20 (m, 9H).

**Step 2: Synthesis of (5-fluoro-2-((trimethylsilyl)ethynyl)phenyl)boronic acid (intermediate 48-3)**

At 20°C, **intermediate 48-2** (1 g), tetrahydroxydiboron (991.72 mg), chloro[(n-butylbis(1-adamantyl)phosphine)-2-(2-aminobiphenyl)]palladium

(246.54 mg) and *N,N*-diisopropylethylamine (2.38 g, 18.44 mmol) were dissolved in methanol (12 mL), and the reaction solution was stirred and reacted at 25°C for 2 hours. After the reaction was completed, to the reaction solution were added ethyl acetate (50 mL) and water (50 mL), and the mixture was extracted with ethyl acetate (25 mL*3). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: 0-10% ethyl acetate/petroleum ether, flow rate: 50 mL/min) to obtain the title compound (400 mg).

**$^1$H NMR (400MHz, DMSO-*d*$_6$)** δ = 7.58 (dd, *J* = 5.8, 8.6 Hz, 1H), 7.52-7.35 (m, 1H), 7.32-7.03 (m, 3H), 0.00 (s, 9H).

**Step 3: Synthesis of methyl 4-(5-fluoro-2-((trimethylsilyl)ethynyl)phenyl)-6-methylpyridine-3-carboxylate (compound 48-4)**

At 20°C, **intermediate 48-3** (400 mg), **intermediate 20-1** (314.43 mg), potassium carbonate (468.25 mg) and 1,1-bis(tert-butylphosphine)ferrocene palladium chloride (123.95 mg) were dissolved in dioxane (10 mL) and water (1 mL). The reaction solution was stirred and reacted at 90°C under nitrogen atmosphere for 2 hours. After the reaction was completed, the organic phase was concentrated to dryness under reduced pressure, and ethyl acetate (200 mL) and water (100 mL) were added. The mixture was washed with water (45 mL*3), and the organic phase was dried over anhydrous sodium sulphate, and filtered. The filtrate was concentrated to dryness under reduced pressure, and the residue was purified by silica gel column chromatography (ISCO®; 20 g SepaFlash® flash silica gel column, gradient: 0-10% ethyl acetate/petroleum ether, flow rate: 50 mL/min) to obtain the title compound (360 mg).

MS m/z (ESI): = 342.3 $[M+H]^+$

**Step 4: Synthesis of 4-(2-ethynyl-5-fluorophenyl)-6-methylpyridine-3-carboxylic acid (intermediate 48-5)**

At 25°C, **intermediate 48-4** (280 mg) and sodium hydroxide (98.40 mg) were dissolved in a solution of methanol (2 mL) and water (1 mL), and the reaction solution was stirred and reacted at 25°C for 2 hours. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (200 mg).

MS m/z (ESI): = 256.1 $[M+H]^+$.

**Step 5: Synthesis of *N*-(6-(4-cyanophenyl)thiazolo[4,5-b]pyrazin-2-yl)-4-(2-ethynyl-5-fluorophenyl)-6-methylpyridine-3-carboxamide (compound 48)**

At 20°C, **intermediate 48-5** (50 mg) and **intermediate 16-3** (49.62 mg) were dissolved in *N,N*-dimethylformamide (1 mL), and to the reaction solution were added HATU (74.48 mg) and *N,N*-diisopropylethylamine (50.63 mg). The reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 48%-68%, 11 minutes) to obtain the title compound (7.77 mg).

**$^1$H NMR (400MHz, DMSO-$d_6$)** $\delta$ = 13.67-13.54 (m, 1H), 9.36 (s, 1H), 9.00 (s, 1H), 8.38 (d, *J* = 8.3 Hz, 2H), 8.03 (s, 2H), 7.61 (dd, *J* = 5.9, 8.4 Hz, 1H), 7.43 (s, 1H), 7.37-7.29 (m, 2H), 4.00-3.98 (m, 1H), 2.63 (s, 3H).

MS m/z (ESI): = 491.1 $[M+H]^+$.

**Example 49, *N*-(6-(4-cyanophenyl)thiazolo[4,5-b]pyrazin-2-yl)-4-(2-ethynyl-6-fluorophenyl)-6-methylpyridine-3-carboxamide (compound 49)**

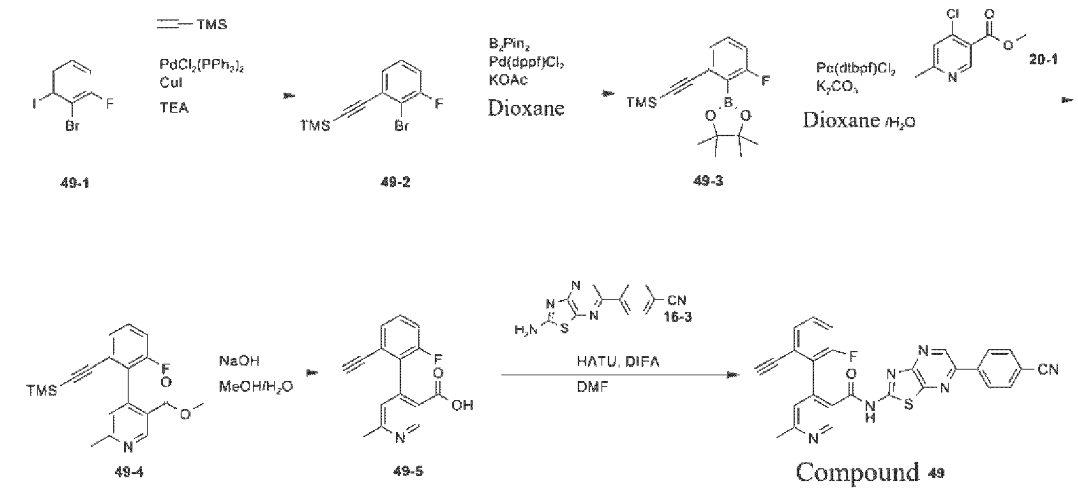

49-1 49-2 49-3

49-4 49-5 Compound 49

**Step 1: Synthesis of ((2-bromo-3-fluorophenyl)ethynyl)trimethylsilane (intermediate 49-2)**

At 25°C, **intermediate 49-1** (9 g) and trimethylsilylacetylene (6.00 g) were dissolved in triethylamine (60 mL), and cuprous iodide (284.83 mg) and dichlorobis(triphenylphosphine)palladium (2.10 g) were added. The reaction solution was stirred and reacted at 20°C for 1 hour. After the reaction was completed, the organic phase was concentrated to dryness under reduced pressure, and ethyl acetate (200 mL) and water (100 mL) were added. The mixture was washed with water (45 mL*3), and the organic phase was dried over anhydrous sodium sulphate, and filtered. The filtrate was concentrated to dryness under reduced pressure, and the residue was purified by silica gel chromatography (100% petroleum ether) to obtain the title compound (8 g).

**$^1$H NMR (400MHz, Methanol-$d_4$)** $\delta$ = 7.47-7.36 (m, 3H), 0.26 (s, 9H)

**Step 2: Synthesis of ((3-fluoro-2-(4,4,5,5-tetramethyl-1,3,2-dioxaborolan-2-yl)phenyl)ethynyl)trimethylsilane (intermediate 49-3)**

At 20°C, **intermediate 49-2** (800 mg), bis(pinacolato)diboron ($B_2Pin_2$) (1.12 g), 1,1-bis(diphenylphosphine) ferrocene palladium chloride (215.84 mg) and potassium acetate (579.01 mg) were dissolved in dioxane (40 mL), and the reaction solution was stirred and reacted at 90°C under nitrogen atmosphere for 2 hours. After the reaction was completed, the organic phase was concentrated to dryness under reduced pressure, and ethyl acetate (200 mL) and water (100 mL) were added. The mixture was washed with water (45 mL*3), and after liquid separation was performed, the organic phase was concentrated to dryness under reduced pressure to obtain the title compound (900 mg).

MS m/z (ESI): = 319.4 $[M+H]^+$.

**Step 3: Synthesis of methyl 4-(2-fluoro-6-((trimethylsilyl)ethynyl)phenyl)-6-methylpyridine-3-carboxylate (intermediate 49-4)**

At 20°C, **intermediate 49-3** (900 mg), **intermediate 20-1** (524.87 mg), potassium carbonate (781.65 mg) and 1,1-bis(tert-butylphosphine)ferrocene palladium chloride (184.30 mg) were dissolved in dioxane (10 mL) and water (2 mL). The reaction solution was stirred and reacted at 90°C under nitrogen atmosphere for 2 hours. After the reaction was completed, the organic phase was concentrated to dryness under reduced pressure, and ethyl acetate (200 mL) and water (100 mL) were added. The mixture was washed with water (45 mL*3), and the organic phase was dried over anhydrous sodium sulphate, and filtered. The filtrate was concentrated to dryness under reduced pressure, and the residue was purified by silica gel chromatography (petroleum ether/ethyl acetate = 3/1) to obtain the title compound (400 mg).

MS m/z (ESI): = 341.9 $[M+H]^+$.

**Step 4: Synthesis of 4-(2-ethynyl-6-fluorophenyl)-6-methylpyridine-3-carboxylic acid (intermediate 49-5)**

At 20°C, **intermediate 49-4** (300 mg) and sodium hydroxide (210.85 mg) were dissolved in a solution of methanol (4 mL) and water (2 mL), and the reaction solution was stirred and reacted at 25°C for 2 hours. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (220 mg).

MS m/z (ESI): = 256.4 $[M+H]^+$.

**Step 5: Synthesis of *N*-(6-(4-cyanophenyl)thiazolo[4,5-b]pyrazin-2-yl)-4-(2-ethynyl-6-fluorophenyl)-6-methylpyridine-3-carboxamide (compound 49)**

At 20°C, **intermediate 49-5** (70 mg) and **intermediate 16-3** (83.35 mg) were dissolved in *N,N*-dimethylformamide (1 mL), and to the reaction solution were added HATU (104.28 mg) and N,N-diisopropylethylamine (88.61 mg). The reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 45%-65%, 11 minutes) to obtain the title compound (23 mg).

**$^1$H NMR (400MHz, DMSO-$d_6$)** $\delta$ = 13.95-13.51 (m, 1H), 9.35 (s, 1H), 9.06 (s, 1H), 8.37 (d, *J* = 8.3 Hz, 2H), 8.01 (d, *J* = 8.3 Hz, 2H), 7.46 (s, 4H), 4.16 (s, 1H), 2.63 (s, 3H).

MS m/z (ESI): = 491.0 $[M+H]^+$.

**Example 50: *N*-(6-(4-cyanophenyl)thiazolo[4,5-*b*]pyrazin-2-yl)-2-(2-methoxyphenyl)pyridine-3-carboxamide (compound 50)**

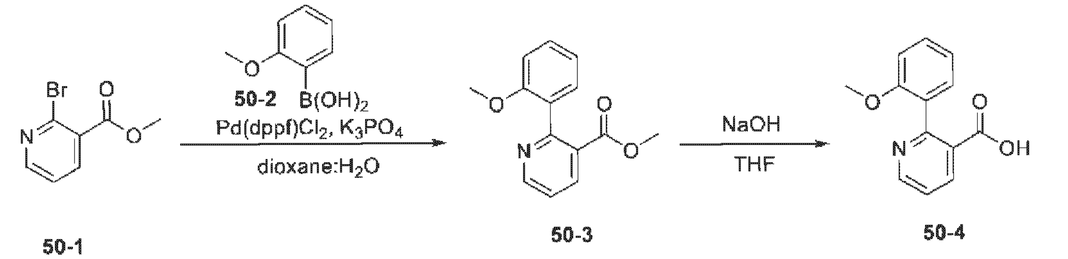


50-1 50-3 50-4

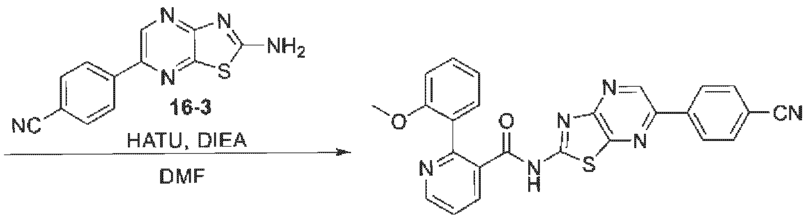


Compound 50

**Step 1: Synthesis of methyl 2-(2-methoxyphenyl)pyridine-3-carboxylate (intermediate 50-3)**

**Intermediate 50-1** (1.00 g), **intermediate 50-2** (1.41 g), $Pd(dppf)Cl_2$ (338 mg), and potassium phosphate (1.97 g) were added to dioxane (10 mL) and water (2 mL), and the reaction solution was stirred at 80°C for 4 hours. The reaction solution was cooled and then poured into water (20 mL), and the mixture was extracted with ethyl acetate (30 mL* 3). The organic layers were combined, washed with water (40 mL * 2) and saturated sodium chloride aqueous solution (40 mL), dried over anhydrous sodium sulphate, and filtered. The filtrate was concentrated under reduced pressure, and the residue was purified by silica gel column chromatography (petroleum ether/ethyl acetate =5/1) to obtain the title compound (1.08 g).
MS m/z(ESI): = 244.1 $[M+H]^+$.

**Step 2: Synthesis of 2-(2-methoxyphenyl)pyridine-3-carboxylic acid (intermediate 50-4)**

**Intermediate 50-3** (1.00 g) was dissolved in tetrahydrofuran (10 mL), and a 2 M sodium hydroxide aqueous solution (10 mL) was added. The reaction solution was stirred at 60°C for 18 hours. Under an ice-water bath, the mixture was adjusted to pH = 6 with a 1 M HCl aqueous solution, and extracted with ethyl acetate (30 mL*3). The organic layers were combined, washed with saturated sodium chloride aqueous solution (20 mL * 2), dried over anhydrous sodium sulphate, and filtered. The filtrate was concentrated under reduced pressure to obtain the title compound (550 mg).
MS m/z(ESI): = 230.2 $[M+H]^+$.

**Step 3: Synthesis of *N*-(6-(4-cyanophenyl)thiazolo[4,5-*b*]pyrazin-2-yl)-2-(2-methoxyphenyl)pyridine-3-carboxamide (compound 50)**

**Intermediate 50-4** (30 mg), HATU (49 mg), and DIEA (33 mg) were added to DMF (1 mL), and the reaction solution was stirred at 25°C for 1 hour. **Intermediate 16-3** (66 mg) was added, and the reaction solution was stirred at 25°C for 18 hours. The reaction solution was poured into water, and the mixture was extracted with ethyl acetate (5 mL * 3). The organic layers were combined, washed with saturated sodium chloride aqueous solution (10 mL* 2), dried over anhydrous sodium sulphate, and filtered. The filtrate was concentrated to dryness under reduced pressure. The residue was purified by preparative high performance liquid chromatography (chromatographic column: Gemini NX C18 3.5 μm*4.6*100 mm; mobile phase: A: 0.05% TFA v/v, B: acetonitrile; B%: 10%-90%, 10 min) to obtain the title compound (1 mg).
MS m/z(ESI): = 465.1 $[M+H]^+$.
**$^1$H NMR(400 MHz, DMSO-*d*$_6$)** δ 8.98(s, 1H), 8.66-8.60(m, 1H), 8.30-8.22(m, 3H), 7.94-7.89(m, 2H), 7.45-7.38(m, 2H), 7.34-7.28(m, 1H), 7.04-6.98(m, 1H), 6.93-6.88(m, 1H), 3.46(s, 3H).

**Example 51: *N*-(6-(4-cyanophenyl)thiazolo[4,5-*b*]pyrazin-2-yl)-3-(2-methoxyphenyl)pyridine-2-carboxamide (compound 51)**

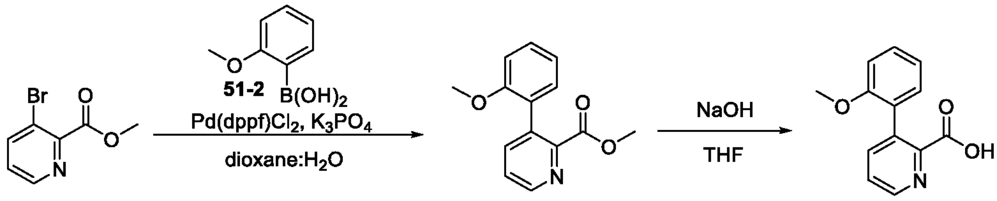


51-1 51-3 51-4

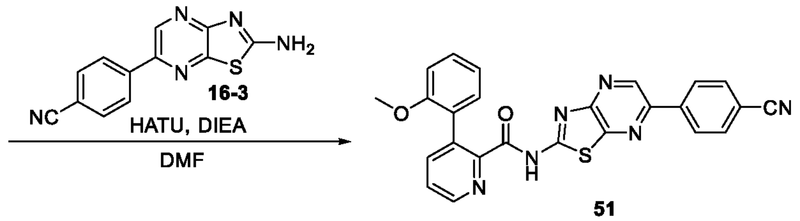


51

**Step 1: Synthesis of methyl 3-(2-methoxyphenyl)pyridine-2-carboxylate (intermediate 51-3)**

Under nitrogen atmosphere, **intermediate 51-1** (1.00 g), **intermediate 51-2** (1.41 g), Pd(dppf)$Cl_2$ (338 mg), and potassium phosphate (1.97 g) were added to dioxane (10 mL) and water (2 mL), and the mixture was stirred at 80°C for 4 h. The reaction solution was cooled and then poured into water (20 mL), and the mixture was extracted with ethyl acetate (30 mL * 3). The organic layers were combined, washed with water (40 mL * 2) and saturated sodium chloride aqueous solution (40 mL), dried over anhydrous sodium sulphate, and filtered. The filtrate was concentrated to dryness under reduced pressure, and the residue was purified by column chromatography (petroleum ether/ethyl acetate =5/1) to obtain the title compound (0.98 g).

MS m/z(ESI): = 244.1 $[M+H]^+$.

**Step 2: Synthesis of 3-(2-methoxyphenyl)pyridine-2-carboxylic acid (intermediate 51-4)**

**Intermediate 51-3** (1.00 g) was dissolved in tetrahydrofuran (10 mL), and a 2 M sodium hydroxide aqueous solution (10 mL) was added. The reaction solution was stirred at 60°C for 18 h. The reaction solution was concentrated to dryness under reduced pressure, adjusted to PH=6 with 1 M HCl hydrochloric acid under an ice-water bath, and extracted with ethyl acetate (30 mL * 3). The organic layers were combined, washed with saturated sodium chloride aqueous solution (20 mL * 2), dried over anhydrous sodium sulphate, and filtered. The filtrate was concentrated to dryness under reduced pressure to obtain the title compound (370 mg).

MS m/z(ESI): = 230.2 $[M+H]^+$.

**Step 3: Synthesis of *N*-(6-(4-cyanophenyl)thiazolo[4,5-*b*]pyrazin-2-yl)-3-(2-methoxyphenyl)pyridine-2-carboxamide (compound 51)**

**Intermediate 51-4** (20 mg), HATU (33 mg), and DIEA (22 mg) were added to DMF (1 mL), and the mixture was stirred at room temperature for 1 h, and then **intermediate 16-3** (44 mg) was added. The reaction solution was stirred at 25°C for 18 h. The reaction solution was poured into water (10 mL), and the mixture was extracted with ethyl acetate (5 mL * 3). The organic layers were combined, washed with saturated sodium chloride aqueous solution (10 mL * 2), dried over anhydrous sodium sulphate, and filtered. The filtrate was concentrated to dryness under reduced pressure. The residue was purified by preparative high performance liquid chromatography (chromatographic column: Gemini NX C18 3.5 μm*4.6*100 mm; mobile phase: A: 0.05% TFA v/v, B: acetonitrile; B%: 10%-90%, 10 min) to obtain the title compound (8 mg).

MS m/z(ESI): = 465.0 $[M+H]^+$.

**$^1$H NMR(400 MHz, DMSO-*$d_6$*)** $\delta$ = 9.31(s, 1H), 8.74-8.64(m, 1H), 8.44-8.31(m, 2H), 8.08-7.95(m, 2H), 7.95-7.84(m, 1H), 7.77-7.64(m, 1H), 7.46-7.29(m, 2H), 7.14-6.94(m, 2H), 3.57(s, 3H).

**Example 52: *N*-(6-(4-cyanophenyl)thiazolo[4,5-*b*]pyrazin-2-yl)-5-(2-methoxyphenyl)pyrimidine-4-carboxamide (compound 52)**

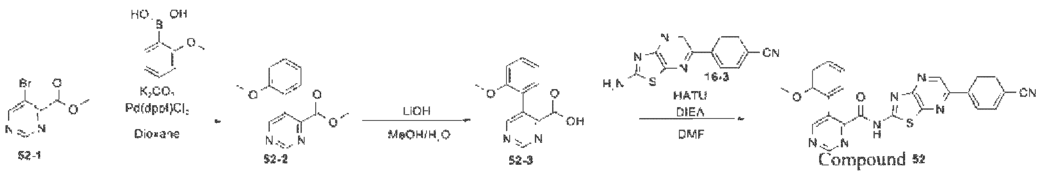


52-1 52-2 52-3 Compound 52

**Step 1: Synthesis of methyl 5-(2-methoxyphenyl)pyrimidine-4-carboxylate (intermediate 52-2)**

Under nitrogen atmosphere, **intermediate 52-1** (200 mg), 2-methoxyphenylboronic acid (323 mg), potassium carbonate (381 mg), and Pd(dppf)$Cl_2$ (67 mg) were dissolved in 1,4-dioxane (3 mL), and the mixture was stirred and reacted at 90°C for 2 h. The reaction solution was concentrated to dryness under reduced pressure, and the residue was purified by silica gel column chromatography (petroleum ether : ethyl acetate =5 : 1) to obtain the title compound (179 mg).
MS m/z(ESI): = 245.2 $[M+H]^+$.

**Step 2: Synthesis of 5-(2-methoxyphenyl)pyrimidine-4-carboxylic acid (intermediate 52-3)**

**Intermediate 52-2** (179 mg) was dissolved in methanol (3 mL), and a solution of lithium hydroxide (153 mg) in water (1 mL) was added. The mixture was stirred and reacted at room temperature for 5 h. The reaction solution was adjusted to pH=4 with dilute hydrochloric acid, with a solid precipitated. The reaction solution was filtered, and the filter cake was dried to obtain the title compound (90 mg).
MS m/z(ESI): = 229.2 $[M-H]^-$.

**Step 3: Synthesis of *N*-(6-(4-cyanophenyl)thiazolo[4,5-*b*]pyrazin-2-yl)-5-(2-methoxyphenyl)pyrimidine-4-carboxamide (compound 52)**

**Intermediate 52-3** (20 mg) was dissolved in *N,N*-dimethylformamide (2 mL), and HATU (33 mg) and DIEA (34 mg) were added. The mixture was stirred and reacted at room temperature for 0.5 h, and then **intermediate 16-3** (22 mg) was added. The resulting mixture was stirred at room temperature for 2 h. The reaction solution was poured into water (10 mL), and the mixture was extracted with ethyl acetate (10 mL*3). The ethyl acetate phases were combined, washed with saturated sodium chloride aqueous solution (50 mL), dried over anhydrous sodium sulphate, and filtered. The organic phase was concentrated to dryness under reduced pressure, and the residue was purified by preparative high performance liquid chromatography [YMC-Actus Triart C18 column, 5 um silica, 30 mm in diameter, 150 mm in length; using a decreasingly polar mixture of water (containing 0.225% formic acid) and acetonitrile as the eluent; acetonitrile gradient proportion: 40%-70%, elution time: 15 minutes] to obtain the title compound (20 mg).
MS m/z(ESI): = 466.1 $[M+H]^+$.
**$^1$H NMR(400 MHz, DMSO-*d*$_6$)** $\delta$ 13.72(s, 1H), 9.36(s, 2H), 9.01(s, 1H), 8.40-8.35(m, 2H), 8.04-8.00(m, 2H), 7.53-7.41(m, 2H), 7.14-7.10(m, 1H), 7.08-7.04(m, 1H), 3.57(s, 3H).

**Example 53: *N*-(6-(4-cyanophenyl)thiazolo[4,5-*b*]pyrazin-2-yl)-5-(2-methoxyphenyl)-2-(trifluoromethyl)pyridine-4-carboxamide (compound 53)**

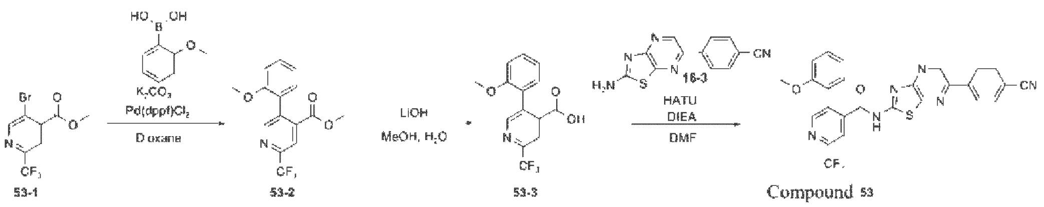


53-1 53-2 53-3 Compound 53

**Step 1: Synthesis of methyl 5-(2-methoxyphenyl)-2-(trifluoromethyl)pyridine-4-carboxylate (intermediate 53-2)**

Under nitrogen atmosphere, **intermediate 53-1** (200 mg), 2-methoxyphenylboronic acid (247 mg), $K_2CO_3$ (291 mg), and Pd(dppf)$Cl_2$ (51 mg) were dissolved in 1,4-dioxane (3 mL), and the mixture was stirred at 90°C for 3 h. The reaction solution was concentrated to dryness under reduced pressure, and the residue was purified by silica gel column chromatography (petroleum ether : ethyl acetate =5 : 1) to obtain the title compound (208 mg).
MS m/z(ESI): = 312.2 $[M+H]^+$.

**Step** 2: **Synthesis of 5-(2-methoxyphenyl)-2-(trifluoromethyl)pyridine-4-carboxylic acid (intermediate 53-3)**

**Intermediate 53-2** (200 mg) was dissolved in methanol (3 mL), and a lithium hydroxide (140 mg lithium hydroxide dissolved in 2 mL of water) solution was added. The mixture was stirred and reacted at room temperature for 5 h. The reaction solution was concentrated to dryness under reduced pressure, and the aqueous phase was adjusted to PH=3 with dilute hydrochloric acid, with a solid precipitated. The reaction solution was filtered, and the filter cake was dried to obtain the title compound (153 mg).
MS m/z(ESI): = 296.2 $[M-H]^-$.

**Step 3: Synthesis of *N*-(6-(4-cyanophenyl)thiazolo[4,5-*b*]pyrazin-2-yl)-5-(2-methoxyphenyl)-2-(trifluoromethyl) pyridine-4-carboxamide (compound 53)**

**Intermediate 53-3** (20 mg) was dissolved in *N,N*-dimethylformamide (2 mL), and HATU (38 mg) and DIEA (35 mg) were added. The mixture was stirred and reacted at room temperature for 0.5 h, and then **intermediate 16-3** (22 mg) was added. The resulting mixture was stirred and reacted at room temperature for 2 h. The reaction solution was poured into water (10 mL), and the mixture was extracted with ethyl acetate (10 mL*3). The organic phases were combined, washed with saturated sodium chloride aqueous solution (50 mL), dried over anhydrous sodium sulphate, and filtered. The filtrate was concentrated to dryness, and the residue was purified by preparative high performance liquid chromatography [YMC-Actus Triart C18 column, 5 um silica, 30 mm in diameter, 150 mm in length; using a decreasingly polar mixture of water (containing 0.225% formic acid) and acetonitrile as the eluent; acetonitrile gradient proportion: 40%-70%, elution time: 15 minutes] to obtain the title compound (25 mg).
MS m/z(ESI): = 531.1 $[M-H]^-$.
**$^1$H** NMR(400 MHz, **DMSO-$d_6$**) $\delta$ 13.71(s, 1H), 9.28(s, 1H), 8.83(s, 1H), 8.36(d, *J* = 8.2 Hz, 2H), 8.30(s, 1H), 8.00(d, *J* = 8.2 Hz, 2H), 7.55-7.45(m, 2H), 7.18-7.08(m, 1H), 7.04(d, *J* = 8.2 Hz, 1H), 3.55(s, 3H).

**Example 54: *N*-(6-(4-cyanophenyl)thiazolo[4,5-*b*]pyrazin-2-yl)-3-fluoro-5-(2-methoxyphenyl)pyridine-4-carboxamide (compound 54)**

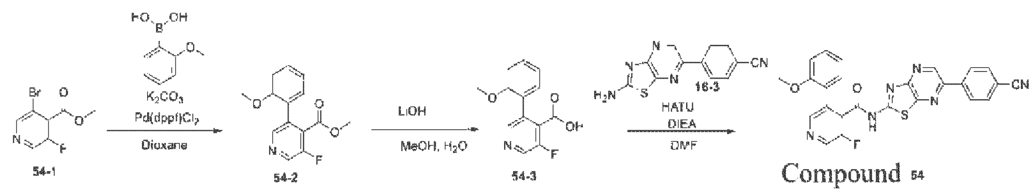


**Step** 1: **Synthesis of methyl 3-fluoro-5-(2-methoxyphenyl)pyridine-4-carboxylate (intermediate 54-2)**

**Intermediate 54-1** (200 mg), 2-methoxyphenylboronic acid (247 mg), potassium carbonate (352 mg), and Pd(dppf)$Cl_2$ (62 mg) were dissolved in 1,4-dioxane (3 mL), and the mixture was stirred at 90°C under nitrogen atmosphere for 3 h. The reaction solution was concentrated to dryness under reduced pressure, and the residue was purified by silica gel column chromatography (petroleum ether : ethyl acetate =5 : 1) to obtain the title compound (220 mg).
MS m/z(ESI): = 262.2 $[M+H]^+$.

**Step 2: Synthesis of 3-fluoro-5-(2-methoxyphenyl)pyridine-4-carboxylic acid (intermediate 54-3)**

**Intermediate 54-2** (210 mg) was dissolved in methanol (3 mL), and a solution of lithium hydroxide (170 mg) in water (1 mL) was added. The mixture was stirred and reacted at room temperature for 5 h. The reaction solution was concentrated to dryness under reduced pressure, and the aqueous phase was adjusted to pH=3 with dilute hydrochloric

acid, with a solid precipitated. The reaction solution was filtered, and the filter cake was dried to obtain the title compound (180 mg).

MS m/z(ESI): = 246.2 $[M-H]^-$.

**Step 3: Synthesis of *N*-(6-(4-cyanophenyl)thiazolo[4,5-*b*]pyrazin-2-yl)-3-fluoro-5-(2-methoxyphenyl)pyridine-4-carboxamide (compound 54)**

**Intermediate 54-3** (20 mg) was dissolved in *N,N*-dimethylformamide (2 mL), and HATU (45 mg) and DIEA (42 mg) were added. The mixture was stirred and reacted at room temperature for 0.5 h, and then **intermediate 16-3** (29 mg) was added. The resulting mixture was stirred and reacted at room temperature for 2 h. The reaction solution was poured into water (10 mL), and the mixture was extracted with ethyl acetate (10 mL*3). The ethyl acetate phases were combined, washed with saturated sodium chloride aqueous solution (50 mL), dried over anhydrous sodium sulphate, and filtered. The filtrate was concentrated to dryness under reduced pressure, and the residue was purified by preparative high performance liquid chromatography (YMC-Actus Triart C18 column, 5 um silica, 30 mm in diameter, 150 mm in length; using a decreasingly polar mixture of water (containing 0.225% formic acid) and acetonitrile as the eluent; acetonitrile gradient proportion: 40%-70%, elution time: 15 minutes) to obtain the title compound (15 mg).

MS m/z(ESI): = 483.1 $[M+H]^+$.

**$^1$H NMR(400 MHz, DMSO-$d_6$)** $\delta$ = 13.80(s, 1H), 9.22(s, 1H), 8.70(s, 1H), 8.48(s, 1H), 8.35(d, *J* = 8.1 Hz, 2H), 7.98(d, *J* = 8.1 Hz, 2H), 7.45-7.31(m, 2H), 7.10-6.95(m, 2H), 3.61(s, 3H).

**Example 55: *N*-(6-(4-cyanophenyl)thiazolo[4,5-*b*]pyrazin-2-yl)-2'-methoxy-[1,1'-biphenyl]-2-carboxamide (compound 55)**

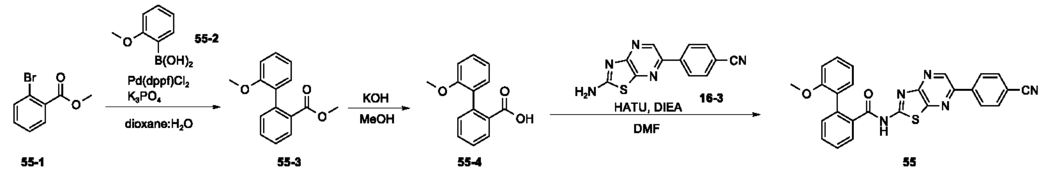


**Step 1: Synthesis of methyl 2'-methoxy-[1,1'-biphenyl]-2-carboxylate (intermediate 55-3)**

**Intermediate 55-1** (1 g), **intermediate 55-2** (1.42 g), $Pd(dppf)Cl_2$ (343 mg), and potassium phosphate (1.98 g) were added to dioxane (10 mL) and water (2 mL), and the reaction solution was stirred at 90°C under nitrogen atmosphere for 18 h. The reaction solution was cooled and then poured into water (20 mL), and the mixture was extracted with ethyl acetate (30 mL * 3). The organic layers were combined, washed with water (40 mL * 2) and saturated sodium chloride aqueous solution (40 mL), dried over anhydrous sodium sulphate, and filtered. The filtrate was concentrated under reduced pressure, and purified by column chromatography (petroleum ether/ethyl acetate =5/1) to obtain the title compound (1.0 g).

MS m/z(ESI): = 243.1 $[M+H]^+$.

**Step 2: Synthesis of 2'-methoxy-[1,1'-biphenyl]-2-carboxylic acid (intermediate 55-4)**

**Intermediate 55-3** (1.0 g) was dissolved in methanol (10 mL), and a solution of potassium hydroxide (3.0 g) in water (10 mL) was added. The reaction solution was stirred at 25°C for 18 h. The reaction solution was concentrated to dryness under reduced pressure, adjusted to pH=6 with a 30 mL 1 M HCl aqueous solution under an ice-water bath, and extracted with ethyl acetate (30 mL* 3). The organic layers were combined, washed with saturated sodium chloride aqueous solution (20 mL * 2), dried over anhydrous sodium sulphate, and filtered. The filtrate was concentrated to dryness under reduced pressure to obtain the title compound (900 mg).

MS m/z(ESI): = 229.1 $[M+H]^+$.

**Step 3: Synthesis of *N*-(6-(4-cyanophenyl)thiazolo[4,5-*b*]pyrazin-2-yl)-2'-methoxy-[1,1'-biphenyl]-2-carboxamide (compound 55)**

**Intermediate 55-4** (40 mg), HATU (66 mg), and DIEA (45 mg) were added to DMF (1 mL), and the reaction solution was stirred at 25°C for 2 h. **Intermediate 16-3** (44 mg) was added, and the reaction solution was stirred at 25°C for

18 h. The reaction solution was poured into water, and the mixture was extracted with ethyl acetate (5 mL * 3). The organic layers were combined, washed with saturated sodium chloride aqueous solution (10 mL * 2), dried over anhydrous sodium sulphate, and filtered. The filtrate was concentrated to dryness under reduced pressure. The residue was purified by preparative high performance liquid chromatography (chromatographic column: Gemini NX C18 3.5 μm*4.6*100 mm; mobile phase: A: 0.05% TFA v/v, B: acetonitrile; B%: 10%-90%, 10 min) to obtain the title compound (11.9 mg).

MS m/z (ESI): 464.1 $[M+H]^+$.

**$^1$H NMR (400 MHz, DMSO-*d*$_6$)** δ 13.24 (s, 1H), 9.33 (s, 1H), 8.41-8.34 (m, 2H), 8.05-7.98 (m, 2H), 7.80-7.73 (m, 1H), 7.71-7.62 (m, 1H), 7.58-7.49 (m, 1H), 7.46-7.39 (m, 1H), 7.39-7.29 (m, 2H), 7.11-7.03 (m, 1H), 6.98-6.91 (m, 1H), 3.51 (s, 3H).

**Example 56: *N*-(6-(4-cyanophenyl)thiazolo[4,5-*b*]pyrazin-2-yl)-6-(2-methoxyphenyl)benzo[*d*][1,3]dioxolyl-5-carboxamide (compound 56)**

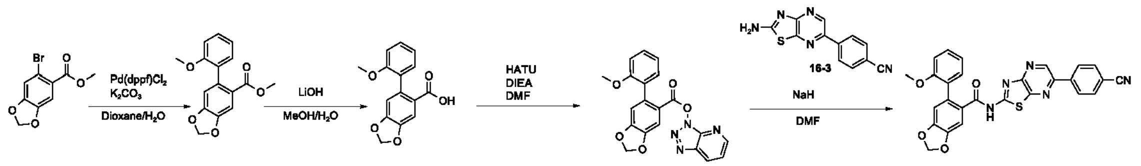


56-1 56-2 56-3 56-4 Compound 56

**Step** 1: **Synthesis of methyl 6-(2-methoxyphenyl)benzo[*d*][1,3]dioxolyl-5-carboxylate (intermediate 56-2)**

Under nitrogen atmosphere, **intermediate 56-1** (400 mg), o-methoxyphenylboronic acid (280 mg), Pd(dppf)$Cl_2$ (400 mg), and potassium carbonate (426 mg) were dissolved in dioxane (5 mL) and water (1 mL), and the mixture was stirred at 100°C for 3 h. The reaction solution was cooled to room temperature, diluted by adding water (5 mL), and extracted with ethyl acetate (10 mL*3). The organic phase was dried over anhydrous sodium sulphate, and filtered. The filtrate was concentrated to dryness under reduced pressure. The residue was purified by silica gel column chromatography (petroleum ether : ethyl acetate =2 : 1) to obtain the title compound (200 mg).

MS m/z(ESI): = 287.1 $[M+H]^+$

**Step 2: Synthesis of 6-(2-methoxyphenyl)benzo[*d*][1,3]dioxolyl-5-carboxylic acid (intermediate 56-3)**

**Intermediate 56-2** (100 mg) was dissolved in MeOH (1 mL) and water (0.2 mL), and lithium hydroxide (42 mg) was added. The mixture was stirred at 60°C for 8 h. To the reaction solution was dropwise added hydrochloric acid (2 M) to adjust pH to 6, and the mixture was extracted with ethyl acetate (5 mL*2). The organic phases were combined, and dried over anhydrous sodium sulphate. After filtration, the organic phase was concentrated to dryness under reduced pressure to obtain the title compound (70 mg).

MS m/z(ESI): = 271.1 $[M-H]^-$

**Step 3: Synthesis of 3*H*-[1,2,3]triazolo[4,5-*b*]pyridin-3-yl6-(2-methoxyphenyl)benzo[*d*][1,3]dioxolyl-5-carboxylate (intermediate 56-4)**

**Intermediate 56-3** (65 mg) was dissolved in DMF (1 mL), and DIEA (62 mg) and HATU (99 mg) were added. The mixture was stirred at 20°C for 8 h. The reaction solution was diluted with DMF (1 mL), and then purified by preparative high performance liquid chromatography (chromatographic column: Gemini NX C18 5 μm*10*150 mm; mobile phase: A: water (0.225% formic acid v/v), B: acetonitrile; B%: 30%-50%, 11 min) to obtain the title compound (20 mg).

MS m/z(ESI): = 391.1 $[M+H]^+$.

**Step 4: Synthesis of *N*-(6-(4-cyanophenyl)thiazolo[4,5-*b*]pyrazin-2-yl)-6-(2-methoxyphenyl)benzo[*d*][1,3]dioxolyl-5-carboxamide (compound 56)**

**Intermediate 56-4** (17.0 mg) and **intermediate 16-3** (11.0 mg) were dissolved in DMF (0.5 mL), and NaH (3.5 mg, 60% wt) was added at 0°C. The reaction solution was stirred at 25°C for 1 h. The reaction solution was quenched by adding saturated ammonium chloride (5 mL), and extracted with ethyl acetate (5 mL*2). The organic phases were combined, and dried over anhydrous sodium sulphate. After filtration, the organic phase was concentrated to dryness under reduced pressure, and the residue was purified by preparative high performance liquid chromatography (chromatographic column: Gemini NX C18 5 μm*10*150 mm; mobile phase: A: water (0.225% formic acid v/v), B: acetonitrile; B%: 30%-50%, 11 min) to obtain the title compound (8.0 mg).

MS m/z(ESI): 508.1 $[M+H]^+$.

**$^1$H NMR(400 MHz, DMSO-$d_6$)** δ 13.06(s, 1H), 9.32(s, 1H), 8.42-8.33(m, 2H), 8.07-7.96(m, 2H), 7.39-7.24(m, 3H), 7.04 -7.00(m, 1H), 6.99-6.88(m, 2H), 6.18(s, 2H), 3.51(s, 3H).

**Example 57: *N*-(6-(4-cyanophenyl)thiazolo[4,5-*b*]pyrazin-2-yl)-6-(2-methoxyphenyl)-[1,2,4]triazolo[1,5-*a*]pyridine-7-carboxamide (compound 57)**

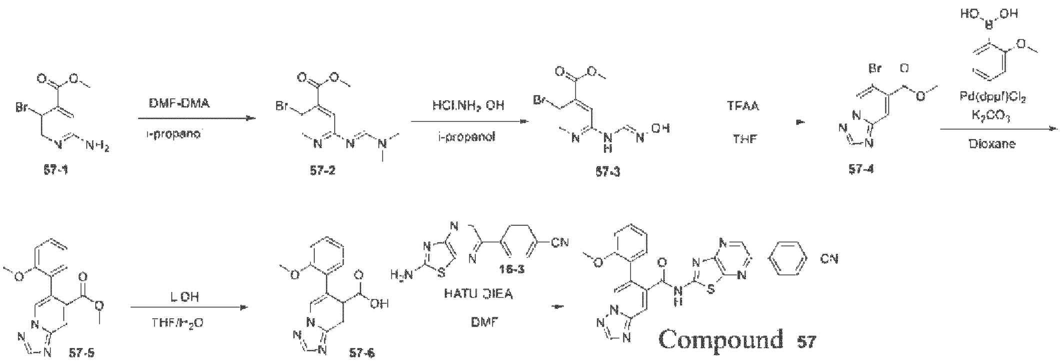


57-1 57-2 57-3 57-4 57-5 57-6 Compound 57

**Step 1: Synthesis of methyl (*E*)-5-bromo-2-(((dimethylamino)methylene)amino)pyridine-4-carboxylate (intermediate 57-2)**

**Intermediate 57-1** (1.3 g) was dissolved in isopropanol (20 mL), and DMF-DMA (2.0 g, 5 mL) was added. After the addition, the mixture was stirred and reacted at 80°C for 3 h. The reaction solution was concentrated to dryness under reduced pressure to obtain the title compound (1.6 g).

MS m/z(ESI): = 286.1$[M+H]^+$.

**Step 2: Synthesis of methyl (*E*)-5-bromo-2-(*N'*-hydroxyformyliminoamino)pyridine-4-carboxylate (intermediate 57-3)**

Hydroxylamine hydrochloride (606 mg) and **intermediate 57-2** (1.20 g) were dissolved in isopropanol (20 mL), and the mixture was stirred and reacted at 50°C for 16 h. The reaction solution was concentrated to dryness under reduced pressure, and the residue was slurried with ethyl acetate (10 mL), and filtered. The filter cake was dried to obtain the title compound (710 mg).

MS m/z(ESI): = 274.1 $[M+H]^+$.

**Step 3: Synthesis of methyl 6-bromo-[1,2,4]triazolo[1,5-*a*]pyridine-7-carboxylate (intermediate 57-4)**

**Intermediate 57-3** (550 mg) was dissolved in tetrahydrofuran (15 mL), and trifluoroacetic anhydride (463 mg) was dropwise added at 0°C. The reaction solution was stirred at 40°C for 4 h. The reaction solution was concentrated to dryness under reduced pressure, and the residue was purified by silica gel column chromatography (petroleum ether : ethyl acetate =10 : 1) to obtain the title compound (200 mg).

MS m/z(ESI): = 256.1$[M+H]^+$.

**Step 4: Synthesis of methyl 6-(2-methoxyphenyl)-[1,2,4]triazolo[1,5-*a*]pyridine-7-carboxylate (intermediate 57-5)**

Under nitrogen atmosphere, to 2-methoxyphenylboronic acid (178 mg) in 1,4-dioxane (3 mL) were successively added **intermediate 57-4** (150 mg), $K_2CO_3$ (242 mg) and Pd(dppf)$Cl_2$ (42 mg), and the mixture was stirred at 100°C for 3 h. The reaction solution was poured into water (10 mL), and the mixture was extracted with ethyl acetate (10 mL*3). The organic phase was washed with saturated sodium chloride aqueous solution (50 mL), dried over anhydrous sodium sulphate, and filtered. The filtrate was concentrated to dryness under reduced pressure, and the residue was purified by column chromatography (petroleum ether : ethyl acetate =10 : 1) to obtain the title compound (150 mg).

MS m/z(ESI): 284.2[M+H]+.

**Step 5: Synthesis of 6-(2-methoxyphenyl)-[1,2,4]triazolo[1,5-*a*]pyridine-7-carboxylic acid (intermediate 57-6)**

**Intermediate 57-5** (120 mg) was dissolved in tetrahydrofuran (3 mL), and a solution of lithium hydroxide (30 mg) in water (1 mL) was added. The mixture was stirred and reacted at room temperature for 3 h. The reaction solution was concentrated to dryness under reduced pressure, and the aqueous phase was adjusted to pH=4 with dilute hydrochloric acid, with a solid precipitated. The reaction solution was filtered, and the filter cake was dried to obtain the title compound (75 mg).
MS m/z(ESI): = 270.2 [M+H]+.

**Step 6: Synthesis of *N*-(6-(4-cyanophenyl)thiazolo[4,5-*b*]pyrazin-2-yl)-6-(2-methoxyphenyl)-[1,2,4]triazolo[1,5-*a*]pyridine-7-carboxamide (compound 57)**

**Intermediate 57-6** (60 mg) was dissolved in *N,N*-dimethylformamide (2 mL), and HATU (126 mg) and DIEA (58 mg) were added. The mixture was stirred and reacted at room temperature for 0.5 h, and then **intermediate 16-3** (84 mg) was added. After the addition, the resulting mixture was stirred and reacted at room temperature for 2 h. After the reaction was completed, the reaction solution was poured into water (10 mL), and the mixture was extracted with ethyl acetate (10 mL*3). The ethyl acetate phases were combined, washed with saturated sodium chloride aqueous solution (50 mL), dried over anhydrous sodium sulphate, and filtered. The filtrate was concentrated to dryness under reduced pressure, and the residue was purified by preparative high performance liquid chromatography (YMC-Actus Triart C18 column, 5 um silica, 30 mm in diameter, 150 mm in length; using a decreasingly polar mixture of water (containing 0.225% formic acid) and acetonitrile as the eluent; acetonitrile gradient proportion: 40%-70%, elution time: 15 minutes) to obtain the title compound (11 mg).
MS m/z(ESI): = 505.1 [M+H]+.
**$^1$H NMR(400 MHz, DMSO-*d*$_6$)** δ 13.67(s, 1H), 9.37(s, 1H), 9.05(s, 1H), 8.70(s, 1H), 8.43-8.36(m, 2H), 8.34(s, 1H), 8.02(d, *J* = 8.4 Hz, 2H), 7.55-7.49(m, 1H), 7.45-7.35(m, 1H), 7.18-7.08(m, 1H), 6.99(d, *J* = 8.2 Hz, 1H), 3.52(s, 3H).

**Example 58: 4-(5-cyano-2-methoxyphenyl)-*N*-(6-(4-cyanophenyl)thiazolo [4,5-*b*]pyrazin-2-yl)-6-methylpyridine-3-carboxamide (compound 58)**

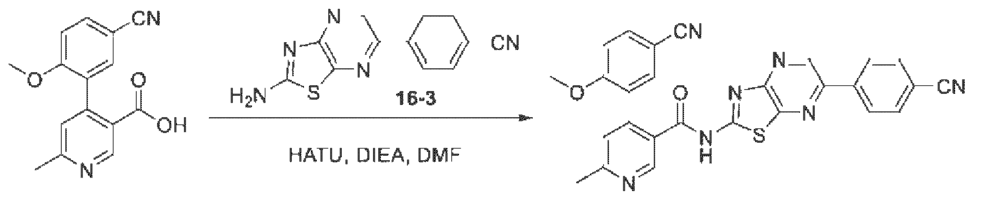


22-3

Compound 58

**Intermediate 22-3** (53.65 mg) was dissolved in DMF (3 mL), and HATU (98.1 mg) and DIEA (77.53 mg) were added. The reaction solution was stirred at room temperature for 1 hour, and then **intermediate 16-3** (101.3 mg) was added. The mixture was stirred at room temperature for additional 2 hours. To the reaction solution was added water (20 mL), and the mixture was extracted with ethyl acetate (15 mL). The organic phase was dried over anhydrous sodium sulphate. After filtration, the filtrate was concentrated to dryness under reduced pressure, and slurried with methanol to obtain the title compound (20 mg).
MS m/z(ESI): = 504.1 [M+H]+.
**$^1$H NMR(400 MHz, DMSO-*d*$_6$)** δ 9.34(s, 1H), 8.85(s, 1H), 8.42-8.36(m, 2H), 8.06-7.99(m, 2H), 7.96-7.89(m, 2H), 7.45(s, 1H), 7.22-7.14(m, 1H), 3.61(s, 3H), 2.61(s, 3H)

**Example 59, *N*-(6-(4-cyanophenyl)thiazolo[4,5-*b*]pyrazin-2-yl)-4-(2-ethynylphenyl)-2-methylpyrimidine-5-carboxamide (compound 59)**

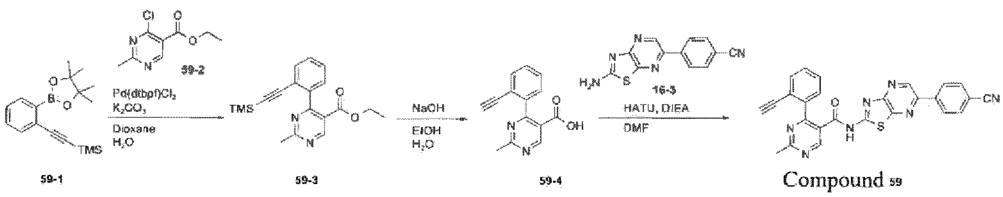


**Step 1: Synthesis of ethyl 2-methyl-4-(2-((trimethylsilyl)ethynyl) phenyl)pyrimidin-5-carboxylate (intermediate 59-3)**

Under nitrogen atmosphere, **intermediate 59-1** (1.0 g) was dissolved in dioxane (10 mL), and **intermediate 59-2** (0.87 g), potassium carbonate (0.92 g), water (1 mL) and dichloro[1,1'-bis(di-tert-butylphosphine)ferrocene palladium (II)
(0.22 g) were added. After the addition, the mixture was warmed to 70°C and stirred for 2 h. The reaction solution was filtered, ethyl acetate (10 mL) was added, and the mixture was extracted with water (10 mL). The organic phase was washed with saturated sodium chloride aqueous solution (300 mL), dried over anhydrous sodium sulphate, and filtered. The filtrate was concentrated to dryness under reduced pressure, and the residue was purified by silica gel column chromatography (petroleum ether/ethyl acetate =10/1) to obtain the title compound (380 mg).
MS m/z(ESI): = 339.1 $[M+H]^+$.

**Step 2: Synthesis of 4-(2-ethynylphenyl)-2-methylpyrimidine-5-carboxylic acid (intermediate 59-4)**

**Intermediate 59-3** (100 mg) was dissolved in ethanol (1 mL), and sodium hydroxide (23.6 mg) and water (0.3 mL) were added. The mixture was warmed to 60°C and stirred for 12 h. The reaction solution was concentrated to dryness under reduced pressure, dissolved in water (60 mL), adjusted to pH 4 with 1 M HCl, and filtered. The filter cake was dried to obtain the title compound (20.0 mg).
MS m/z(ESI): = 239.1 $[M+H]^-$.

**Step 3: Synthesis of *N*-(6-(4-cyanophenyl)thiazolo[4,5-*b*]pyrazin-2-yl)-4-(2-ethynylphenyl)-2-methylpyrimidine-5-carboxamide (compound 59)**

**Intermediate 59-4** (12.0 mg) was dissolved in anhydrous *N,N*-dimethylformamide (1 mL), and HATU (19.0 mg) and DIEA (13.0 mg) were added. After the addition, the mixture was stirred at room temperature for 2 h, and **intermediate 16-3** (21.3 mg) was added. After the addition, the resulting mixture was stirred at room temperature for 12 h. The reaction solution was purified by preparative high-pressure liquid chromatography (YMC-Actus Triart C18 column, 5 um silica, 30 mm in diameter, 150 mm in length; using a decreasingly polar mixture of water (containing 0.05% $NH_4HCO_3$) and acetonitrile as the eluent; acetonitrile gradient proportion: 55%-80%, elution time: 13 minutes) to obtain the title compound (4.0 mg).
MS m/z(ESI): = 474.0 $[M+H]^+$.
**$^1$H NMR(400 MHz, DMSO-*d*$_6$)** δ 9.31(s, 1H), 9.23(s, 1H), 8.38-8.36(m, 2H), 8.02-7.99(m, 2H), 7.57-7.50(m, 4H), 4.02(s, 1H), 2.77(s, 3H).

**Example 60: *N*-(6-(4-cyanophenyl)thiazolo[4,5-*b*]pyridin-2-yl)-4-(2-ethynylphenyl)-6-methylpyridine-3-carboxamide (compound 60)**

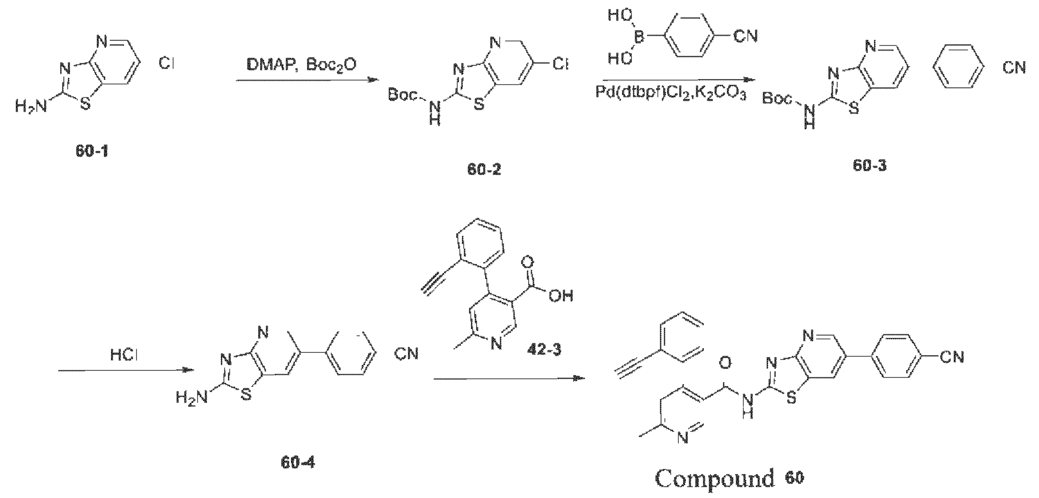


60-1 60-2 60-3 60-4 42-3 Compound 60

**Step 1: Synthesis of tert-butyl (6-chlorothiazolo[4,5-*b*]pyridin-2-yl)carbamate (intermediate 60-2)**

**Intermediate 60-1** (100 mg), $Boc_2O$ (129 mg) and DMAP (13 mg) were dissolved in DCM (3 mL), and the mixture was stirred at 20°C for 1 hour. To the reaction solution were added ethyl acetate (10 mL) and water (20 mL). The organic phase was washed with water (10 mL*2), dried over anhydrous sodium sulphate, and filtered. The filtrate was concentrated to dryness under reduced pressure. The residue was purified by silica gel column chromatography (petroleum ether/ethyl acetate =1/1) to obtain the title compound (130 mg).

MS m/z(ESI): = 286.1 $[M+H]^+$.

**Step 2: Synthesis of tert-butyl (6-(4-cyanophenyl)thiazolo[4,5-*b*]pyridin-2-yl)carbamate (intermediate 60-3)**

Under nitrogen atmosphere, **intermediate 60-2** (120 mg), $Pd(dtbpf)Cl_2$ (57 mg), potassium carbonate (174 mg) and 4-cyanophenylboronic acid (123 mg) were dissolved in dioxane (2 mL) and water (0.5 mL), and the mixture was warmed to 70°C and stirred for 16 h. The reaction solution was cooled to room temperature, quenched by adding water (20 mL), and extracted with ethyl acetate (20 mL*3). The organic phases were combined, dried over anhydrous sodium sulphate, and filtered. The filtrate was concentrated to dryness under reduced pressure, and the residue was purified by silica gel column chromatography (EA/PE=(10:1)) to obtain the title compound (90 mg).

MS m/z(ESI): = 353.2 $[M+H]^+$.

**Step 3: Synthesis of 4-(2-aminothiazolo[4,5-*b*]pyridin-6-yl)benzonitrile (intermediate 60-4)**

**Intermediate 60-3** (80 mg) was dissolved in hydrogen chloride in ethyl acetate (4 M, 5 mL), and the mixture was stirred at room temperature for 1 h. The reaction solution was concentrated to dryness under reduced pressure, and the residue was purified by silica gel column chromatography (DCM/MeOH = 10 : 1) to obtain the title compound (45 mg).

MS m/z(ESI): = 253.0 $[M+H]^+$.

**Step 4: Synthesis of *N*-(6-(4-cyanophenyl)thiazolo[4,5-*b*]pyridin-2-yl)-4-(2-ethynylphenyl)-6-methylpyridine-3-carboxamide (compound 60)**

**Intermediate 60-4** (30 mg), **intermediate 42-3** (28 mg), DIEA (31 mg) and HATU (40 mg) were dissolved in DMF (1 mL), and the mixture was warmed to 80°C and stirred for 16 h. The reaction solution was cooled to room temperature, quenched by adding water (10 mL), and extracted with ethyl acetate (10 mL* 3). The organic phases were combined, dried over anhydrous sodium sulphate, and filtered. The filtrate was concentrated to dryness under reduced pressure, and the residue was purified by preparative high performance liquid chromatography [YMC-Actus Triart C18 column, 5 um silica, 30 mm in diameter, 150 mm in length; using a decreasingly polar mixture of water (containing 0.05% *N*H4HCO3) and acetonitrile as the eluent; acetonitrile gradient proportion: 30%-80%, elution time: 20 minutes] to obtain the title compound (3.8 mg).

MS m/z(ESI): = 472.0 $[M+H]^+$.

**$^1$H** NMR(400 MHz, **DMSO-*d*$_6$**)δ 13.32(s, 1H), 8.98(s, 1H), 8.90(s, 1H), 8.73(s, 1H), 8.05-7.90(m, 4H), 7.57-7.51(m, 1H), 7.51-7.46(m, 1H), 7.45-7.38(m, 1H), 7.37-7.30(m, 2H), 3.97(s, 1H), 2.59(s, 3H).

**Example 61, *N*-(6-(5-cyanopyridin-2-yl)thiazolo[4,5-b]pyrazin-2-yl)-2'-chloro-5'-methoxy-6-methyl-[4,4'-bipyridine]-3-carboxamide (compound 61)**

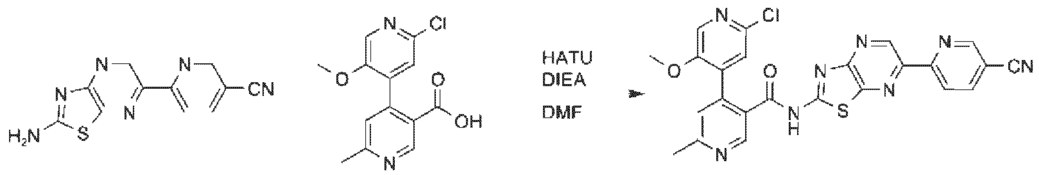


26-3 20-4 Compound 61

At 20°C, intermediate **26-3** (100 mg) was dissolved in *N,N*-dimethylformamide (5 mL), and to the reaction solution were added **intermediate 20-4** (76.72 mg), HATU (104.68 mg) and N,N-diisopropylethylamine (71.16 mg). The reaction solution was stirred and reacted at 45°C for 2 hours. After the reaction was completed, the reaction solution was purified by high performance liquid chromatography (chromatographic column: Boston Prime C18 150*30 mm*5 um; mobile phase: A: water (0.05% ammonia water), B: acetonitrile; B%: 20%-40%, 11 minutes) to obtain the title compound (6.2 mg).

**$^1$H NMR (400 MHz, DMSO-*d*$_6$)** $\delta$ = 13.85-13.59 (m, 1H), 9.57 (s, 1H), 9.18 (s, 1H), 8.93 (s, 1H), 8.55-8.51 (m, 1H), 8.50-8.46 (m, 1H), 8.18 (s, 1H), 7.61 (s, 1H), 7.48 (s, 1H), 3.64 (s, 3H), 2.62 (s, 3H)

MS m/z (ESI): = 515.1 $[M+H]^+$.

**Example 62, *N*-(6-(5-chloropyridin-2-yl)thiazolo[4,5-b]pyrazin-2-yl)-4-(5-cyano-2-methoxyphenyl)-6-methylpyridine-3-carboxamide (compound 62)**

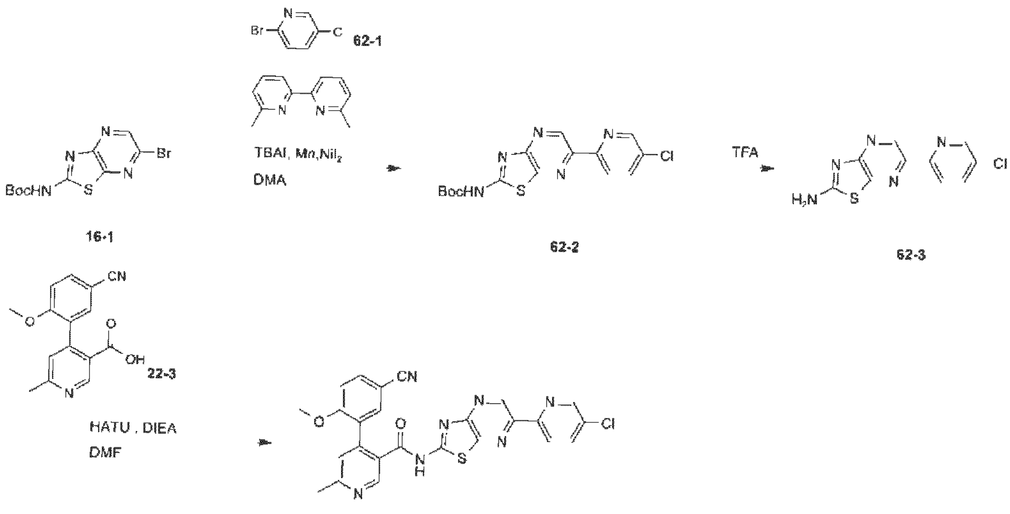


62-1 16-1 62-2 62-3 22-3

Compound 62

**Step 1: Synthesis of tert-butyl (6-(5-chloropyridin-2-yl)thiazolo[4,5-b]pyrazin-2-yl)carbamate (intermediate 62-2)**

At 25°C, **intermediate 16-1** (3 g), intermediate **62-1** (1.74 g), 6,6'-dimethyl-2,2'-bipyridine (166.89 mg), tetrabutylammonium iodide (5.02 g), manganese powder (1.99 g), and nickel iodide (283.07 mg) were dissolved in *N,N*-dimethylacetamide (150 mL), and the reaction solution was stirred at 100°C under nitrogen atmosphere for 16 hours. After the reaction was completed, to the reaction solution were added dichloromethane (150 mL) and methanol (20 mL). After filtration, the organic phase was concentrated to dryness under reduced pressure, and to the residue was added water (200 mL). The mixture was filtered, and the filter cake was dried to obtain the title compound (1 g).

MS m/z (ESI): = 363.9 $[M+H]^+$.

**Step 2: Synthesis of 6-(5-chloropyridin-2-yl)thiazolo[4,5-*b*]pyrazin-2-amine (intermediate 62-3)**

At 20°C, **intermediate 62-2** (1 g) was dissolved in dichloromethane (10 mL), and then to the reaction solution was added trifluoroacetic acid (2.04 mL). The reaction solution was stirred and reacted at 25°C for 2 hours. After the reaction was completed, the reaction solution was concentrated to dryness under reduced pressure. A saturated sodium bicarbonate solution was added to the residue to neutralize the remaining trifluoroacetic acid, and the mixture was filtered to obtain the title compound (700 mg).
MS m/z (ESI): = 264.1 $[M+H]^+$.

**Step 3: Synthesis of *N*-(6-(5-chloropyridin-2-yl)thiazolo[4,5-b]pyrazin-2-yl)-4-(5-cyano-2-methoxyphenyl)-6-methylpyridine-3-carboxamide (compound 62)**

At 20°C, **intermediate 22-3** (100 mg) was dissolved in *N,N* dimethylformamide (1.5 mL), and to the reaction solution were added HATU (141.74 mg), *N,N* diisopropylethylamine (144.53 mg) and **intermediate 62-3** (117.96 mg). The reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase; mobile phase: A: water (0.05% ammonia water), B: acetonitrile; B%: 45%-65%, 11 minutes) to obtain the title compound (30 mg).
**$^1$H NMR (400 MHz, DMSO-*d*$_6$)** δ = 13.61-13.43 (m, 1H), 9.52 (s, 1H), 8.84 (s, 1H), 8.82-8.78 (m, 1H), 8.42-8.37 (m, 1H), 8.15-8.11 (m, 1H), 7.96-7.91 (m, 2H), 7.49-7.46 (m, 1H), 7.22-7.16 (m, 1H), 3.61 (s, 3H), 2.62 (s, 3H)
MS m/z (ESI): = 514.1 $[M+H]^+$

**Example 63, *N*-(6-(4-chlorophenyl)thiazolo[4,5-*b*]pyrazin-2-yl)-2'-cyano-5'-methoxy-6-methyl-[4,4'-bipyridine]-3-carboxamide (compound 63)**

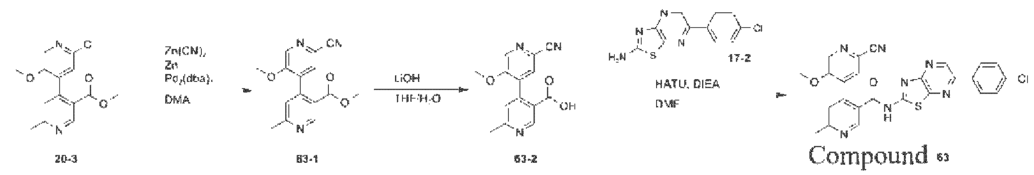


20-3 63-1 63-2 Compound 63

**Step 1: Synthesis of methyl 2'-cyano-5'-methoxy-6-methyl-[4,4'-bipyridine]-3-carboxylate (intermediate 63-1)**

At 20°C, **intermediate 20-3** (400 mg) was dissolved in *N,N*-dimethylacetamide (5 mL), and to the reaction solution were added zinc cyanide (208.60 mg), zinc powder (17.87 mg), tris(dibenzylideneacetone)dipalladium (37.54 mg) and 1,1-bis(diphenylphosphino)ferrocene (37.88 mg). Under nitrogen atmosphere, the reaction solution was stirred and reacted at 110°C for 1 hour. After the reaction was completed, to the reaction solution was added water (30 mL), and the mixture was extracted with ethyl acetate (20 mL*3). The organic phases were combined, and dried over anhydrous sodium sulphate. After filtration, the organic phase was concentrated to dryness under reduced pressure. The residue was purified by column chromatography (petroleum ether/ethyl acetate =1/1) to obtain the title compound (380 mg).
MS m/z (ESI): = 284.2 $[M+H]^+$.

**Step 2: Synthesis of 2'-cyano-5'-methoxy-6-methyl-[4,4'-bipyridine]-3-carboxylic acid (intermediate 63-2)**

At 20°C, **intermediate 63-1** (380 mg) was dissolved in a solution of tetrahydrofuran (3 mL)/water (3 mL), and to the reaction solution was added lithium hydroxide (64.25 mg). The reaction solution was stirred and reacted at 20°C for 4 hours. After the reaction was completed, the reaction solution was adjusted to pH 4 with dilute hydrochloric acid, and water (5 mL) was added. The mixture was extracted with ethyl acetate (10 mL*3). The organic phases were combined, and concentrated to dryness under reduced pressure, to obtain the title compound (350 mg).
MS m/z (ESI): = 270.1 $[M+H]^+$.

**Step 3: Synthesis of *N*-(6-(4-chlorophenyl)thiazolo[4,5-b]pyrazin-2-yl)-2'-cyano-5'-methoxy-6-methyl-[4,4'-bipyridine]-3-carboxamide (compound 63)**

At 20°C, **intermediate 63-2** (150 mg) was dissolved in *N,N*-dimethylformamide (3 mL), and to the reaction solution were added **intermediate 17-2** (73.18 mg), HATU (211.82 mg) and N,N-diisopropylethylamine (72.00 mg). The reaction solution was stirred and reacted at 45°C for 4 hours. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 53%-73%, 11 minutes) to obtain the title compound (65.5 mg).

**$^1$H NMR (400 MHz, DMSO-$d_6$)** δ = 13.55 (s, 1H), 9.27 (s, 1H), 8.93 (s, 1H), 8.58 (s, 1H), 8.25-8.17 (m, 3H), 7.62 (d, *J* = 8.4 Hz, 2H), 7.56-7.51 (m, 1H), 3.76 (s, 3H), 2.64 (s, 3H)

MS m/z (ESI): = 514.0 [M+H]$^+$.

**Example 64, *N*-(6-(4-cyanophenyl)thiazolo[4,5-b]pyrazin-2-yl)-5'-ethynyl-2',6-dimethyl-[4,4'-bipyridine]-3-carboxamide (compound 64)**

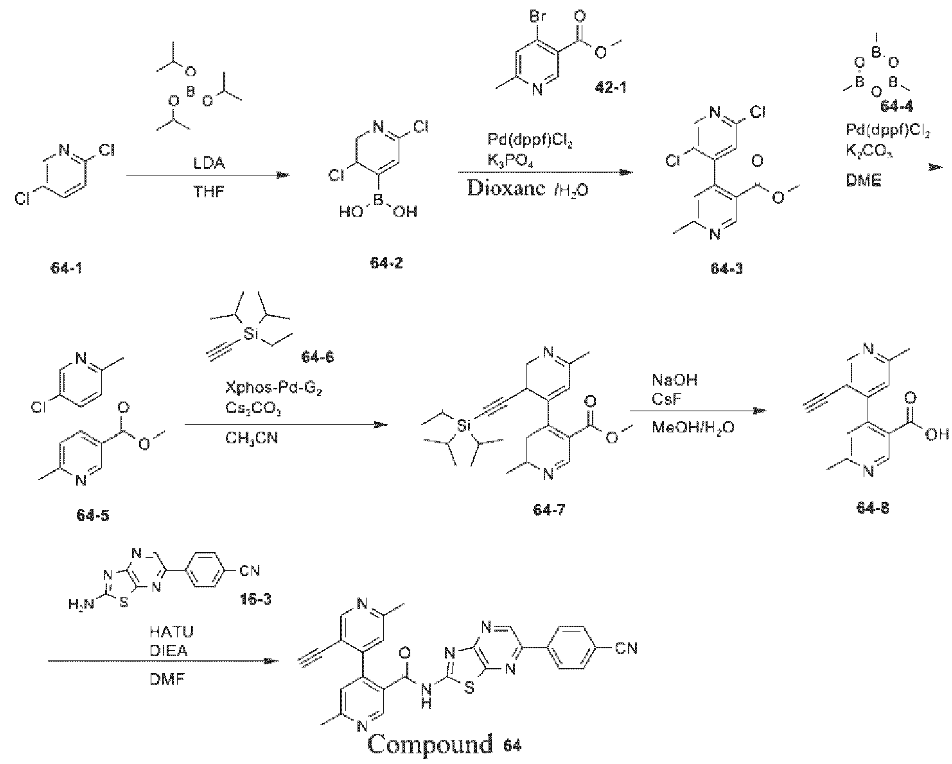


**Step 1: Synthesis of (2,5-dichloropyridin-4-yl)boronic acid (intermediate 64-2)**

**Intermediate 64-1** (7.0 g) was dissolved in anhydrous tetrahydrofuran (120 mL), and the mixture was cooled to -78°C. Under nitrogen atmosphere, to the reaction solution was slowly added a solution of lithium diisopropylamide in tetrahydrofuran (47.30 mL, 2 M). The reaction solution was stirred at -78°C under nitrogen atmosphere for 1 h. To the reaction solution was then slowly added triisopropyl borate (17.79 g). After the reaction was completed, the reaction solution was quenched by adding water (20 mL), and extracted with ethyl acetate (50 mL*3). The organic phase was then concentrated to dryness under reduced pressure. To the residue was added 30 mL of (petroleum ether : ethyl acetate = 1 : 1), and the mixture was stirred at room temperature for 30 minutes, and filtered. The filter cake was dried to obtain the title compound (7.0 g).

MS m/z (ESI): 191.8 [M+H]$^+$.

**Step 2: Synthesis of methyl 2',5'-dichloro-6-methyl-[4,4'-bipyridine]-3-carboxylate (intermediate 64-3)**

**Intermediate 64-2** (1.00 g) and **intermediate 42-1** (1.20 g) were dissolved in dioxane (10 mL) and water (2 mL), and to the mixture was added potassium phosphate (2.21 g). Under nitrogen atmosphere, to the reaction solution was added (1,1'-bis(diphenylphosphino)ferrocene)palladium dichloride (381.48 mg). The reaction solution was stirred at 90°C under nitrogen atmosphere for 1 h. After the reaction was completed, the reaction solution was filtered, and the filtrate was concentrated to dryness under reduced pressure. The residue was purified by silica gel column chromatography (ISCO®; 12 g SepaFlash® flash silica gel column, gradient: 0-25% tetrahydrofuran/petroleum ether, flow rate: 60 mL/min) to obtain the title compound (490.0 mg).
MS m/z (ESI): 296.9 $[M+H]^+$.

**Step 3: Synthesis of methyl 5'-chloro-2',6-dimethyl-[4,4'-bipyridine]-3-carboxylate (intermediate 64-5)**

**Intermediate 64-3** (490 mg) and **intermediate 64-4** (455.43 mg) were dissolved in 1,2-dimethoxyethane (5 mL), and to the mixture was added potassium carbonate (455.43 mg). Under nitrogen atmosphere, to the reaction solution was added (1, 1'-bis(diphenylphosphino)ferrocene)palladium dichloride (12.66 mg). The reaction solution was stirred at 110°C under nitrogen atmosphere for 2 h. After the reaction was completed, the reaction solution was filtered, and the filtrate was concentrated to dryness under reduced pressure. The residue was purified by silica gel column chromatography (ISCO®; 12 g SepaFlash® flash silica gel column, gradient: 0-40% tetrahydrofuran/petroleum ether, flow rate: 50 mL/min) to obtain the title compound (350.0 mg).
MS m/z (ESI): 277.0 $[M+H]^+$.

**Step 4: Synthesis of methyl 2',6-dimethyl-5'-((triisopropylsilyl)ethynyl)-[4,4'-bipyridine]-3-carboxylate (intermediate 64-7)**

**Intermediate 64-5** (200 mg) and **intermediate 64-6** (263.63 mg) were dissolved in acetonitrile (2 mL), and to the mixture was added caesium carbonate (612.27 mg). Under nitrogen atmosphere, to the reaction solution was added chloro(2-dicyclohexylphosphino-2,4,6-triisopropyl-1,1-biphenyl)[2-(2-amino-1,1-biphenyl)]palladium (II) (56.87 mg). The reaction solution was stirred at 90°C under nitrogen atmosphere for 1 h. After the reaction was completed, the reaction solution was filtered, and the filtrate was concentrated to dryness under reduced pressure. The residue was purified by silica gel column chromatography (ISCO®; 12 g SepaFlash® flash silica gel column, gradient: 0-30% tetrahydrofuran/petroleum ether, flow rate: 50 mL/min) to obtain the title compound (200 mg).
MS m/z (ESI): 423.2 $[M+H]^+$.

**Step 5: Synthesis of 5'-ethynyl-2',6-dimethyl-[4,4'-bipyridine]-3-carboxylic acid (intermediate 64-8)**

**Intermediate 64-7** (200 mg) was dissolved in anhydrous methanol (2 mL), and to the mixture was added caesium fluoride (79.07 mg). The reaction solution was stirred at 65°C for 16 h. The reaction solution was cooled to room temperature, and then a solution of sodium hydroxide (56.78 mg) in water (0.5 mL) was added. The reaction solution was stirred at 25°C for 3 h. After the reaction was completed, the reaction solution was concentrated to dryness under reduced pressure, and to the residue was added 10 mL of dichloromethane : methanol (10 : 1). The mixture was stirred for 10 min, and filtered. The filtrate was concentrated under reduced pressure to remove the solvent, to obtain the title compound (100 mg).
MS m/z (ESI): 253.0 $[M+H]^+$.

**Step 6: Synthesis of *N*-(6-(4-cyanophenyl)thiazolo[4,5-b]pyrazin-2-yl)-5'-ethynyl-2',6-dimethyl-[4,4'-bipyridine]-3-carboxamide (compound 64)**

**Intermediate 64-8** (100.00 mg) was dissolved in anhydrous *N'N*-dimethylformamide (2 mL), and to the mixture were added HATU (165.80 mg) and N,N-diisopropylethylamine (153.70 mg). The reaction solution was stirred under nitrogen atmosphere for 30 minutes. **Intermediate 16-3** (110.44 mg) was then added, and subsequently the mixture was stirred at 25°C under nitrogen atmosphere for 1 h. After the reaction was completed, the reaction solution was filtered, and purified by preparative high performance liquid chromatography (C18-1 column, 5 um in particle size, 30 mm in diameter, 150 mm in length; using a decreasingly polar reaction solution of water (containing 0.225% formic acid) and acetonitrile as the eluent; acetonitrile gradient proportion: 15%-55%, elution time: 9 minutes) to obtain the title compound (16 mg).
MS m/z (ESI): 488.1 [M+H]+.
**$^1$H NMR (400MHz, DMSO-d6)** $\delta$ = 13.70 (s, 1H), 9.34 (s, 1H), 9.06 (s, 1H), 8.58 (s, 1H), 8.38 (d, *J* = 8.5 Hz, 2H), 8.02 (d, *J* = 8.5 Hz, 2H), 7.40 (s, 1H), 7.37 (s, 1H), 4.15 (s, 1H), 2.63 (s, 3H), 2.57 (s, 3H)

**Example 65, *N*-(6-(5-chloropyridin-2-yl)thiazolo[4,5-b]pyrazin-2-yl)-2'-cyano-5'-methoxy-6-methyl-[4,4'-bipyridine]-3-carboxamide (compound 65)**

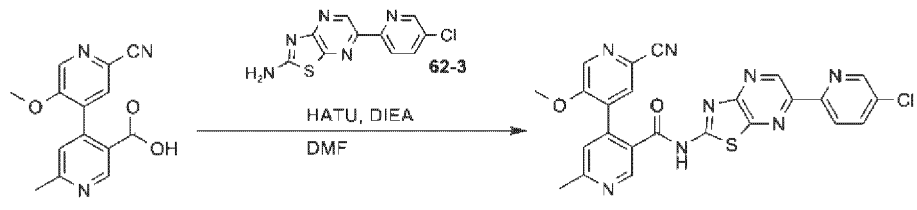


63-2

Compound 65

At 20°C, **intermediate 63-2** (150 mg) was dissolved in *N,N*-dimethylformamide (2 mL), and to the reaction solution were added **intermediate 62-3** (146.91 mg), HATU (211.82 mg) and *N,N*-diisopropylethylamine (144.00 mg). The reaction solution was stirred and reacted at 40°C for 6 hours. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 48%-68%, 11 minutes) to obtain the title compound (40 mg).

**$^1$H NMR (400 MHz, DMSO-$d_6$)** δ = 13.61 (s, 1H), 9.50 (s, 1H), 8.93 (s, 1H), 8.78 (s, 1H), 8.57 (s, 1H), 8.37 (d, *J* = 8.3 Hz, 1H), 8.20 (s, 1H), 8.15 - 8.08 (m, 1H), 7.52 (s, 1H), 3.76 (s, 3H), 2.63 (s, 3H)

MS m/z (ESI): = 515.0 $[M+H]^+$.

**Example 66, *N*-(6-(5-chloropyridin-2-yl)thiazolo[4,5-b]pyrazin-2-yl)-5'-methoxy-2',6-dimethyl-[4,4'-bipyridine]-3-carboxamide (compound 66)**

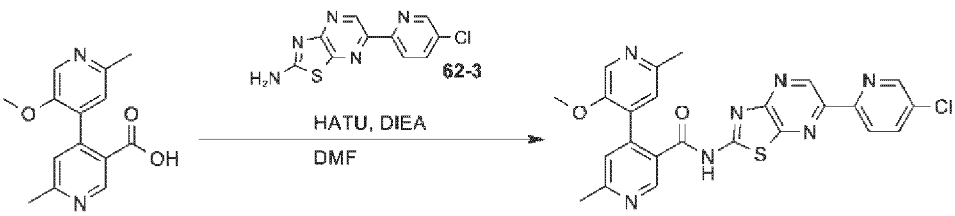


21-3

Compound 66

At 20°C, **intermediate 21-3** (100 mg) was dissolved in *N,N* dimethylformamide (1.5 mL), and to the reaction solution were added HATU (147.22 mg), *N,N* diisopropylethylamine (150.12 mg) and **intermediate 62-3** (122.52 mg). The reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 28%-48%, 11 minutes) to obtain the title compound (25 mg).

**$^1$H NMR (400 MHz, DMSO-$d_6$)** δ = 13.68-13.54 (m, 1H), 9.52 (s, 1H), 8.86 (s, 1H), 8.80 (d, *J* = 2.2 Hz, 1H), 8.39 (d, *J* = 8.7 Hz, 1H), 8.22 (s, 1H), 8.13 (dd, *J* = 2.4, 8.6 Hz, 1H), 7.44 (s, 1H), 7.41-7.37 (m, 1H), 3.61 (s, 3H), 2.62 (s, 3H).

MS m/z (ESI): = 504.1 $[M+H]^+$.

**Example 67, *N*-(6-(4-chlorophenyl)thiazolo[4,5-b]pyrazin-2-yl)-5'-methoxy-2',6-dimethyl-[4,4'-bipyridine]-3-carboxamide (compound 67)**

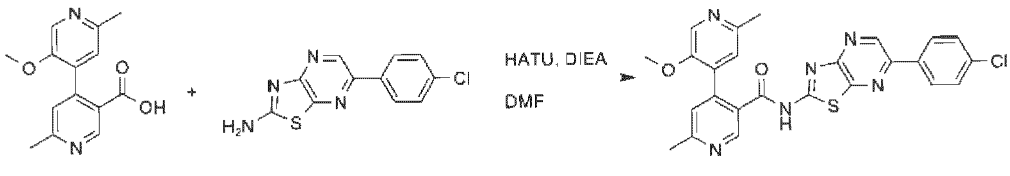


21-3 17-2 Compound 67

At 20°C, **intermediate 21-3** (200 mg) was dissolved in *N,N*-dimethylformamide (2 mL), and to the reaction solution were added **intermediate 17-2** (235.94 mg), HATU (347.35 mg) and *N,N*-diisopropylethylamine (196.78 mg). The reaction solution was stirred and reacted at 70°C for 6 hours. After the reaction was completed, the reaction solution was purified by high performance liquid chromatography (chromatographic column: Boston Prime C18 150*30 mm*5 um; mobile phase: A: water (0.05% ammonia water), B: acetonitrile; B%: 23%-43%, 11 minutes) to obtain the title compound (130 mg).

**$^1$H NMR (400 MHz, DMSO-$d_6$)** $\delta$ = 13.49 (s, 1H), 9.24 (s, 1H), 8.84 (s, 1H), 8.23-8.17 (m, 3H), 7.61 (d, *J* = 8.6 Hz, 2H), 7.42 (s, 1H), 7.33 (s, 1H), 3.59 (s, 3H), 2.61 (s, 3H).

MS m/z (ESI): = 503.1 $[M+H]^+$.

**Example 68, *N*-[6-[5-(1-cyano-1-methyl-ethyl)pyridin-2-yl]thiazolo[4,5-b]pyrazin-2-yl]-4-(5-methoxy-2-methyl-pyridine-4-yl)-6-methyl-pyridine-3-carboxamide (compound 68)**

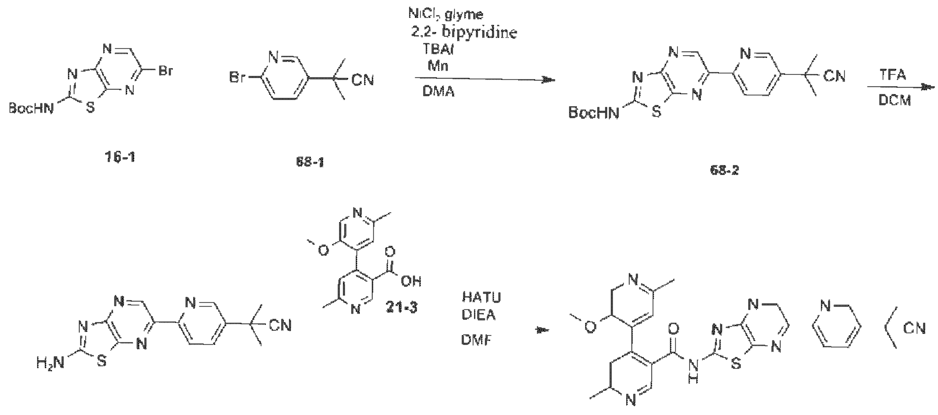


16-1 68-1 68-2 68-3 Compound 68

**Step 1: Synthesis of tert-butyl *N*-[6-[5-(1-cyano-1-methyl-ethyl)pyridin-2-yl]thiazolo[4,5-b]pyrazin-2-yl]carbamate (intermediate 68-2)**

At 25°C, **intermediate 16-1** (1.5 g) was dissolved in *N,N*-dimethylacetamide (15 mL), and to the reaction solution were added **intermediate 68-1** (1.02 g), tetrabutylammonium iodide (2.51 g), nickel (II) chloride ethylene glycol dimethyl ether complex (199.03 mg), 2,2'-bipyridine (70.74 mg) and manganese powder (995.29 mg). Under nitrogen atmosphere, the reaction solution was stirred and reacted at 100°C for 16 hours. After the reaction was completed, to the reaction solution were added dichloromethane (250 mL) and methanol (25 mL). After filtration, the filtrate was concentrated to dryness under reduced pressure. To the residue was added water (50 mL), and the mixture was extracted with dichloromethane (50 mL*3). The organic phases were combined, and dried over anhydrous sodium sulphate. After filtration, the organic phase was concentrated to dryness under reduced pressure, and the residue was purified by silica gel column chromatography (ISCO®; 40 g SepaFlash® Silica Flash chromatographic column, gradient: 0-45% tetrahydrofuran/dichloromethane @30 mL/minute) to obtain the title compound (106 mg).

MS m/z (ESI): 397.0 $[M+H]^+$.

**Step 2: Synthesis of 2-[6-(2-aminothiazolo[4,5-*b*]pyrazin-6-yl)pyridin-3-yl]-2-methyl-propanenitrile (intermediate 68-3)**

At 25°C, **intermediate 68-2** (100 mg) was dissolved in dichloromethane (3 mL), and to the reaction solution was

added trifluoroacetic acid (28.76 mg). The reaction solution was stirred and reacted at 20°C for 2 hours. After the reaction was completed, the reaction solution was concentrated to dryness under reduced pressure. To the residue was added water (1 mL), and the mixture was adjusted to pH 7 with a saturated sodium bicarbonate solution, and filtered. The filter cake was dried to obtain the title compound (65 mg).

MS m/z (ESI): 297.3 [M+H]$^+$.

**Step 3: Synthesis of *N*-[6-[5-(1-cyano-1-methyl-ethyl)pyridin-2-yl]thiazolo[4,5-b]pyrazin-2-yl]-4-(5-methoxy-2-methylpyridine-4-yl)-6-methylpyridine-3-carboxamide (compound 68)**

At 25°C, **intermediate 68-3** (65 mg) was dissolved in *N,N*-dimethylformamide (4 mL), and to the reaction solution were added **intermediate 21-3** (56.65 mg), HATU (83.40 mg) and *N,N*-diisopropylethylamine (56.69 mg). The reaction solution was stirred and reacted at 20°C for 16 hours. After the reaction was completed, the reaction solution was purified by preparative high performance liquid chromatography (chromatographic column: Boston Prime C18 150*30 mm*5 um; mobile phase: [A: water (0.225% formic acid), B: acetonitrile]; B%: 23%-43%, 11 minutes) to obtain the title compound (36.96 mg).

MS m/z (ESI): 537.2 [M+H]$^+$.

**$^1$H NMR (400 MHz, DMSO-*d*$_6$)** δ 13.57 (s, 1H), 9.55 (s, 1H), 8.92 (d, *J* = 2.2 Hz, 1H), 8.85 (s, 1H), 8.42 (d, *J* = 8.4 Hz, 1H), 8.20 (s, 1H), 8.15 (dd, *J* = 2.4, 8.4 Hz, 1H), 7.44 (s, 1H), 7.34 (s, 1H), 3.60 (s, 3H), 2.62 (s, 3H), 1.80 (s, 6H)

**Example 69, N-(6-(tetrahydrofuran-3-yl)thiazolo[4,5-b]pyrazin-2-yl)-5'-methoxy-2',6-dimethyl-[4,4'-bipyridine]-3-carboxamide (compound 69)**

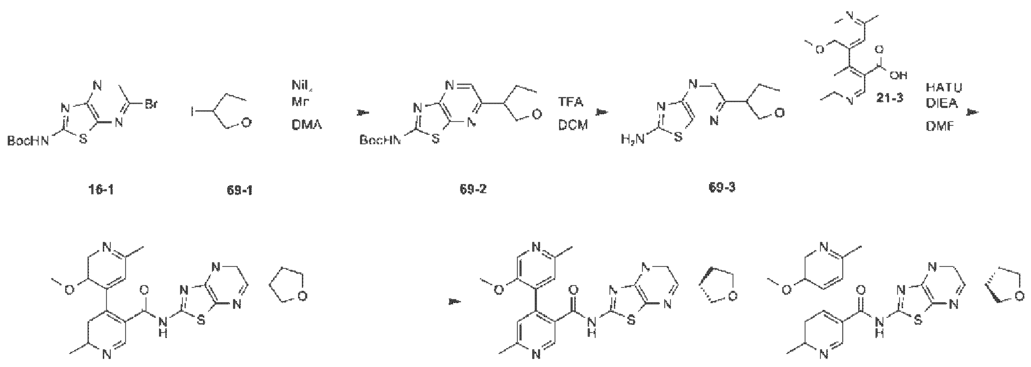


Compound 69

**Step 1: Synthesis of tert-butyl *N*-(6-tetrahydrofuran-3-ylthiazolo[4,5-b]pyrazin-2-yl)carbamate (intermediate 69-2)**

**Intermediate 16-1** (2 g) was dissolved in *N,N*-dimethylacetamide (120 mL), and to the reaction solution were added **intermediate 69-1** (3.59 g), nickel iodide (377.43 mg), 4,4'-di-tert-butyl-2,2'-bipyridine (324.16 mg) and manganese powder (1.16 g). Under nitrogen atmosphere, the reaction solution was stirred and reacted at 100°C for 16 hours. After the reaction was completed, to the reaction solution were added dichloromethane (250 mL) and methanol (25 mL). After filtration, the filtrate was concentrated to dryness under reduced pressure. To the residue was added water (50 mL), and the mixture was extracted with dichloromethane (50 mL*3). The organic phases were combined, and dried over anhydrous sodium sulphate. After filtration, the organic phase was concentrated to dryness under reduced pressure, and the residue was purified by silica gel column chromatography (ISCO®; 80 g SepaFlash® Silica Flash chromatographic column, gradient: 0-15% tetrahydrofuran/dichloromethane @60 mL/minute) to obtain the title compound (250 mg).

MS m/z (ESI): 322.9 [M+H]$^+$.

**Step 2: Synthesis of 6-(tetrahydrofuran-3-yl)thiazolo[4,5-*b*]pyrazin-2-amine (intermediate 69-3)**

At 25°C, **intermediate 69-2** (250 mg) was dissolved in dichloromethane (4 mL), and to the reaction solution was added trifluoroacetic acid (7.70 g). The reaction solution was stirred and reacted at 25°C for 2 hours. After the reaction was completed, the reaction solution was concentrated to dryness under reduced pressure. To the residue was added water (1 mL), and the mixture was adjusted to pH 7 with a saturated sodium bicarbonate solution, and filtered. The filter cake was

dried to obtain the title compound (560 mg).

MS m/z (ESI): 223.2 [M+H]+.

**Step 3: Synthesis of N-(6-(tetrahydrofuran-3-yl)thiazolo[4,5-b]pyrazin-2-yl)-5'-methoxy-2',6-dimethyl-[4,4'-bipyridine]-3-carboxamide (compound 69)**

At 25°C, **intermediate 69-3** (68.85 mg) was dissolved in *N,N*-dimethylformamide (4 mL), and to the reaction solution were added **intermediate 21-3** (80 mg), HATU (117.78 mg) and N,N-diisopropylethylamine (80.06 mg). The reaction solution was stirred and reacted at 25°C for 16 hours. After the reaction was completed, the reaction solution was purified by preparative high performance liquid chromatography (chromatographic column: Boston Prime C18 150*30 mm*5 um; mobile phase: [A: water (0.225% formic acid), B: acetonitrile]; B%: 13%-33%, 11 minutes) to obtain the title compound (40 mg).

Compound 69 was resolved by preparative supercritical fluid chromatography (DAICEL CHIRALCEL OJ column, 10 μm silica, 30 mm in diameter, 250 mm in length; using ethanol (containing 0.1% ammonia water) as the eluent) to obtain **compound 69-P1** (12.64 mg, RT = 4.958 min) and **compound 69-P2** (8.57 mg, RT = 5.209 min).

**Compound 69-P1:**

MS m/z (ESI): 463.1 [M+H]+.

**$^1$H NMR (400 MHz, DMSO-$d_6$)** δ 13.38 (s, 1H), 8.84 (s, 1H), 8.61 (s, 1H), 8.21 (s, 1H), 7.43 (s, 1H), 7.38 (s, 1H), 4.16-4.08 (m, 1H), 4.01-3.93 (m, 1H), 3.85 (q, *J* = 7.4 Hz, 1H), 3.80-3.72 (m, 2H), 3.58 (s, 3H), 2.62 (s, 3H), 2.52 (s, 3H), 2.40-2.30 (m, 1H), 2.24-2.13 (m, 1H)

**Compound 69-P2:**

MS m/z (ESI): 463.1 [M+H]+

**$^1$H NMR (400 MHz, DMSO-$d_6$)** δ 13.37 (s, 1H), 8.84 (s, 1H), 8.59 (s, 1H), 8.18 (s, 1H), 7.41 (s, 1H), 7.32 (s, 1H), 4.16-4.07 (m, 1H), 4.01-3.93 (m, 1H), 3.85 (q, *J* = 7.6 Hz, 1H), 3.80-3.71 (m, 2H), 3.57 (s, 3H), 2.61 (s, 3H), 2.52-2.51 (m, 3H), 2.40-2.30 (m, 1H), 2.24-2.13 (m, 1H)

**Example 70, *N*-(6-(4-cyano-2-fluorophenyl)thiazolo[4,5-b]pyrazin-2-yl)-5'-methoxy-2',6-dimethyl-[4,4'-bipyridine]-3-carboxamide (compound 70)**

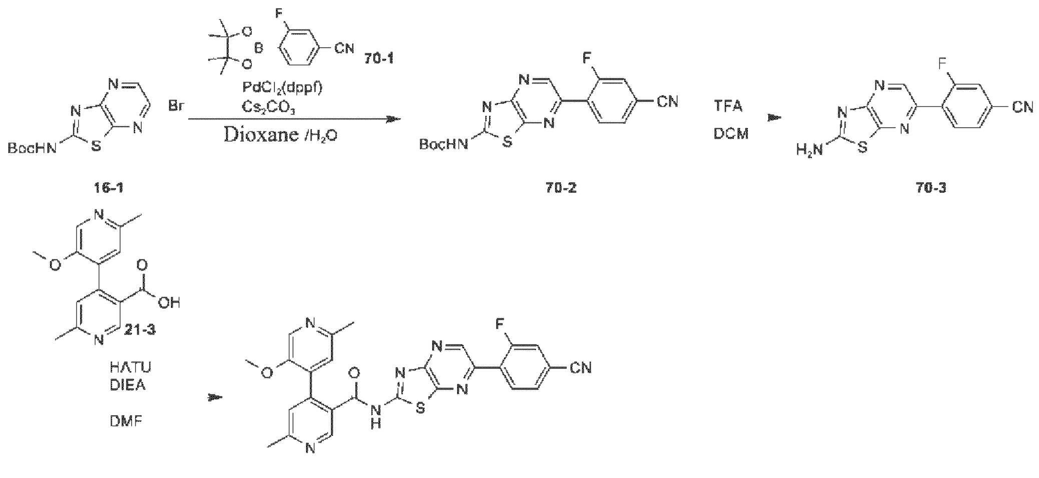


Compound 70

**Step 1: Synthesis of tert-butyl (6-(4-cyano-2-fluorophenyl)thiazolo[4,5-b]pyrazin-2-yl)carbamate (intermediate 70-2)**

**Intermediate 16-1** (300 mg) and **intermediate 70-1** (246.19 mg) were dissolved in dioxane (5 mL) and water (1 mL), and to the mixture was added caesium carbonate (590.27 mg). Under nitrogen atmosphere, to the reaction solution was added (1, 1'-bis(diphenylphosphino)ferrocene)palladium dichloride (66.28 mg). The reaction solution was stirred at 90°C under nitrogen atmosphere for 2 h. After the reaction was completed, the reaction solution was filtered, and the filtrate

was concentrated to dryness under reduced pressure. The residue was purified by silica gel column chromatography (ISCO®; 12 g SepaFlash® flash silica gel column, gradient: 0-40% petroleum ether/ethyl acetate, flow rate: 50 mL/min) to obtain the title compound (170 mg).

MS m/z (ESI): 372.0 [M+H]+.

**Step 2: Synthesis of 4-(2-aminothiazolo[4,5-*b*]pyrazin-6-yl)-3-fluorobenzonitrile (intermediate 70-3)**

**Intermediate 70-2** (150 mg) was dissolved in anhydrous dichloromethane (1 mL) and trifluoroacetic acid (1 mL), and the reaction solution was stirred at 25°C for 2 h. After the reaction was completed, the reaction solution was concentrated under reduced pressure to remove dichloromethane, and the residue was dissolved in ethyl acetate, and washed by adding a saturated sodium bicarbonate aqueous solution. The organic phase was concentrated to dryness under reduced pressure to obtain the title compound (100 mg).

MS m/z (ESI): 271.9 [M+H]+.

**Step 3: Synthesis of *N*-(6-(4-cyano-2-fluorophenyl)thiazolo[4,5-b]pyrazin-2-yl)-5'-methoxy-2',6-dimethyl-[4,4'-bipyridine]-3-carboxamide (compound 70)**

**Intermediate 21-3** (76.17 mg) was dissolved in anhydrous *N'N*-dimethylformamide (2 mL), and to the mixture were added HATU (123.35 mg) and N,N-diisopropylethylamine (114.34 mg). The reaction solution was stirred under nitrogen atmosphere for 30 min. **Intermediate 70-3** (80 mg) was then added, and the reaction solution was stirred at 25°C under nitrogen atmosphere for 2 h. After the reaction was completed, the reaction solution was filtered, and purified by preparative high performance liquid chromatography (Boston Green ODS C18 column, 5 um silica, 30 mm in diameter, 150 mm in length; using a decreasingly polar reaction solution of water (containing 0.225% formic acid) and acetonitrile as the eluent; acetonitrile gradient proportion: 19%-49%, elution time: 12 minutes) to obtain the title compound (12 mg).

MS m/z (ESI): 512.2 [M+H]+.

**$^1$H NMR (400MHz, DMSO-$d_6$)** $\delta$ = 13.62 (s, 1H), 9.10 (d, *J* = 2.1 Hz, 1H), 8.87 (s, 1H), 8.23 (s, 1H), 8.21-8.17 (m, 1H), 8.14-8.08 (m, 1H), 7.90 (dd, *J* = 1.4, 8.1 Hz, 1H), 7.45 (s, 1H), 7.40 (s, 1H), 3.61 (s, 3H), 2.63 (s, 3H).

**Example 71, 4-(5-chloro-2-methoxyphenyl)-*N*-(6-(5-chloropyridin-2-yl)thiazolo[4,5-b]pyrazin-2-yl)-6-methylpyridine-3-carboxamide (compound 71)**

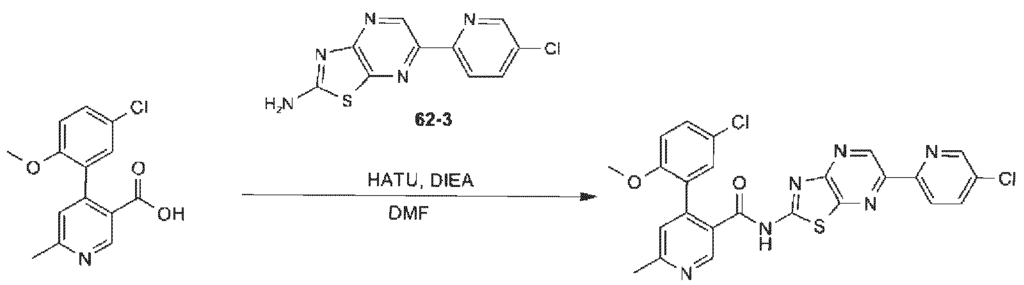


62-3

23-3

Compound 71

At 20°C, **intermediate 23-3** (100 mg) was dissolved in *N,N* dimethylformamide (2 mL), and to the reaction solution were added HATU (136.92 mg), *N,N* diisopropylethylamine (46.54 mg) and **intermediate 62-3** (94.96 mg). The reaction solution was stirred and reacted at 25°C for 2 hours. After the reaction was completed, to the reaction solution were added ethyl acetate (50 mL) and water (100 mL), and the mixture was extracted with ethyl acetate (25 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.05% ammonia water), B: acetonitrile; B%: 53%-73%, 11 minutes) to obtain the title compound (35 mg).

**$^1$H NMR (400 MHz, DMSO-d6)** $\delta$ = 13.47 (s, 1H), 9.49 (s, 1H), 8.81-8.76 (m, 2H), 8.38 (d, *J* = 8.6 Hz, 1H), 8.11 (dd, *J* = 2.4, 8.6 Hz, 1H), 7.52-7.45 (m, 2H), 7.43 (s, 1H), 7.02 (d, *J* = 8.9 Hz, 1H), 3.52 (s, 3H), 2.61 (s, 3H).

MS m/z (ESI): = 523.0 [M+H]+.

**Example 72, *N*-(6-(4-cyanophenyl)thiazolo[4,5-b]pyrazin-2-yl)-2'-cyclopropyl-5'-methoxy-6-methyl-[4,4'-bipyridine]-3-carboxamide (compound 72)**

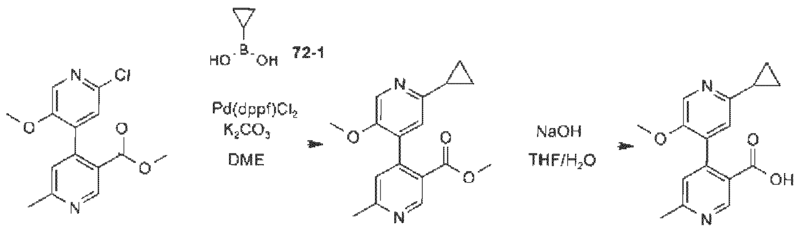


20-3 72-2 72-3

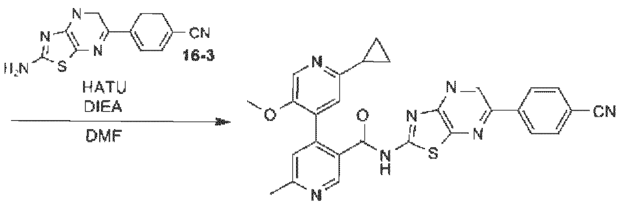


Compound 72

**Step 1: Synthesis of methyl 2'-cyclopropyl-5'-methoxy-6-methyl-[4,4'-bipyridine]-3-carboxylate (intermediate 72-2)**

Under nitrogen atmosphere, **intermediate 20-3** (500 mg) was dissolved in 1,2-dimethoxyethane (10 mL), and to the reaction solution were added **intermediate 72-**1 (1.47 g), 1,1-bis(diphenylphosphine)ferrocene palladium chloride (124.99 mg) and potassium carbonate (472.16 mg). Subsequently, the reaction solution was stirred at 90°C under nitrogen atmosphere for 2 hours. After the reaction was completed, to the reaction solution was added water (20 mL), and the mixture was extracted with ethyl acetate (30 mL*3). The organic phases were combined, and dried over anhydrous sodium sulphate. After filtration, the organic phase was concentrated to dryness under reduced pressure, and the residue was purified by silica gel column chromatography (ISCO®; 40 g SepaFlash® Silica Flash chromatographic column, gradient: 0-30% tetrahydrofuran/petroleum ether @30 mL/minute) to obtain the title compound (270 mg).
MS m/z (ESI): 299.5 $[M+H]^+$.

**Step 2: Synthesis of 2'-cyclopropyl-5'-methoxy-6-methyl-[4,4'-bipyridine]-3-carboxylic acid (intermediate 72-3)**

**Intermediate 72-2** (250 mg) was added to tetrahydrofuran (8 mL) and water (4 mL), and to the reaction solution was added sodium hydroxide (335.17 mg). The reaction solution was stirred at 25°C for 16 hours. After the reaction was completed, the reaction solution was adjusted to pH 3, and concentrated to dryness under reduced pressure. The residue was washed with dichloromethane/methanol (10/1, 20 mL), and filtered. The filtrate was concentrated under reduced pressure to obtain the title compound (700 mg).
MS m/z (ESI): 285.1 $[M+H]^+$.

**Step 3: Synthesis of *N*-(6-(4-cyanophenyl)thiazolo[4,5-b]pyrazin-2-yl)-2'-cyclopropyl-5'-methoxy-6-methyl-[4,4'-bipyridine]-3-carboxamide (compound 72)**

Under nitrogen atmosphere, **intermediate 72-3** (112.25 mg) was added to *N,N*-dimethylformamide (4 mL), and to the reaction solution were added **intermediate 16-3** (100 mg), HATU (150.12 mg) and *N,N*-diisopropylethylamine (102.05 mg). Subsequently, the reaction solution was stirred at 25°C under nitrogen atmosphere for 2 hours. After the reaction was completed, the reaction solution was purified by preparative high performance liquid chromatography (chromatographic column: Boston Prime C18 150*30 mm*5 um; mobile phase: [A: water (0.225% formic acid), B: acetonitrile]; B%: 30%-50%, 11 minutes) to obtain the title compound (43.04 mg).
MS m/z (ESI): 520.1 $[M+H]^+$.
**$^1$H NMR (400MHz, DMSO-$d_6$)** δ 13.52 (s, 1H), 9.35 (s, 1H), 8.85 (s, 1H), 8.38 (d, *J* = 8.1 Hz, 2H), 8.16 (s, 1H), 8.01 (d, *J* = 8.2 Hz, 2H), 7.46 (s, 1H), 7.35 (s, 1H), 3.58 (s, 3H), 2.62 (s, 3H), 2.16 (s, 1H), 0.98-0.87 (m, 4H)

**Example 73, *N*-(6-(4-cyanophenyl)thiazolo[4,5-*b*]pyrazin-2-yl)-5'-methoxy -6-methyl-2'-(trifluoromethyl)-[4,4'-bipyridine]-3-carboxamide (compound 73)**

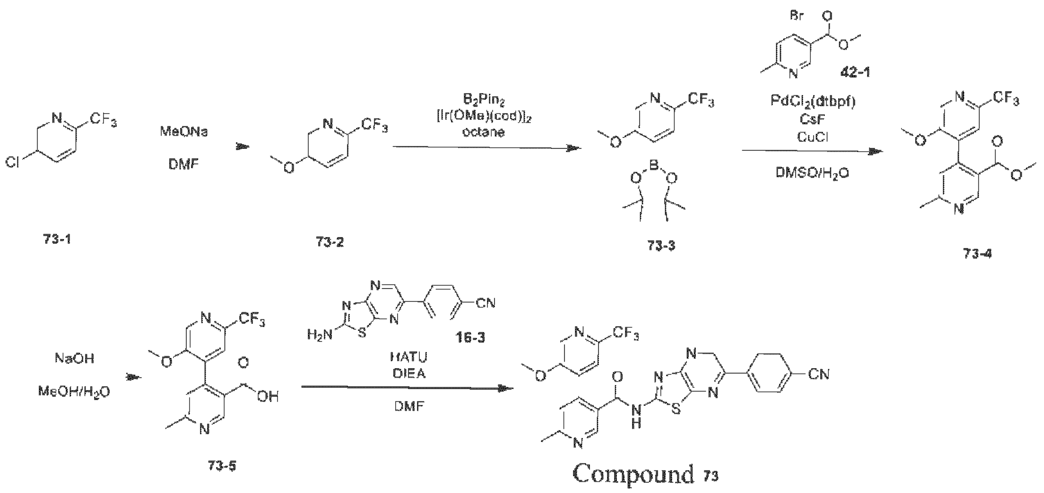


73-1 73-2 73-3 73-4 73-5 Compound 73

**Step 1: Synthesis of 5-methoxy-2-(trifluoromethyl)pyridine (intermediate 73-2)**

**Intermediate 73-1** (10 g) and sodium methoxide (5.95 g) were dissolved in N,N-dimethylformamide (150 mL). Under nitrogen atmosphere, the reaction solution was stirred and reacted at 80°C for 16 h. After the reaction was completed, the reaction solution was cooled to room temperature, diluted with water (200 mL), and extracted with ethyl acetate (145 ml * 3). The organic phases were combined, and dried over anhydrous sodium sulphate. After filtration, the organic phase was concentrated to dryness under reduced pressure. The residue was purified by silica gel column chromatography (ISCO®; 80 g SepaFlash® flash silica gel column, gradient: 0-10% ethyl acetate/petroleum ether, flow rate: 60 mL/min) to obtain the title compound (2.8 g).

MS m/z(ESI): = 178.1 $[M+H]^+$.

**Step 2: Synthesis of 5-methoxy-4-(4,4,5,5-tetramethyl-1,3,2-dioxaborolan-2-yl)-2-(trifluoromethyl)pyridine (intermediate 73-3)**

**Intermediate 73-2** (1 g), bis(pinacolato)diboron (1.72 g), bis(1,5-cyclooctadiene)di-μ-methoxydiiridium (I) ($[Ir(OMe)(cod)]_2$) (187.12 mg, 282.29 umol), and tris[3,5-bis(trifluoromethyl)phenyl]phosphine (378.42 mg) were dissolved in n-octane (20 mL). Under nitrogen atmosphere, the reaction solution was stirred and reacted at 80°C for 16 h. After the reaction was completed, the reaction solution was cooled to room temperature. The reaction solution was diluted with water (50 mL), and extracted with ethyl acetate (45 ml * 3). The organic phases were combined, and dried over anhydrous sodium sulphate. After filtration, the organic phase was concentrated to dryness under reduced pressure. The residue was purified by silica gel column chromatography (ISCO®; 40 g SepaFlash® flash silica gel column, gradient: 0-15% ethyl acetate/petroleum ether, flow rate: 60 mL/min) to obtain the title compound (400 mg).

**$^1$H NMR(400 MHz, DMSO-$d_6$)**δ = 8.61-8.51(s, 1H), 7.83-7.78(s, 1H), 4.01-3.95(d, 3H), 1.33-1.29(s, 12H)

**Step 3: Synthesis of methyl 5'-methoxy-6-methyl-2'-(trifluoromethyl)-[4,4'-bipyridine]-3-carboxylate (intermediate 73-4)**

**Intermediate 73-3** (390 mg), **intermediate 42-1** (444.05 mg), 1,1-bis(tert-butylphosphine)ferrocene palladium chloride (83.86 mg), caesium fluoride (390.93 mg) and cuprous chloride (254.78 mg) were dissolved in dimethyl sulfoxide (10 mL) and water (1 mL). Under nitrogen atmosphere, the reaction solution was stirred and reacted at 90°C for 2 hours. After the reaction was completed, the reaction solution was cooled to room temperature, diluted by adding water (20 mL), and extracted with ethyl acetate (15 mL*3). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: 0-20% ethyl acetate/petroleum ether, flow rate: 50 mL/min) to obtain the title compound (150 mg).

MS m/z(ESI): = 327.0 $[M+H]^+$.

**Step 4: Synthesis of 5'-methoxy-6-methyl-2'-(trifluoromethyl)-[4,4'-bipyridine]-3-carboxylic acid (intermediate 73-5)**

**Intermediate 73-4** (150 mg) and sodium hydroxide (55.17 mg) were dissolved in methanol (1 mL) and water (0.2 mL), and the reaction solution was stirred and reacted at 25°C for 2 hours. After the reaction was completed, the reaction solution was adjusted to pH 7 with dilute hydrochloric acid, and concentrated to dryness under reduced pressure. The residue was dissolved in methanol (1 mL). After filtration, the filtrate was concentrated to dryness under reduced pressure to obtain the title compound (120 mg).

MS m/z(ESI): = 313.3 [M+H]+

**Step 5: Synthesis of *N*-(6-(4-cyanophenyl)thiazolo[4,5-*b*]pyrazin-2-yl)-5'-methoxy-6-methyl-2'-(trifluoromethyl)-[4,4'-bipyridine]-3-carboxamide (compound 73)**

**Intermediate 73-5** (100 mg), **intermediate 16-3** (81.12 mg), HATU (121.77 mg), and DIEA (124.18 mg) were dissolved in *N,N*-dimethylformamide (2 mL). The reaction was stirred and reacted at 25°C for 2 hours. After the reaction was completed, to the reaction solution was added water (50 mL), and the mixture was extracted with ethyl acetate (15 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 45%-65%, 11 minutes) to obtain the title compound (43 mg).

**$^1$H NMR(400 MHz, DMSO-*d*$_6$)** $\delta$ = 13.70-13.53(m, 1H), 9.37(s, 1H), 8.92(s, 1H), 8.58-8.54(s, 1H), 8.44-8.33(d, 2H), 8.05-7.98(m, 3H), 7.58-7.52(s, 1H), 3.75(s, 3H), 2.64(s, 3H).

MS m/z(ESI): = 548.1 [M+H]+

**Example 74, *N*-(6-(4-cyanophenyl)thiazolo[4,5-b]pyrazin-2-yl)-5'-(difluoromethoxy)-2',6-dimethyl-[4,4'-bipyridine]-3-carboxamide (compound 74)**

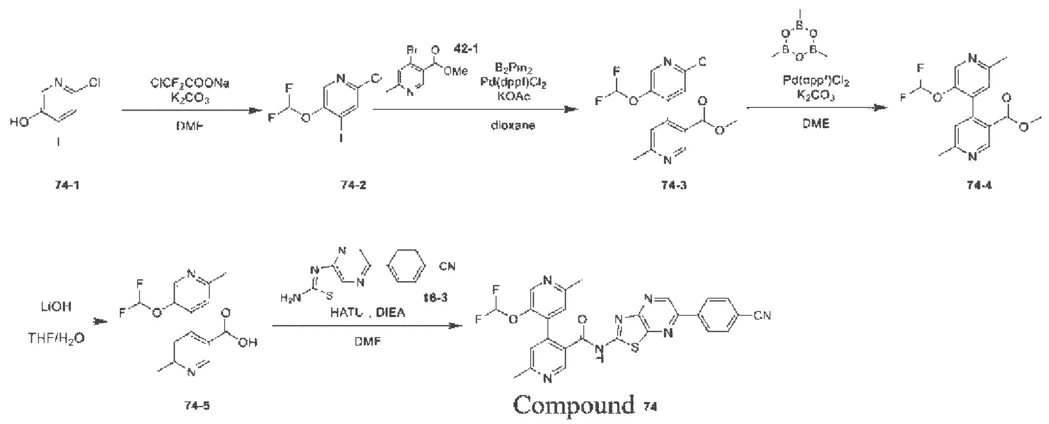

74-1 74-2 74-3 74-4 74-5 Compound 74

**Step 1: Synthesis of 2-chloro-5-(difluoromethoxy)-4-iodopyridine (intermediate 74-2)**

**Intermediate 74-1** (9.6 g), sodium difluorochloroacetate (11.46 g), and potassium carbonate (7.79 g) were dissolved in N,N-dimethylformamide (60 mL). Under nitrogen atmosphere, the reaction solution was stirred and reacted at 90°C for 3 hours. After the reaction was completed, to the reaction solution was added water (200 mL), and the mixture was extracted with ethyl acetate (100 mL*3). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: 0-5% ethyl acetate/petroleum ether, flow rate: 80 mL/min) to obtain the title compound (8 g).

MS m/z(ESI): = 305.9 [M+H]+

**Step 2: Synthesis of methyl 2'-chloro-5'-(difluoromethoxy)-6-methyl-[4,4'-bipyridine]-3-carboxylate (intermediate 74-3)**

**Intermediate 74-2** (200 mg), **intermediate 42-1** (165.74 mg), potassium acetate (192.78 mg), 1,1-bis(diphenylphosphine)ferrocene palladium chloride (95.82 mg), and bis(pinacolato)diboron (249.41 mg) were dissolved in dioxane (4 mL). The reaction solution was stirred and reacted at 90°C for 16 hours. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 35%-55%, 12 minutes) to obtain the title compound (30 mg).

MS m/z(ESI): = 329.3 $[M+H]^+$.

**Step 3: Synthesis of methyl 5'-(difluoromethoxy)-2',6-dimethyl-[4,4'-bipyridine]-3-carboxylate (intermediate 74-4)**

**Intermediate 74-3** (30 mg), trimethylboroxine (22.91 mg), potassium carbonate (25.23 mg), and 1,1-bis(diphenylphosphine)ferrocene palladium chloride (6.68 mg) were dissolved in 1,2-dimethoxyethane (0.5 mL). The reaction solution was stirred and reacted at 110°C for 1 hour. After the reaction was completed, the reaction solution was concentrated to dryness under reduced pressure to obtain the title compound (30 mg).

MS m/z(ESI): = 309.3 $[M+H]^+$.

**Step 4: Synthesis of 5'-(difluoromethoxy)-2',6-dimethyl-[4,4'-bipyridine]-3-carboxylic acid (intermediate 74-5)**

**Intermediate 74-4** (30 mg) was dissolved in tetrahydrofuran (1 mL), and a solution of lithium hydroxide (244.97 mg) in water (1 mL) was added. The reaction solution was stirred and reacted at 20°C for 1 hour. After the reaction was completed, the reaction solution was adjusted to pH 7 with dilute hydrochloric acid, and extracted with ethyl acetate (10 mL*3). The organic phases were combined, and dried over anhydrous sodium sulphate. After filtration, the organic phase was concentrated to dryness under reduced pressure to obtain the title compound (28 mg).

MS m/z(ESI): = 294.9 $[M+H]^+$

**Step 5: Synthesis of *N*-(6-(4-cyanophenyl)thiazolo[4,5-*b*]pyrazin-2-yl)-5'-(difluoromethoxy)-2',6-dimethyl-[4,4'-bipyridine]-3-carboxamide (compound 74)**

**Intermediate 74-5** (20 mg), **intermediate 16-3** (17.22 mg), HATU (31.01 mg) and DIEA (17.57 mg) were dissolved in N,N-dimethylformamide (1 mL). Under nitrogen atmosphere, the reaction solution was stirred and reacted at 25°C for 1 h. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 38%-58%, 12 minutes) to obtain the title compound (12 mg).

**$^1$H NMR(400 MHz, DMSO-*$d_6$*)** δ 9.36(s, 1H), 8.99(s, 1H), 8.42-8.32(m, 3H), 8.02(d, *J* = 8.5 Hz, 2H), 7.47(d, *J* =1.8 Hz, 2H), 6.96(t, *J* =72 Hz, 1H), 2.64(s, 3H), 2.56(s, 3H)

MS m/z(ESI): = 530.0 $[M+H]^+$.

**Example 75, 5'-methoxy-*N*-[6-(5-methoxypyridin-2-yl)thiazolo[4,5-*b*]pyrazin-2-yl]-2',6-dimethyl-[4,4'-bipyridine]-3-carboxamide (compound 75)**

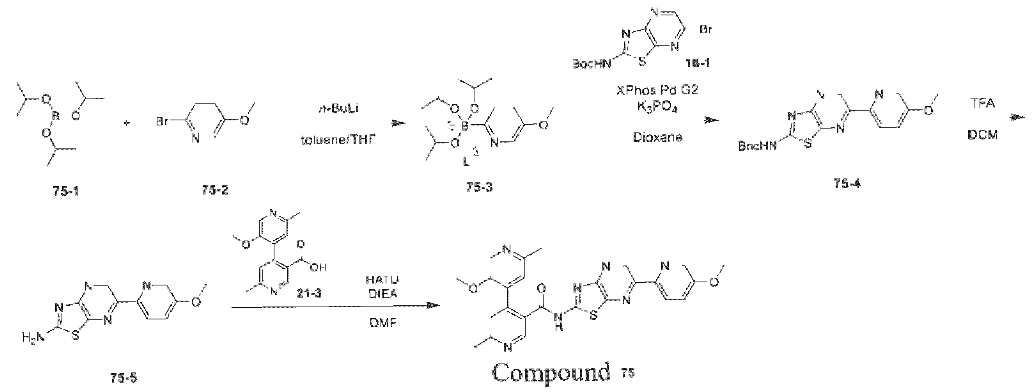


75-1 75-2 75-3 75-4 75-5 Compound 75

**Step 1: Synthesis of lithium triisopropoxy(5-methoxypyridin-2-yl)borate (intermediate 75-3)**

At 20°C, **intermediate 75-2** (300 mg) and **intermediate 75-1** (330.09 mg) were dissolved in a solution of toluene (4 mL)/tetrahydrofuran (1 mL), and n-butyllithium (702.05 uL) was slowly dropwise added to the reaction solution at - 78°C under nitrogen atmosphere. After the dropwise addition was completed, the reaction solution was stirred and reacted at -78°C for 0.5 hours, and then warmed to room temperature, and stirred and reacted for 16 hours. After the reaction was completed, the reaction solution was quenched by adding water, and concentrated to dryness under reduced pressure to obtain the title compound (670 mg).

**Step 2: Synthesis of tert-butyl *N*-[6-(5-methoxypyridin-2-yl)thiazolo[4,5-*b*]pyrazin-2-yl]carbamate (intermediate 75-4)**

At 20°C, **intermediate 75-3** (570 mg) was dissolved in a solution of dioxane (2.5 mL)/water (0.5 mL), and to the mixture were added **intermediate 16-1** (622.76 mg), potassium phosphate (1.20 g) and chloro(2-dicyclohexylphosphino-2,4,6-triisopropyl-1,1-biphenyl)[2-(2-amino-1,1-biphenyl)]palladium (II) (147.95 mg). Under nitrogen atmosphere, the reaction solution was stirred and reacted at 100°C for 3 hours. After the reaction was completed, the reaction solution was filtered, and the filtrate was concentrated to dryness under reduced pressure. The residue was purified by column chromatography (dichloromethane/tetrahydrofuran =15/1) to obtain the title compound (40 mg).

MS m/z(ESI): = 360.1 $[M+H]^+$.

**Step 3: Synthesis of 6-(5-methoxypyridin-2-yl)thiazolo[4,5-*b*]pyrazin-2-amine (intermediate 75-5)**

At 20°C, **intermediate 75-4** (100 mg) was dissolved in dichloromethane (2 mL), and to the mixture was added trifluoroacetic acid (206.68 uL). The reaction solution was stirred and reacted at 20°C for 2 hours. After the reaction was completed, the reaction solution was concentrated to dryness under reduced pressure. A saturated sodium bicarbonate solution was used to neutralize the remaining trifluoroacetic acid, and the mixture was filtered to obtain the title compound (60 mg).

MS m/z(ESI): = 260.0 $[M+H]^+$.

**Step 4: Synthesis of 5'-methoxy-*N*-[6-(5-methoxypyridin-2-yl)thiazolo[4,5-*b*]pyrazin-2-yl]-2',6-dimethyl-[4,4'-bipyridine]-3-carboxamide (compound 75)**

At 20°C, **intermediate 75-5** (60 mg) was dissolved in *N,N*-dimethylformamide (1 mL), and to the mixture were added **intermediate 21-3** (89.65 mg), HATU (96.79 mg) and DIEA (59.81 mg). The reaction solution was stirred and reacted at 20°C for 4 hours. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 18%-38%, 11 minutes) to obtain the title compound (15.5 mg).

MS m/z(ESI): = 500.1 $[M+H]^+$.

**$^1$H NMR(400 MHz, DMSO-$d_6$)** $\delta$ = 13.49(s, 1H), 9.46(s, 1H), 8.85(s, 1H), 8.45(d, *J* = 2.9 Hz, 1H), 8.34(d, *J* = 9.0 Hz, 1H), 8.20(s, 1H), 7.59(dd, *J* = 2.8, 8.9 Hz, 1H), 7.43(s, 1H), 7.34(s, 1H), 3.92(s, 3H), 3.60(s, 3H), 2.62(s, 3H)

**Example 76, *N*-(6-(4-cyanophenyl)thiazolo[4,5-*b*]pyrazin-2-yl)-3'-fluoro-5'-methoxy-2',6-dimethyl-[4,4'-bipyridine]-3-carboxamide (compound 76)**

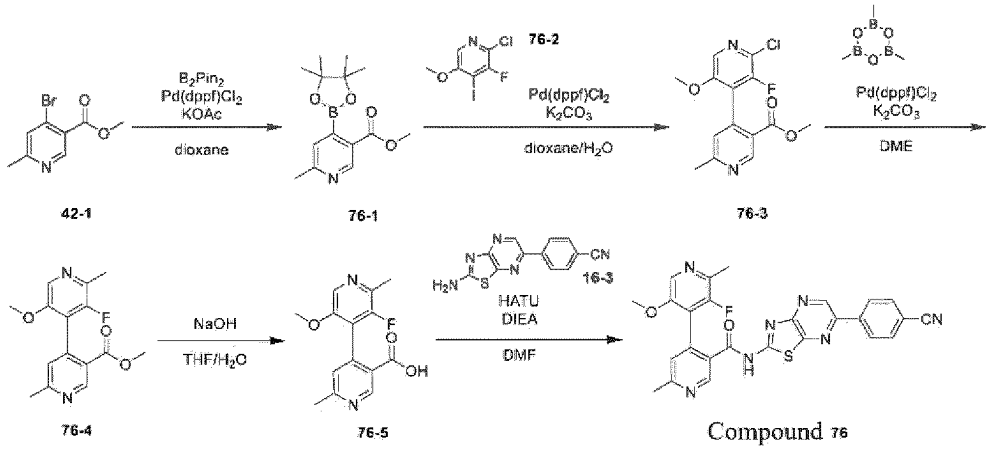


**Step 1: Synthesis of methyl 6-methyl-4-(4,4,5,5-tetramethyl-1,3,2-dioxaborolan-2-yl)pyridine-3-carboxylate (intermediate 76-1)**

**Intermediate 42-1** (2 g), potassium acetate (2.56 g), 1,1-bis(diphenylphosphine)ferrocene palladium chloride (636.10 mg), and bis(pinacolato)diboron (3.31 g) were dissolved in dioxane (20 mL). The reaction solution was stirred and reacted at 100°C for 12 hours. After the reaction was completed, the reaction solution was filtered, and then the organic phase was concentrated to dryness under reduced pressure to obtain the title compound (2 g).

**Step 2: Synthesis of methyl 2'-chloro-3'-fluoro-5'-methoxy-6-methyl-[4,4'-bipyridine]-3-carboxylate (intermediate 76-3)**

**Intermediate 76-1** (1.74 g), **intermediate 76-2** (1.5 g), potassium carbonate (2.16 g), and 1,1-bis(diphenylphosphine)ferrocene palladium chloride (763.63 mg) were dissolved in a mixed solution of dioxane (20 mL) and water (5 mL). The reaction solution was stirred and reacted at 90°C for 12 hours. After the reaction was completed, to the reaction solution was added water (20 mL), and the mixture was extracted with ethyl acetate (10 mL*3). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: 0-30% tetrahydrofuran/petroleum ether, flow rate: 60 mL/min) to obtain the title compound (230 mg).

MS m/z(ESI): = 311.1 $[M+H]^+$

**Step 3: Synthesis of methyl 3'-fluoro-5'-methoxy-2',6-dimethyl-[4,4'-bipyridine]-3-carboxylate (intermediate 76-4)**

**Intermediate 76-3** (230 mg), trimethylboroxine (185.85 mg), potassium carbonate (204.61 mg), and 1,1-bis(diphenylphosphine)ferrocene palladium chloride (54.16 mg) were dissolved in 1,2-dimethoxyethane (0.5 mL). The reaction solution was stirred and reacted at 110°C for 2 hours. After the reaction was completed, to the reaction solution was added water (20 mL), and the mixture was extracted with ethyl acetate (10 mL*3). The organic phases were combined, and dried over anhydrous sodium sulphate. After filtration, the organic phase was concentrated to dryness under reduced pressure. The residue was purified by silica gel column chromatography (ISCO®; 20 g SepaFlash® flash silica gel column, gradient: 0-30% tetrahydrofuran/petroleum ether, flow rate: 60 mL/min) to obtain the title compound (200 mg).

MS m/z(ESI): = 291.0 $[M+H]^+$.

**Step 4: Synthesis of 3'-fluoro-5'-methoxy-2',6-dimethyl-[4,4'-bipyridine]-3-carboxylic acid (intermediate 76-5)**

**Intermediate 76-4** (200 mg) was dissolved in tetrahydrofuran (10 mL), and a solution of sodium hydroxide (275.57 mg) in water (5 mL) was added. The reaction solution was stirred and reacted at 25°C for 2 hours. After the reaction

was completed, the reaction solution was adjusted to pH 5 with dilute hydrochloric acid, and extracted with ethyl acetate (10 mL*3). The organic phases were combined, and dried over anhydrous sodium sulphate. After filtration, the organic phase was concentrated to dryness under reduced pressure to obtain the title compound (170 mg).

MS m/z(ESI): = 277.2 $[M+H]^+$

**Step 5: Synthesis of *N*-(6-(4-cyanophenyl)thiazolo[4,5-*b*]pyrazin-2-yl)-3'-fluoro-5'-methoxy-2',6-dimethyl-[4,4'-bipyridine]-3-carboxamide (compound 76)**

**Intermediate 76-5** (170 mg), **intermediate 16-3** (233.79 mg), HATU (350.97 mg) and DIEA (159.06 mg) were dissolved in *N,N*-dimethylformamide (2 mL). Under nitrogen atmosphere, the reaction solution was stirred and reacted at 25°C for 1 h. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 27%-57%, 8 minutes) to obtain the title compound (3.6 mg).

**$^1$H NMR(400 MHz, DMSO-$d_6$)** δ = 13.67(s, 1H), 9.35(s, 1H), 9.02(s, 1H), 8.38(d, *J*=8.4 Hz, 2H), 8.19(s, 1H), 8.02(d, *J*=8.4 Hz, 2H), 7.45(s, 1H), 3.69(s, 3H), 2.62(s, 3H), 2.45(m, 3H).

MS m/z(ESI): = 512.1 $[M+H]^+$.

**Example 77, 4-(5-chloro-2-methoxyphenyl)-*N*-[6-(5-cyclopropylpyridin-2-yl)thiazolo[4,5-*b*]pyrazin-2-yl]-6-methylpyridine-3-carboxamide (compound 77)**

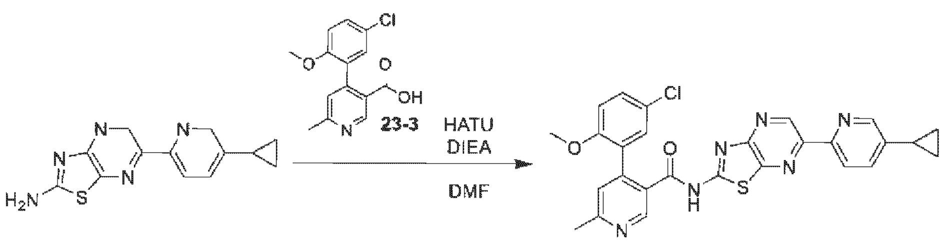


22-2

Compound 77

At 20°C, **intermediate 22-2** (150 mg) was dissolved in *N,N*-dimethylformamide (2.5 mL), and to the mixture were added **intermediate 23-3** (154.67 mg), HATU (211.77 mg) and DIEA (143.96 mg). The reaction solution was stirred and reacted at 20°C for 6 hours. After the reaction was completed, the reaction solution was purified by high performance liquid chromatography (chromatographic column: Boston Green ODS 150*30 mm*5 um; mobile phase: A: water (0.225% formic acid), B: acetonitrile; B%: 55%-85%, 14 minutes) to obtain the title compound (91 mg).

**$^1$H NMR(400 MHz, DMSO-$d_6$)** δ = 13.42(s, 1H), 9.49(s, 1H), 8.80(s, 1H), 8.54(s, 1H), 8.22(d, *J* = 7.7 Hz, 1H), 7.60(d, *J* = 7.5 Hz, 1H), 7.54-7.39(m, 3H), 7.01(d, *J* = 8.6 Hz, 1H), 3.52(s, 3H), 2.61(s, 3H), 2.05-1.98(m, 1H), 1.09-1.01(m, 2H), 0.91-0.86(s, 2H)

MS m/z(ESI): = 529.1 $[M+H]^+$.

**Example 78, 4-(5-chloro-2-methoxyphenyl)-*N*-(6-(5-(2-hydroxyprop-2-yl)pyridin-2-yl)thiazolo[4,5-*b*]pyrazin-2-yl)-6-methylpyridine-3-carboxamide (compound 78)**

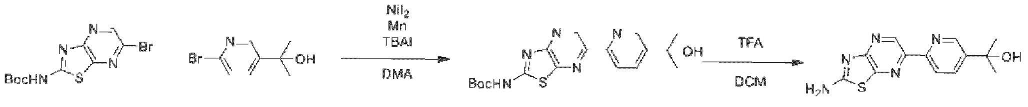


16-1 78-1 78-2 78-3

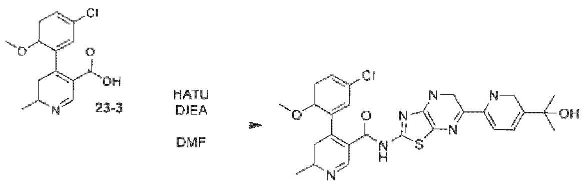


23-3

Compound 78

**Step 1: Synthesis of tert-butyl (6-(5-(2-hydroxypropan-2-yl)pyridin-2-yl)thiazolo[4,5-*b*]pyrazin-2-yl)carbamate (intermediate 78-2)**

At 20°C, **intermediate 16-1** (1.53 g) was dissolved in *N,N*-dimethylacetamide (20 mL), and to the mixture were added **intermediate 78-1** (1 g), nickel iodide (144.63 mg), tetrabutylammonium iodide (2.56 g), 6,6-dimethyl-2,2'-bipyridine (85.27 mg) and manganese powder (1.02 g). Under nitrogen atmosphere, the reaction solution was stirred and reacted at 90°C for 8 hours. After the reaction was completed, to the reaction solution were added dichloromethane (200 mL) and methanol (20 mL). After filtration, the organic phase was concentrated to dryness under reduced pressure. To the residue was added water (200 mL), and the mixture was filtered. The filter cake was purified by column chromatography (dichloromethane/tetrahydrofuran =15/1) to obtain the title compound (60 mg).

MS m/z(ESI): = 388.3 $[M+H]^+$.

**Step 2: Synthesis of 2-(6-(2-aminothiazolo[4,5-*b*]pyrazin-6-yl)pyridin-3-yl)prop-2-ol (intermediate 78-3)**

At 20°C, **intermediate 78-2** (60 mg) was dissolved in dichloromethane (2 mL), and to the mixture was added trifluoroacetic acid (115.03 uL). The reaction solution was stirred and reacted at 20°C for 2 hours. After the reaction was completed, the reaction solution was concentrated to dryness under reduced pressure. The reaction solution was adjusted to pH = 7 with a saturated sodium bicarbonate solution, and filtered. The filter cake was dried to obtain the title compound (60 mg).

MS m/z(ESI): = 288.3 $[M+H]^+$.

**Step 3: Synthesis of 4-(5-chloro-2-methoxyphenyl)-*N*-(6-(5-(2-hydroxyprop-2-yl)pyridin-2-yl)thiazolo[4,5-*b*]pyrazin-2-yl)-6-methylpyridine-3-carboxamide (compound 78)**

At 20°C, **intermediate 78-3** (40 mg) was dissolved in *N,N*-dimethylformamide (1 mL), and to the mixture were added **intermediate 23-3** (42.52 mg), HATU (52.93 mg) and DIEA (35.98 mg). The reaction solution was stirred and reacted at 20°C for 4 hours. After the reaction was completed, the reaction solution was purified by high performance liquid chromatography (chromatographic column: Boston Prime C18 150*30 mm*5 um; mobile phase: A: water (0.225% formic acid), B: acetonitrile; B%: 35%-55%, 11 minutes) to obtain the title compound (15 mg).

**$^1$H NMR(400 MHz, DMSO-$d_6$)** $\delta$ = 13.42(s, 1H), 9.53(s, 1H), 8.84(s, 1H), 8.80(s, 1H), 8.32(d, *J* = 8.3 Hz, 1H), 8.05(dd, *J* = 1.9, 8.3 Hz, 1H), 7.51-7.45(m, 2H), 7.42(s, 1H), 7.02(d, *J* = 8.8 Hz, 1H), 5.36(s, 1H), 3.53(s, 3H), 2.61(s, 3H), 1.52(s, 6H)

MS m/z(ESI): = 547.2 $[M+H]^+$.

**Example 79, 4-(5-chloro-2-methoxyphenyl)-*N*-(6-(4-cyclopropylphenyl) thiazolo[4,5-*b*]pyrazin-2-yl)-6-methylpyridine-3-carboxamide (compound 79)**

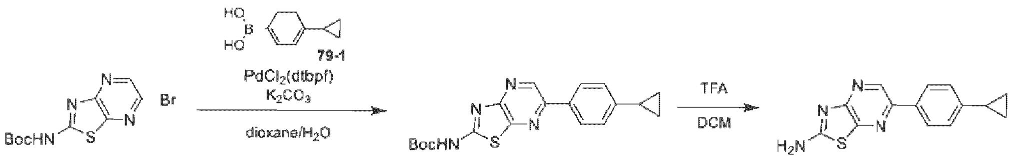


16-1 79-2 79-3

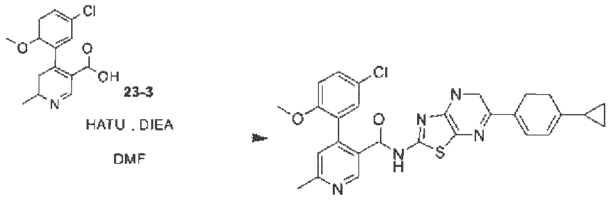


Compound 79

**Step 1: Synthesis of tert-butyl (6-(4-cyclopropylphenyl)thiazolo[4,5-*b*]pyrazin-2-yl)carbamate (intermediate 79-2)**

**Intermediate 16-1** (1.50 g), **intermediate 79-1** (1.10 g), potassium carbonate (1.88 g), and 1,1-bis(tert-butylphosphine)ferrocene palladium chloride (295.19 mg, 452.91 μmol) were dissolved in dioxane (15 mL) and water (3 mL). Under nitrogen atmosphere, the reaction solution was stirred and reacted at 90°C for 2 h. After the reaction was completed, the reaction solution was cooled to room temperature, diluted with water (50 mL), and extracted with ethyl acetate (45 ml * 3). The organic phases were combined, and dried over anhydrous sodium sulphate. After filtration, the organic phase was concentrated to dryness under reduced pressure. The residue was purified by silica gel column chromatography (ISCO®; 40 g SepaFlash® flash silica gel column, gradient: 0-30% tetrahydrofuran/petroleum ether, flow rate: 40 mL/min) to obtain the title compound (750 mg).

MS m/z(ESI): = 369.4 $[M+H]^+$.

**Step 2: Synthesis of 6-(4-cyclopropylphenyl)thiazolo[4,5-*b*]pyrazin-2-amine (intermediate 79-3)**

**Intermediate 79-2** (700 mg) and trifluoroacetic acid (7.68 g) were dissolved in dichloromethane (10 mL), and the reaction solution was stirred and reacted at 25°C for 16 h. After the reaction was completed, the reaction solution was concentrated to dryness under reduced pressure, and then adjusted to pH = 7 with a saturated sodium bicarbonate solution, and filtered. The filter cake was dried to obtain the title compound (600 mg).

MS m/z(ESI): = 269.3 $[M+H]^+$.

**Step 3: Synthesis of 4-(5-chloro-2-methoxyphenyl)-*N*-(6-(4-cyclopropylphenyl)thiazolo[4,5-*b*]pyrazin-2-yl)-6-methylpyridine-3-carboxamide (compound 79)**

**Intermediate 79-3** (150 mg), **intermediate 23-3** (155.24 mg), HATU (212.55 mg) and DIEA (72.25 mg) were dissolved in *N,N*-dimethylformamide (1 mL), and the reaction solution was stirred and reacted at 25°C for 2 hours. After the reaction was completed, the reaction solution was diluted by adding water (10 mL), and extracted with ethyl acetate (10 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 60%-80%, 11 minutes) to obtain the title compound (67.1 mg).

**$^1$H NMR(400 MHz, DMSO-$d_6$)** δ = 13.33-13.27(m, 1H), 9.19(s, 1H), 8.78(s, 1H), 8.08-8.02(m, 2H), 7.51-7.39(m, 3H), 7.28-7.19(m, 2H), 7.05-6.96(m, 1H), 3.51(s, 3H), 2.60(s, 3H), 2.05-1.92(m, 1H), 1.08-0.96(m, 2H), 0.84-0.68(m, 2H).

MS m/z(ESI): = 528.2 $[M+H]^+$.

**Example 80, 4-(5-chloro-2-methoxyphenyl)-*N*-(6-(4-(difluoromethoxy) phenyl)thiazolo[4,5-*b*]pyrazin-2-yl)-6-methylpyridine-3-carboxamide (compound 80)**

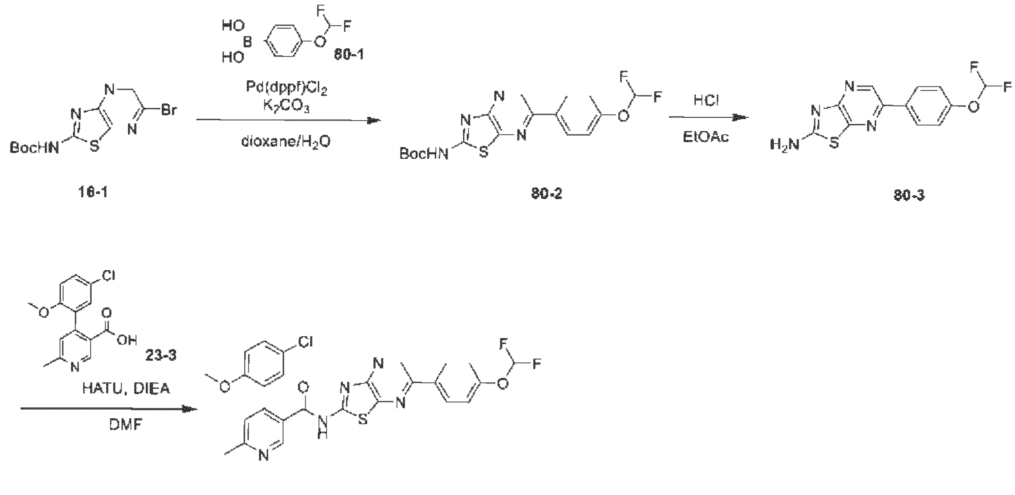


Compound 80

**Step 1: Synthesis of tert-butyl (6-(4-(difluoromethoxy)phenyl)thiazolo[4,5-*b*]pyrazin-2-yl)carbamate (intermediate 80-2)**

**Intermediate 16-1** (1.13 g), **intermediate 80-1** (957.59 mg), potassium carbonate (1.41 g), and 1,1-bis(diphenylphosphine)ferrocene palladium chloride (248.55 mg) were dissolved in dioxane (10 mL) and water (2 mL). Under nitrogen atmosphere, the reaction solution was stirred and reacted at 90°C for 2 h. After the reaction was completed, the reaction solution was cooled to room temperature, diluted with water (50 mL), and extracted with ethyl acetate (45 ml*3). The organic phases were combined, and dried over anhydrous sodium sulphate. After filtration, the organic phase was concentrated to dryness under reduced pressure to obtain the title compound (1.16 g).

MS m/z(ESI): = 395.0 $[M+H]^+$.

**Step 2: Synthesis of (6-(4-(difluoromethoxy)phenyl)thiazolo[4,5-*b*]pyrazin-2-amine (intermediate 80-3)**

**Intermediate 80-2** (510 mg) was dissolved in hydrochloric acid/ethyl acetate (4M) (8 mL), and the reaction solution was stirred and reacted at 25°C for 16 h. After the reaction was completed, the reaction solution was concentrated to dryness under reduced pressure to obtain the title compound (380 mg).

MS m/z(ESI): = 295.1 $[M+H]^+$.

**Step 3: Synthesis of 4-(5-chloro-2-methoxyphenyl)-*N*-(6-(4-(difluoromethoxy)phenyl)thiazolo[4,5-*b*]pyrazin-2-yl)-6-methylpyridine-3-carboxamide (compound 80)**

**Intermediate 80-3** (150 mg), **intermediate 23-3** (141.55 mg), HATU (193.81 mg) and DIEA (65.88 mg) were dissolved in *N,N*-dimethylformamide (2 mL), and the reaction solution was stirred and reacted at 25°C for 2 hours. After the reaction was completed, the reaction solution was diluted by adding water (10 mL), and extracted with ethyl acetate (10 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 53%-73%, 11 minutes) to obtain the title compound (124.8 mg).

**$^1$H NMR(400 MHz, DMSO-$d_6$)**$\delta$ = 13.35(s, 1H), 9.25(s, 1H), 8.80(s, 1H), 8.25(d, *J* = 8.8 Hz, 2H), 7.58-7.18(m, 6H), 7.02(d, *J* = 8.8 Hz, 1H), 3.52(s, 3H), 2.61(s, 3H).

MS m/z(ESI): = 554.1 $[M+H]^+$.

**Example 81, 5'-methoxy-2',6-dimethyl-*N*-(6-(4-(trifluoromethoxy)phenyl) thiazolo[4,5-*b*]pyrazin-2-yl)-[4,4'-bipyridine]-3-carboxamide (compound 81)**

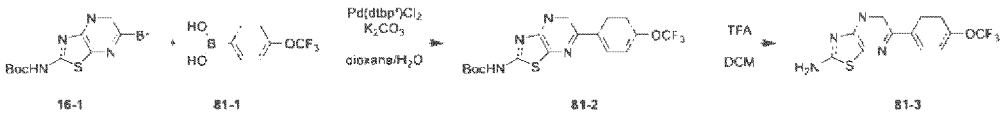


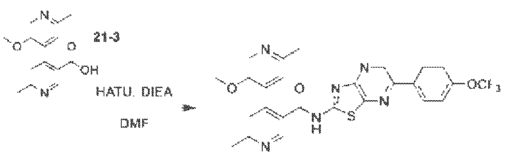


Compound 81

**Step 1: Synthesis of tert-butyl (6-(4-(trifluoromethoxy)phenyl) thiazolo [4,5-*b*]pyrazin-2-yl)carbamate (intermediate 81-2)**

**Intermediate 16-1** (1 g), **intermediate 81-1** (932.67 mg), 1,1'-bis(di-tert-butylphosphine)ferrocene palladium dichloride (196.79 mg), and potassium carbonate (1.25 g) were dissolved in dioxane (10 mL)/water (10 mL). Under nitrogen atmosphere, the reaction solution was stirred at 90°C for 2 h. After the reaction was completed, the reaction solution was cooled to room temperature, diluted with water (30 mL), and extracted with ethyl acetate (10 ml*3). The organic phases were combined, and dried over anhydrous sodium sulphate. After filtration, the organic phase was concentrated to dryness under reduced pressure. The residue was purified by silica gel column chromatography (ISCO®; 4 g SepaFlash® flash silica gel column, gradient: 0-30% ethyl acetate/petroleum ether, flow rate: 30 mL/min) to obtain the title compound (730 mg).

MS m/z(ESI): 412.9 $[M+H]^+$.

**Step 2: Synthesis of 6-(4-(trifluoromethoxy)phenyl)thiazolo[4,5-*b*]pyrazin-2-amine (intermediate 81-3)**

**Intermediate 81-2** (270 mg) was dissolved in dichloromethane (2 mL), and to the solution was added trifluoroacetic acid (2 mL). The reaction solution was stirred and reacted at 90°C for 2 hours. After the reaction was completed, the reaction solution was adjusted to pH 9 with a sodium bicarbonate solution, and extracted with ethyl acetate (33 mL *3). The organic phases were combined, and dried over anhydrous sodium sulphate. After filtration, the organic phase was concentrated to dryness under reduced pressure to obtain the title compound (350 mg).

MS m/z(ESI): 312.9 $[M+H]^+$

**Step 3: Synthesis of 5'-methoxy-2',6-dimethyl-*N*-(6-(4-(trifluoromethoxy) phenyl)thiazolo[4,5-*b*]pyrazin-2-yl)-[4,4'-bipyridine]-3-carboxamide (compound 81)**

**Intermediate 21-3** (164.87 mg), HATU (220.83 mg), and DIEA (136.47 mg) were dissolved in *N,N*-dimethylformamide (2 mL). The reaction solution was stirred and reacted at 50°C for 30 minutes. **Intermediate 81-3** (150 mg) was added to the reaction solution. The reaction solution was stirred and reacted at 50°C for 2 hours. The reaction solution was directly purified by high performance liquid chromatography (chromatographic column: Boston Prime C18 150*30 mm*5 um; mobile phase: A: water (0.225% formic acid), B: acetonitrile; B%: 25%-45%, 11 minutes) to obtain the title compound (74.7 mg).

**$^1$H NMR(400MHz, DMSO-$d_6$)** $\delta$= 13.43(s, 1H), 9.22(s, 1H), 8.88(s, 1H), 8.29(d, *J* = 8.8 Hz, 2H), 8.20(s, 1H), 7.54(d, *J* = 8.1 Hz, 2H), 7.39(s, 1H), 7.30(s, 1H), 3.60(s, 3H), 2.61(s, 3H)

MS m/z(ESI): = 553.1 $[M+H]^+$

**Example 82, *N*-(6-(4-cyclopropylphenyl)thiazolo[4,5-*b*]pyrazin-2-yl)-5'-methoxy-2',6-dimethyl-[4,4'-bipyridine]-3-carboxamide (compound 82)**

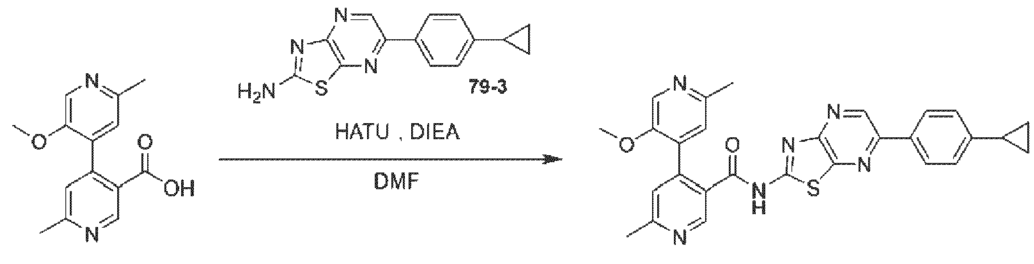


21-3

Compound 82

**Intermediate 21-3** (144.37 mg), **intermediate 79-3** (150 mg), HATU (212.55 mg), and DIEA (72.25 mg) were dissolved in *N,N*-dimethylformamide (1 mL). The mixture was stirred and reacted at 25°C for 2 hours. After the reaction was completed, to the reaction solution was added water (10 mL), and the mixture was extracted with ethyl acetate (15 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 23%-43%, 11 minutes) to obtain the title compound (94.4 mg).

**$^1$H NMR(400 MHz, DMSO-*d*$_6$)**$\delta$ = 13.55-13.31(m, 1H), 9.17(s, 1H), 8.86(s, 1H), 8.19(s, 1H), 8.05(d, *J* = 8.3 Hz, 2H), 7.41(s, 1H), 7.32(s, 1H), 7.24(d, *J* = 8.3 Hz, 2H), 3.59(s, 3H), 2.61(s, 3H), 2.06-1.94(m, 1H), 1.08-0.96(m, 2H), 0.82-0.71(m, 2H).

MS m/z(ESI): = 509.2 [M+H]$^+$

**Example 83, 2'-cyclopropyl-*N*-(6-(4-cyclopropylphenyl)thiazolo[4,5-6*b*pyrazin-2-yl)-5'-methoxy-6-methyl-[4,4'-bipyridine]-3-carboxamide (compound 83)**

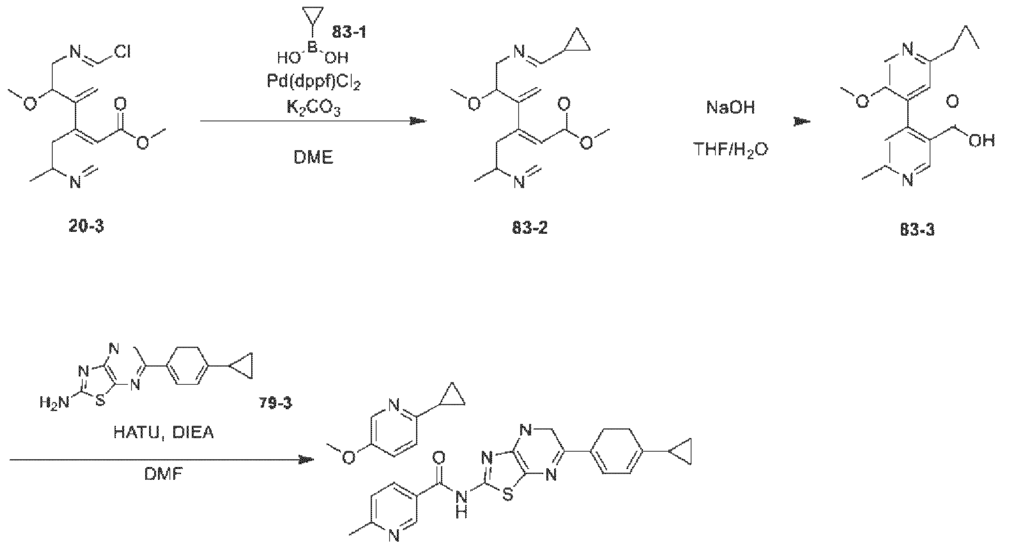


20-3

83-2

83-3

Compound 83

**Step 1: Synthesis of methyl 2'-cyclopropyl-5'-methoxy-6-methyl-[4,4'-bipyridine]-3-carboxylate (intermediate 83-2)**

**Intermediate 20-3** (1 g), **intermediate 83-1** (2.93 g), potassium carbonate (944.29 mg), and 1,1-bis(diphenylphosphine)ferrocene palladium chloride (249.97 mg) were dissolved in 1,2-dimethoxyethane (16 mL). Under nitrogen atmosphere, the reaction solution was stirred and reacted at 90°C for 2 h. After the reaction was completed, the reaction solution was cooled to room temperature, diluted with water (50 mL), and extracted with ethyl acetate (45 ml * 3). The organic phases were combined, and dried over anhydrous sodium sulphate. After filtration, the organic phase was concentrated to dryness under reduced pressure. The residue was purified by silica gel column chromatography (ISCO®; 40 g SepaFlash® flash silica gel column, gradient: 0-30% tetrahydrofuran/petroleum ether, flow rate: 30 mL/min) to obtain

the title compound (400 mg).

MS m/z(ESI): = 298.9 [M+H]+.

**Step 2: Synthesis of 2'-cyclopropyl-5'-methoxy-6-methyl-[4,4'-bipyridine]-3-carboxylic acid (intermediate 83-3)**

**Intermediate 83-2** (400 mg) and sodium hydroxide (268.13 mg) were dissolved in tetrahydrofuran (5 mL) and water (3 mL), and the reaction solution was stirred and reacted at 40°C for 16 h. After the reaction was completed, the reaction solution was adjusted to pH=7 with dilute hydrochloric acid, and then concentrated to dryness under reduced pressure. The residue was dissolved in a solution of dichloromethane/petroleum ether =10/1 (5 mL), and filtered. The filtrate was concentrated to dryness under reduced pressure to obtain the title compound (330 mg).

MS m/z(ESI): = 285.1 [M+H]+

**Step 3: Synthesis of 2'-cyclopropyl-*N*-(6-(4-cyclopropylphenyl) thiazolo[4,5-*b*]pyrazin-2-yl)-5'-methoxy-6-methyl-[4,4'-bipyridine]-3-carboxamide (compound 83)**

**Intermediate 83-3** (158.93 mg), **intermediate 79-3** (150 mg), HATU (212.55 mg) and DIEA (72.25 mg) were dissolved in *N,N*-dimethylformamide (2 mL), and the reaction solution was stirred and reacted at 25°C for 2 hours. After the reaction was completed, the reaction solution was diluted by adding water (10 mL), and extracted with ethyl acetate (10 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 43%-63%, 11 minutes) to obtain the title compound (66.9 mg).

**$^1$H NMR(400 MHz, DMSO-*$d_6$*)**$\delta$ = 13.51-13.33(m, 1H), 9.20(s, 1H), 8.84(s, 1H), 8.16(s, 1H), 8.06(d, *J* = 8.3 Hz, 2H), 7.46(s, 1H), 7.35(s, 1H), 7.27-7.22(m, 2H), 3.58(s, 3H), 2.62(s, 3H), 2.21-2.11(m, 1H), 2.06-1.95(m, 1H), 1.07-1.00(m, 2H), 0.98-0.88(m, 4H), 0.80-0.72(m, 2H).

MS m/z(ESI): = 535.2 [M+H]+.

Example **84, *n*-(6-(4-cyanophenyl)thiazolo[4,5-*b*]pyrazin-2-yl)-4-(5-cyclopropyl-2-methoxyphenyl)-6-methylpyridine-3-carboxamide (compound 84)**

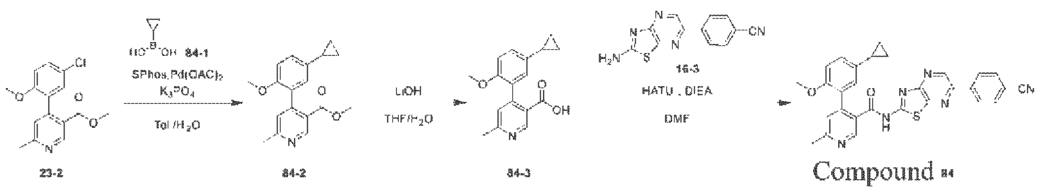


**Step 1: Synthesis of methyl 4-(5-cyclopropyl-2-methoxyphenyl)-6-methylpyridine-3-carboxylate (intermediate 84-2)**

At 20°C, **intermediate 23-2** (2 g) was dissolved in a solution of toluene (20 mL)/water (2 mL), and to the mixture were added **intermediate 84-1** (965.77 mg), 2-dicyclohexylphosphine-2,6-dimethoxybiphenyl (SPhos) (230.79 mg), palladium acetate (63.11 mg) and potassium phosphate (3.58 g). Under nitrogen atmosphere, the reaction solution was stirred and reacted at 95°C for 1 hour. After the reaction was completed, the reaction solution was concentrated under reduced pressure to remove toluene. To the reaction solution was added water (50 mL), and the mixture was extracted with ethyl acetate (40 mL*3). The organic phases were combined, and dried over anhydrous sodium sulphate. After filtration, the organic phase was concentrated to dryness under reduced pressure. The residue was purified by column chromatography (petroleum ether/ethyl acetate =1/1) to obtain the title compound (2.5 g).

MS m/z(ESI): = 298.3 [M+H]+.

**Step 2: Synthesis of 4-(5-cyclopropyl-2-methoxyphenyl)-6-methylpyridine-3-carboxylic acid (intermediate 84-3)**

At 20°C, **intermediate 84-2** (2.5 g) was dissolved in a solution of tetrahydrofuran (20 mL)/water (20 mL), and to the mixture was added lithium hydroxide (503.37 mg). The reaction solution was stirred and reacted at 20°C for 6 hours. After the reaction was completed, the reaction solution was adjusted to pH=4 with a 4 M dilute hydrochloric acid aqueous

solution, and filtered. The filter cake was dried to obtain the title compound (1.7 g).

MS m/z(ESI): = 284.3 [M+H]+.

**Step 3: Synthesis of *N*-(6-(4-cyanophenyl)thiazolo[4,5-*b*]pyrazin-2-yl)-4-(5-cyclopropyl-2-methoxyphenyl)-6-methylpyridine-3-carboxamide (compound 84)**

At 20°C, **intermediate 84-3** (246.09 mg) was dissolved in *N,N*-dimethylformamide (3 mL), and to the mixture were added **intermediate 16-3** (200 mg), HATU (330.27 mg) and DIEA (204.11 mg). The reaction solution was stirred and reacted at 40°C for 4 hours. After the reaction was completed, the reaction solution was purified by high performance liquid chromatography (chromatographic column: Xtimate C18 150*40 mm*5 um; mobile phase: A: water (0.05% ammonia water), B: acetonitrile; B%: 30%-90%, 9 minutes) to obtain the title compound (260 mg).

**$^1$H NMR(400 MHz, DMSO-*d*$_6$)** δ = 13.39(s, 1H), 9.33(s, 1H), 8.73(s, 1H), 8.36(d, *J* = 8.4 Hz, 2H), 8.00(d, *J* = 8.4 Hz, 2H), 7.38(s, 1H), 7.15-7.08(m, 2H), 6.86(d, *J* = 8.6 Hz, 1H), 3.47(s, 3H), 2.60(s, 3H), 1.99-1.94(m, 1H), 0.98-0.89(m, 2H), 0.74-0.65(m, 2H)

MS m/z(ESI): = 519.2 [M+H]+.

**Example 85, 4-(5-cyclopropyl-2-methoxyphenyl)-*N*-(6-(4-(difluoromethoxy)phenyl)thiazolo[4,5-*b*]pyrazin-2-yl)-6-methylpyridine-3-carboxamide (compound 85)**

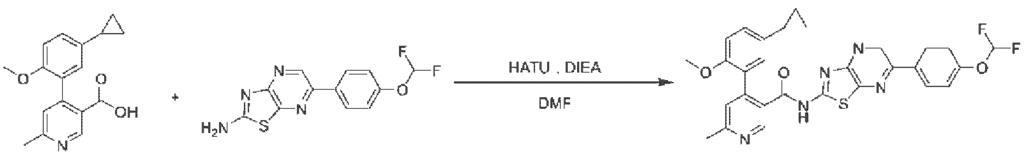


84-3 80-3 Compound 85

At 20°C, **intermediate 84-3** (158.86 mg) was dissolved in *N,N*-dimethylformamide (2 mL), and to the mixture were added **intermediate 80-3** (150 mg), HATU (213.19 mg) and DIEA (131.76 mg). The reaction solution was stirred and reacted at 40°C for 4 hours. After the reaction was completed, the reaction solution was purified by high performance liquid chromatography (chromatographic column: Xtimate C18 150*40 mm*5 um; mobile phase: A: water (0.225% formic acid), B: acetonitrile; B%: 44%-84%, 9 minutes) to obtain the title compound (65 mg).

**$^1$H NMR(400 MHz, DMSO-*d*$_6$)** δ = 13.30(s, 1H), 9.23(s, 1H), 8.73(s, 1H), 8.23(d, *J* = 8.7 Hz, 2H), 7.55-7.18(m, 4H ), 7.14-7.07(m, 2H), 6.86(d, *J* = 8.3 Hz, 1H), 3.47(s, 3H), 2.60(s, 3H), 2.01-1.96(m, 1H), 0.98-0.93(m, 2H), 0.87-0.81(m, 2H)

MS m/z(ESI): = 560.2 [M+H]+.

**Example 86, *N*-(6-(4-cyanophenyl)thiazolo[4,5-*b*]pyrazin-2-yl)-4-(2-methoxy-5-(trifluoromethyl)phenyl)-6-methylpyridine-3-carboxamide (compound 86)**

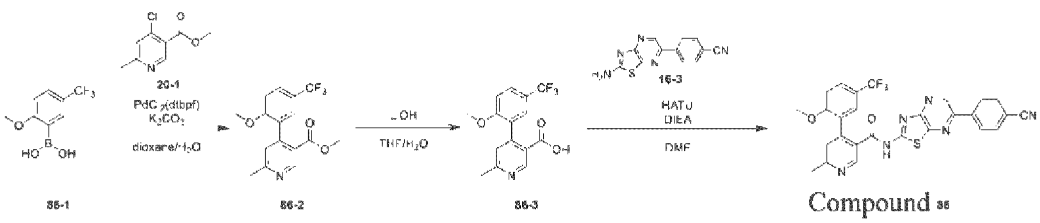


86-1 86-2 86-3 Compound 86

**Step 1: Synthesis of methyl 4-(2-methoxy-5-(trifluoromethyl)phenyl)-6-methylpyridine-3-carboxylate (intermediate 86-2)**

**Intermediate 86-1** (980 mg), **intermediate 20-1** (826.97 mg), 1,1-bis(tert-butylphosphine)ferrocene palladium chloride (290.38 mg), and potassium carbonate (1.23 g) were dissolved in dioxane (10 mL)/water (2 mL). Under nitrogen atmosphere, the reaction solution was stirred at 90°C for 22 h. After the reaction was completed, the reaction solution was

cooled to room temperature, diluted with water (100 mL), and extracted with ethyl acetate (33 ml*3). The organic phases were combined, and dried over anhydrous sodium sulphate. After filtration, the organic phase was concentrated to dryness under reduced pressure. The residue was purified by silica gel column chromatography (ISCO®; 12 g SepaFlash® flash silica gel column, gradient: 0-26% ethyl acetate/petroleum ether, flow rate: 36 mL/min) to obtain the title compound (1.28 g).

MS m/z(ESI): = 326.1 $[M+H]^+$

**Step 2: Synthesis of 4-(2-methoxy-5-(trifluoromethyl)phenyl)-6-methylpyridine-3-carboxylic acid (intermediate 86-3)**

**Intermediate 86-2** (1.08 g) was dissolved in tetrahydrofuran (10 mL)/water (10 mL), and lithium hydroxide (174.94 mg) was added. The reaction solution was stirred and reacted at 20°C for 16 hours. After the reaction was completed, the reaction solution was diluted with water (150 mL), and extracted with ethyl acetate (50 mL *3). The organic phases were combined, washed with saturated sodium chloride aqueous solution (30 mL *3), and dried over anhydrous sodium sulphate. After filtration, the organic phase was concentrated to dryness under reduced pressure to obtain the title compound (1 g).

MS m/z(ESI): 312.1 $[M+H]^+$

**Step 3: Synthesis of *N*-(6-(4-cyanophenyl)thiazolo[4,5-*b*]pyrazin-2-yl)-4-(2-methoxy-5-(trifluoromethyl)phenyl)-6-methylpyridine-3-carboxamide (compound 86)**

**Intermediate 86-3** (400 mg), HATU (488.64 mg) and DIEA (830.44 mg) were dissolved in *N*,*N*-dimethylformamide (5 mL), and the reaction solution was stirred and reacted at 50°C for 30 minutes. **Intermediate 16-3** (325.50 mg) was added to the reaction solution. The reaction solution was stirred and reacted at 50°C for 4 hours. After the reaction was completed, the reaction solution was directly purified by high performance liquid chromatography (chromatographic column: Boston Prime C18 150*30 mm*5 um; mobile phase: A: water (0.225% formic acid), B: acetonitrile; B%: 30%-50%, 11 minutes) to obtain the title compound (49.7 mg).

**$^1$H NMR(400MHz, DMSO-$d_6$)** δ= 13.50(s, 1H), 9.36(s, 1H), 8.84(s, 1H), 8.38(d, *J* = 8.6 Hz, 2H), 8.02(d, *J* = 8.4 Hz, 2H), 7.82-7.76(m, 2H), 7.46(s, 1H), 7.20(d, *J* = 8.6 Hz, 1H), 3.61(s, 3H), 2.62(s, 3H)

MS m/z(ESI): = 547.1 [M+H]

**Example 87, *N*-(6-(4-cyanophenyl)thiazolo[4,5-*b*]pyrazin-2-yl)-4-(2,5-dichlorophenyl)-6-methylpyridine-3-carboxamide (compound 87)**

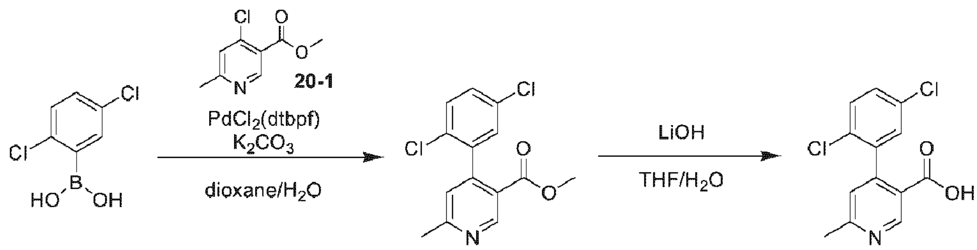


87-1 87-2 87-3

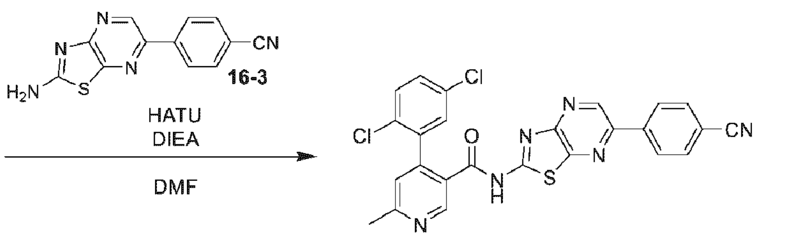


Compound 87

**Step 1: Synthesis of methyl 4-(2,5-dichlorophenyl)-6-methylpyridine-3-carboxylate (intermediate 87-2)**

**Intermediate 87-1** (500 mg), **intermediate 20-1** (486.34 mg), potassium carbonate (724.27 mg), and 1,1-bis(tert-butylphosphine)ferrocene palladium chloride (170.78 mg) were dissolved in dioxane (2 mL) and water (0.4 mL). Under nitrogen atmosphere, the reaction solution was stirred and reacted at 90°C for 2 h. After the reaction was completed, the reaction solution was cooled to room temperature, diluted with water (10 mL), and extracted with ethyl acetate (15 ml * 3). The organic phases were combined, and dried over anhydrous sodium sulphate. After filtration, the organic phase was concentrated to dryness under reduced pressure. The residue was purified by silica gel column chromatography (ISCO®;
40 g SepaFlash® flash silica gel column, gradient: 0-30% ethyl acetate/petroleum ether, flow rate: 40 mL/min) to obtain the title compound (800 mg).
MS m/z(ESI): = 296.2 $[M+H]^+$.

**Step 2: Synthesis of 4-(2,5-dichlorophenyl)-6-methylpyridine-3-carboxylic acid (intermediate 87-3)**

**Intermediate 87-2** (800 mg) and lithium hydroxide (249.39 mg) were dissolved in tetrahydrofuran (6 mL) and water (3 mL), and the reaction solution was stirred and reacted at 25°C for 4 h. After the reaction was completed, the reaction solution was adjusted to pH=7 with dilute hydrochloric acid, and then concentrated to dryness under reduced pressure. The residue was dissolved in a solution of dichloromethane/petroleum ether =10/1 (5 mL), and filtered. The filtrate was concentrated to dryness under reduced pressure to obtain the title compound (620 mg).
MS m/z(ESI): = 282.2 $[M+H]^+$.

**Step 3: Synthesis of *N*-(6-(4-cyanophenyl)thiazolo[4,5-*b*]pyrazin-2-yl)-4-(2,5-dichlorophenyl)-6-methylpyridine-3-carboxamide (compound 87)**

**Intermediate 87-3** (150 mg), **intermediate 16-3** (134.67 mg), HATU (202.16 mg) and DIEA (206.15 mg) were dissolved in *N*,*N*-dimethylformamide (3 mL), and the reaction solution was stirred and reacted at 25°C for 2 hours. After the reaction was completed, the reaction solution was diluted by adding water (15 mL), and extracted with ethyl acetate (10 mL*3). The organic phases were combined, and dried over anhydrous sodium sulphate. After filtration, the organic phase was concentrated to dryness under reduced pressure. The residue was purified by high performance liquid chromatography (chromatographic column: Boston Prime C18 150*30 mm*5 um; mobile phase: A: water (0.225% formic acid), B: acetonitrile; B%: 28%-48%, 11 minutes) to obtain the title compound (158.1 mg).
**$^1$H NMR(400 MHz, DMSO-$d_6$)** δ = 9.32(s, 1H), 9.06(s, 1H), 8.37(d, *J* = 8.5 Hz, 2H), 8.01(d, *J*= 8.5 Hz, 2H), 7.58-7.53(m, 3H), 7.43-7.34(m, 1H), 2.63(s, 3H).
MS m/z(ESI): = 517.1 $[M+H]^+$.

**Example 88, 4-(5-chloro-2-(trifluoromethyl)phenyl)-*N*-(6-(4-cyanophenyl) thiazolo[4,5-*b*]pyrazin-2-yl)-6-methylpyridine-3-carboxamide (compound 88)**

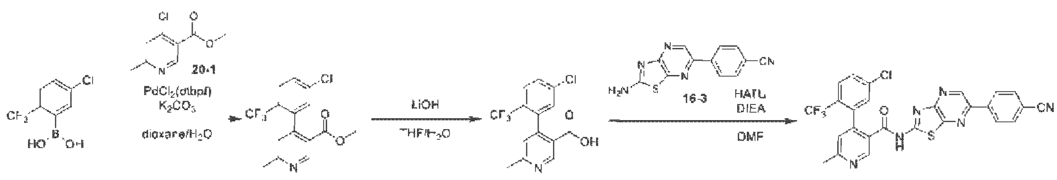


88-1 88-2 88-3 Compound 88

**Step 1: Synthesis of methyl 4-(5-chloro-2-(trifluoromethyl)phenyl)-6-methylpyridine-3-carboxylate (intermediate 88-2)**

At 20°C, **intermediate 88-1** (400 mg) was dissolved in a solution of dioxane (8 mL)/water (1.6 mL), and to the mixture were added **intermediate 20-1** (330.89 mg), 1,1-bis(tert-butylphosphine)ferrocene palladium chloride (116.19 mg) and potassium carbonate (492.77 mg). Under nitrogen atmosphere, the reaction solution was stirred and reacted at 90°C for 1 hour. After the reaction was completed, the reaction solution was concentrated to dryness under reduced pressure. The residue was purified by column chromatography (petroleum ether/ethyl acetate =3/1) to obtain the title compound (540 mg).
MS m/z(ESI): = 330.2 $[M+H]^+$.

**Step 2: Synthesis of 4-(5-chloro-2-(trifluoromethyl)phenyl)-6-methylpyridine-3-carboxylic acid (intermediate 88-3)**

At 20°C, **intermediate 2** (540 mg) was dissolved in a solution of tetrahydrofuran (5 mL)/water (5 mL), and to the mixture was added lithium hydroxide (98.06 mg). The reaction solution was stirred and reacted at 20°C for 6 hours. After the reaction was completed, the reaction solution was adjusted to pH=4 with a 4 M dilute hydrochloric acid aqueous solution, and extracted with ethyl acetate (20 mL * 3). The organic phase was concentrated to dryness under reduced pressure to obtain the title compound (500 mg).
MS m/z(ESI): = 316.2 $[M+H]^+$.

**Step 3: Synthesis of 4-(5-chloro-2-(trifluoromethyl)phenyl)-*N*-(6-(4-cyanophenyl)thiazolo[4,5-*b*]pyrazin-2-yl)-6-methylpyridine-3-carboxamide (compound 88)**

At 20°C, **intermediate 88-3** (205.65 mg) was dissolved in *N*,*N*-dimethylformamide (3 mL), and to the mixture were added **intermediate 16-3** (150 mg), HATU (225.18 mg) and DIEA (153.08 mg). The reaction solution was stirred and reacted at 40°C for 4 hours. After the reaction was completed, the reaction solution was purified by high performance liquid chromatography (chromatographic column: Boston Prime C18 150*30 mm*5 um; mobile phase: A: water (0.05% ammonia water), B: acetonitrile; B%: 30%-50%, 11 minutes) to obtain the title compound (106.6 mg).
**$^1$H NMR(400 MHz, DMSO-$d_6$)**$\delta$ = 13.81-13.64(m, 1H), 9.35(s, 1H), 9.07(s, 1H), 8.37(d, *J* = 8.5 Hz, 2H), 8.02(d, *J* = 8.4 Hz, 2H), 7.87-7.83(m, 2H), 7.81-7.77(m, 1H), 7.45(s, 1H), 2.64(s, 3H)
MS m/z(ESI): = 551.1 $[M+H]^+$.

**Example 89, 4-(5-chloro-2-(difluoromethoxy)phenyl)-*N*-(6-(4-cyanophenyl) thiazolo[4,5-*b*]pyrazin-2-yl)-6-methylpyridine-3-carboxamide (compound 89)**

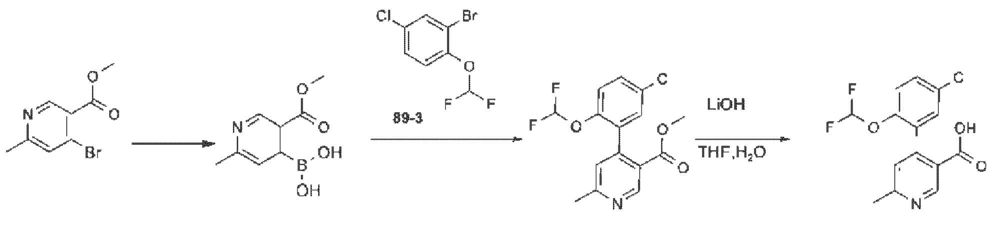


89-1 89-2 89-4 89-5

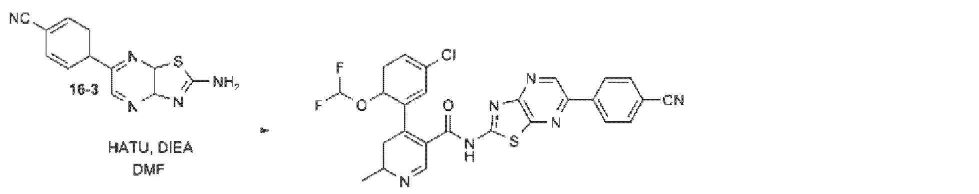


Compound 89

**Step 1: Synthesis of (5-methoxycarbonyl-2-methylpyridine-4-yl)boronic acid (intermediate 89-2)**

Under nitrogen atmosphere, **intermediate 89-1** (10 g), 1,1'-bis(diphenylphosphino)ferrocene palladium dichloride (II) (3.1 g), bis(pinacolato)diboron (13.2 g) and potassium acetate (8.5 g) were added to dioxane (100 mL), and the reaction solution was stirred at 100°C for 18 hours. After the completion of the reaction detected by LCMS, the reaction solution was filtered, and the filtrate was concentrated under reduced pressure to obtain the title compound (22 g).

**Step 2: Synthesis of methyl 4-(5-chloro-2-(difluoromethoxy)phenyl)-6-methylpyridine-3-carboxylate (intermediate 89-4)**

Under nitrogen atmosphere, **intermediate 89-3** (990 mg), **intermediate 89-2** (1 g), 1,1'-bis(diphenylphosphino)ferrocene palladium dichloride (II) (187 mg) and potassium phosphate (1.08 g) were added to dioxane (15 mL) and $H_2O$ (5 mL), and the reaction solution was stirred at 100°C for 18 hours. The reaction solution was cooled to room temperature, quenched by adding water (30 mL), and extracted with ethyl acetate (40 mL * 3). The organic layers were combined, washed with saturated sodium chloride aqueous solution (15 mL *2), dried over anhydrous sodium sulphate, and filtered. The filtrate was concentrated to dryness under reduced pressure, and the residue was purified by column chromatography (petroleum ether/ethyl acetate =5/1) to obtain the title compound (230 mg).

**Step 3: Synthesis of 4-(5-chloro-2-(difluoromethoxy)phenyl)-6-methylpyridine-3-carboxylic acid (intermediate 89-5)**

**Intermediate 89-4** (200 mg) and lithium hydroxide (29 mg) were added to THF (3 mL) and $H_2O$ (1 mL), and the resulting reaction solution was stirred at 50°C for 4 h. The reaction solution was cooled to room temperature, adjusted to pH=6 by adding 1 M hydrochloric acid, and extracted with ethyl acetate (40 mL * 3). The organic layers were combined, washed with saturated sodium chloride aqueous solution (15 mL *2), dried over anhydrous sodium sulphate, and filtered. The filtrate was concentrated to dryness under reduced pressure, and the residue was purified by column chromatography (DCM/MeOH=10/1) to obtain the title compound (120 mg).

**Step 4: Synthesis of 4-(5-chloro-2-(difluoromethoxy)phenyl)-*N*-(6-(4-cyanophenyl)thiazolo[4,5-*b*]pyrazin-2-yl)-6-methylpyridine-3-carboxamide (compound 89)**

**Intermediate 16-3** (121 mg), **89-5** (100 mg), HATU (132 mg) and DIEA (82 mg) were added to DMF (3 mL). Under nitrogen atmosphere, the reaction solution was stirred at 25°C for 18 hours. After the reaction was completed, the reaction solution was filtered, and the filtrate was concentrated to dryness under reduced pressure. The residue was purified by high performance liquid chromatography (chromatographic column: Waters Xbridge BEH C18 100*30 mm*10 um; mobile phase: A: water (0.05% formic acid), B: acetonitrile; B%: 40%-80%, 10 minutes) to obtain the title compound (67 mg).
**$^1$H NMR(400 MHz, DMSO-$d_6$)** $\delta$ 13.61(s, 1H), 9.34(s, 1H), 8.96(s, 1H), 8.41-8.34(m, 2H), 8.05-7.98(m, 2H), 7.63-7.55(m, 2H), 7.43(s, 1H), 7.34-6.71(m, 2H), 2.62(s, 3H).
MS m/z(ESI): = 549.1 $[M+H]^+$.

**Example 90, 4-(5-chloro-2-(trifluoromethoxy)phenyl)-*N*-(6-(4-cyanophenyl)thiazolo[4,5-*b*]pyrazin-2-yl)-6-methylpyridine-3-carboxamide (compound 90)**

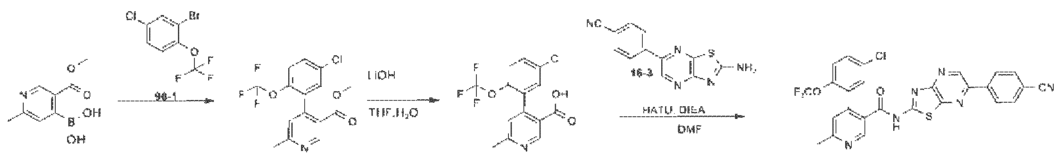


89-2 90-2 90-3 Compound 90

**Step 1: Synthesis of methyl 4-(5-chloro-2-(trifluoromethoxy)phenyl)-6-methylpyridine-3-carboxylate (intermediate 90-2)**

**Intermediate 90-1** (745 mg), **intermediate 89-2** (1 g), potassium phosphate (761 mg) and 1,1'-bis(diphenylphosphino)ferrocene palladium dichloride (II) (131 mg) were added to dioxane (15 mL) and $H_2O$ (4 mL). Under nitrogen atmosphere, the reaction solution was stirred at 100°C for 18 hours. The reaction solution was cooled to room temperature, quenched by adding water (30 mL), and extracted with ethyl acetate (40 mL * 3). The organic layers were combined, washed with saturated sodium chloride aqueous solution (15 mL *2), dried over anhydrous sodium sulphate, and filtered. The filtrate was concentrated to dryness under reduced pressure, and the residue was purified by column chromatography (petroleum ether/ethyl acetate =5/1) to obtain the title compound (320 mg).

**Step 2: Synthesis of 4-(5-chloro-2-(trifluoromethoxy)phenyl)-6-methylpyridine-3-carboxylic acid (intermediate 90-3)**

**Intermediate 90-2** (300 mg) and lithium hydroxide (83 mg) were added to a mixed solvent of THF (3 mL) and $H_2O$ (1 mL), and the reaction solution was stirred at 50°C for 4 h. The reaction solution was cooled to room temperature,

adjusted to pH 6 by adding 1 M hydrochloric acid, and extracted with ethyl acetate (40 mL * 3). The organic layers were combined, washed with saturated sodium chloride aqueous solution (15 mL *2), dried over anhydrous sodium sulphate, and filtered. The filtrate was concentrated to dryness under reduced pressure, and the residue was purified by column chromatography (DCM/MeOH=10/1) to obtain the title compound (220 mg).

**Step 3: Synthesis of 4-(5-chloro-2-(trifluoromethoxy)phenyl)-*N*-(6-(4-cyanophenyl)thiazolo[4,5-*b*]pyrazin-2-yl)-6-methylpyridine-3-carboxamide (compound 90)**

**Intermediate 16-3** (229 mg), **intermediate 90-3** (200 mg), HATU (250 mg) and DIEA (155 mg) were added to DMF (4 mL). Under nitrogen atmosphere, the reaction solution was stirred at 25°C for 18 hours. After the reaction was completed, the reaction solution was filtered, and the filtrate was concentrated to dryness under reduced pressure. The residue was purified by high performance liquid chromatography (chromatographic column: Waters Xbridge BEH C18 100*30 mm*10 um; mobile phase: A: water (0.05% formic acid), B: acetonitrile; B%: 40%-80%, 10 minutes) to obtain the title compound (110 mg).
**$^1$H NMR(400 MHz, DMSO-*d*$_6$)** δ = 13.71(s, 1H), 9.35(s, 1H), 8.99(s, 1H), 8.42-8.34(m, 2H), 8.06-7.99(m, 2H), 7.76-7.71(m, 1H), 7.70-7.62(m, 1H), 7.52-7.42(m, 2H), 2.63(s, 3H).
MS m/z(ESI): = 567.0 [M+H]$^+$.

**Example 91: 4-(5-chloro-2-(methoxy-*d*$_3$)phenyl)-*N*-(6-(4-cyanophenyl) thiazolo[4,5-*b*]pyrazin-2-yl)-6-methylpyridine-3-carboxamide (compound 91)**

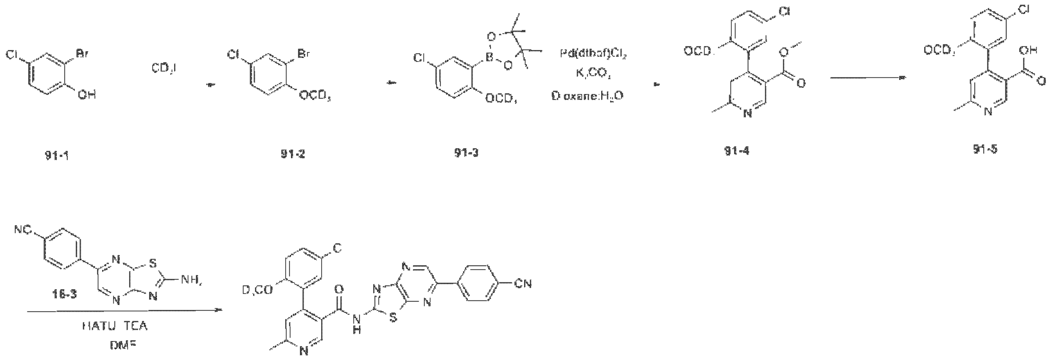


91-1 91-2 91-3 91-4 91-5

Compound 91

**Step 1: Synthesis of 2-bromo-4-chloro-1-(methoxy-*d*$_3$)benzene (intermediate 91-2)**

**Intermediate 91-1** (2.0 g), potassium carbonate (4.0 g) and deuterated iodomethane (2.1 g) were dissolved in NMP (15 mL), and the resulting reaction solution was stirred at 100°C for 2 hours. After the reaction was completed, to the reaction solution were added ethyl acetate (100 mL) and water (50 mL), and the organic phase was washed with saturated sodium chloride aqueous solution (30 mL*2), separated, and dried over anhydrous sodium sulphate. After filtration, the organic phase was concentrated to dryness under reduced pressure. The residue was purified by silica gel column chromatography (petroleum ether/ethyl acetate =100/1) to obtain the title compound (2.1 g).
**$^1$H NMR(400 MHz, DMSO-*d*$_6$)**δ 7.68(d, *J* = 2.5 Hz, 1H), 7.43(dd, *J* = 8.9, 2.5 Hz, 1H), 7. 13(d, *J* = 8.9 Hz, 1H).

**Step 2: Synthesis of 2-(5-chloro-2-(methoxy-*d*$_3$)phenyl)-4,4,5,5-tetramethyl-1,3,2-dioxaborane (intermediate 91-3)**

Under nitrogen atmosphere, **intermediate 91-2** (500 mg), Pd(dtbpf)Cl2 (325 mg), potassium acetate (655 mg) and bis(pinacolato)diboron (678 mg) were dissolved in dioxane (10 mL), and the mixture was warmed to 70°C and stirred for 16 h. The reaction solution was cooled to room temperature, quenched by adding water (20 mL), and extracted with ethyl acetate (20 mL *3). The organic phases were combined, dried over anhydrous sodium sulphate, and filtered. The filtrate was concentrated to dryness under reduced pressure to obtain the title compound (600 mg).
MS m/z(ESI): = 272.1 [M+H]$^+$.

**Step 3: Synthesis of methyl 4-(5-chloro-2-(methoxy-*d*$_3$)phenyl)-6-methylpyridine-3-carboxylate (intermediate 91-4)**

Under nitrogen atmosphere, **intermediate 91-3** (600 mg), Pd(dtbpf)$Cl_2$ (161 mg), potassium carbonate (916 mg) and methyl 4-bromo-6-methylpyridine-3-carboxylate (508 mg) were dissolved in dioxane (10 mL) and water (2.5 mL), and the mixture was warmed to 90°C and stirred for 2 h. The reaction solution was cooled to room temperature, quenched by adding water (20 mL), and extracted with ethyl acetate (20 mL *3). The organic phases were combined, with? dried, and filtered. The filtrate was concentrated to dryness under reduced pressure to obtain the title compound (480 mg).
MS m/z(ESI): = 295.1 $[M+H]^+$.

**Step 4: Synthesis of 4-(5-chloro-2-(methoxy-*d*$_3$)phenyl)-6-methylpyridine-3-carboxylic acid (compound 91-5)**

**Intermediate 91-4** (301 mg) and lithium hydroxide (490 mg) were dissolved in tetrahydrofuran (5 mL) and water (5 mL), and the mixture was warmed to 40°C and stirred for 16 h. The reaction solution was cooled to room temperature, adjusted to pH=6 with 1 M hydrochloric acid, and extracted with ethyl acetate (20 mL *3). The organic phases were combined, dried over anhydrous sodium sulphate, and filtered. The filtrate was concentrated to dryness under reduced pressure, and the residue was purified by silica gel column chromatography (DCM/MeOH=10/1) to obtain the title compound (128 mg).
MS m/z(ESI): = 281.1 $[M+H]^+$

**Step 5: Synthesis of 4-(5-chloro-2-(methoxy-*d*$_3$)phenyl)-*N*-(6-(4-cyanophenyl)thiazolo[4,5-*b*]pyrazin-2-yl)-6-methylpyridine-3-carboxamide (compound 91)**

**Intermediate 91-5** (116 mg), **intermediate 16-3** (105 mg), triethylamine (125 mg) and HATU(164 mg) were dissolved in DMF (2 mL), and the mixture was warmed to 40°C and stirred for 2 h. The reaction solution was cooled to room temperature, quenched by adding water (10 mL), and extracted with ethyl acetate (20 mL *3). The organic phases were combined, dried over anhydrous sodium sulphate, and filtered. The filtrate was concentrated to dryness under reduced pressure, and the residue was purified by high performance liquid chromatography (YMC-Actus Triart C18 column, 5 um silica, 30 mm in diameter, 150 mm in length; using a decreasingly polar mixture of water (containing 0.225% formic acid) and acetonitrile as the eluent; acetonitrile gradient proportion: 40%-70%, elution time: 15 minutes) to obtain the title compound (64.1 mg).
MS m/z(ESI): = 516.1 $[M+H]^+$
**$^1$H NMR(400 MHz, DMSO-*d*$_6$)**$\delta$ 13.44(s, 1H), 9.35(s, 1H), 8.80(s, 1H), 8.43-8.34(m, 2H), 8.07-7.96(m, 2H), 7.52-7.44(m, 2H), 7.42(s, 1H), 7.01(d, *J*= 8.7 Hz, 1H), 2.60(s, 3H).

**Example 92, *N*-(6-(4-cyanophenyl)thiazolo[4,5-*b*]pyrazin-2-yl)-2-(5-methoxy-2-methylpyridine-4-yl)-4-methylbenzamide (compound 92)**

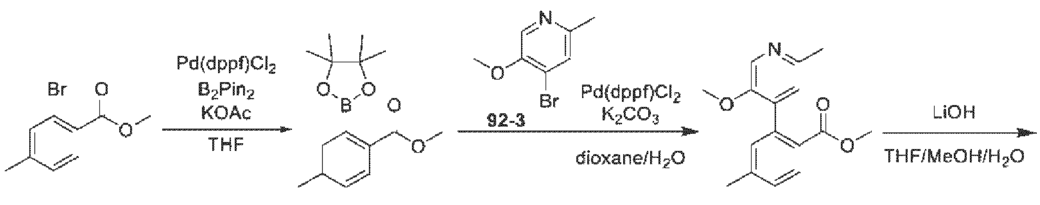


92-1 92-2 92-4

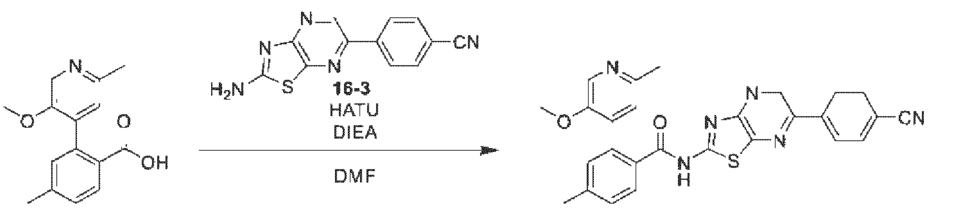


92-5 Compound 92

**Step 1: Synthesis of methyl 4-methyl-2-(4,4,5,5-tetramethyl-1,3,2-dioxaboran-2-yl)benzoate (intermediate 92-2)**

At 20°C, **intermediate 92-1** (5.00 g) was dissolved in tetrahydrofuran (100 mL), and to the reaction solution were added bis(pinacolato)diboron (7.21 g), potassium acetate (6.42 g) and 1,1'-bis(diphenylphosphino)ferrocene palladium dichloride (II) (1.60 g). Under nitrogen atmosphere, the reaction solution was stirred at 70°C for 10 hours. After the reaction was completed, the reaction solution was filtered, and the filtrate was concentrated to dryness under reduced pressure. The residue was purified by silica gel column chromatography (petroleum ether/ethyl acetate =20/1) to obtain the title compound (5.30 g).

MS m/z (ESI): = 277.1 $[M+H]^+$.

**Step 2: Synthesis of methyl 2-(5-methoxy-2-methylpyridine-4-yl)-4-benzoate (intermediate 92-4)**

At 20°C, **intermediate 92-3** (1.00 g) was dissolved in a solution of dioxane (25 mL)/water (5 mL), and to the reaction solution were added **intermediate 92-2** (1.78

g), potassium carbonate (2.05 g) and 1,1'-bis(diphenylphosphino)ferrocene palladium dichloride (II) (361 mg). Under nitrogen atmosphere, the reaction solution

was stirred at 90°C for 12 hours. After the reaction was completed, the reaction solution was filtered, and the filtrate was concentrated to dryness under reduced pressure. The residue was purified by silica gel column chromatography (petroleum ether/ethyl acetate =5/1) to obtain the title compound (1.30 g).

MS m/z (ESI): = 272.2 $[M+H]^+$.

**Step 3: Synthesis of 2-(5-methoxy-2-methylpyridine-4-yl)-4-methylbenzoic acid (intermediate 92-5)**

At 20°C, **intermediate 92-4** (700 mg) was dissolved in a solution of tetrahydrofuran (6 mL)/methanol (2 mL)/water (2 mL), and to the reaction solution was added lithium hydroxide (247 mg). The reaction solution was stirred at 50°C for 48 hours. After the reaction was completed, the reaction solution was concentrated under reduced pressure, and the residue was adjusted to pH=3 with dilute hydrochloric acid, and filtered. The filter cake was dried to obtain the title compound (400 mg).

MS m/z (ESI): = 258.1 $[M+H]^+$.

**Step 4: Synthesis of *N*-(6-(4-cyanophenyl)thiazolo[4,5-*b*]pyrazin-2-yl)-2-(5-methoxy-2-methylpyridine-4-yl)-4-methylbenzamide (compound 92)**

At 20°C, **intermediate 92-5** (380 mg) was dissolved in DMF(5 mL), and to the reaction solution were added **intermediate 16-3** (374 mg), HATU (557 mg) and DIEA (572 mg). The reaction solution was stirred at 50°C for 48 hours. After the reaction was completed, the reaction solution was filtered. The filter cake was washed twice with DMF (2 mL), and dried to obtain the title compound (160 mg).

**$^1$H NMR (400 MHz, DMSO-$d_6$)** δ 13.31 (s, 1H), 9.34 (s, 1H), 8.40-8.34 (m, 2H), 8.12 (s, 1H), 8.03-7.95 (m, 2H), 7.72 (d, *J*= 7.8 Hz, 1H), 7.43 - 7.37 (m, 1H), 7.30 (s, 1H), 7.26 (s, 1H), 3.56 (s, 3H), 2.48 (s, 3H), 2.44 (s, 3H).

MS m/z (ESI): = 493.1 $[M+H]^+$.

**Example 93, 5'-chloro-*N*-(6-(4-cyanophenyl)thiazolo[4,5-*b*]pyrazin-2-yl)-2'-methoxy-5-methyl-[1,1'-biphenyl]-2-carboxamide (compound 93)**

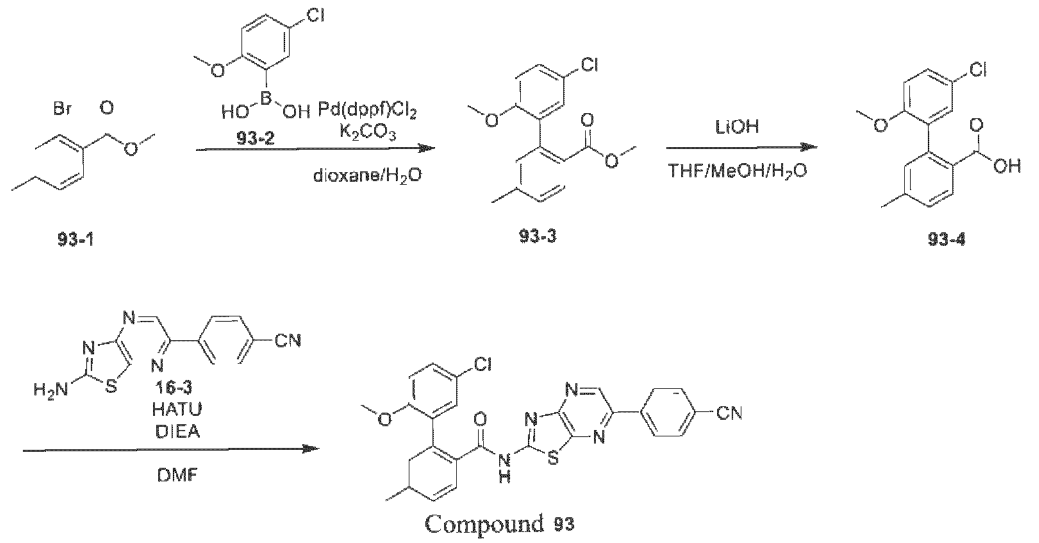


93-1 93-3 93-4

Compound 93

**Step 1: Synthesis of methyl 5'-chloro-2'-methoxy-5-methyl-[1,1'-biphenyl]-2-carboxylate (intermediate 93-3)**

At 20°C, **intermediate 93-1** (5.00 g) was dissolved in a solution of dioxane (100 mL)/water (20 mL), and to the reaction solution were added **intermediate 93-2** (4.88 g), potassium carbonate (9.04 g) and 1,1'-bis(diphenylphosphino) ferrocene palladium dichloride (II) (1.60 g). Under nitrogen atmosphere, the reaction solution was stirred at 90°C for 12 hours. After the reaction was completed, the reaction solution was filtered, and the filtrate was concentrated to dryness under reduced pressure. The residue was purified by column chromatography (petroleum ether/ethyl acetate =10/1) to obtain the title compound (6.10 g).

MS m/z (ESI): = 291.1 $[M+H]^+$.

**Step 2: Synthesis of 5'-chloro-2'-methoxy-5-methyl-[1,1'-biphenyl]-2-carboxylic acid (intermediate 93-4)**

At 20°C, **intermediate 93-3** (1.00 g) was dissolved in a solution of tetrahydrofuran (10 mL)/methanol (5 mL)/water (5 mL), and to the reaction solution was added lithium hydroxide (1.65 g). The reaction solution was stirred at 50°C for 40 hours. After the reaction was completed, the reaction solution was concentrated under reduced pressure, and the residue was adjusted to pH=3 with dilute hydrochloric acid, and filtered. The filter cake was dried to obtain the title compound (800 mg).

MS m/z (ESI): = 277.1 $[M+H]^+$.

**Step 3: Synthesis of 5'-chloro-*N*-(6-(4-cyanophenyl)thiazolo[4,5-*b*]pyrazin-2-yl)-2'-methoxy-5-methyl-[1,1'-biphenyl]-2-carboxamide (compound 93)**

At 20°C, **intermediate 93-4** (200 mg) was dissolved in DMF (5 mL), and to the reaction solution were added **intermediate 16-3** (238 mg), HATU (273 mg) and DIEA (280 mg). The reaction solution was stirred at 50°C for 36 hours. After the reaction was completed, the reaction solution was filtered, and the filtrate was concentrated to dryness under reduced pressure. The residue was purified by preparative high performance liquid chromatography (YMC-Actus Triart C18 column, 5 um silica, 30 mm in diameter, 150 mm in length; using a decreasingly polar mixture of water (containing 7 mmol $NH_4HCO_3$) and acetonitrile as the eluent; acetonitrile gradient proportion: 50%-90%, elution time: 10 minutes) to obtain the title compound (65 mg).

**$^1$H NMR (400 MHz, DMSO-*d*$_6$)** δ 13.20 (s, 1H), 9.33 (s, 1H), 8.37 (d, *J*= 8.3 Hz, 2H), 8.01 (d, *J*= 8.2 Hz, 2H), 7.68 (d, *J*= 7.8 Hz, 1H), 7.44 - 7.34 (m, 3H), 7.27 (s, 1H), 6.95 (d, *J*= 9.5 Hz, 1H), 3.49 (s, 3H), 2.43 (s, 3H).

MS m/z (ESI): = 512.1 $[M+H]^+$.

**Example 94, 4-[5-chloro-2-(cyclopropoxy)phenyl]-N-[6-(4-cyanophenyl) thiazolo[4,5-b]pyrazin-2-yl]-6-methyl-pyridine-3-carboxamide (compound 94)**

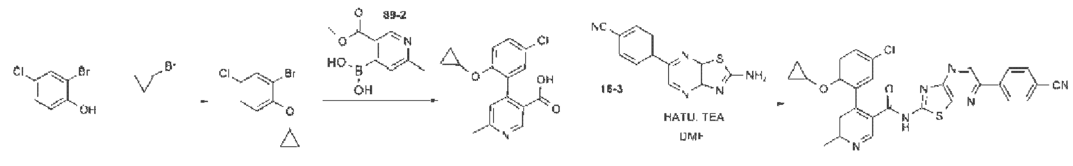


94-1 94-2 94-4 Compound 94

**Step 1: Synthesis of 2-bromo-4-chloro-1-cyclopropoxybenzene (intermediate 94-2)**

Under nitrogen atmosphere, **intermediate 94-1** (0.7 g), bromocyclopropane (2.0 g) and caesium carbonate (2.7 g) were dissolved in dimethyl sulfoxide (20 mL). After the addition, the mixture was warmed to 150°C and stirred for 5 h. The reaction solution was poured into ethyl acetate (50 mL) and water (50 mL), and the mixture was extracted. The aqueous phase was extracted with ethyl acetate (50 mL*2). The organic phases were combined, washed twice with saturated sodium chloride aqueous solution (30 mL), dried over anhydrous sodium sulphate, and filtered. The filtrate was concentrated to dryness under reduced pressure, and the residue was purified by silica gel column chromatography (petroleum ether/ethyl acetate =20/1) to obtain the title compound (0.6 g).
MS m/z (ESI): = 247.1 $[M+H]^+$.

**Step 2: Synthesis of 4-(5-chloro-2-cyclopropoxyphenyl)-6-methylpyridine-3-carboxylic acid (intermediate 94-4)**

Under nitrogen atmosphere, **intermediate 94-2** (300 mg), **intermediate 89-2** (671 mg), potassium carbonate (502 mg) and $Pd(dppf)Cl_2$ (88 mg) were dissolved in dioxane (8 mL) and water (2 mL). After the addition, the mixture was warmed to 70°C and stirred for 16 h. The reaction solution was poured into ethyl acetate (50 mL) and water (50 mL), and the mixture was extracted. The aqueous phase was extracted with ethyl acetate (50 mL*2). The organic phases were combined, washed twice with saturated sodium chloride aqueous solution (30 mL), dried over anhydrous sodium sulphate, and filtered. The filtrate was concentrated to dryness under reduced pressure, and the residue was purified by silica gel column chromatography (petroleum ether/ethyl acetate =1/1) to obtain the title compound (80 mg).
MS m/z (ESI): = 304.1 $[M+H]^+$.

**Step 3: Synthesis of 4-(5-chloro-2-cyclopropoxyphenyl)-*N*-(6-(4-cyanophenyl)thiazolo[4,5-*b*]pyrazin-2-yl)-6-methylpyridine-3-carboxamide (compound 94)**

**Intermediate 94-4** (72 mg) was dissolved in anhydrous *N*,*N*-dimethylformamide (3 mL), and HATU (89 mg) and triethylamine (72 mg) were added. After the addition, the mixture was stirred at room temperature for 2 h, and then **intermediate 16-3** (60 mg) was added. After the addition, the resulting mixture was stirred at room temperature for 2 h. The reaction solution was purified by preparative high performance liquid chromatography (YMC-Actus Triart C18 column, 5 um silica, 30 mm in diameter, 150 mm in length; using a decreasingly polar mixture of water (containing 0.05% $NH_4HCO_3$) and acetonitrile as the eluent; acetonitrile gradient proportion: 55%-80%, elution time: 13 minutes) to obtain the title compound (10 mg).
MS m/z (ESI): = 539.1 $[M+H]^+$.
**$^1$H NMR (400 MHz, DMSO-*d*$_6$)** $\delta$ 13.48 (s, 1H), 9.31 (s, 1H), 8.83 (s, 1H), 8.37 (d, *J* = 8.1 Hz, 2H), 8.00 (d, *J* = 8.1 Hz, 2H), 7.58-7.40 (m, 2H), 7.34 (s, 1H), 7.26 (d, *J*= 8.7 Hz, 1H), 3.65-3.52 (m, 1H), 2.59 (s, 2H), 0.63-0.23 (m, 4H).

**Test Examples of Biological Activity and Related Properties**

The compounds in the following test examples were all prepared according to the methods of the above examples of the present application.

Test example 1: POLQ Enzyme Activity Inhibition Experiment

**Brief introduction to the experimental principle:** The N-terminal active peptide segment (M1-N899) of POLQ with ATPase activity was co-incubated with compounds, and then reacted with substrate dT50 under the action of ATP to generate ADP, which participated in the subsequent NADH oxidative coupling enzymatic reaction and catalysed the NADH reaction to generate NAD+. The Envision microplate reader from Perkin Elmer Inc was used to measure the reduction of OD value of NADH at 340 nm, thus reflecting the enzyme activity.
**Experimental instruments:** Echo 650 pipetting system from Labcyte Inc; Envision microplate reader from

Perkin Elmer Inc; 5810R centrifuge from Eppendorf Inc, and BSD-YX3400 thermostatic shaker from Boxun Inc.

**Experimental materials:**

| Reagent | Brand |
|---|---|
| POLQ (N) | Synthesized by Pharmaron Inc |
| ATP | Sigma |
| dT50 | Synthesized by Genscript Inc |
| NADH | Roche |
| PEP | Sigma |
| Lactate dehydrogenase | Sigma |
| Pyruvate kinase | Sigma |
| 384-well plate | Greiner Bio-One |

**Experimental method:** The POLQ enzyme was diluted to 100 nM with a reaction buffer (20 mM Tris HCl (pH 7.80), 80 mM KCl, 10 mM $MgCl_2$, 1 mM DTT, 0.01% w/v bovine serum albumin, 0.01% v/v Tween-20, and 5% v/v glycerol). The compounds to be tested were diluted to different concentrations in dimethyl sulfoxide (DMSO) using the Echo 650 pipetting system, and transferred to a 384-well plate. 20 μL/well of 100 nM POLQ was added, and the mixture was incubated at room temperature for 15 minutes. A reaction mixture solution was formulated, and the concentration of each component in the reaction mixture solution was: 100 μM ATP, 300 nM dT50, 300 μM NADH, 6 mM PEP, 10 U/mL lactate dehydrogenase and 20 U/mL pyruvate kinase. 20 μL/well of reaction mixture solution was added to start the enzyme reaction. The final concentration of the compounds in the reaction system started from 10 μM, and was subjected to 3-fold gradient dilution. The concentration range was from 10 μM to 0.0005 μM, and the final concentration of DMSO in the system was 0.2% v/v. The 384-well plate was reacted at room temperature for 20 minutes, and then the OD value at 340 nm was read with an Envision microplate reader.

**Data analysis:**

The inhibition rate was calculated, and XLfit software was used to perform fitting to obtain $IC_{50}$ of the compounds.
In the experiment, a blank group and a DMSO group were set up, respectively. In the blank group, the reaction system was 0.2% v/v DMSO and the reaction mixture solution, and the inhibition rate was considered to be 100% in this case. In the DMSO group, the reaction system was 0.2% v/v DMSO, POLQ (N) (100 nM) and the reaction mixture solution, and the inhibition rate was considered to be 0 in this case.

$$\text{Inhibition rate} = (100-100*(OD_{max}-OD_{compound})/(OD_{max}-OD_{min}))\%,$$

where $OD_{max}$ refers to the OD value of well containing the reaction mixture solution and 0.2% v/v DMSO; $OD_{compound}$ refers to the OD value of well containing the compound, enzyme and reaction mixture solution; and $OD_{min}$ refers to the OD value of the well containing the enzyme, reaction mixture solution and 0.2% v/v DMSO.
The biological activity of the compounds of the present application was measured by means of the above test, and the measured $IC_{50}$ values were shown in Table 1 below.

Table 1 $IC_{50}$ of inhibition on POLQ enzyme activity by example compounds

| Example compound No. | $IC_{50}$ (nM) | Example compound No. | $IC_{50}$ (nM) |
|---|---|---|---|
| Compound 1 | +++ | Compound 49 | ++++ |
| Compound 2 | ++++ | Compound 50 | ++++ |
| Compound 3 | ++++ | Compound 51 | +++ |
| Compound 4 | ++++ | Compound 52 | ++++ |
| Compound 5 | ++++ | Compound 53 | +++ |

(continued)

| Example compound No. | $IC_{50}$ (nM) | Example compound No. | $IC_{50}$ (nM) |
|---|---|---|---|
| Compound 6 | ++++ | Compound 54 | ++++ |
| Compound 7 | ++++ | Compound 55 | ++++ |
| Compound 8 | ++++ | Compound 56 | ++++ |
| Compound 9 | ++++ | Compound 57 | ++++ |
| Compound 10 | ++++ | Compound 58 | ++++ |
| Compound 11 | ++++ | Compound 59 | ++++ |
| Compound 12 | ++++ | Compound 60 | ++++ |
| Compound 13 | ++++ | Compound 61 | ++++ |
| Compound 14 | ++++ | Compound 62 | ++++ |
| Compound 15 | ++++ | Compound 63 | ++++ |
| Compound 16 | ++++ | Compound 64 | ++++ |
| Compound 17 | ++++ | Compound 65 | ++++ |
| Compound 18 | ++++ | Compound 66 | ++++ |
| Compound 19 | ++++ | Compound 67 | ++++ |
| Compound 20 | ++++ | Compound 68 | ++++ |
| Compound 21 | ++++ | Compound 69-P1 | ++++ |
| Compound 22 | ++++ | Compound 69-P2 | ++++ |
| Compound 23 | ++++ | Compound 70 | ++++ |
| Compound 24 | ++++ | Compound 71 | ++++ |
| Compound 25 | ++++ | Compound 72 | ++++ |
| Compound 26 | ++++ | Compound 73 | ++++ |
| Compound 27 | ++++ | Compound 74 | ++++ |
| Compound 28 | ++++ | Compound 75 | ++++ |
| Compound 29 | ++++ | Compound 76 | ++++ |
| Compound 30 | ++++ | Compound 77 | ++++ |
| Compound 31 | ++++ | Compound 78 | ++++ |
| Compound 32 | ++++ | Compound 79 | ++++ |
| Compound 33 | ++++ | Compound 80 | ++++ |
| Compound 34 | ++++ | Compound 81 | ++++ |
| Compound 35 | ++++ | Compound 82 | ++++ |
| Compound 36 | ++++ | Compound 83 | ++++ |
| Compound 37 | ++++ | Compound 84 | ++++ |
| Compound 38 | ++++ | Compound 85 | ++++ |
| Compound 39 | +++ | Compound 86 | ++++ |
| Compound 40 | ++++ | Compound 87 | ++++ |
| Compound 41 | ++++ | Compound 88 | ++++ |
| Compound 42 | ++++ | Compound 89 | ++++ |
| Compound 43 | ++++ | Compound 90 | ++++ |
| Compound 44 | ++++ | Compound 91 | ++++ |

(continued)

| Example compound No. | $IC_{50}$ (nM) | Example compound No. | $IC_{50}$ (nM) |
|---|---|---|---|
| Compound 45 | ++++ | Compound 92 | ++++ |
| Compound 46 | ++++ | Compound 93 | ++++ |
| Compound 47 | ++++ | Compound 94 | ++++ |
| Compound 48 | ++++ | | |

In the Table above, the symbols used to indicate inhibitory activity have the following meanings:

"++++" indicates that the $IC_{50}$ range of the enzyme inhibitory activity of the compound to be tested is: $IC_{50}$ < 100 nM.
"+++" indicates that the $IC_{50}$ range of the enzyme inhibitory activity of the compound to be tested is: $100 \leq IC_{50} < 500$ nM.
"++" indicates that the $IC_{50}$ range of the enzyme inhibitory activity of the compound to be tested is: $500 \leq IC_{50} < 1000$ nM.

**Test example 2: Inhibitory Experiment of Compounds on Tumour Cell Proliferation**

**Brief introduction to the experimental principle:** The compounds were co-incubated with tumour cells for 7 days, and then a CTG kit from Promega Inc was used to quantify ATP in viable cells, thus reflecting the effect of the compounds on tumour cell proliferation.
**Experimental instruments:** Envision microplate reader from Perkin Elmer Inc; 5810R centrifuge from Eppendorf Inc, and Automated Cell Counter from Countstar Inc.

**Experimental materials:**

| Reagent | Brand | Catalogue number |
|---|---|---|
| DLD-1 parental cell | Horizon | HD PAR-008 |
| DLD-1 BRCA2 (-/-) cell line | Horizon | HD 105-007 |
| RPMI 1640 medium | Gibco | A10491-01 |
| FBS | Gibco | 10099-141C |
| 0.25% Trypsin-EDTA | Gibco | 25200-172 |
| PBS | HyClone | SH30256.01 |
| 96-well plate | Corning | 3610 |
| CelltiterGlo assay kit (CTG) | Promega | G7573 |

**Experimental method:** DLD-1 parental cells or DLD-1 BRCA2 (-/-) cells were diluted with RPMI 1640 medium containing 10% FBS, and then added to a 96-well plate (90 μL/well), with the number of cells being 600 cells/well or 1200 cells/well, respectively, and cultured overnight in a 37°C, 5% $CO_2$ incubator. The compounds to be tested were diluted to different concentrations in dimethyl sulfoxide (DMSO), and then added to a 96-well plate. The final concentration of the compounds in the reaction system started from 25 μM, and was subjected to 4-fold gradient dilution. The concentration range of the compounds was from 25 μM to 0.0004 μM, and the final concentration of DMSO was 0.25% v/v. After 7 days of incubation, 50 μL/well CTG was added, and the mixture was incubated at room temperature for 10 minutes. Subsequently, the luminescence (Lum) signal value was read with an Envision microplate reader, and the inhibition rate and half inhibitory concentration ($IC_{50}$) were calculated.

**Data analysis:**

The inhibition rate was calculated, and XLfit software was used to perform fitting to obtain $IC_{50}$ of the compounds.
In the experiment, a blank well and a DMSO well were set up, respectively. The blank well was 100 μL of RPMI

1640 Medium containing 10% FBS, and the inhibition rate of the compound on tumour cell growth was considered to be 100% in this case. The DMSO well was cell well with 0.25% v/v DMSO, and the inhibition rate of the compound on tumour cell growth was considered to be 0 in this case.

$$\text{Inhibition rate}= 100^* (Lum_{max}-Lum_{compound}) / (Lum_{max}-Lum_{min})\%,$$

where $Lum_{max}$ refers to the luminescence signal value of the well containing cells and 0.25% v/v DMSO; $Lum_{compound}$ refers to the luminescence signal value of the well containing the compound and cells; and $Lum_{min}$ refers to the luminescence signal value of the well containing the medium and 0.25% v/v DMSO.

The growth inhibition on tumour cells by the compounds of the present application was determined by means of the above test, and the measured $IC_{50}$ values.

Table 2 $IC_{50}$ of growth inhibition on tumour cells by example compounds

| Example compound No. | DLD-1 BRCA2 (-/-) $IC_{50}$ (nM) | DLD-1 parental cell $IC_{50}$ (nM) |
|---|---|---|
| Compound 9 | ++++ | - |
| Compound 16 | +++ | - |
| Compound 20 | ++++ | - |
| Compound 21 | ++++ | - |
| Compound 23 | ++++ | - |
| Compound 24 | ++++ | - |
| Compound 25 | ++++ | - |
| Compound 26 | ++++ | - |
| Compound 27 | ++++ | - |
| Compound 30 | ++++ | - |
| Compound 32 | +++ | - |
| Compound 33 | ++++ | - |
| Compound 34 | ++++ | - |
| Compound 42 | +++ | - |
| Compound 46 | ++++ | - |
| Compound 48 | +++ | - |
| Compound 58 | ++++ | - |
| Compound 61 | ++++ | - |
| Compound 62 | ++++ | - |
| Compound 63 | +++ | - |
| Compound 68 | ++++ | - |
| Compound 72 | ++++ | - |
| Compound 73 | ++++ | - |
| Compound 74 | ++++ | - |
| Compound 75 | ++++ | - |
| Compound 77 | ++++ | - |
| Compound 78 | ++++ | - |
| Compound 79 | ++++ | - |
| Compound 80 | ++++ | - |
| Compound 81 | ++++ | - |

(continued)

| Example compound No. | DLD-1 BRCA2 (-/-) $IC_{50}$ (nM) | DLD-1 parental cell $IC_{50}$ (nM) |
|---|---|---|
| Compound 82 | ++++ | - |
| Compound 83 | ++++ | - |
| Compound 84 | ++++ | - |
| Compound 85 | ++++ | - |
| Compound 86 | ++++ | - |
| Compound 87 | ++++ | - |
| Compound 91 | ++++ | - |
| Compound 92 | ++++ | - |
| Compound 93 | ++++ | - |
| Compound 94 | ++++ | - |

In the Table above, the symbols used to indicate inhibitory activity have the following meanings:

"++++" indicates that the $IC_{50}$ range of the cell inhibitory activity of the compound to be tested is: $IC_{50}$ < 200 nM.
"+++" indicates that the $IC_{50}$ range of the cell inhibitory activity of the compound to be tested is: 200nM ≤ $IC_{50}$ < 500 nM.
"++" indicates that the $IC_{50}$ range of the cell inhibitory activity of the compound to be tested is: 500 nM ≤ $IC_{50}$ < 1000 nM.
"+" indicates that the $IC_{50}$ range of the cell inhibitory activity of the compound to be tested is: 1000 nM ≤ $IC_{50}$ < 10000 nM.
"-" indicates that the $IC_{50}$ range of the cell inhibitory activity of the compound to be tested is: $IC_{50}$ ≥ 10000 nM.

Upon testing, the compounds of the present application had good inhibitory effect on tumour cells with BRCA2 mutation, and had good selectivity.

**Test example 3: Experiment on Inhibition of Cellular MMEJ Pathway by Compounds**

**Brief introduction to the experimental principle:** POLQ was a key protein in the cellular MMEJ repair process. The NanoLuciferase MMEJ repair reporter system was transfected into HEK293T cells. When the MMEJ repair pathway in the cells was in the normal progress, the NanoLuciferase reporter protein was correctly expressed, and the cellular luminescence signal could be detected. The BMG multifunctional microplate reader from BMG LABTECH Inc was used to measure the reduction of the cellular luminescence, thus reflecting the inhibition of the cellular MMEJ pathway by the compounds.

**Experimental instruments:** Incucyte viable cell imaging system from ESCD Inc; Echo 655 pipetting system from Labcyte Inc; BMG multifunctional microplate reader from BMG LABTECH Inc; and Neon transfection system from Invitrogen Inc.

**Experimental materials:**

| Reagent | Brand | Catalogue number |
|---|---|---|
| MMEJ luciferase substrate | Synthesized by ICE Bioscience Inc | / |
| HEK293T cell | ATCC | CRL-3216 |
| Nano-Glo Luciferase Assay kit | Promega | N1130 |
| DMEM | Gibco | 11995-065 |
| DMSO | Solarbio | D8371 |
| DPBS | Gibco | 14190250 |
| TrypL Express | Gibco | 25200-072 |

(continued)

| Reagent | Brand | Catalogue number |
|---|---|---|
| 384-well plate | Coming | 3764 |
| Neon™ Transfection System 100 μL Kit | Invitrogen | MPK10096 |

**Experimental method:** The compounds to be tested were diluted to different concentrations in dimethyl sulfoxide (DMSO) using the Echo 655 pipetting system, and transferred to a 384-well plate. The final concentration of the compounds in the reaction system started from 10 μM, and was subjected to 3-fold gradient dilution. The final concentration of DMSO was 0.1%. HEK293T cells were collected, and the MMEJ luciferase substrate was transfected into the cells with Neon transfection system. Subsequently, 4000 transfected HEK293T cells/well were diluted with DMEM medium containing 10% FBS, and then added to the 384-well plate (25 μL/well). The compounds and cells were cultured in a 37°C, 5% $CO_2$ incubator for 24 hours, and then 40 μL/well NanoGlo substrate buffer was added to measure the growth inhibition on tumour cells by the compounds, and the inhibition rate and half inhibitory concentration ($IC_{50}$) were calculated.

**Data analysis:**

The compound inhibition rate was calculated, and XLfit software was used to perform fitting to obtain $IC_{50}$ of the compounds.

In the experiment, a blank well and a DMSO well were set up, respectively. To the blank well was added 10 μM positive compound ART558 (doi: 10.1038/s41467-021-23463-8), and the inhibition rate of the compound was considered to be 100% in this case. To the DMSO well was added 0.1% DMSO, and the inhibition rate of the compound was considered to be 0 in this case.

Compound inhibition rate (%) = (100* (DMSO well-Compound to be tested well) / (DMSO well-Blank well))%

Upon testing, the example compounds of the present application had strong inhibitory activity on the POLQ-mediated MMEJ pathway in cells. It is expected that the compounds can effectively inhibit the target POLQ in tumours and the related pathway MMEJ, and then exert corresponding pharmacological effects.

**Test example 4: Determination of Metabolic Stability of Compounds in Hepatocytes**

The metabolic stability of the compounds of the present application in hepatocytes is determined by the following test method.

I. Experimental materials and instruments

1. Caucasian human hepatocytes (Biopredic BQHPCH10), cynomolgus monkey hepatocytes (RILD HP-SXH-02M), beagle dog hepatocytes (BioIVT M00205), SD rat hepatocytes (BioIVT M00005) and CD-1 mouse hepatocytes (BioIVT M00505)
2. AOPI dyes (Nexcelom 200710-01-01)
3. Dexamethasone (NIFDC 100129-201506)
4. DPBS (10×) (Gibco by Life Technologies 2060570)
5. Foetal bovine serum (FBS) (Corning 35081001)
6. GlutaMAXTM-1 (100×) (Gibco by Life Technologies 2186980)
7. HEPES (Sigma RNBJ1276)
8. Human recombinant insulin (Gibco by Life Technologies 2090407)
9. Isotonic Percoll (GE Healthcare 10288259)
10. Verapamil (Sigma MKBV4993V)
11. Williams' Medium E (Sigma RNBJ3314)
12. AB Sciex API4000 liquid chromatography-mass spectrometry

II. Experimental steps

1. Hepatocyte thawing medium was prepared according to the information in the Table below. 49.5 mL of Williams' Medium E and 0.5 mL of GlutaMAXTM-1 (100×) were mixed as an incubation medium. Hepatocyte thawing medium and incubation medium were placed in a 37°C water bath and pre-heated for at least 15 minutes prior to use. A tube of hepatocytes stored at an ultra-low temperature was taken and the hepatocytes were ensured to be at a frozen state at a low temperature prior to thawing. The hepatocytes were rapidly placed in a 37°C water bath and gently shaken until all the ice crystals were dispersed, and then after 70% ethanol was sprayed, the hepatocytes were transferred to a biosafety cabinet. The content of the tube of hepatocytes was poured into a centrifugal tube containing 50 mL of thawing medium and centrifuged at 100 g for 10 minutes. After centrifugation, the thawing medium was aspirated, and a sufficient amount of incubation medium was added to obtain a cell suspension with a cell density of about $1.5\times10^6$ cells/mL. The hepatocytes were counted, and the viable cell density was determined using Cellometer Vision, and the survival rate of the hepatocytes must be higher than 75%. The hepatocyte suspension was diluted by using the incubation medium to a viable cell density of $0.5 \times 10^6$ viable cells/mL.

| Reagent | Initial concentration | Final concentration | Volume of reagent |
|---|---|---|---|
| Williams' Medium E | - | - | 31.2 mL |
| Isotonic Percoll | - | 30% | 13.5 mL |
| DPBS (10×) | - | - | 1.5 mL |
| GlutaMAXTM-1 (100×) | 200 mM/100× | 2 mM | 500 μL |
| HEPES | 1 M | 15 mM | 750 μL |
| FBS | - | 5% | 2.5 mL |
| Recombinant human insulin | 4 mg/mL | 4 μg/mL | 50 μL |
| Dexamethasone | 10 mM | 1 μM | μL |

2. 247.5 μL of viable cell suspension or medium was transferred into a 96-well deep-well plate, and the deep-well plate was vortexed and placed in an incubator and pre-heated for 10 minutes. Incubation of all the samples was performed in parallel duplicate. 2.5 μL of 100 μM compound to be tested or the control drug verapamil was added to each well to start the reaction, and the deep-well plate was placed onto the vortexer of the incubator again. 25 μL of incubation sample was taken at 0, 15, 30, 60, 90 and 120 minutes, respectively, and 125 μL of acetonitrile containing the internal standard was added to terminate the reaction. The mixture was vortexed for 10 minutes and centrifuged at 3220 g, 4°C for 30 minutes. After centrifugation, 100 μL of the supernatant was transferred into the loading plate and 150 μL of pure water was added and mixed uniformly for LC-MS/MS analysis.

All the data was calculated by the Microsoft Excel software. The peak area was detected by extracting the ion chromatogram. The *in vitro* half-life ($t_{1/2}$) of the compound was determined by performing linear fitting of the natural logarithm of elimination percent of the compound and time.

The *in vitro* half-life ($t_{1/2}$) was calculated by means of the slope:
*in vitro*

$$t_{1/2} = 0.693 / k.$$

The *in vitro* intrinsic clearance (unit: μL/min/mg protein) was calculated using the following formula:
*in vitro*

$$CL_{int} = k \text{ x volume of incubation } (\mu L)/\text{amount of proteins (mg)},$$

where $CL_{int}$ is the inherent clearance rate; *k* is the elimination rate constant; volume of incubation is the incubation volume (μL); and amount of proteins is the protein amount (mg).

Upon testing, the example compounds of the present application were metabolically stable in various species of hepatocytes. It is expected that the compounds are relatively stable when metabolized by the liver *in vivo* and are relatively less affected by the first-pass effect of the liver.

## Claims

1. A compound represented by formula (I) or a pharmaceutically acceptable salt thereof:

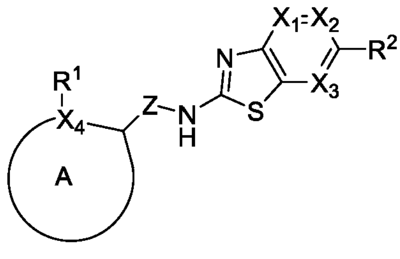

(I)

**characterized in that**,

$X_1$, $X_2$ and $X_3$ are independently selected from CH or N;
$X_4$ is selected from C or N;
Z is selected from C(=O) or $CH_2$;
ring A is selected from 5- to 10-membered heteroaryl, $C_6$-$C_{14}$ aryl or 4- to 12-membered heterocyclyl, wherein the 5- to 10-membered heteroaryl, $C_6$-$C_{14}$ aryl or 4- to 12-membered heterocyclyl is optionally substituted with $R^{1a}$;
each $R^{1a}$ is independently selected from halogen, hydroxyl, -NRR', cyano, carboxyl, =O, -C(=O)NRR', $C_1$-$C_{10}$ alkyl, $C_1$-$C_{10}$ alkoxy, $C_1$-$C_{10}$ alkoxyacyl, $C_3$-$C_{10}$ cycloalkyl, $C_2$-$C_{10}$ alkynyl, $C_2$-$C_{10}$ alkenyl, 4- to 12-membered heterocyclyl or 5- to 10-membered heteroaryl, wherein the $C_1$-$C_{10}$ alkyl, $C_1$-$C_{10}$ alkoxy, $C_1$-$C_{10}$ alkoxyacyl, $C_3$-$C_{10}$ cycloalkyl, $C_2$-$C_{10}$ alkynyl, $C_2$-$C_{10}$ alkenyl, 4- to 12-membered heterocyclyl or 5- to 10-membered heteroaryl is optionally substituted with $R^{1b}$;
$R^1$ is selected from $C_6$-$C_{14}$ aryl, 5- to 10-membered heteroaryl, 3- to 18-membered heterocyclyl or $C_4$-$C_{10}$ cycloalkenyl, wherein the $C_6$-$C_{14}$ aryl, 5- to 10-membered heteroaryl, 3- to 18-membered heterocyclyl or $C_4$-$C_{10}$ cycloalkenyl is optionally substituted with $R^{2a}$;
each $R^{2a}$ is independently selected from halogen, cyano, =O, hydroxyl, -NRR', -C(=O)NRR', $C_1$-$C_{10}$ alkyl, $C_1$-$C_{10}$ alkoxy, $C_3$-$C_{10}$ cycloalkyloxy, $C_1$-$C_{10}$ alkylacyl, $C_1$-$C_{10}$ alkylsulfonyl, $C_2$-$C_{10}$ alkynyl, $C_2$-$C_{10}$ alkenyl, $C_3$-$C_{10}$ cycloalkyl, 4- to 12-membered heterocyclyl, 4- to 8-membered heterocyclylalkyl or 4- to 8-membered heterocyclyloxy, wherein the $C_1$-$C_{10}$ alkyl, $C_1$-$C_{10}$ alkoxy, $C_3$-$C_{10}$ cycloalkyloxy, $C_1$-$C_{10}$ alkylacyl, $C_1$-$C_{10}$ alkylsulfonyl, $C_2$-$C_{10}$ alkynyl, $C_2$-$C_{10}$ alkenyl, $C_3$-$C_{10}$ cycloalkyl, 4- to 12-membered heterocyclyl, 4- to 8-membered heterocyclylalkyl or 4- to 8-membered heterocyclyloxy is optionally substituted with $R^{2b}$;
$R^2$ is selected from $C_6$-$C_{14}$ aryl, 5- to 10-membered heteroaryl, $C_3$-$C_{10}$ cycloalkyl or 4- to 12-membered heterocyclyl, wherein the $C_6$-$C_{14}$ aryl, 5- to 10-membered heteroaryl, $C_3$-$C_{10}$ cycloalkyl or 4- to 12-membered heterocyclyl is optionally substituted with $R^{3a}$;
each $R^{3a}$ is independently selected from halogen, -NRR', hydroxyl, cyano, =O, $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5-to 10-membered heteroaryl or 4- to 8-membered heterocyclyl, wherein the $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5- to 10-membered heteroaryl or 4- to 8-membered heterocyclyl is optionally substituted with $R^{3b}$;
R and R' are independently selected from hydrogen, $C_3$-$C_{10}$ cycloalkyl, $C_1$-$C_{10}$ alkylcarbonyl, 4- to 8-membered heterocyclyl or $C_1$-$C_{10}$ alkyl, wherein the $C_3$-$C_{10}$ cycloalkyl, $C_1$-$C_{10}$ alkylcarbonyl, 4- to 8-membered heterocyclyl or $C_1$-$C_{10}$ alkyl is optionally substituted with $R^{4b}$;
$R^{1b}$, $R^{2b}$, $R^{3b}$, and $R^{4b}$ are each independently selected from deuterium, halogen, carboxyl, hydroxyl, =O, cyano, -C(=O)NRR', sulfonyl, -S(=O)$_2$NRR', -NRR', $C_1$-$C_{10}$ alkyl or $C_1$-$C_{10}$ alkoxy.

2. The compound represented by formula (I) or the pharmaceutically acceptable salt thereof according to claim 1, **characterized in that**, at least one of $X_1$, $X_2$ and $X_3$ is N; or, $X_1$ and $X_2$ are independently selected from CH or N, and $X_3$ is N; or, $X_1$ and $X_2$ are independently CH, and $X_3$ is N; or, $X_1$ and $X_3$ are independently N, and $X_2$ is CH; or, $X_1$ is CH, and $X_2$ and $X_3$ are independently N; or, $X_1$ is N, and $X_2$ and $X_3$ are independently CH.

3. The compound represented by formula (I) or the pharmaceutically acceptable salt thereof according to claim 1 or 2, **characterized in that**, $X_4$ is C and/or Z is C(=O).

4. The compound represented by formula (I) or the pharmaceutically acceptable salt thereof according to any one of

claims 1 to 3, **characterized in that**, ring A is selected from 5- to 10-membered heteroaryl, $C_6$-$C_{10}$ aryl or 4- to 10-membered heterocyclyl, wherein the 5- to 10-membered heteroaryl, $C_6$-$C_{10}$ aryl or 4- to 10-membered heterocyclyl is optionally substituted with $R^{1a}$; or,

ring A is selected from 5- to 10-membered heteroaryl, $C_6$-$C_{10}$ aryl or 6- to 10-membered heterocyclyl, wherein the 5- to 10-membered heteroaryl, $C_6$-$C_{10}$ aryl or 6- to 10-membered heterocyclyl is optionally substituted with $R^{1a}$; or,
ring A is selected from 5- to 9-membered heteroaryl, phenyl or 9-membered heterocyclyl, wherein the 5- to 9-membered heteroaryl, phenyl or 5- to 9-membered heterocyclyl is optionally substituted with $R^{1a}$; or,
ring A is selected from 5- to 9-membered heteroaryl having 1, 2 or 3 nitrogen atoms, phenyl or 9-membered heterocyclyl, wherein the 5- to 9-membered heteroaryl, phenyl or 9-membered heterocyclyl is optionally substituted with $R^{1a}$; or,
ring A is selected from imidazolyl, 6- to 9-membered heteroaryl, phenyl or 9-membered heterocyclyl, wherein the imidazolyl, 6- to 9-membered heteroaryl, phenyl or 9-membered heterocyclyl is optionally substituted with $R^{1a}$; or,
ring A is selected from pyridyl, imidazolyl, imidazo[1,2-a]pyridyl, pyrimidyl, [1,2,4]triazolo[1,5-a]pyridyl, phenyl or

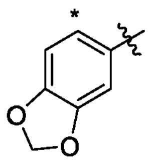

,

wherein the pyridyl, imidazolyl, imidazo[1,2-a]pyridyl, pyrimidyl, [1,2,4]triazolo[1,5-a]pyridyl, phenyl or

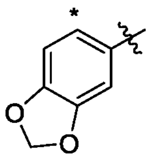

is optionally substituted with $R^{1a}$.

5. The compound represented by formula (I) or the pharmaceutically acceptable salt thereof according to any one of claims 1 to 4, **characterized in that**, $R^{1a}$ is selected from halogen, cyano or $C_1$-$C_6$ alkyl, wherein the $C_1$-$C_6$ alkyl is optionally substituted with $R^{1b}$; or, $R^{1a}$ is selected from halogen, cyano or $C_1$-$C_3$ alkyl, wherein the $C_1$-$C_3$ alkyl is optionally substituted with halogen; or, $R^{1a}$ is selected from fluorine, chlorine, cyano, $CF_3$ or methyl.

6. The compound represented by formula (I) or the pharmaceutically acceptable salt thereof according to any one of claims 1 to 5, **characterized in that**, $R^{1b}$ is halogen; or, $R^{1b}$ is fluorine.

7. The compound represented by formula (I) or the pharmaceutically acceptable salt thereof according to any one of claims 1 to 6, **characterized in that**, ring A is selected from

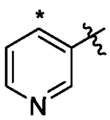 , 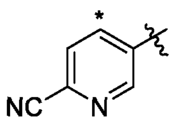 , 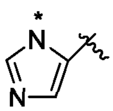 , 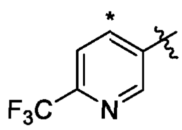 ,

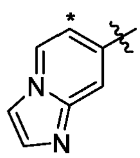 , 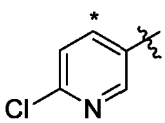 , 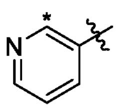 , 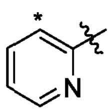 , 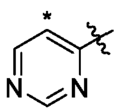 , 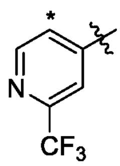 , 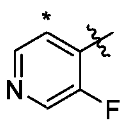 ,

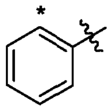, 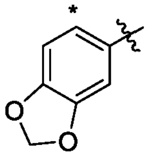, 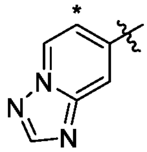, 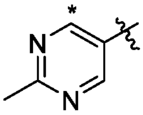, 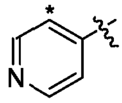, 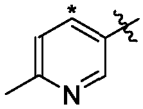

or

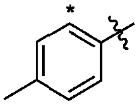,

wherein * indicates linking to $R^1$; or, ring A is selected from

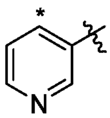, 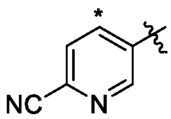, 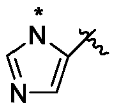, 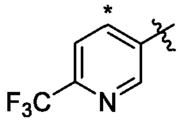,

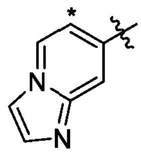, 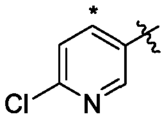, 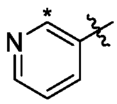, 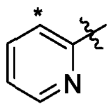, 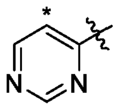, 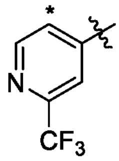, 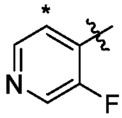,

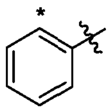, 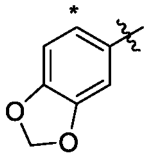, 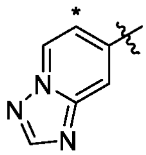, 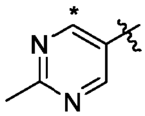, 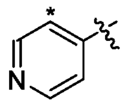 or 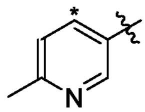,

wherein * indicates linking to $R^1$; or, ring A is selected from

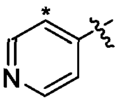 or 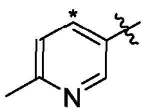,

wherein * indicates linking to $R^1$.

**8.** The compound represented by formula (I) or the pharmaceutically acceptable salt thereof according to any one of claims 1 to 7, **characterized in that**, $R^1$ is selected from $C_6$-$C_{10}$ aryl, 5- to 10-membered heteroaryl, 4- to 10-membered heterocyclyl or $C_5$-$C_7$ cycloalkenyl, wherein the $C_6$-$C_{10}$ aryl, 5- to 10-membered heteroaryl, 4- to 10-membered heterocyclyl or $C_5$-$C_7$ cycloalkenyl is optionally substituted with $R^{2a}$; or,

$R^1$ is selected from $C_6$-$C_{10}$ aryl, 5- to 10-membered heteroaryl or 4- to 10-membered heterocyclyl, wherein the $C_6$-$C_{10}$ aryl, 5- to 10-membered heteroaryl or 4- to 10-membered heterocyclyl is optionally substituted with $R^{2a}$; or,

$R^1$ is selected from phenyl, 6-membered heteroaryl or 6-membered heterocyclyl, wherein the phenyl, 6-membered heteroaryl or 6-membered heterocyclyl is optionally substituted with $R^{2a}$; or,

$R^1$ is selected from phenyl, pyridyl or morpholinyl, wherein the phenyl, pyridyl or morpholinyl is optionally substituted with $R^{2a}$.

**9.** The compound represented by formula (I) or the pharmaceutically acceptable salt thereof according to any one of claims 1 to 8, **characterized in that**, $R^{2a}$ is selected from halogen, cyano, $C_1$-$C_6$ alkyl, $C_2$-$C_6$ alkynyl, $C_3$-$C_6$ cycloalkyl, $C_1$-$C_6$ alkoxy or $C_3$-$C_6$ cycloalkyloxy, wherein the $C_1$-$C_6$ alkyl, $C_2$-$C_6$ alkynyl, $C_3$-$C_6$ cycloalkyl, $C_1$-$C_6$ alkoxy or $C_3$-$C_6$ cycloalkyloxy is optionally substituted with $R^{2b}$; or

$R^{2a}$ is selected from halogen, cyano, $C_1$-$C_3$ alkyl, $C_2$-$C_4$ alkynyl, $C_3$-$C_4$ cycloalkyl, $C_1$-$C_3$ alkoxy or $C_3$-$C_4$ cycloalkyloxy, wherein the $C_1$-$C_3$ alkyl, $C_2$-$C_4$ alkynyl, $C_3$-$C_4$ cycloalkyl, $C_1$-$C_3$ alkoxy or $C_3$-$C_4$ cycloalkyloxy is optionally substituted with $R^{2b}$; or,
$R^{2a}$ is selected from halogen, cyano, $C_1$-$C_3$ alkyl, $C_2$-$C_4$ alkynyl, $C_3$-$C_4$ cycloalkyl, $C_1$-$C_3$ alkoxy or $C_3$-$C_4$ cycloalkyloxy, wherein the $C_1$-$C_3$ alkyl or $C_1$-$C_3$ alkoxy is optionally substituted with $R^{2b}$; or,
$R^{2a}$ is selected from halogen, cyano, $C_1$-$C_6$ alkyl, $C_2$-$C_6$ alkynyl, $C_3$-$C_6$ cycloalkyl or $C_1$-$C_6$ alkoxy, wherein the $C_1$-$C_6$ alkyl, $C_2$-$C_6$ alkynyl, $C_3$-$C_6$ cycloalkyl or $C_1$-$C_6$ alkoxy is optionally substituted with $R^{2b}$; or,
$R^{2a}$ is selected from halogen, cyano, $C_1$-$C_3$ alkyl, $C_2$-$C_4$ alkynyl, $C_3$-$C_4$ cycloalkyl or $C_1$-$C_3$ alkoxy, wherein the $C_1$-$C_3$ alkyl, $C_2$-$C_4$ alkynyl, $C_3$-$C_4$ cycloalkyl or $C_1$-$C_3$ alkoxy is optionally substituted with $R^{2b}$; or,
$R^{2a}$ is selected from halogen, cyano, $C_1$-$C_3$ alkyl, $C_2$-$C_4$ alkynyl, $C_3$-$C_4$ cycloalkyl or $C_1$-$C_3$ alkoxy, wherein the $C_1$-$C_3$ alkyl or $C_1$-$C_3$ alkoxy is optionally substituted with $R^{2b}$.

**10.** The compound represented by formula (I) or the pharmaceutically acceptable salt thereof according to any one of claims 1 to 9, **characterized in that**, $R^{2b}$ is selected from halogen or deuterium; or, $R^{2b}$ is halogen; or, $R^{2b}$ is fluorine; or, $R^{2b}$ is selected from fluorine or deuterium.

**11.** The compound represented by formula (I) or the pharmaceutically acceptable salt thereof according to any one of claims 1 to 10, **characterized in that**, $R^2$ is selected from $C_6$-$C_{10}$ aryl, 5- to 10-membered heteroaryl, $C_3$-$C_6$ cycloalkyl or 4- to 10-membered heterocyclyl, wherein the $C_6$-$C_{10}$ aryl, 5- to 10-membered heteroaryl, $C_3$-$C_6$ cycloalkyl or 4- to 10-membered heterocyclyl is optionally substituted with $R^{3a}$; or,

$R^2$ is selected from $C_6$-$C_{10}$ aryl, 5- to 10-membered heteroaryl or 4- to 10-membered heterocyclyl, wherein the $C_6$-$C_{10}$ aryl, 5- to 10-membered heteroaryl or 4- to 10-membered heterocyclyl is optionally substituted with $R^{3a}$; or,
$R^2$ is selected from phenyl, 5- to 6-membered heteroaryl or 5- to 6-membered heterocyclyl, wherein the phenyl, 5- to 6-membered heteroaryl or 5- to 6-membered heterocyclyl is optionally substituted with $R^{3a}$; or,
$R^2$ is selected from phenyl, 6-membered heteroaryl or 5- to 6-membered heterocyclyl, wherein the phenyl, 6-membered heteroaryl or 5- to 6-membered heterocyclyl is optionally substituted with $R^{3a}$; or,
$R^2$ is selected from phenyl, pyridyl, tetrahydrofuryl or

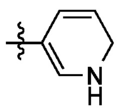
,

wherein the phenyl, pyridyl, tetrahydrofuryl or

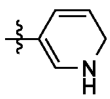

is optionally substituted with $R^{3a}$; or,
$R^2$ is selected from phenyl, pyridyl or tetrahydrofuryl, wherein the phenyl, pyridyl or tetrahydrofuryl is optionally substituted with $R^{3a}$; or,
$R^2$ is selected from phenyl or pyridyl, wherein the phenyl or pyridyl is optionally substituted with $R^{3a}$.

**12.** The compound represented by formula (I) or the pharmaceutically acceptable salt thereof according to any one of claims 1 to 11, **characterized in that**, each $R^{3a}$ is independently selected from halogen, -NRR', hydroxyl, cyano, $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5- to 10-membered heteroaryl or 4- to 8-membered heterocyclyl, wherein the $C_1$-$C_{10}$ alkyl, $C_1$-$C_{10}$ alkoxy, $C_3$-$C_{10}$ cycloalkyl, $C_2$-$C_{10}$ alkynyl, $C_2$-$C_{10}$ alkenyl, 5- to 10-membered heteroaryl or 4- to 8-membered heterocyclyl is optionally substituted with $R^{3b}$; or,

each $R^{3a}$ is independently selected from halogen, cyano, =O, $C_1$-$C_6$ alkyl, $C_1$-$C_6$ alkoxy or $C_3$-$C_6$ cycloalkyl,

wherein the $C_1$-$C_6$ alkyl, $C_1$-$C_6$ alkoxy or $C_3$-$C_6$ cycloalkyl is optionally substituted with $R^{3b}$; or, each $R^{3a}$ is independently selected from halogen, cyano, =O, $C_1$-$C_6$ alkyl, $C_1$-$C_6$ alkoxy or $C_3$-$C_6$ cycloalkyl, wherein the $C_1$-$C_6$ alkyl or $C_1$-$C_6$ alkoxy is optionally substituted with $R^{3b}$; or, each $R^{3a}$ is independently selected from halogen, cyano, $C_1$-$C_6$ alkyl or $C_1$-$C_6$ alkoxy, wherein the $C_1$-$C_6$ alkyl or $C_1$-$C_6$ alkoxy is optionally substituted with $R^{3b}$; or, each $R^{3a}$ is independently selected from fluorine, chlorine, bromine, iodine, cyano, $C_1$-$C_3$ alkyl or $C_1$-$C_3$ alkoxy.

**13.** The compound represented by formula (I) or the pharmaceutically acceptable salt thereof according to any one of claims 1 to 12, **characterized in that**, $R^{3b}$ is selected from halogen, cyano or hydroxyl; or, $R^{3b}$ is selected from fluorine, cyano or hydroxyl.

**14.** The compound represented by formula (I) or the pharmaceutically acceptable salt thereof according to claim 1, **characterized in that**, the compound represented by formula (I) or the pharmaceutically acceptable salt thereof is selected from the following compound or a pharmaceutically acceptable salt thereof:

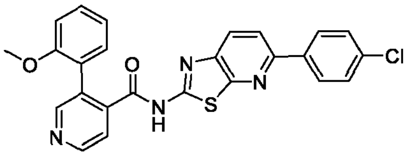
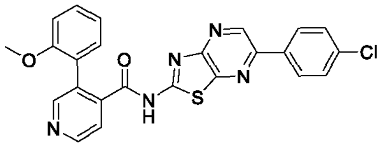

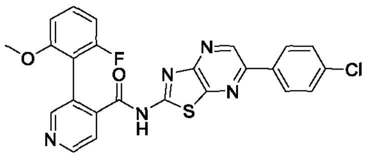
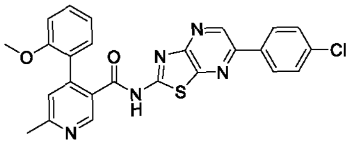

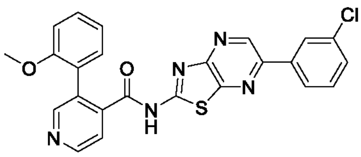
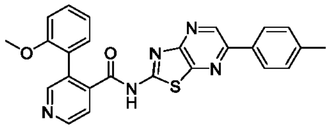

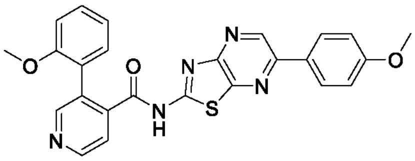
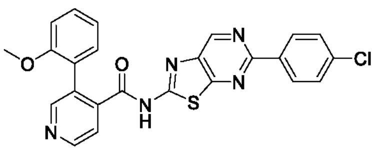

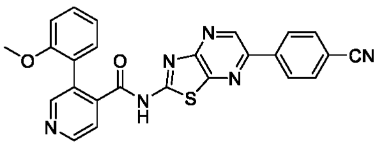
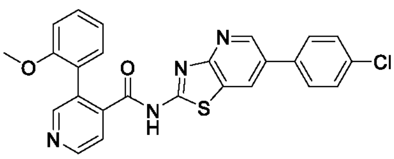

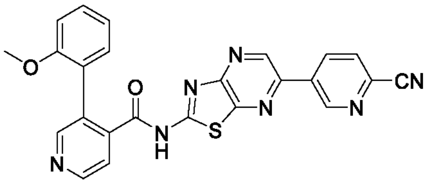
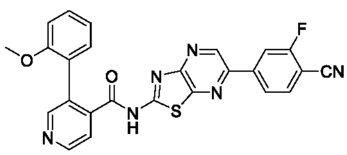

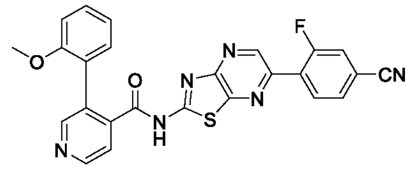
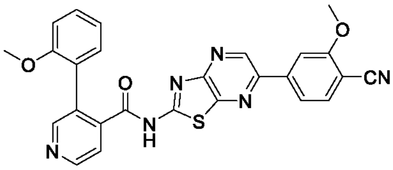

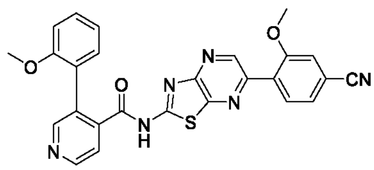
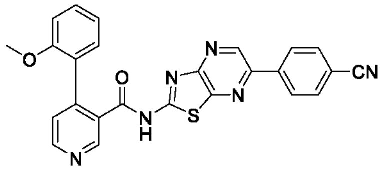

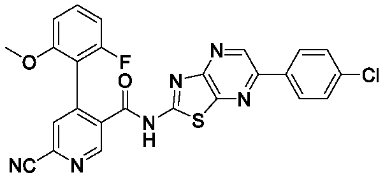
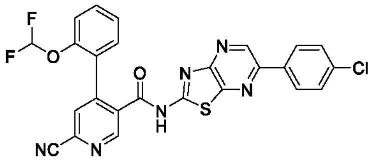

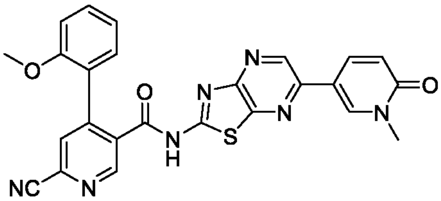
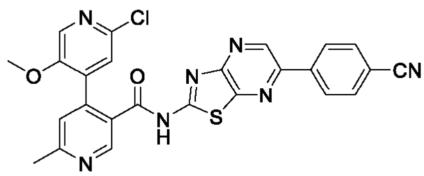

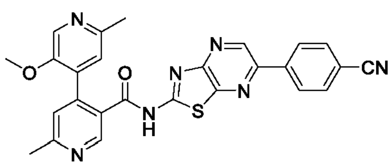
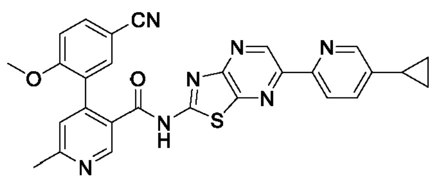

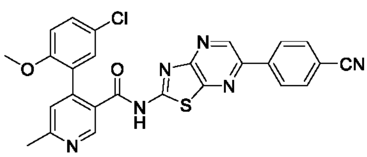
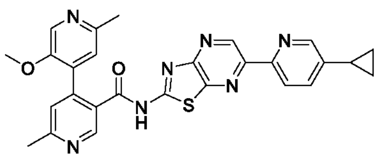

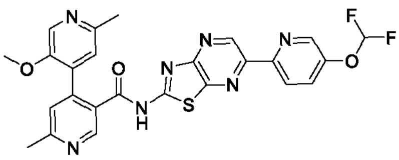
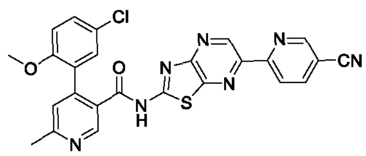

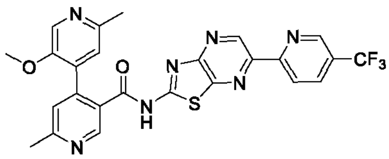
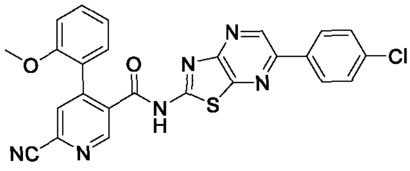

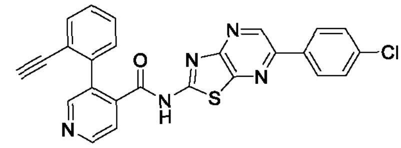
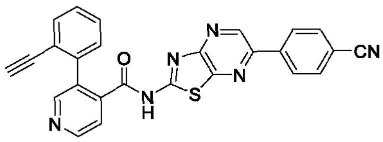

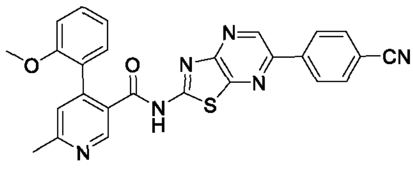
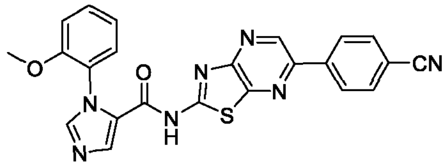

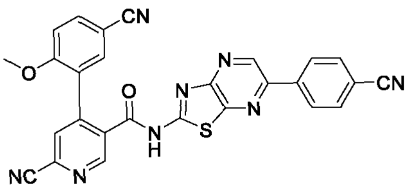
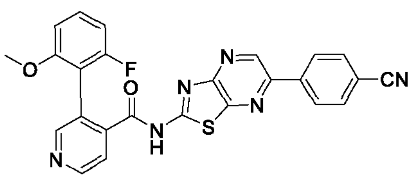

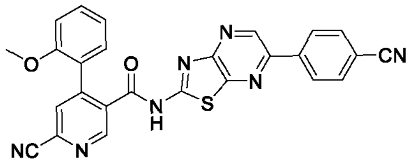
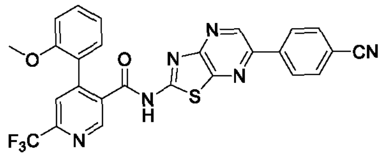

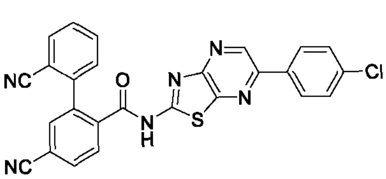
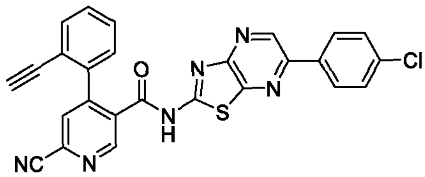

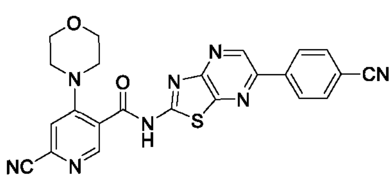
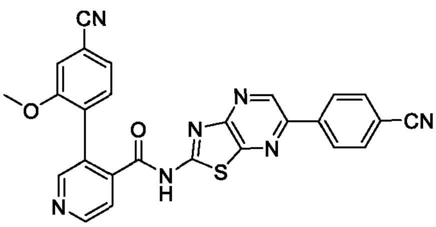

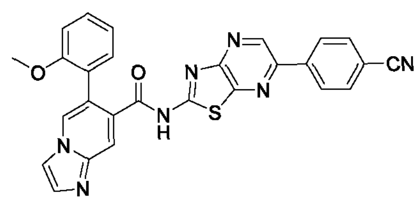
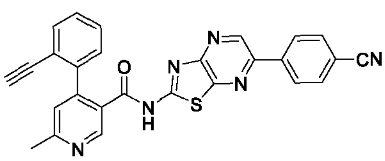

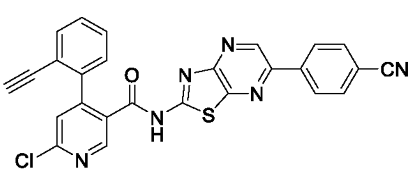
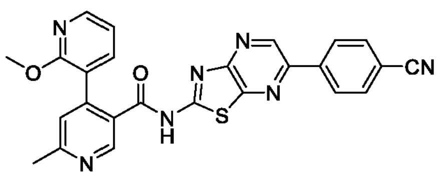

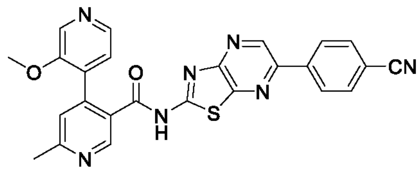

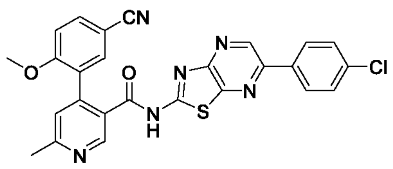

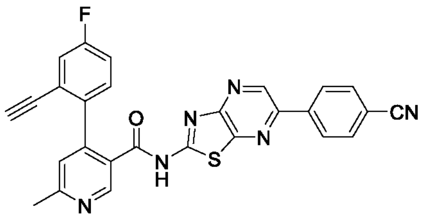

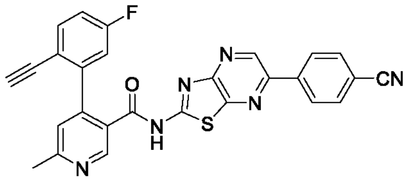

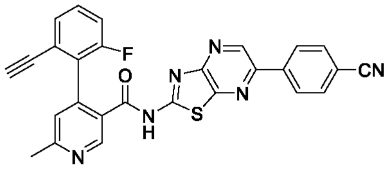

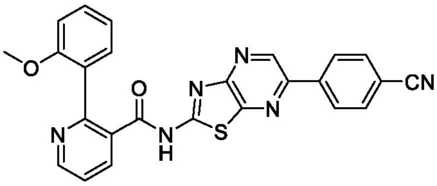

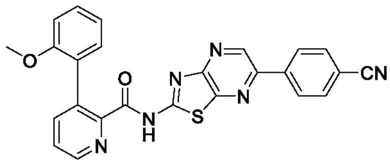

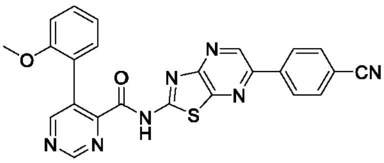

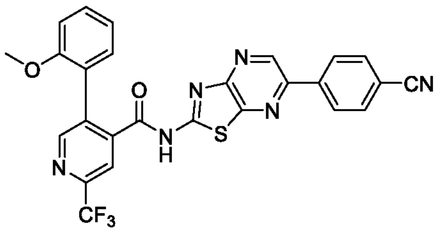

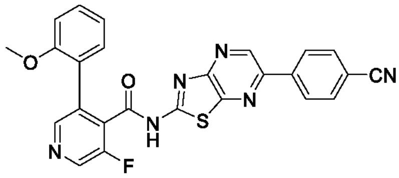

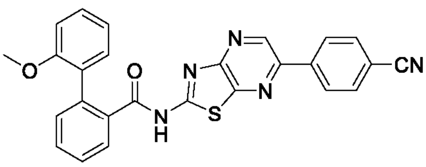

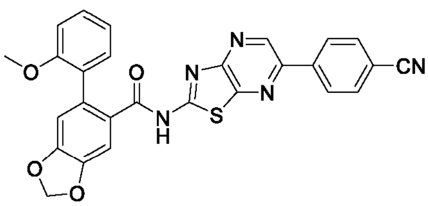

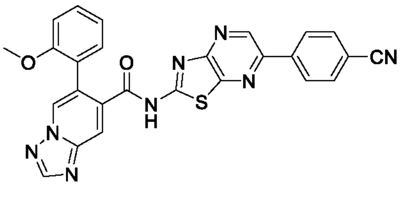

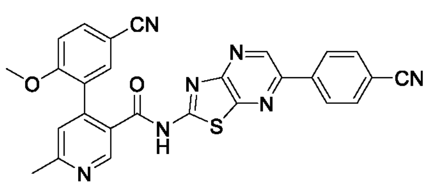

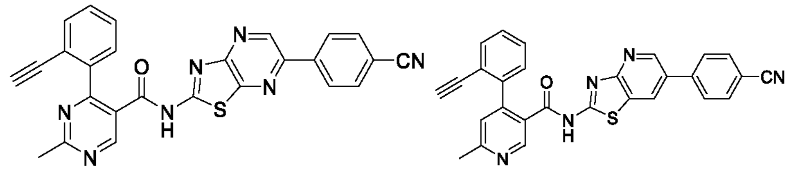

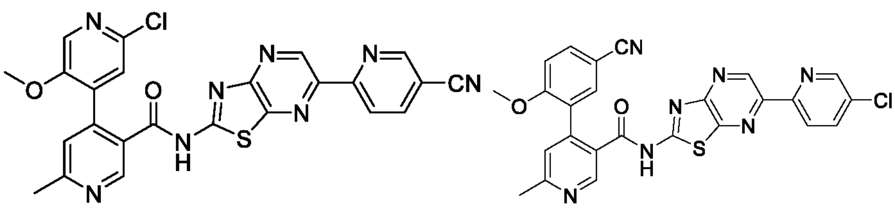

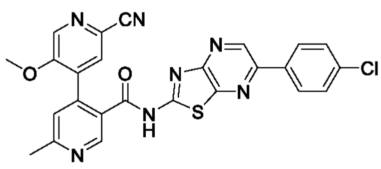
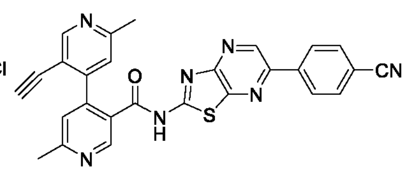

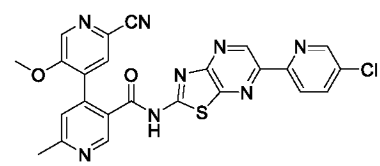
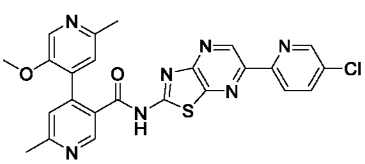

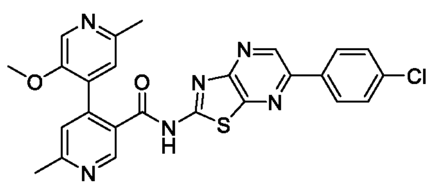
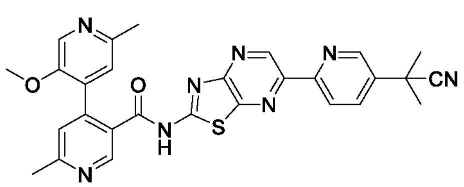

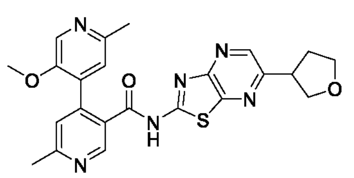
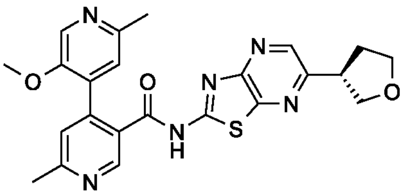

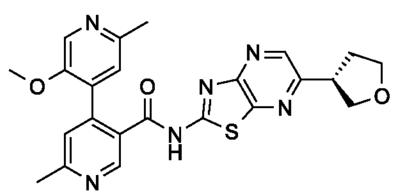
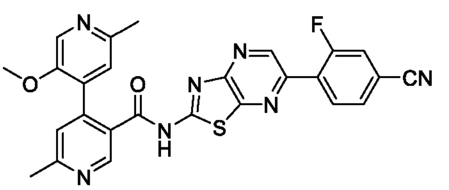

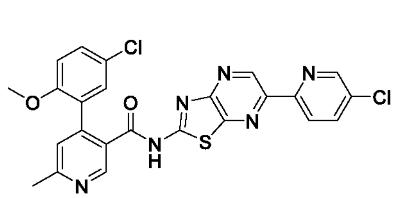
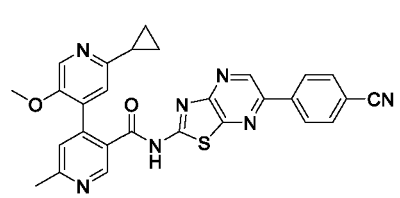

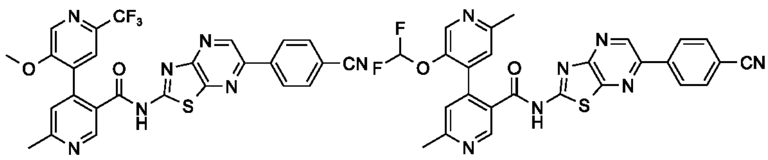

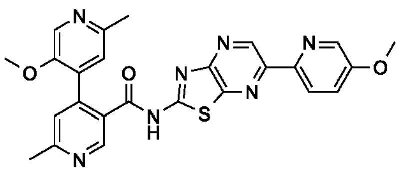

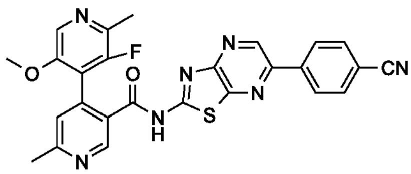

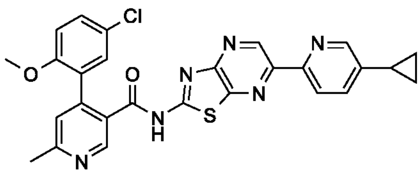

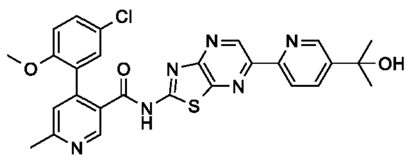

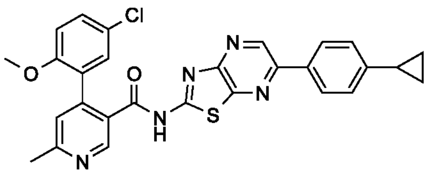

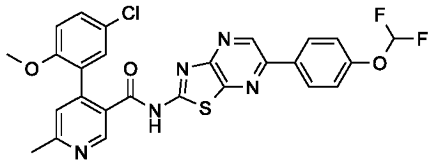

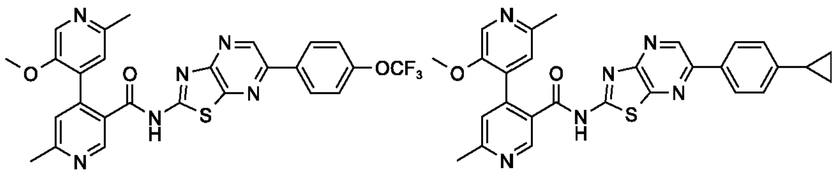

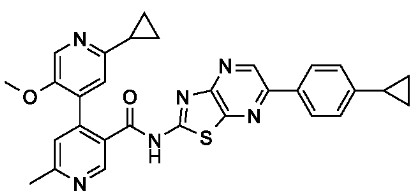

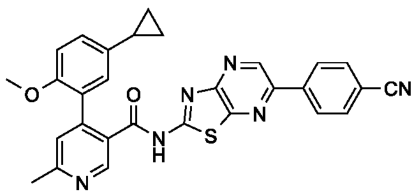

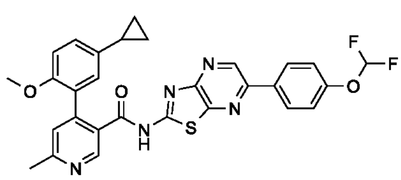

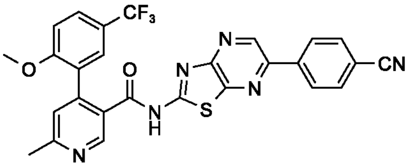

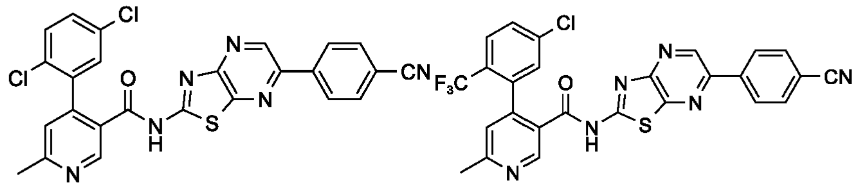

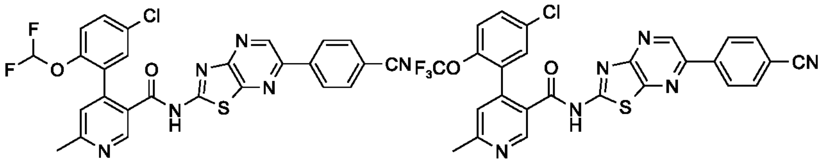

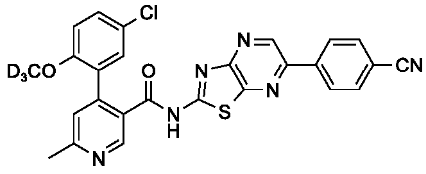

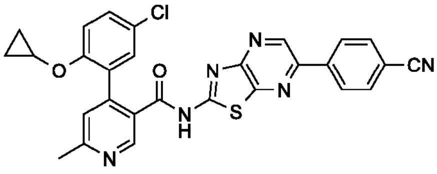

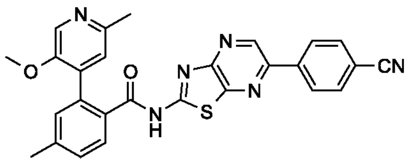

or

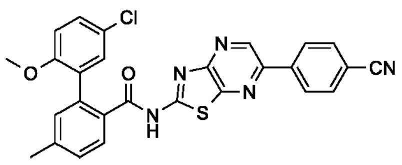

**15.** A pharmaceutical composition, **characterized in that** the pharmaceutical composition comprises the compound or the pharmaceutically acceptable salt thereof according to any one of claims 1 to 14, and a pharmaceutically acceptable excipient.

**16.** The compound or the pharmaceutically acceptable salt thereof according to any one of claims 1 to 14, or the pharmaceutical composition according to claim 15, for use in the preparation of a medicament for preventing or treating diseases mediated by DNA polymerase θ.

**17.** A method for preventing or treating diseases mediated by DNA polymerase θ, **characterized in that** the method comprises administering the compound or the pharmaceutically acceptable salt thereof according to any one of claims 1 to 14, or the pharmaceutical composition according to claim 15 to an individual in need thereof.

**18.** The compound or the pharmaceutically acceptable salt thereof according to any one of claims 1 to 14, or the pharmaceutical composition according to claim 15, for use in preventing or treating diseases mediated by DNA polymerase θ.

**19.** The compound or the pharmaceutically acceptable salt thereof according to any one of claims 1 to 14, or the pharmaceutical composition according to claim 15, for use in the prevention or treatment of diseases mediated by DNA polymerase θ.

**20.** The use according to claim 16 or 19, or the method according to claim 17, or the compound or the pharmaceutically acceptable salt thereof or the pharmaceutical composition according to claim 18, **characterized in that** the diseases mediated by DNA polymerase θ are diseases in which DNA polymerase θ is overexpressed.

**21.** The use according to claim 16 or 19, or the method according to claim 17, or the compound or the pharmaceutically acceptable salt thereof or the pharmaceutical composition according to claim 18, **characterized in that** the diseases mediated by DNA polymerase θ are cancer.

**22.** The use, method or compound or pharmaceutically acceptable salt thereof or pharmaceutical composition according to claim 21, **characterized in that** the cancer is with a reduction or absence of BRCA gene expression, the absence of the BRCA gene, or reduced function of BRCA protein.

**INTERNATIONAL SEARCH REPORT**

International application No.
**PCT/CN2023/072041**

**A. CLASSIFICATION OF SUBJECT MATTER**

C07D513/04(2006.01)i;C07D417/12(2006.01)i;C07D417/14(2006.01)i;A61K31/428(2006.01)i;A61K31/429(2006.01)i; A61P35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNTXT, ENTXT, CNKI, REGISTRY (STN), CAPLUS (STN): 脱氧核糖核酸, 聚合酶, 西塔, 抑制剂, 噻唑, 癌, 肿瘤, DNA, polymerase, 0, theta, inhibitor, thiazole, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| X | WO 2020160213 A1 (IDEAYA BIOSCIENCES, INC.) 06 August 2020 (2020-08-06) abstract, claim 1, description paragraphs [0010], [0055], [0080], [0253]-[0255] | 1-22 |
| A | WO 2020243459 A1 (IDEAYA BIOSCIENCES, INC.) 03 December 2020 (2020-12-03) entire document | 1-22 |
| A | WO 2021028643 A1 (ARTIOS PHARMA LIMITED) 18 February 2021 (2021-02-18) entire document | 1-22 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 May 2023** | **15 May 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/072041**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17, 19-22**
because they relate to subject matter not required to be searched by this Authority, namely:

The method of claims 17 and 19-22 belongs to methods for treatment of diseases. However, the present search report is provided on the basis of the corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/072041**

| Patent document cited in search report | | | Publication date (day/month/year) | Patent family member(s) | | | Publication date (day/month/year) |
|---|---|---|---|---|---|---|---|
| WO | 2020160213 | A1 | 06 August 2020 | None | | | |
| WO | 2020243459 | A1 | 03 December 2020 | TW | 202110834 | A | 16 March 2021 |
| | | | | CA | 3141134 | A1 | 03 December 2020 |
| | | | | US | 2023078112 | A1 | 16 March 2023 |
| | | | | JP | 2022535227 | A | 05 August 2022 |
| | | | | AU | 2020282768 | A1 | 23 December 2021 |
| | | | | BR | 112021024101 | A2 | 08 February 2022 |
| | | | | EP | 3976608 | A1 | 06 April 2022 |
| WO | 2021028643 | A1 | 18 February 2021 | EP | 4010080 | A1 | 15 June 2022 |
| | | | | JP | 2022548822 | A | 22 November 2022 |
| | | | | US | 2022298134 | A1 | 22 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210038783
- CN 202211207114
- WO 2020243459 A


### Non-patent literature cited in the description


- **KENT et al.** *Nature Structural & Molecular Biology*, 2015, vol. 22 (3), 230-237
- **MATEOS-GOMEZ et al.** *Nature*, 2015, vol. 518 (7538), 254-257
- **WOOD** ; **DOUBLIE**. *DNA Repair*, 2016, vol. 44, 22-32
- **CECCALDI et al.** *Nature*, 2015, vol. 518 (7538), 258-262
- **HIGGINS et al.** *Oncotarget*, 2010, vol. 1, 175-184
- **LEMEE et al.** *PNAS*, 2010, vol. 107 (30), 13390-13395
- **KAWAMURA et al.** *International Journal of Cancer*, 2004, vol. 109 (1), 9-16
- **MAEHR**. *J.Chem.Ed.*, 1985, vol. 62, 114-120